# EUROPEAN PATENT SPECIFICATION

(11) **EP 3 986 873 B1**
(45) Date of publication and mention of the grant of the patent: **15.11.2023**
(21) Application number: 20734158.7
(22) Date of filing: 19.06.2020
(51) Int. Cl.: C07D 257/04, C07D 207/12, C07D 261/08, C07D 285/08, C07D 249/08, C07D 271/06, C07C 259/00, C07C 261/04, C07C 259/14, C07C 311/51, A61K 31/40, A61K 31/41, A61K 31/433, A61K 31/165, A61K 31/277, A61P 21/00, A61P 21/02, A61P 21/04

(54) **5-[(1S)-1-(4-BROMOPHENOXY)ETHYL]-2H-TETRAZOLE DERIVATIVES AND RELATED COMPOUNDS AS CLC-1 ION CHANNEL INHIBITORS FOR TREATING NEUROMUSCULAR DISORDERS**
5-[(1S)-1-(4-BROMOPHENOXY)ETHYL]-2H-TETRAZOL-DERIVATE UND ÄHNLICHE VERBINDUNGEN ALS CLC-1 IONEN-KANNAL INHIBITOREN ZUR BEHANDLUNG NEUROMUSKULÄRER ERKRANKUNGEN
DÉRIVÉS DE 5-[(1S)-1-(4-BROMOPHÉNOXY)ÉTHYL]-2H-TETRAZOLEET COMPOSÉS SIMILAIRES EN TANT QU'INHIBITEURS DU CANAL IONIQUE CLC-1 POUR LE TRAITEMENT DE TROUBLES NEUROMUSCULAIRES

(30) Priority: 19.06.2019 US 201962863487 P; 01.07.2019 EP 19183622
(43) Date of publication of application: 27.04.2022
(73) Proprietor: NMD Pharma A/S, 8200 Århus N (DK)
(72) Inventor: KNUTSEN, Lars, J.S., Frinton-on-Sea Essex, CO13 0DH (GB); BROCH-LIPS, Martin, 8541 Skødstrup (DK); COOPER, Martin, E, Nottingham NG4 1AQ (GB); LABELLE, Marc, deceased (GB); PEDERSEN, Thomas, Holm, 8240 Risskov (DK); DUDEKULA, Dastagiri, Winnipeg, Manitoba, R3Y 2A6 (CA); SARASWAT, Neerja, Winnipeg Manitoba, R3X 1R4 (CA); TAJ, Rafiq, A., Winnipeg, Manitoba, R3Y 0E9 (CA)
(74) Representative: Høiberg P/S
(86) International application number: PCT/EP2020/067070
(87) International publication number: WO 2020/254558

(56) References cited:
- SANTOSH KURHADE ET AL: "Synthesis of a novel tetracyclic azaindolo[2,1-][1,4]benzoxazine ring system", TETRAHEDRON LETTERS, ELSEVIER LTD, AMSTERDAM, NL, vol. 52, no. 16, 7 February 2011 (2011-02-07), pages 1874-1877, XP028165600, ISSN: 0040-4039, DOI: 10.1016/J.TETLET.2011.02.020 [retrieved on 2011-02-09]
- AROMATARIS E C: "Pharmacology of the CIC-1 Chloride channel", INTERNET CITATION, 23 August 2010 (2010-08-23), pages 1-179, XP002781720, Retrieved from the Internet: URL:https://digital.library.adelaide.edu.a u/dspace/bitstream/2440/58973/8/02whole.pd f [retrieved on 2018-06-06]

## Description

### Technical field

The present disclosure relates to compounds and their use in treating, ameliorating and/or preventing neuromuscular disorders, including the reversal of drug-induced neuromuscular blockade. The compounds as defined herein preferably inhibit the CIC-1 ion channel. The disclosure further relates to methods of treating, preventing and/or ameliorating neuromuscular disorders, by administering said composition to a person in need thereof.

### Background

Walking, breathing, and eye movement are examples of essential everyday physiological activities that are powered by the contractile activity of skeletal muscle. Skeletal muscles are inherently in a resting state and contractile activity occurs exclusively in response to commands from the central nervous system (CNS). Such neuronal commands take the form of action potentials that travel from the brain to the muscle fibres in several steps. The neuromuscular junction (NMJ) is a highly specialized membrane area on muscle fibres where motor neurons come into close contact with the muscle fibres, and it is at the NMJ where neuronal action potentials are transmitted to muscular action potentials in a one-to-one fashion *via* synaptic transmission.

Neuromuscular transmission refers to the sequence of cellular events at the NMJ whereby an action potential in the lower motor neuron is transmitted to a corresponding action potential in a muscle fibre (Wood SJ, Slater CR. Safety factor at the neuromuscular junction. Prog. Neurobiol. 2001, 64, 393-429). When a neuronal action potential arrives at the pre-synaptic terminal it triggers influx of Ca²⁺ through voltage gated P/Q-type Ca²⁺ channels in the nerve terminal membrane. This influx causes a rise in cytosolic Ca²⁺ in the nerve terminal that triggers exocytosis of acetylcholine (ACh). Released ACh next diffuses across the synaptic cleft to activate nicotinic ACh receptors in the post-synaptic, muscle fibre membrane. Upon activation, ACh receptors convey an excitatory current flow of Na⁺ into the muscle fibre, which results in a local depolarization of the muscle fibre at the NMJ that is known as the endplate potential (EPP). If the EPP is sufficiently large, voltage gated Na⁺ channels in the muscle fibre will activate and an action potential in the muscle fibre will ensue. This action potential then propagates from the NMJ throughout the muscle fibre and triggers release of Ca²⁺ release from the sarcoplasmic reticulum. The released Ca²⁺ activates the contractile proteins within the muscle fibres, thus resulting in contraction of the fibre.

Failure of neuromuscular transmission can arise from both pre-synaptic dysfunction [Lambert Eaton syndrome (Titulaer MJ, Lang B, Verschuuren JJ. Lambert-Eaton myasthenic syndrome: from clinical characteristics to therapeutic strategies. Lancet Neurol. 2011, 10, 1098-107)*,* amyotrophic lateral sclerosis (Killian JM, Wilfong AA, Burnett L, Appel SH, Boland D. Decremental motor responses to repetitive nerve stimulation in ALS. Muscle Nerve, 1994, 17, 747-754), spinal muscular atrophy (Wadman RI, Vrancken AF, van den Berg LH, van der Pol WL. Dysfunction of the neuromuscular junction in spinal muscular atrophy types 2 and 3. Neurology, 2012, 79, 2050-2055) and as a result of post-synaptic dysfunction as occurs in myasthenia gravis (Le Panse R, Berrih-Aknin S. Autoimmune myasthenia gravis: autoantibody mechanisms and new developments on immune regulation. Curr Opin Neurol., 2013, 26, 569-576)]. Failure to excite and/or propagate action potentials in muscle can also arise from reduced muscle excitability such as in critical illness myopathy (CIM) (Latronico, N., Bolton, C.F. Critical illness polyneuropathy and myopathy: a major cause of muscle weakness and paralysis. Lancet Neurol. 2011, 10, 931-941). In Lambert Eaton syndrome, an autoimmune attack against the pre-synaptic P/Q-type Ca²⁺ channels results in markedly reduced Ca²⁺ influx into the nerve terminal during the pre-synaptic action potential and consequently a reduced release of ACh into the synaptic cleft. In myasthenia gravis, the most common finding is an autoimmune attack on the post-synaptic membrane either against the nicotinic ACh receptors or the musk-receptor in the muscle fibre membrane. Congenital forms of myasthenia are also known. Common to disorders with neuromuscular transmission failure (Lambert Eaton syndrome, amyotrophic lateral sclerosis, spinal muscular atrophy and myasthenia gravis) is that the current flow generated by ACh receptor activation is markedly reduced, and EPPs therefore become insufficient to trigger muscle fibre action potentials.

Neuromuscular blocking agents also reduce EPP by antagonizing ACh receptors. In CIM with reduced muscle excitability, the EPP may be of normal amplitude but they are still insufficient to trigger muscle fibre action potentials because the membrane potential threshold for action potential excitation has become more depolarized because of loss of function of voltage gated Na⁺ channels in the muscle fibres.

While ACh release (Lambert Eaton, amyotrophic lateral sclerosis, spinal muscular atrophy), ACh receptor function (myasthenia gravis, neuromuscular blockade) and function of voltage gated Na⁺ channels (CIM) are essential components in the synaptic transmission at NMJ, the magnitude of the EPP is also affected by inhibitory currents flowing in the NMJ region of muscle fibres. These currents tend to outbalance excitatory current through ACh receptors and, expectedly, they thereby tend to reduce EPP amplitude. The most important ion channel for carrying such inhibitory membrane currents in muscle fibres is the muscle-specific CIC-1 Cl⁻ ion channel (Kwieciński H, Lehmann-Horn F, R̈üdel R. Membrane currents in human intercostal muscle at varied extracellular potassium. *Muscle Nerve.* **1984,** 7, 465-469; Kwieciński H, Lehmann-Horn F, Rüdel R. Drug-induced myotonia in human intercostal muscle. *Muscle Nerve.* **1988,** 11, 576-581; Pedersen, T.H., F. de Paoli, and O.B. Nielsen. Increased excitability of acidified skeletal muscle: role of chloride conductance. J. Gen. Physiol., 2005, 125, 237-246).

ACh esterase (AChE) inhibitors are traditionally used in the treatment of myasthenia gravis. This treatment leads to improvement in most patients but it is associated with side effects, some of which are serious (Mehndiratta MM, Pandey S, Kuntzer T. Acetylcholinesterase inhibitor treatment for myasthenia gravis. Cochrane Database Syst Rev. 2014, Oct 13;10). Because ACh is an import neurotransmitter in the autonomic nervous system, delaying its breakdown can lead to gastric discomfort, diarrhoea, salivation and muscle cramping. Overdosing is a serious concern as it can lead to muscle paralysis and respiratory failure, a situation commonly referred to as cholinergic crisis. Despite the serious side effects of AChE inhibitors, these drugs are today the treatment of choice for a number of disorders involving neuromuscular impairment. In patients where pyridostigmine (a parasympathomimetic and a reversible AChE inhibitor) is insufficient, corticosteroid treatment (prednisone) and immunosuppressive treatment (azathioprine) is used. Plasma exchange can be used to obtain a fast but transient improvement.

Unfortunately, all of the currently employed drug regimens for treatment of myasthenia gravis are associated with deleterious long-term consequences (Howard, J.F. Jr.

Adverse drug effects on neuromuscular transmission. Semin Neurol. 1990, 10, 89 - 102) despite research to identify new treatments (Gilhus, N.E. New England Journal of Medicine, 2016, 375, 2570-2581).

Kurhade, S. et al., Tetrahedron Lett. (2011), 52, 1874-1877 relates to synthesis of compounds comprising a tetracyclic azaindolo[2,1-c][1,4]benzoxaxzine ring system, e.g. (2R)-2-(2,4-difluorophenoxy)-N-methoxy-N-methyl-propanamide.

Aromataris, E. C. (2010) "Pharmacology of the CIC-1 Chloride channel" is a PhD thesis from the University of Adelaide disclosing for example 2-[phenoxy]propanoic acid derivatives as CIC-1 chloride channel inhibitors and their effects on CIC-1 fast and slow gating.

The CIC-1 ion channel (Pedersen, T.H., Riisager, A., Vincenzo de Paoli, F., Chen, T-Y, Nielsen, O.B. Role of physiological CIC-1 Cl- ion channel regulation for the excitability and function of working skeletal muscle. J. Gen. Physiol. 2016, 147, 291 - 308) is emerging as a target for potential drugs, although its potential has been largely unrealised.

### Summary

The present disclosure comprises a new series of compounds that alleviate disorders of the neuromuscular junction through inhibition of CIC-1 channels.

It has been found that a set of compounds that inhibit CIC-1 ion channels are capable of restoring neuromuscular transmission, as evidenced by the data generated by investigation of the compound set in biological models described herein. Compounds of the disclosure thus constitute a new group of potential drugs that can be used to treat or ameliorate muscle weakness and muscle fatigue in neuromuscular junction disorders caused by disease or by neuromuscular blocking agents.

The present disclosure thus concerns the discovery of CIC-1 ion channel inhibitors with application in the treatment of a range of conditions, such as reversal of block, ALS and myasthenic conditions, in which muscle activation by the nervous system is compromised and symptoms of weakness and fatigue are prominent.

In one aspect, the disclosure concerns a compound of Formula (I): 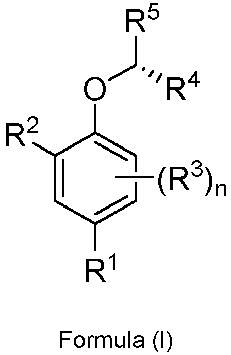 wherein:
- R¹ is selected from the group consisting of F, CI, Br and I;
- R² is selected from the group consisting of H, F, Cl, Br, !, C₁₋₅ alkyl optionally substituted with one or more, identical or different, substituents R⁶, C₂₋₅ alkenyl optionally substituted with one or more, identical or different, substituents R⁶, C₂₋₅ alkynyl optionally substituted with one or more, identical or different, substituents R⁶, C₃₋₅ cycloalkyl optionally substituted with one or more, identical or different, substituents R⁶, phenyl optionally substituted with one or more, identical or different, substituents R⁹, and 5-6 membered aromatic heterocycle optionally substituted with one or more, identical or different, substituents R⁷;
- R³ is selected from the group consisting of deuterium and F;
- R⁴ is selected from the group consisting of C₁₋₅ alkyl, C₂₋₅ alkenyl, C₂₋₅ alkynyl and C₃₋₅ cycloalkyl each of which may be optionally substituted with one or more, identical or different, substituents R⁷;
- R⁵ is selected from the group consisting of 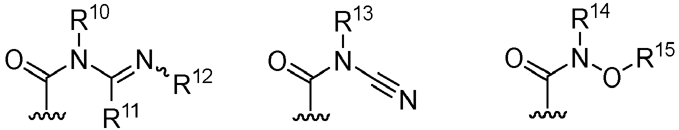 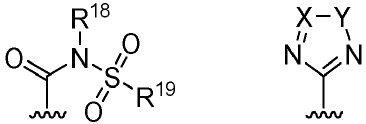
- R⁶ is independently selected from the group consisting of C₃₋₅ cycloalkyl, -O-C₁₋₅ alkyl, -O-C₃₋₅ cycloalkyl, -S-C₁₋₅ alkyl and -S-C₃₋₅ cycloalkyl each of which may optionally substituted with one or more, identical or different, substituents R⁸, deuterium, F and -CN;
- R⁷ is independently selected from the group consisting of C₃₋₅ cycloalkyl, -O-C₁₋₅ alkyl, -O-C₃₋₅ cycloalkyl, -S-C₁₋₅ alkyl and -S-C₃₋₅ cycloalkyl each of which may optionally substituted with one or more, identical or different, substituents R⁸, deuterium, F and -CN;
- R⁸ is independently selected from the group consisting of deuterium, OH, OMe and F;
- R⁹ is independently selected from the group consisting of deuterium, methoxy, nitro, cyano, CF₃, Cl, Br, I and F;
- R¹⁰ is selected from the group consisting of H, C₁₋₅ alkyl optionally substituted with one or more, identical or different, substituents R⁸, C₃₋₅ cycloalkyl optionally substituted with one or more, identical or different, substituents R⁸, -C(=O)-C₁₋₅ alkyl optionally substituted with one or more, identical or different, substituents R⁸ and -C(=O)-C₃₋₅ cycloalkyl optionally substituted with one or more, identical or different, substituents R⁸;
- R¹¹ is selected from the group consisting of C₁₋₅ alkyl optionally substituted with one or more, identical or different, substituents R⁸, C₃₋₅ cycloalkyl optionally substituted with one or more, identical or different, substituents R⁸ and phenyl optionally substituted with one or more, identical or different, substituents R⁹;
- R¹² is selected from the group consisting of -OH, -OC₁₋₅ alkyl optionally substituted with one or more, identical or different, substituents R⁸ and -OC₃₋₅ cycloalkyl optionally substituted with one or more, identical or different, substituents R⁸;
- R¹³ is selected from the group consisting of H, C₁₋₅ alkyl optionally substituted with one or more, identical or different, substituents R⁸, C₃₋₅ cycloalkyl optionally substituted with one or more, identical or different, substituents R⁸, -C(=O)-C₁₋₅ alkyl optionally substituted with one or more, identical or different, substituents R⁸ and -C(=O)-C₃₋₅ cycloalkyl optionally substituted with one or more, identical or different, substituents R⁸;
- R¹⁴ is selected from the group consisting of H, C₁₋₅ alkyl optionally substituted with one or more, identical or different, substituents R¹⁷, C₃₋₅ cycloalkyl optionally substituted with one or more, identical or different, substituents R⁸, -C(=O)-C₁₋₅ alkyl optionally substituted with one or more, identical or different, substituents R⁸ and -C(=O)-C₃₋₅ cycloalkyl optionally substituted with one or more, identical or different, substituents R⁸;
- R¹⁵ is selected from the group consisting of H, C₁₋₅ alkyl optionally substituted with one or more, identical or different, substituents R¹⁶, C₃₋₆ cycloalkyl optionally substituted with one or more, identical or different, substituents R¹⁶, phenyl optionally substituted with one or more, identical or different, substituents R⁹, and 4-6 membered heterocycle optionally substituted with one or more, identical or different, substituents R¹⁷ with the proviso that when R¹⁵ is H then R¹⁴ is not H;
- R¹⁶ is independently selected from the group consisting of F, C!, Br, I, -CN, =O, C₃₋₆ cycloalkyl optionally substituted with one or more, identical or different, substituents R⁸, -O-C₁₋₅ alkyl optionally substituted with one or more, identical or different, substituents R⁸, -O-C₃₋₅ cycloalkyl optionally substituted with one or more, identical or different, substituents R⁸, -S-C₁₋₅ alkyl optionally substituted with one or more, identical or different, substituents R⁸, -S-C₃₋₅ cycloalkyl optionally substituted with one or more, identical or different, substituents R⁸, -SO-C₁₋₅ alkyl optionally substituted with one or more, identical or different, substituents R⁸, -SO-C₃₋₅ cycloalkyl optionally substituted with one or more, identical or different, substituents R⁸, -SO₂-C₁₋₅ alkyl optionally substituted with one or more, identical or different, substituents R⁸, - SO₂-C₃₋₅ cycloalkyl optionally substituted with one or more, identical or different, substituents R⁸, -C(=O)-C₁₋₅ alkyl optionally substituted with one or more, identical or different substituents R⁸, -C(=O)-OC₁₋₅ alkyl optionally substituted with one or more, identical or different, substituents R⁸, -NH-C(=O)-OC₁₋₅ alkyl optionally substituted with one or more, identical or different, substituents R⁸, -C(=O)-C₃₋₅ cycloalkyl optionally substituted with one or more, identical or different, substituents R⁸, -NH-C(=O)-C₁₋₅ alkyl optionally substituted with one or more, identical or different, substituents R⁸, -NH-C(=O)-C₃₋₅ cycloalkyl optionally substituted with one or more, identical or different, substituents R⁸, -C(=O)-NH-C₁₋₅ alkyl optionally substituted with one or more, identical or different, substituents R⁸, -C(=O)-NH-C₃₋₅ cycloalkyl optionally substituted with one or more, identical or different, substituents R⁸, phenyl optionally substituted with one or more, identical or different, substituents R⁹, pyrrolidin-1-yl optionally substituted with one or more, identical or different, substituents R¹⁷ and 4-6 membered heterocycle optionally substituted with one or more, identical or different, substituents R¹⁷;
- R¹⁷ is independently selected from the group consisting of F, C!, Br, I, -CN, =O, C₁₋₅ alkyl optionally substituted with one or more, identical or different, substituents R⁸, C₃₋₆ cycloalkyl optionally substituted with one or more, identical or different, substituents R⁸, -C(=O)-C₁₋₅ alkyl optionally substituted with one or more, identical or different, substituents R⁸, -C(=O)-OC₁₋₅ alkyl optionally substituted with one or more, identical or different, substituents R⁸, -C(=O)-C₃₋₅ cycloalkyl optionally substituted with one or more, identical or different, substituents R⁸, -NH-C(=O)-C₁₋₅ alkyl optionally substituted with one or more, identical or different, substituents R⁸, -NH-C(=O)-C₃₋₅ cycloalkyl optionally substituted with one or more, identical or different, substituents R⁸, -C(=O)-NH-C₁₋₅ alkyl optionally substituted with one or more, identical or different, substituents R⁸ and -C(=O)-NH-C₃₋₅ cycloalkyl optionally substituted with one or more, identical or different, substituents R⁸;
- R¹⁸ is selected from the group consisting of H, C₁₋₅ alkyl optionally substituted with one or more, identical or different, substituents R⁸, C₃₋₅ cycloalkyl optionally substituted with one or more, identical or different, substituents R⁸, -C(=O)-C₁₋₅ alkyl optionally substituted with one or more, identical or different, substituents R⁸ and -C(=O)-C₃₋₅ cycloalkyl optionally substituted with one or more, identical or different, substituents R⁸;

- R¹⁹ is selected from the group consisting of C₁₋₅ alkyl optionally substituted with one or more, identical or different, substituents R⁸, C₃₋₅ cycloalkyl optionally substituted with one or more, identical or different, substituents R⁸ and phenyl optionally substituted with one or more, identical or different, substituents R⁹;
- X is selected from the group consisting of N and CR²⁰;
- Y is selected from the group consisting of NH, O and S;
- R²⁰ is selected from the group consisting of H, NH₂, C₁₋₅ alkyl optionally substituted with one or more, identical or different, substituents R⁸, C₃₋₅ cycloalkyl optionally substituted with one or more, identical or different, substituents R⁸, -OC₁₋₅ alkyl optionally substituted with one or more, identical or different, substituents R⁸, -OC₃₋₅ cycloalkyl optionally substituted with one or more, identical or different, substituents R⁸, -NH-C(=O)C₁₋₅ alkyl optionally substituted with one or more, identical or different, substituents R⁸ and -NH-SO₂-C₁₋₅ alkyl optionally substituted with one or more, identical or different, substituents R⁸;
- n is an integer 0, 1, 2 or 3;
or a pharmaceutically acceptable salt, hydrate, polymorph, tautomer, or solvate thereof.

In another aspect, the disclosure concerns a compound as defined herein for use in treating, ameliorating and/or preventing a neuromuscular disorder, and/or for use in reversing and/or ameliorating a neuromuscular blockade. In yet another aspect, the disclosure concerns a composition comprising a compound as defined herein.

The invention is defined by the claims. Any subject matter falling outside the scope of the claims is provided for information purposes only.

### Description of Drawings

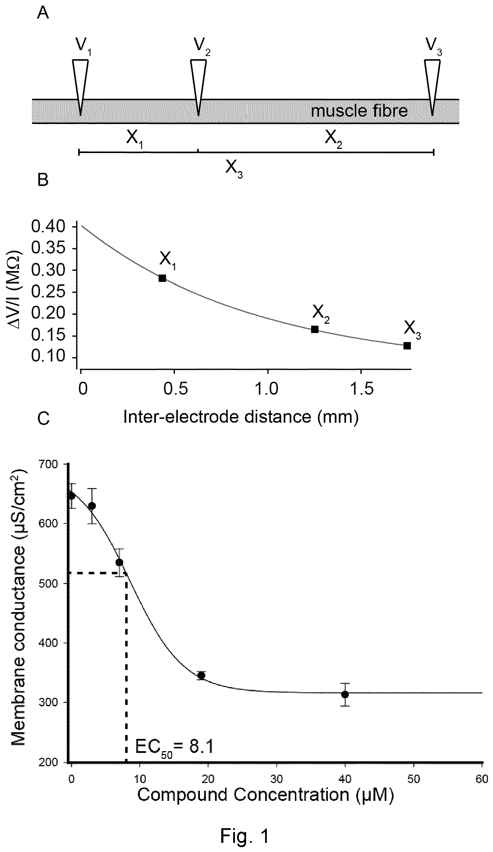

**Figure 1****.** Panel A shows a schematic representation of the positioning of the three microelectrodes (V₁, V₂ and V₃) when inserted in a single skeletal muscle fibre for Gₘ determination. Please note that the drawing illustrates only the impaled fibre although it is part of an intact muscle that contains many such fibres. All electrodes recorded the membrane potential of the fibre and the two peripheral electrodes were used to inject current (-30 nA, 50 ms). The electrodes were inserted with known inter-electrode distances (X₁, X₂ and X₃). After insertion, current was passed first via the V₁ electrode and then via the V₃ electrode. The resulting deflections in the membrane voltage were measured by the other electrodes. The steady state deflections in membrane potential were measured and divided by the magnitude of the injected current (-30 nA) to obtain transfer resistances. These were next plotted against inter-electrode distances, and fitted to an exponential function (Panel B), from which Gₘ could be calculated using linear cable theory. The approach described in panel A and B, was repeated for several muscle fibres in the muscle during exposure at increasing concentrations of compound E-8, with approx. 10 fibres at each concentration. Average Gₘ at each concentration was plotted as a function of compound concentration in panel C, and fitted to a 4-parameter sigmoidal function from which the EC₅₀ value for the compound was obtained (dashed line)

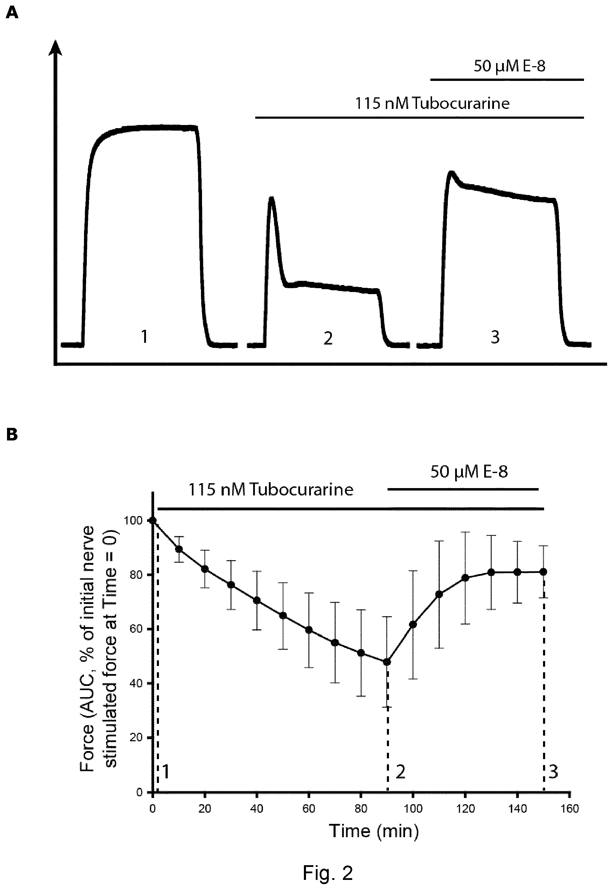

**Figure 2****.** Panel A shows representative force traces before and after exposure to compound E-8. Force traces from a representative muscle stimulated to contract in 1) control condition before addition of neuromuscular blocking agent, 2) the force response to stimulation after 90 minutes incubation with Tubocurarine. Here the muscle displays severe neuromuscular transmission impediment, and 3) The muscle force response after addition of 50 µM) compound E-8. Panel B shows average force (AUC) from 3 muscles relative to their initial force. The traces presented in panel A (1, 2, 3), correspond to the dotted lines in panel B, respectively. Thus, force is lost due to 90 min incubation in tubocurarine and is subsequently recovered when compound E-8 is added.

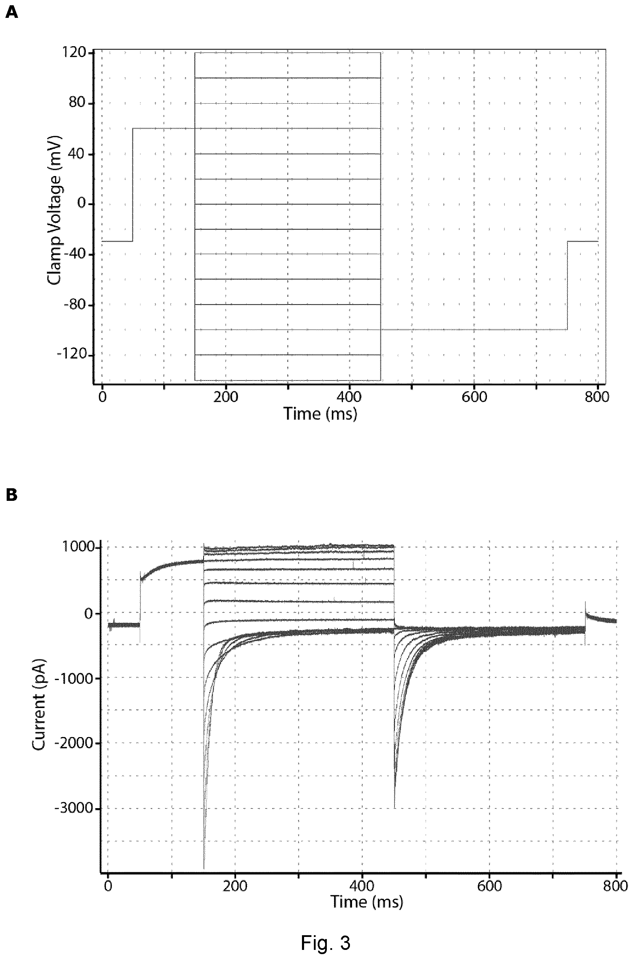

**Figure 3****:** Panel A illustrates the voltage protocol used to evoke currents in whole cell patches of CHO cells expressing human CIC-1 channels. Panel B shows representative whole cell current traces from a patched CHO cell expressing human CIC-1 channels. Currents were evoked by applying the voltage protocol shown in Panel A.

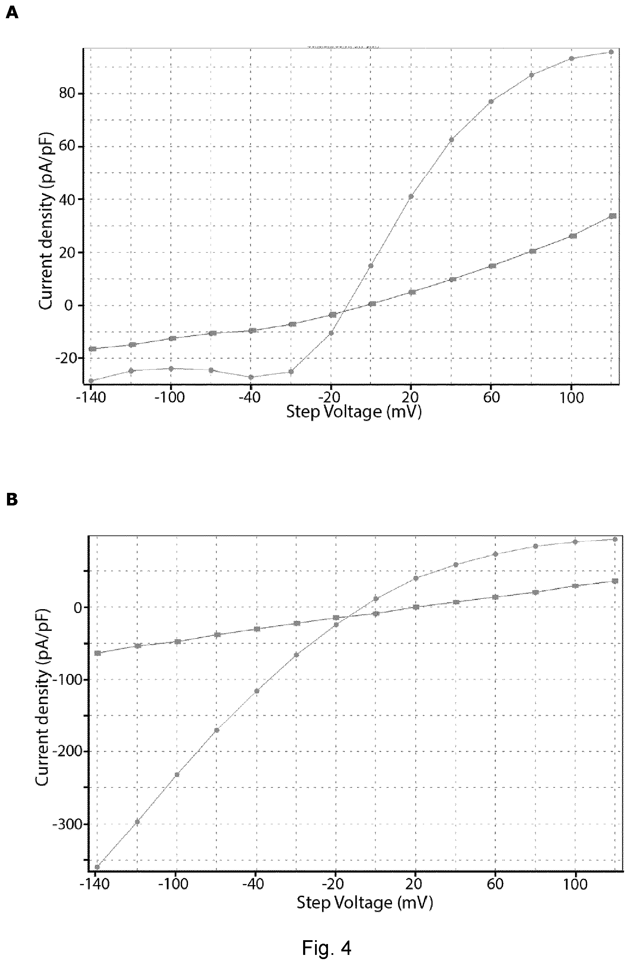

**Figure 4****:** Panel A shows a representative I/V plot of constant current density in a CIC-1 expressing CHO cell before (circles) and after (squares) application of 100 µM) of the CIC-1 inhibitor, 9-anthracenecarboxylic acid (9-AC, Sigma A89405). Panel B shows a I/V plot of instant tail current density from the same CIC-1 expressing CHO cell as illustrated in Panel A, before (circles) and after (squares) application of 100 µM) 9-AC.

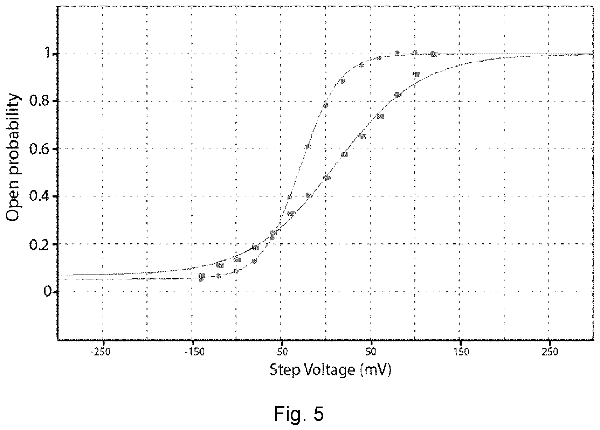

**Figure 5:** Figure 5 shows representative plots of normalized instant tail currents from a CIC-1 expressing CHO cell patch before (circles) and after (squares) application of 100 µM) 9-AC. The instant tail currents at each voltage step were normalized to the maximal tail current obtained following the (+)120 mV voltage step and fitted to a Boltzmann function to determine the half activation potential, V_{1/2}.

### Detailed description

### Definitions

The terms "C₁₋₃ alkyl" and "C₁₋₅ alkyl" refers to a branched or unbranched alkyl group having from one to three or one to five carbon atoms respectively, including but not limited to methyl, ethyl, prop-1-yl, prop-2-yl, 2-methyl-prop-1-yl, 2-methyl-prop-2-yl, 2,2-dimethyl-prop-1-yl, but-1-yl, but-2-yl, 3-methyl-but-1-yl, 3-methyl-but-2-yl, pent-1-yl, pent-2-yl and pent-3-yl.

The term "C₂₋₅ alkenyl" refers to a branched or unbranched alkenyl group having from one to five carbon atoms, two of which are connected by a double bond, including but not limited to ethenyl, propenyl, isopropenyl, butenyl, isobutenyl, pentenyl and isopentenyl.

The term "C₂₋₅ alkynyl" refers to a branched or unbranched alkynyl group having from one to five carbon atoms, two of which are connected by a triple bond, including but not limited to ethynyl, prop-1-ynyl, prop-2-ynyl, but-1-ynyl, but-2-ynyl, but-3-ynyl, buta-1,3-diynyl, pent-1-ynyl, pent-2-ynyl, pent-3-ynyl, pent-4-ynyl, penta-2,4-diynyl and penta-1,3-diynyl.

The term "C₃₋₅ cycloalkyl" and "C₃₋₆ cycloalkyl" refers to a group having three to five or three to six carbon atoms respectively including a monocyclic or bicyclic carbocycle, including but not limited to cyclopropyl, cyclobutyl, cyclopentyl and cyclohexyl.

The term "5-6 membered aromatic heterocycle" refers to an aromatic group having five or six carbon atoms respectively wherein between 1 and 3 carbon atoms in the ring have been replaced with a heteroatom selected from the group comprising nitrogen, sulphur and oxygen. Binding to the heterocycle may be at the position of the heteroatom or via a carbon atom of the heterocycle. 5-membered aromatic heterocycles include but are not limited to furan, thiophene, pyrrole, imidazole, pyrazole, oxazole, thiazole, isoxazole, isothiazole, 1,2,3-triazole, 1,2,4-triazole, 1,2,5-oxadiazole, 1,2,3-oxadiazole, 1,2,4-oxadiazole, 1,3,4-oxadiazole, 1,2,5-thiadiazole, 1,2,3-thiadiazole, 1,2,4-thiadiazole, and 1,3,4-thiadiazole. 6-membered heterocycles include but are not limited to pyridine, pyrazine, pyrimidine and pyridazine.

The term "4-6 membered heterocycle" refers to an aromatic group having four, five or six carbon atoms respectively wherein between 1 and 3 carbon atoms in the ring have been replaced with a heteroatom selected from the group comprising nitrogen, sulphur and oxygen. Binding to the heterocycle may be at the position of the heteroatom or via a carbon atom of the heterocycle.
4-membered heterocycles include but are not limited to oxetane, azetidine and thietane.
5-membered heterocycles include but are not limited to furan, thiophene, pyrrole, imidazole, pyrazole, oxazole, thiazole, isoxazole, isothiazole, 1,2,3-triazole, 1,2,4-triazole, 1,2,5-oxadiazole, 1,2,3-oxadiazole, 1,2,4-oxadiazole, 1,3,4-oxadiazole, 1,2,5-thiadiazole, 1,2,3-thiadiazole, 1,2,4-thiadiazole, 1,3,4-thiadiazole, dihydrofuran, dihydrothiophene, 3-pyrroline, 2-pyrroline, 2-imidazoline, 2-pyrazolidine, dihydro-oxazole, dihydro-thiazole, dihydro-isoxazole, dihydro-isothiazole, dihydro-1,2,3-triazole, dihydro-1,2,4-triazole, dihydro-1,2,5-oxadiazole, dihydro-1,2,3-oxadiazole, dihydro-1,2,4-oxadiazole, dihydro-1,3,4-oxadiazole, dihydro-1,2,5-thiadiazole, dihydro-1,2,3-thiadiazole, dihydro-1,2,4-thiadiazole, dihydro-1,3,4-thiadiazole, tetrahydrofuran, tetrahydrothiophene, pyrrolidine, imidazolidine, pyrazolidine, oxazolidine, thiazolidine, isoxazolidine, isothiazolidine, 1,2,3-triazolidine, 1,2,4-triazolidine, 1,2,5-oxadiazolidine, 1,2,3-oxadiazolidine, 1,3,4-oxadiazolidine, 1,2,5-thiadiazolidine, 1,2,3-thiadiazolidine, 1,3,4-thiadiazolidine, 1,2-oxathiolane, 1,3-oxathiolane, 2-oxazolidinone and 2-pyrrolidinone.
6-membered heterocycles include but are not limited to pyridine, pyrazine, pyrimidine, pyridazine, tetrahydropyran, thiane, piperidine, 1, 4-dioxane, morpholine, 1,4-oxathiane, 1,4-diathiane and piperazine.

The term "half-life" as used herein is the time it takes for the compound to lose one-half of its pharmacologic activity. The term "plasma half-life" is the time that it takes the compound to lose one-half of its pharmacologic activity in the blood plasma.

The term "treatment" refers to the combating of a disease or disorder. "Treatment" or "treating," as used herein, includes any desirable effect on the symptoms or pathology of a disease or condition as described herein, and may include even minimal changes or improvements in one or more measurable markers of the disease or condition being treated. "Treatment" or "treating" does not necessarily indicate complete eradication or cure of the disease or condition, or associated symptoms thereof. In some embodiments, the term "treatment" encompasses amelioration and prevention.

The term "amelioration" refers to moderation in the severity of the symptoms of a disease or condition. Improvement in a patient's condition, or the activity of making an effort to correct, or at least make more acceptable, conditions that are difficult to endure related to patient's conditions is considered "ameliorative" treatment.

The term "prevent" or "preventing" refers to precluding, averting, obviating, forestalling, stopping, or hindering something from happening, especially by advance action.

The term "reversal" or "reversing" refers to the ability of a compound to restore nerve-stimulated force in skeletal muscle exposed either ex *vivo* or *in vivo* to a non-depolarizing neuromuscular blocking agent or another pharmaceutical that is able to depress neuromuscular transmission
The term "non-depolarizing blockers" refers to pharmaceutical agents that antagonize the activation of acetylcholine receptors at the post-synaptic muscle fibre membrane by blocking the acetylcholine binding site on the receptor. These agents are used to block neuromuscular transmission and induce muscle paralysis in connection with surgery.

The term "ester hydrolysing reagent" refers to a chemical reagent which is capable of converting an ester functional group to a carboxylic acid with elimination of the alcohol moiety of the original ester, including but not limited to acid, base, a fluoride source, PBr₃, PCI₃ and lipase enzymes.

The term "recovery of force in muscle with neuromuscular dysfunction" refers to the ability of a compound to recover contractile force in nerve-stimulated healthy rat muscle after exposure to submaximal concentration of (115 nM) tubocurarine for 90 mins. Recovery of force is quantified as the percentage of the force prior to tubocurarine that is recovered by the compound.

The term "total membrane conductance (Gm)" is the electrophysiological measure of the ability of ions to cross the muscle fibre surface membrane. It reflects the function of ion channels that are active in resting muscle fibres of which CIC-1 is known to contribute around 80 % in most animal species.

### Compounds

It is within the scope of the present disclosure to provide a compound for use in treating, ameliorating and/or preventing neuromuscular disorders characterized in that the neuromuscular function is reduced. As disclosed herein, inhibition of CIC-1 improves or restores neuromuscular function. The compounds of the present disclosure comprise compounds capable of inhibiting the CIC-1 channel thereby improving or restoring neuromuscular function. In one embodiment, the EC₅₀ of the compound is <50 µM, such as <40 µM, such as <30 µM, such as <20 µM, such as <15 µM, or such as <10 µM. In one embodiment, the recovery of force in muscles with neuromuscular dysfunction is >5%, such as >10%, such as >15%, such as >20%, such as >25%, such as >30% or such as >35%.

In one aspect, the disclosure concerns a compound of Formula (I): 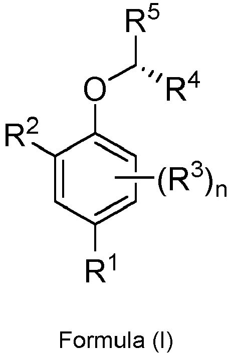 wherein:
- R¹ is selected from the group consisting of F, CI, Br and I;
- R² is selected from the group consisting of H, F, Cl, Br, !, C₁₋₅ alkyl optionally substituted with one or more, identical or different, substituents R⁶, C₂₋₅ alkenyl optionally substituted with one or more, identical or different, substituents R⁶, C₂₋₅ alkynyl optionally substituted with one or more, identical or different, substituents R⁶, C₃₋₅ cycloalkyl optionally substituted with one or more, identical or different, substituents R⁶, phenyl optionally substituted with one or more, identical or different, substituents R⁹, and 5-6 membered aromatic heterocycle optionally substituted with one or more, identical or different, substituents R⁷;
- R³ is selected from the group consisting of deuterium and F;
- R⁴ is selected from the group consisting of C₁₋₅ alkyl, C₂₋₅ alkenyl, C₂₋₅ alkynyl and C₃₋₅ cycloalkyl each of which may be optionally substituted with one or more, identical or different, substituents R⁷;
- R⁵ is selected from the group consisting of 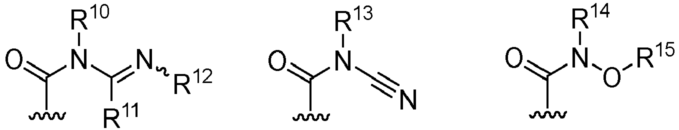 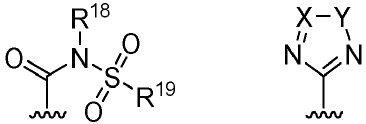
- R⁶ is independently selected from the group consisting of C₃₋₅ cycloalkyl, -O-C₁₋₅ alkyl, -O-C₃₋₅ cycloalkyl, -S-C₁₋₅ alkyl and -S-C₃₋₅ cycloalkyl each of which may optionally substituted with one or more, identical or different, substituents R⁸, deuterium, F and -CN;
- R⁷ is independently selected from the group consisting of C₃₋₅ cycloalkyl, -O-C₁₋₅ alkyl, -O-C₃₋₅ cycloalkyl, -S-C₁₋₅ alkyl and -S-C₃₋₅ cycloalkyl each of which may optionally substituted with one or more, identical or different, substituents R⁸, deuterium, F and -CN;
- R⁸ is independently selected from the group consisting of deuterium, OH, OMe and F;
- R⁹ is independently selected from the group consisting of deuterium, methoxy, nitro, cyano, CF₃, Cl, Br, I and F;
- R¹⁰ is selected from the group consisting of H, C₁₋₅ alkyl optionally substituted with one or more, identical or different, substituents R⁸, C₃₋₅ cycloalkyl optionally substituted with one or more, identical or different, substituents R⁸, -C(=O)-C₁₋₅ alkyl optionally substituted with one or more, identical or different, substituents R⁸ and -C(=O)-C₃₋₅ cycloalkyl optionally substituted with one or more, identical or different, substituents R⁸;
- R¹¹ is selected from the group consisting of C₁₋₅ alkyl optionally substituted with one or more, identical or different, substituents R⁸, C₃₋₅ cycloalkyl optionally substituted with one or more, identical or different, substituents R⁸ and phenyl optionally substituted with one or more, identical or different, substituents R⁹;
- R¹² is selected from the group consisting of -OH, -OC₁₋₅ alkyl optionally substituted with one or more, identical or different, substituents R⁸ and -OC₃₋₅ cycloalkyl optionally substituted with one or more, identical or different, substituents R⁸;
- R¹³ is selected from the group consisting of H, C₁₋₅ alkyl optionally substituted with one or more, identical or different, substituents R⁸, C₃₋₅ cycloalkyl optionally substituted with one or more, identical or different, substituents R⁸, -C(=O)-C₁₋₅ alkyl optionally substituted with one or more, identical or different, substituents R⁸ and -C(=O)-C₃₋₅ cycloalkyl optionally substituted with one or more, identical or different, substituents R⁸;
- R¹⁴ is selected from the group consisting of H, C₁₋₅ alkyl optionally substituted with one or more, identical or different, substituents R¹⁷, C₃₋₅ cycloalkyl optionally substituted with one or more, identical or different, substituents R⁸, -C(=O)-C₁₋₅ alkyl optionally substituted with one or more, identical or different, substituents R⁸ and -C(=O)-C₃₋₅ cycloalkyl optionally substituted with one or more, identical or different, substituents R⁸;
- R¹⁵ is selected from the group consisting of H, C₁₋₅ alkyl optionally substituted with one or more, identical or different, substituents R¹⁶, C₃₋₆ cycloalkyl optionally substituted with one or more, identical or different, substituents R¹⁶, phenyl optionally substituted with one or more, identical or different, substituents R⁹, and 4-6 membered heterocycle optionally substituted with one or more, identical or different, substituents R¹⁷ with the proviso that when R¹⁵ is H then R¹⁴ is not H;
- R¹⁶ is independently selected from the group consisting of F, C!, Br, I, -CN, =O, C₃₋₆ cycloalkyl optionally substituted with one or more, identical or different, substituents R⁸, -O-C₁₋₅ alkyl optionally substituted with one or more, identical or different, substituents R⁸, -O-C₃₋₅ cycloalkyl optionally substituted with one or more, identical or different, substituents R⁸, -S-C₁₋₅ alkyl optionally substituted with one or more, identical or different, substituents R⁸, -S-C₃₋₅ cycloalkyl optionally substituted with one or more, identical or different, substituents R⁸, -SO-C₁₋₅ alkyl optionally substituted with one or more, identical or different, substituents R⁸, -SO-C₃₋₅ cycloalkyl optionally substituted with one or more, identical or different, substituents R⁸, -SO₂-C₁₋₅ alkyl optionally substituted with one or more, identical or different, substituents R⁸, - SO₂-C₃₋₅ cycloalkyl optionally substituted with one or more, identical or different, substituents R⁸, -C(=O)-C₁₋₅ alkyl optionally substituted with one or more, identical or different substituents R⁸, -C(=O)-OC₁₋₅ alkyl optionally substituted with one or more, identical or different, substituents R⁸, -NH-C(=O)-OC₁₋₅ alkyl optionally substituted with one or more, identical or different, substituents R⁸, -C(=O)-C₃₋₅ cycloalkyl optionally substituted with one or more, identical or different, substituents R⁸, -NH-C(=O)-C₁₋₅ alkyl optionally substituted with one or more, identical or different, substituents R⁸, -NH-C(=O)-C₃₋₅ cycloalkyl optionally substituted with one or more, identical or different, substituents R⁸, -C(=O)-NH-C₁₋₅ alkyl optionally substituted with one or more, identical or different, substituents R⁸, -C(=O)-NH-C₃₋₅ cycloalkyl optionally substituted with one or more, identical or different, substituents R⁸, phenyl optionally substituted with one or more, identical or different, substituents R⁹, pyrrolidin-1-yl optionally substituted with one or more, identical or different, substituents R¹⁷ and 4-6 membered heterocycle optionally substituted with one or more, identical or different, substituents R¹⁷;
- R¹⁷ is independently selected from the group consisting of F, C!, Br, I, -CN, =O, C₁₋₅ alkyl optionally substituted with one or more, identical or different, substituents R⁸, C₃₋₆ cycloalkyl optionally substituted with one or more, identical or different, substituents R⁸, -C(=O)-C₁₋₅ alkyl optionally substituted with one or more, identical or different, substituents R⁸, -C(=O)-OC₁₋₅ alkyl optionally substituted with one or more, identical or different, substituents R⁸, -C(=O)-C₃₋₅ cycloalkyl optionally substituted with one or more, identical or different, substituents R⁸, -NH-C(=O)-C₁₋₅ alkyl optionally substituted with one or more, identical or different, substituents R⁸, -NH-C(=O)-C₃₋₅ cycloalkyl optionally substituted with one or more, identical or different, substituents R⁸, -C(=O)-NH-C₁₋₅ alkyl optionally substituted with one or more, identical or different, substituents R⁸ and -C(=O)-NH-C₃₋₅ cycloalkyl optionally substituted with one or more, identical or different, substituents R⁸;
- R¹⁸ is selected from the group consisting of H, C₁₋₅ alkyl optionally substituted with one or more, identical or different, substituents R⁸, C₃₋₅ cycloalkyl optionally substituted with one or more, identical or different, substituents R⁸, -C(=O)-C₁₋₅ alkyl optionally substituted with one or more, identical or different, substituents R⁸ and -C(=O)-C₃₋₅ cycloalkyl optionally substituted with one or more, identical or different, substituents R⁸;
- R¹⁹ is selected from the group consisting of C₁₋₅ alkyl optionally substituted with one or more, identical or different, substituents R⁸, C₃₋₅ cycloalkyl optionally substituted with one or more, identical or different, substituents R⁸ and phenyl optionally substituted with one or more, identical or different, substituents R⁹;
- X is selected from the group consisting of N and CR²⁰;
- Y is selected from the group consisting of NH, O and S;
- R²⁰ is selected from the group consisting of H, NH₂, C₁₋₅ alkyl optionally substituted with one or more, identical or different, substituents R⁸, C₃₋₅ cycloalkyl optionally substituted with one or more, identical or different, substituents R⁸, -OC₁₋₅ alkyl optionally substituted with one or more, identical or different, substituents R⁸, -OC₃₋₅ cycloalkyl optionally substituted with one or more, identical or different, substituents R⁸, -NH-C(=O)C₁₋₅ alkyl optionally substituted with one or more, identical or different, substituents R⁸ and -NH-SO₂-C₁₋₅ alkyl optionally substituted with one or more, identical or different, substituents R⁸;
- n is an integer 0, 1, 2 or 3;
or a pharmaceutically acceptable salt, hydrate, polymorph, tautomer, or solvate thereof.

In one aspect, the disclosure concerns a compound of Formula (I): 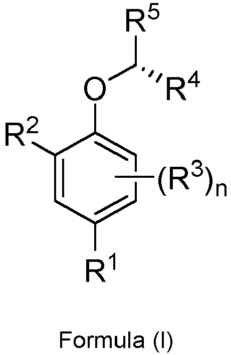 wherein:
- R¹ is selected from the group consisting of F, CI, Br and I;
- R² is selected from the group consisting of H, F, Cl, Br, !, C₁₋₅ alkyl optionally substituted with one or more, identical or different, substituents R⁶, C₂₋₅ alkenyl optionally substituted with one or more, identical or different, substituents R⁶, C₂₋₅ alkynyl optionally substituted with one or more, identical or different, substituents R⁶, C₃₋₅ cycloalkyl optionally substituted with one or more, identical or different, substituents R⁶, phenyl optionally substituted with one or more, identical or different, substituents R⁹, and 5-6 membered aromatic heterocycle optionally substituted with one or more, identical or different, substituents R⁷;
- R³ is selected from the group consisting of deuterium and F;
- R⁴ is selected from the group consisting of C₁₋₅ alkyl, C₂₋₅ alkenyl, C₂₋₅ alkynyl and C₃₋₅ cycloalkyl each of which may be optionally substituted with one or more, identical or different, substituents R⁷;
- R⁵ is selected from the group consisting of 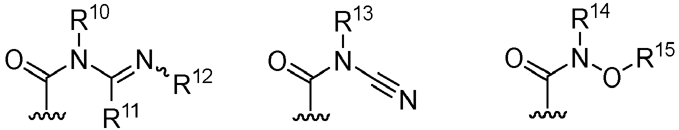 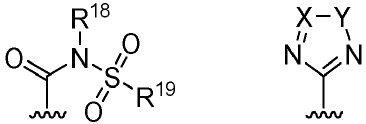
- R⁶ is independently selected from the group consisting of C₃₋₅ cycloalkyl, -O-C₁₋₅ alkyl, -O-C₃₋₅ cycloalkyl, -S-C₁₋₅ alkyl and -S-C₃₋₅ cycloalkyl each of which may optionally substituted with one or more, identical or different, substituents R⁸, deuterium, F and -CN;
- R⁷ is independently selected from the group consisting of C₃₋₅ cycloalkyl, -O-C₁₋₅ alkyl, -O-C₃₋₅ cycloalkyl, -S-C₁₋₅ alkyl and -S-C₃₋₅ cycloalkyl each of which may optionally substituted with one or more, identical or different, substituents R⁸, deuterium, F and -CN;
- R⁸ is independently selected from the group consisting of deuterium, OH, OMe and F;
- R⁹ is independently selected from the group consisting of deuterium, methoxy, nitro, cyano, CF₃, Cl, Br, I and F;
- R¹⁰ is selected from the group consisting of H, C₁₋₅ alkyl optionally substituted with one or more, identical or different, substituents R⁸, C₃₋₅ cycloalkyl optionally substituted with one or more, identical or different, substituents R⁸, -C(=O)-C₁₋₅ alkyl optionally substituted with one or more, identical or different, substituents R⁸ and -C(=O)-C₃₋₅ cycloalkyl optionally substituted with one or more, identical or different, substituents R⁸;
- R¹¹ is selected from the group consisting of C₁₋₅ alkyl optionally substituted with one or more, identical or different, substituents R⁸, C₃₋₅ cycloalkyl optionally substituted with one or more, identical or different, substituents R⁸ and phenyl optionally substituted with one or more, identical or different, substituents R⁹;
- R¹² is selected from the group consisting of -OH, -OC₁₋₅ alkyl optionally substituted with one or more, identical or different, substituents R⁸ and -OC₃₋₅ cycloalkyl optionally substituted with one or more, identical or different, substituents R⁸;
- R¹³ is selected from the group consisting of H, C₁₋₅ alkyl optionally substituted with one or more, identical or different, substituents R⁸, C₃₋₅ cycloalkyl optionally substituted with one or more, identical or different, substituents R⁸, -C(=O)-C₁₋₅ alkyl optionally substituted with one or more, identical or different, substituents R⁸ and -C(=O)-C₃₋₅ cycloalkyl optionally substituted with one or more, identical or different, substituents R⁸;
- R¹⁴ is selected from the group consisting of H, C₁₋₅ alkyl optionally substituted with one or more, identical or different, substituents R¹⁷, C₃₋₅ cycloalkyl optionally substituted with one or more, identical or different, substituents R⁸, -C(=O)-C₁₋₅ alkyl optionally substituted with one or more, identical or different, substituents R⁸ and -C(=O)-C₃₋₅ cycloalkyl optionally substituted with one or more, identical or different, substituents R⁸;
- R¹⁵ is selected from the group consisting of H, C₁₋₅ alkyl optionally substituted with one or more, identical or different, substituents R¹⁶, C₃₋₆ cycloalkyl optionally substituted with one or more, identical or different, substituents R¹⁶, phenyl optionally substituted with one or more, identical or different, substituents R⁹, and 4-6 membered heterocycle optionally substituted with one or more, identical or different, substituents R¹⁷ with the proviso that when R¹⁵ is H then R¹⁴ is not H;
- R¹⁶ is independently selected from the group consisting of F, C!, Br, I, -CN, =O, C₃₋₆ cycloalkyl optionally substituted with one or more, identical or different, substituents R⁸, -O-C₁₋₅ alkyl optionally substituted with one or more, identical or different, substituents R⁸, -O-C₃₋₅ cycloalkyl optionally substituted with one or more, identical or different, substituents R⁸, -S-C₁₋₅ alkyl optionally substituted with one or more, identical or different, substituents R⁸, -S-C₃₋₅ cycloalkyl optionally substituted with one or more, identical or different, substituents R⁸, -SO-C₁₋₅ alkyl optionally substituted with one or more, identical or different, substituents R⁸, -SO-C₃₋₅ cycloalkyl optionally substituted with one or more, identical or different, substituents R⁸, -SO₂-C₁₋₅ alkyl optionally substituted with one or more, identical or different, substituents R⁸, - SO₂-C₃₋₅ cycloalkyl optionally substituted with one or more, identical or different, substituents R⁸, -C(=O)-C₁₋₅ alkyl optionally substituted with one or more, identical or different substituents R⁸, -C(=O)-OC₁₋₅ alkyl optionally substituted with one or more, identical or different, substituents R⁸, -NH-C(=O)-OC₁₋₅ alkyl optionally substituted with one or more, identical or different, substituents R⁸, -C(=O)-C₃₋₅ cycloalkyl optionally substituted with one or more, identical or different, substituents R⁸, -NH-C(=O)-C₁₋₅ alkyl optionally substituted with one or more, identical or different, substituents R⁸, -NH-C(=O)-C₃₋₅ cycloalkyl optionally substituted with one or more, identical or different, substituents R⁸, -C(=O)-NH-C₁₋₅ alkyl optionally substituted with one or more, identical or different, substituents R⁸, -C(=O)-NH-C₃₋₅ cycloalkyl optionally substituted with one or more, identical or different, substituents R⁸, phenyl optionally substituted with one or more, identical or different, substituents R⁹, pyrrolidin-1-yl optionally substituted with one or more, identical or different, substituents R¹⁷ and 4-6 membered heterocycle optionally substituted with one or more, identical or different, substituents R¹⁷;
- R¹⁷ is independently selected from the group consisting of F, C!, Br, I, -CN, =O, C₁₋₅ alkyl optionally substituted with one or more, identical or different, substituents R⁸, C₃₋₆ cycloalkyl optionally substituted with one or more, identical or different, substituents R⁸, -C(=O)-C₁₋₅ alkyl optionally substituted with one or more, identical or different, substituents R⁸, -C(=O)-OC₁₋₅ alkyl optionally substituted with one or more, identical or different, substituents R⁸, -C(=O)-C₃₋₅ cycloalkyl optionally substituted with one or more, identical or different, substituents R⁸, -NH-C(=O)-C₁₋₅ alkyl optionally substituted with one or more, identical or different, substituents R⁸, -NH-C(=O)-C₃₋₅ cycloalkyl optionally substituted with one or more, identical or different, substituents R⁸, -C(=O)-NH-C₁₋₅ alkyl optionally substituted with one or more, identical or different, substituents R⁸ and -C(=O)-NH-C₃₋₅ cycloalkyl optionally substituted with one or more, identical or different, substituents R⁸;
- R¹⁸ is selected from the group consisting of H, C₁₋₅ alkyl optionally substituted with one or more, identical or different, substituents R⁸, C₃₋₅ cycloalkyl optionally substituted with one or more, identical or different, substituents R⁸, -C(=O)-C₁₋₅ alkyl optionally substituted with one or more, identical or different, substituents R⁸ and -C(=O)-C₃₋₅ cycloalkyl optionally substituted with one or more, identical or different, substituents R⁸;

- R¹⁹ is selected from the group consisting of C₁₋₅ alkyl optionally substituted with one or more, identical or different, substituents R⁸, C₃₋₅ cycloalkyl optionally substituted with one or more, identical or different, substituents R⁸ and phenyl optionally substituted with one or more, identical or different, substituents R⁹;
- X is selected from the group consisting of N and CR²⁰;
- Y is selected from the group consisting of NH, O and S;
- R²⁰ is selected from the group consisting of H, NH₂, C₁₋₅ alkyl optionally substituted with one or more, identical or different, substituents R⁸, C₃₋₅ cycloalkyl optionally substituted with one or more, identical or different, substituents R⁸, -OC₁₋₅ alkyl optionally substituted with one or more, identical or different, substituents R⁸, -OC₃₋₅ cycloalkyl optionally substituted with one or more, identical or different, substituents R⁸, -NH-C(=O)C₁₋₅ alkyl optionally substituted with one or more, identical or different, substituents R⁸ and -NH-SO₂-C₁₋₅ alkyl optionally substituted with one or more, identical or different, substituents R⁸;
- n is an integer 0, 1, 2 or 3;
or a pharmaceutically acceptable salt, hydrate, polymorph, tautomer, or solvate thereof.

In one embodiment, the compound is of Formula (II) 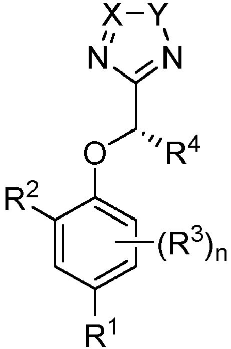 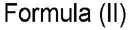 wherein:
- R¹ is selected from the group consisting of F, CI, Br and I;
- R² is selected from the group consisting of H, F, Cl, Br, !, C₁₋₅ alkyl optionally substituted with one or more, identical or different, substituents R⁶, C₂₋₅ alkenyl optionally substituted with one or more, identical or different, substituents R⁶, C₂₋₅ alkynyl optionally substituted with one or more, identical or different, substituents R⁶, C₃₋₅ cycloalkyl optionally substituted with one or more, identical or different, substituents R⁶, phenyl optionally substituted with one or more, identical or different, substituents R⁹, and 5-6 membered aromatic heterocycle optionally substituted with one or more, identical or different, substituents R⁷;
- R³ is selected from the group consisting of deuterium and F;
- R⁴ is selected from the group consisting of C₁₋₅ alkyl, C₂₋₅ alkenyl, C₂₋₅ alkynyl and C₃₋₅ cycloalkyl each of which may be optionally substituted with one or more, identical or different, substituents R⁷;
- R⁶ is independently selected from the group consisting of C₃₋₅ cycloalkyl, -O-C₁₋₅ alkyl, -O-C₃₋₅ cycloalkyl, -S-C₁₋₅ alkyl and -S-C₃₋₅ cycloalkyl each of which may optionally substituted with one or more, identical or different, substituents R⁸, deuterium, F and -CN;
- R⁷ is independently selected from the group consisting of C₃₋₅ cycloalkyl, -O-C₁₋₅ alkyl, -O-C₃₋₅ cycloalkyl, -S-C₁₋₅ alkyl and -S-C₃₋₅ cycloalkyl each of which may optionally substituted with one or more, identical or different, substituents R⁸, deuterium, F and -CN;
- R⁸ is independently selected from the group consisting of deuterium, OH, OMe and F;
- R⁹ is independently selected from the group consisting of deuterium, methoxy, nitro, cyano, CF₃, Cl, Br, I and F;
- X is selected from the group consisting of N and CR²⁰;
- Y is selected from the group consisting of NH, O and S;
- R²⁰ is selected from the group consisting of H, NH₂, C₁₋₅ alkyl optionally substituted with one or more, identical or different, substituents R⁸, C₃₋₅ cycloalkyl optionally substituted with one or more, identical or different, substituents R⁸, -OC₁₋₅ alkyl optionally substituted with one or more, identical or different, substituents R⁸, -OC₃₋₅ cycloalkyl optionally substituted with one or more, identical or different, substituents R⁸, -NH-C(=O)C₁₋₅ alkyl optionally substituted with one or more, identical or different, substituents R⁸ and -NH-SO₂-C₁₋₅ alkyl optionally substituted with one or more, identical or different, substituents R⁸;
- n is an integer 0, 1, 2 or 3;
or a pharmaceutically acceptable salt, hydrate, polymorph, tautomer, or solvate thereof.

In one embodiment, the compound is of Formula (IVa) or Formula (IVb) 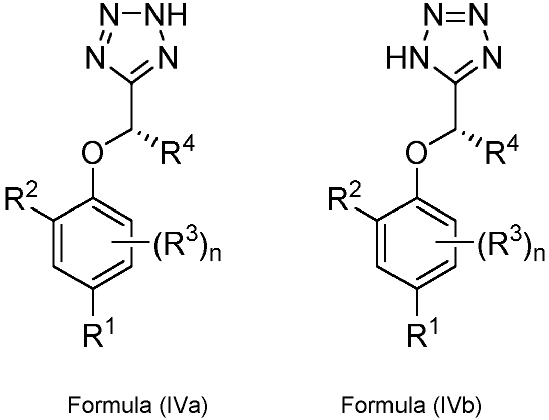 wherein:
- R¹ is selected from the group consisting of F, CI, Br and I;
- R² is selected from the group consisting of H, F, Cl, Br, !, C₁₋₅ alkyl optionally substituted with one or more, identical or different, substituents R⁶, C₂₋₅ alkenyl optionally substituted with one or more, identical or different, substituents R⁶, C₂₋₅ alkynyl optionally substituted with one or more, identical or different, substituents R⁶, C₃₋₅ cycloalkyl optionally substituted with one or more, identical or different, substituents R⁶, phenyl optionally substituted with one or more, identical or different, substituents R⁹, and 5-6 membered aromatic heterocycle optionally substituted with one or more, identical or different, substituents R⁷;
- R³ is selected from the group consisting of deuterium and F;
- R⁴ is selected from the group consisting of C₁₋₅ alkyl, C₂₋₅ alkenyl, C₂₋₅ alkynyl and C₃₋₅ cycloalkyl each of which may be optionally substituted with one or more, identical or different, substituents R⁷;
- R⁶ is independently selected from the group consisting of C₃₋₅ cycloalkyl, -O-C₁₋₅ alkyl, -O-C₃₋₅ cycloalkyl, -S-C₁₋₅ alkyl and -S-C₃₋₅ cycloalkyl each of which may optionally substituted with one or more, identical or different, substituents R⁸, deuterium, F and -CN;
- R⁷ is independently selected from the group consisting of C₃₋₅ cycloalkyl, -O-C₁₋₅ alkyl, -O-C₃₋₅ cycloalkyl, -S-C₁₋₅ alkyl and -S-C₃₋₅ cycloalkyl each of which may optionally substituted with one or more, identical or different, substituents R⁸, deuterium, F and -CN;
- R⁸ is independently selected from the group consisting of deuterium, OH, OMe and F;
- R⁹ is independently selected from the group consisting of deuterium, methoxy, nitro, cyano, CF₃, Cl, Br, I and F; and
- n is an integer 0, 1, 2 or 3;
or a pharmaceutically acceptable salt, hydrate, polymorph, tautomer, or solvate thereof.

It is understood by the skilled person that Formulas (lVa) and (IVb) are tautomers of each other.

In one embodiment, the compound is of Formula (V), 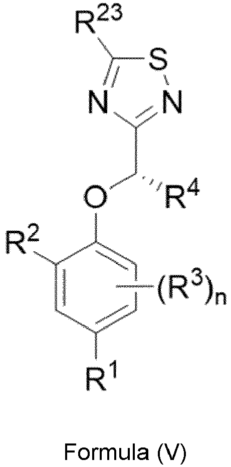 wherein:
- R¹ is selected from the group consisting of F, CI, Br and I;
- R² is selected from the group consisting of H, F, Cl, Br, I, C₁₋₅ alkyl optionally substituted with one or more, identical or different, substituents R⁶, C₂₋₅ alkenyl optionally substituted with one or more, identical or different, substituents R⁶, C₂₋₅ alkynyl optionally substituted with one or more, identical or different, substituents R⁶, C₃₋₅ cycloalkyl optionally substituted with one or more, identical or different, substituents R⁶, phenyl optionally substituted with one or more, identical or different, substituents R⁹, and 5-6 membered aromatic heterocycle optionally substituted with one or more, identical or different, substituents R⁷;
- R³ is selected from the group consisting of deuterium and F;
- R⁴ is selected from the group consisting of C₁₋₅ alkyl, C₂₋₅ alkenyl, C₂₋₅ alkynyl and C₃₋₅ cycloalkyl each of which may be optionally substituted with one or more, identical or different, substituents R⁷;
- R⁶ is independently selected from the group consisting of C₃₋₅ cycloalkyl, -O-C₁₋₅ alkyl, -O-C₃₋₅ cycloalkyl, -S-C₁₋₅ alkyl and -S-C₃₋₅ cycloalkyl each of which may optionally substituted with one or more, identical or different, substituents R⁸, deuterium, F and -CN;
- R⁷ is independently selected from the group consisting of C₃₋₅ cycloalkyl, -O-C₁₋₅ alkyl, -O-C₃₋₅ cycloalkyl, -S-C₁₋₅ alkyl and -S-C₃₋₅ cycloalkyl each of which may optionally substituted with one or more, identical or different, substituents R⁸, deuterium, F and -CN;
- R⁸ is independently selected from the group consisting of deuterium, OH, OMe and F;
- R⁹ is independently selected from the group consisting of deuterium, methoxy, nitro, cyano, CF₃, Cl, Br, I and F;
- R²³ is selected from the group consisting of H, NH₂, C₁₋₅ alkyl optionally substituted with one or more, identical or different, substituents R⁸, C₃₋₅ cycloalkyl optionally substituted with one or more, identical or different, substituents R⁸, -OC₁₋₅ alkyl optionally substituted with one or more, identical or different, substituents R⁸, -OC₃₋₅ cycloalkyl optionally substituted with one or more, identical or different, substituents R⁸, -NH-C(=O)C₁₋₅ alkyl optionally substituted with one or more, identical or different, substituents R⁸ and -NH-SO₂-C₁₋₅ alkyl optionally substituted with one or more, identical or different, substituents R⁸;
- n is an integer 0, 1, 2 or 3;
or a pharmaceutically acceptable salt, hydrate, polymorph, tautomer, or solvate thereof.

In one embodiment, the compound is of Formula (VI), 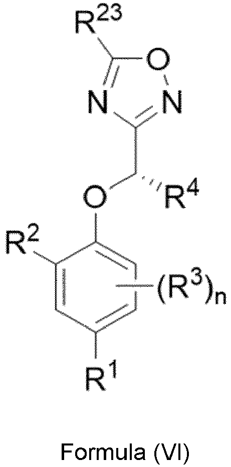 wherein:
- R¹ is selected from the group consisting of F, CI, Br and I;
- R² is selected from the group consisting of H, F, Cl, Br, !, C₁₋₅ alkyl optionally substituted with one or more, identical or different, substituents R⁶, C₂₋₅ alkenyl optionally substituted with one or more, identical or different, substituents R⁶, C₂₋₅ alkynyl optionally substituted with one or more, identical or different, substituents R⁶, C₃₋₅ cycloalkyl optionally substituted with one or more, identical or different, substituents R⁶, phenyl optionally substituted with one or more, identical or different, substituents R⁹, and 5-6 membered aromatic heterocycle optionally substituted with one or more, identical or different, substituents R⁷;
- R³ is selected from the group consisting of deuterium and F;
- R⁴ is selected from the group consisting of C₁₋₅ alkyl, C₂₋₅ alkenyl, C₂₋₅ alkynyl and C₃₋₅ cycloalkyl each of which may be optionally substituted with one or more, identical or different, substituents R⁷;
- R⁶ is independently selected from the group consisting of C₃₋₅ cycloalkyl, -O-C₁₋₅ alkyl, -O-C₃₋₅ cycloalkyl, -S-C₁₋₅ alkyl and -S-C₃₋₅ cycloalkyl each of which may optionally substituted with one or more, identical or different, substituents R⁸, deuterium, F and -CN;
- R⁷ is independently selected from the group consisting of C₃₋₅ cycloalkyl, -O-C₁₋₅ alkyl, -O-C₃₋₅ cycloalkyl, -S-C₁₋₅ alkyl and -S-C₃₋₅ cycloalkyl each of which may optionally substituted with one or more, identical or different, substituents R⁸, deuterium, F and -CN;
- R⁸ is independently selected from the group consisting of deuterium, OH, OMe and F;
- R⁹ is independently selected from the group consisting of deuterium, methoxy, nitro, cyano, CF₃, Cl, Br, I and F;
- R²³ is selected from the group consisting of H, NH₂, C₁₋₅ alkyl optionally substituted with one or more, identical or different, substituents R⁸, C₃₋₅ cycloalkyl optionally substituted with one or more, identical or different, substituents R⁸, -OC₁₋₅ alkyl optionally substituted with one or more, identical or different, substituents R⁸, -OC₃₋₅ cycloalkyl optionally substituted with one or more, identical or different, substituents R⁸, -NH-C(=O)C₁₋₅ alkyl optionally substituted with one or more, identical or different, substituents R⁸ and -NH-SO₂-C₁₋₅ alkyl optionally substituted with one or more, identical or different, substituents R⁸;
- n is an integer 0, 1, 2 or 3;
or a pharmaceutically acceptable salt, hydrate, polymorph, tautomer, or solvate thereof.

In one embodiment, the compound is of Formula (VII), 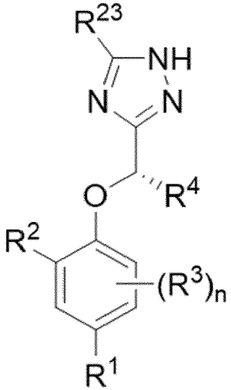 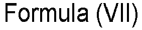 wherein:
- R¹ is selected from the group consisting of F, CI, Br and I;
- R² is selected from the group consisting of H, F, Cl, Br, !, C₁₋₅ alkyl optionally substituted with one or more, identical or different, substituents R⁶, C₂₋₅ alkenyl optionally substituted with one or more, identical or different, substituents R⁶, C₂₋₅ alkynyl optionally substituted with one or more, identical or different, substituents R⁶, C₃₋₅ cycloalkyl optionally substituted with one or more, identical or different, substituents R⁶, phenyl optionally substituted with one or more, identical or different, substituents R⁹, and 5-6 membered aromatic heterocycle optionally substituted with one or more, identical or different, substituents R⁷;
- R³ is selected from the group consisting of deuterium and F;
- R⁴ is selected from the group consisting of C₁₋₅ alkyl, C₂₋₅ alkenyl, C₂₋₅ alkynyl and C₃₋₅ cycloalkyl each of which may be optionally substituted with one or more, identical or different, substituents R⁷;
- R⁶ is independently selected from the group consisting of C₃₋₅ cycloalkyl, -O-C₁₋₅ alkyl, -O-C₃₋₅ cycloalkyl, -S-C₁₋₅ alkyl and -S-C₃₋₅ cycloalkyl each of which may optionally substituted with one or more, identical or different, substituents R⁸, deuterium, F and -CN;
- R⁷ is independently selected from the group consisting of C₃₋₅ cycloalkyl, -O-C₁₋₅ alkyl, -O-C₃₋₅ cycloalkyl, -S-C₁₋₅ alkyl and -S-C₃₋₅ cycloalkyl each of which may optionally substituted with one or more, identical or different, substituents R⁸, deuterium, F and -CN;
- R⁸ is independently selected from the group consisting of deuterium, OH, OMe and F;
- R⁹ is independently selected from the group consisting of deuterium, methoxy, nitro, cyano, CF₃, Cl, Br, I and F;
- R²³ is selected from the group consisting of H, NH₂, C₁₋₅ alkyl optionally substituted with one or more, identical or different, substituents R⁸, C₃₋₅ cycloalkyl optionally substituted with one or more, identical or different, substituents R⁸, -OC₁₋₅ alkyl optionally substituted with one or more, identical or different, substituents R⁸, -OC₃₋₅ cycloalkyl optionally substituted with one or more, identical or different, substituents R⁸, -NH-C(=O)C₁₋₅ alkyl optionally substituted with one or more, identical or different, substituents R⁸ and -NH-SO₂-C₁₋₅ alkyl optionally substituted with one or more, identical or different, substituents R⁸;
- n is an integer 0, 1, 2 or 3;
or a pharmaceutically acceptable salt, hydrate, polymorph, tautomer, or solvate thereof.

In one embodiment, the compound is of Formula (V), Formula (VI) or Formula (VII), wherein R¹, R², R³, R⁴, R⁶, R⁷, R⁸, R⁹, R²³ and n are as defined herein.

In one embodiment, the compound is of Formula (III) 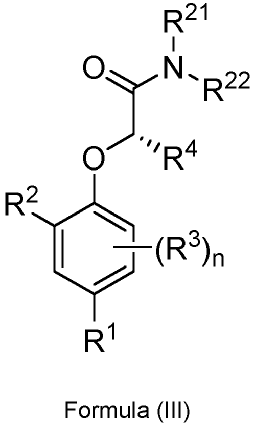 wherein:
- R¹ is selected from the group consisting of F, CI, Br and I;
- R² is selected from the group consisting of H, F, Cl, Br, !, C₁₋₅ alkyl optionally substituted with one or more, identical or different, substituents R⁶, C₂₋₅ alkenyl optionally substituted with one or more, identical or different, substituents R⁶, C₂₋₅ alkynyl optionally substituted with one or more, identical or different, substituents R⁶, C₃₋₅ cycloalkyl optionally substituted with one or more, identical or different, substituents R⁶, phenyl optionally substituted with one or more, identical or different, substituents R⁹, and 5-6 membered aromatic heterocycle optionally substituted with one or more, identical or different, substituents R⁷;
- R³ is selected from the group consisting of deuterium and F;
- R⁴ is selected from the group consisting of C₁₋₅ alkyl, C₂₋₅ alkenyl, C₂₋₅ alkynyl and C₃₋₅ cycloalkyl each of which may be optionally substituted with one or more, identical or different, substituents R⁷;
- R⁶ is independently selected from the group consisting of C₃₋₅ cycloalkyl, -O-C₁₋₅ alkyl, -O-C₃₋₅ cycloalkyl, -S-C₁₋₅ alkyl and -S-C₃₋₅ cycloalkyl each of which may optionally substituted with one or more, identical or different, substituents R⁸, deuterium, F and -CN;
- R⁷ is independently selected from the group consisting of C₃₋₅ cycloalkyl, -O-C₁₋₅ alkyl, -O-C₃₋₅ cycloalkyl, -S-C₁₋₅ alkyl and -S-C₃₋₅ cycloalkyl each of which may optionally substituted with one or more, identical or different, substituents R⁸, deuterium, F and -CN;
- R⁸ is independently selected from the group consisting of deuterium, OH, OMe and F;
- R⁹ is independently selected from the group consisting of deuterium, methoxy, nitro, cyano, CF₃, Cl, Br, I and F;
- R¹¹ is selected from the group consisting of C₁₋₅ alkyl optionally substituted with one or more, identical or different, substituents R⁸, C₃₋₅ cycloalkyl optionally substituted with one or more, identical or different, substituents R⁸ and phenyl optionally substituted with one or more, identical or different, substituents R⁹;
- R¹² is selected from the group consisting of -OH, -OC₁₋₅ alkyl optionally substituted with one or more, identical or different, substituents R⁸ and -OC₃₋₅ cycloalkyl optionally substituted with one or more, identical or different, substituents R⁸;
- R¹⁵ is selected from the group consisting of H, C₁₋₅ alkyl optionally substituted with one or more, identical or different, substituents R¹⁶, C₃₋₆ cycloalkyl optionally substituted with one or more, identical or different, substituents R¹⁶, phenyl optionally substituted with one or more, identical or different, substituents R⁹, and 4-6 membered heterocycle optionally substituted with one or more, identical or different, substituents R¹⁷ with the proviso that when R¹⁵ is H then R²¹ is not H;
- R¹⁶ is independently selected from the group consisting of F, C!, Br, I, -CN, =O, C₃₋₆ cycloalkyl optionally substituted with one or more, identical or different, substituents R⁸, -O-C₁₋₅ alkyl optionally substituted with one or more, identical or different, substituents R⁸, -O-C₃₋₅ cycloalkyl optionally substituted with one or more, identical or different, substituents R⁸, -S-C₁₋₅ alkyl optionally substituted with one or more, identical or different, substituents R⁸, -S-C₃₋₅ cycloalkyl optionally substituted with one or more, identical or different, substituents R⁸, -SO-C₁₋₅ alkyl optionally substituted with one or more, identical or different, substituents R⁸, -SO-C₃₋₅ cycloalkyl optionally substituted with one or more, identical or different, substituents R⁸, -SO₂-C₁₋₅ alkyl optionally substituted with one or more, identical or different, substituents R⁸, - SO₂-C₃₋₅ cycloalkyl optionally substituted with one or more, identical or different, substituents R⁸, -C(=O)-C₁₋₅ alkyl optionally substituted with one or more, identical or different substituents R⁸, -C(=O)-OC₁₋₅ alkyl optionally substituted with one or more, identical or different, substituents R⁸, -NH-C(=O)-OC₁₋₅ alkyl optionally substituted with one or more, identical or different, substituents R⁸, -C(=O)-C₃₋₅ cycloalkyl optionally substituted with one or more, identical or different, substituents R⁸, -NH-C(=O)-C₁₋₅ alkyl optionally substituted with one or more, identical or different, substituents R⁸, -NH-C(=O)-C₃₋₅ cycloalkyl optionally substituted with one or more, identical or different, substituents R⁸, -C(=O)-NH-C₁₋₅ alkyl optionally substituted with one or more, identical or different, substituents R⁸, -C(=O)-NH-C₃₋₅ cycloalkyl optionally substituted with one or more, identical or different, substituents R⁸, phenyl optionally substituted with one or more, identical or different, substituents R⁹, pyrrolidin-1-yl optionally substituted with one or more, identical or different, substituents R¹⁷ and 4-6 membered heterocycle optionally substituted with one or more, identical or different, substituents R¹⁷;
- R¹⁷ is independently selected from the group consisting of F, C!, Br, I, -CN, =O, C₁₋₅ alkyl optionally substituted with one or more, identical or different, substituents R⁸, C₃₋₆ cycloalkyl optionally substituted with one or more, identical or different, substituents R⁸, -C(=O)-C₁₋₅ alkyl optionally substituted with one or more, identical or different, substituents R⁸, -C(=O)-OC₁₋₅ alkyl optionally substituted with one or more, identical or different, substituents R⁸, -C(=O)-C₃₋₅ cycloalkyl optionally substituted with one or more, identical or different, substituents R⁸, -NH-C(=O)-C₁₋₅ alkyl optionally substituted with one or more, identical or different, substituents R⁸, -NH-C(=O)-C₃₋₅ cycloalkyl optionally substituted with one or more, identical or different, substituents R⁸, -C(=O)-NH-C₁₋₅ alkyl optionally substituted with one or more, identical or different, substituents R⁸ and -C(=O)-NH-C₃₋₅ cycloalkyl optionally substituted with one or more, identical or different, substituents R⁸;
- R¹⁹ is selected from the group consisting of C₁₋₅ alkyl optionally substituted with one or more, identical or different, substituents R⁸, C₃₋₅ cycloalkyl optionally substituted with one or more, identical or different, substituents R⁸ and phenyl optionally substituted with one or more, identical or different, substituents R⁹;
- R²¹ is selected from the group consisting of H, C₁₋₅ alkyl optionally substituted with one or more, identical or different, substituents R⁸, C₃₋₅ cycloalkyl optionally substituted with one or more, identical or different, substituents R⁸, -C(=O)-C₁₋₅ alkyl optionally substituted with one or more, identical or different, substituents R⁸ and -C(=O)-C₃₋₅ cycloalkyl optionally substituted with one or more, identical or different, substituents R⁸;
- R²² is selected from the group consisting of -C(R¹¹)=NR¹², -CN, -OR¹⁵ and SO₂R¹⁹; and
- n is an integer 0, 1, 2 or 3;
or a pharmaceutically acceptable salt, hydrate, polymorph, tautomer, or solvate thereof.

In one embodiment, the compound is of Formula (VIII) 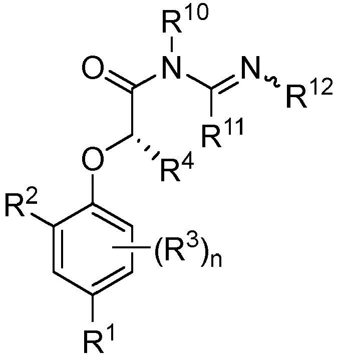 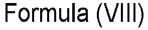 wherein:
- R¹ is selected from the group consisting of F, CI, Br and I;
- R² is selected from the group consisting of H, F, Cl, Br, !, C₁₋₅ alkyl optionally substituted with one or more, identical or different, substituents R⁶, C₂₋₅ alkenyl optionally substituted with one or more, identical or different, substituents R⁶, C₂₋₅ alkynyl optionally substituted with one or more, identical or different, substituents R⁶, C₃₋₅ cycloalkyl optionally substituted with one or more, identical or different, substituents R⁶, phenyl optionally substituted with one or more, identical or different, substituents R⁹, and 5-6 membered aromatic heterocycle optionally substituted with one or more, identical or different, substituents R⁷;
- R³ is selected from the group consisting of deuterium and F;
- R⁴ is selected from the group consisting of C₁₋₅ alkyl, C₂₋₅ alkenyl, C₂₋₅ alkynyl and C₃₋₅ cycloalkyl each of which may be optionally substituted with one or more, identical or different, substituents R⁷;
- R⁶ is independently selected from the group consisting of C₃₋₅ cycloalkyl, -O-C₁₋₅ alkyl, -O-C₃₋₅ cycloalkyl, -S-C₁₋₅ alkyl and -S-C₃₋₅ cycloalkyl each of which may optionally substituted with one or more, identical or different, substituents R⁸, deuterium, F and -CN;
- R⁷ is independently selected from the group consisting of C₃₋₅ cycloalkyl, -O-C₁₋₅ alkyl, -O-C₃₋₅ cycloalkyl, -S-C₁₋₅ alkyl and -S-C₃₋₅ cycloalkyl each of which may optionally substituted with one or more, identical or different, substituents R⁸, deuterium, F and -CN;
- R⁸ is independently selected from the group consisting of deuterium, OH, OMe and F;
- R⁹ is independently selected from the group consisting of deuterium, methoxy, nitro, cyano, CF₃, Cl, Br, I and F;
- R¹⁰ is selected from the group consisting of H, C₁₋₅ alkyl optionally substituted with one or more, identical or different, substituents R⁸, C₃₋₅ cycloalkyl optionally substituted with one or more, identical or different, substituents R⁸, -C(=O)-C₁₋₅ alkyl optionally substituted with one or more, identical or different, substituents R⁸ and -C(=O)-C₃₋₅ cycloalkyl optionally substituted with one or more, identical or different, substituents R⁸;
- R¹¹ is selected from the group consisting of C₁₋₅ alkyl optionally substituted with one or more, identical or different, substituents R⁸, C₃₋₅ cycloalkyl optionally substituted with one or more, identical or different, substituents R⁸ and phenyl optionally substituted with one or more, identical or different, substituents R⁹;
- R¹² is selected from the group consisting of -OH, -OC₁₋₅ alkyl optionally substituted with one or more, identical or different, substituents R⁸ and -OC₃₋₅ cycloalkyl optionally substituted with one or more, identical or different, substituents R⁸; and
- n is an integer 0, 1, 2 or 3;
or a pharmaceutically acceptable salt, hydrate, polymorph, tautomer, or solvate thereof.

In one embodiment, the compound is of Formula (IX) 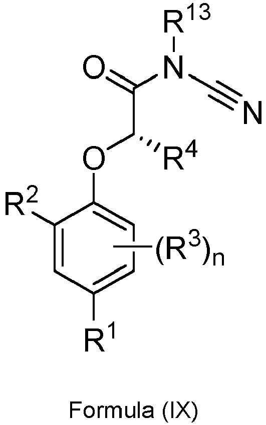 wherein:
- R¹ is selected from the group consisting of F, CI, Br and I;
- R² is selected from the group consisting of H, F, Cl, Br, !, C₁₋₅ alkyl optionally substituted with one or more, identical or different, substituents R⁶, C₂₋₅ alkenyl optionally substituted with one or more, identical or different, substituents R⁶, C₂₋₅ alkynyl optionally substituted with one or more, identical or different, substituents R⁶, C₃₋₅ cycloalkyl optionally substituted with one or more, identical or different, substituents R⁶, phenyl optionally substituted with one or more, identical or different, substituents R⁹, and 5-6 membered aromatic heterocycle optionally substituted with one or more, identical or different, substituents R⁷;
- R³ is selected from the group consisting of deuterium and F;
- R⁴ is selected from the group consisting of C₁₋₅ alkyl, C₂₋₅ alkenyl, C₂₋₅ alkynyl and C₃₋₅ cycloalkyl each of which may be optionally substituted with one or more, identical or different, substituents R⁷;
- R⁶ is independently selected from the group consisting of C₃₋₅ cycloalkyl, -O-C₁₋₅ alkyl, -O-C₃₋₅ cycloalkyl, -S-C₁₋₅ alkyl and -S-C₃₋₅ cycloalkyl each of which may optionally substituted with one or more, identical or different, substituents R⁸, deuterium, F and -CN;
- R⁷ is independently selected from the group consisting of C₃₋₅ cycloalkyl, -O-C₁₋₅ alkyl, -O-C₃₋₅ cycloalkyl, -S-C₁₋₅ alkyl and -S-C₃₋₅ cycloalkyl each of which may optionally substituted with one or more, identical or different, substituents R⁸, deuterium, F and -CN;
- R⁸ is independently selected from the group consisting of deuterium, OH, OMe and F;
- R⁹ is independently selected from the group consisting of deuterium, methoxy, nitro, cyano, CF₃, Cl, Br, I and F;
- R¹³ is selected from the group consisting of H, C₁₋₅ alkyl optionally substituted with one or more, identical or different, substituents R⁸, C₃₋₅ cycloalkyl optionally substituted with one or more, identical or different, substituents R⁸, -C(=O)-C₁₋₅ alkyl optionally substituted with one or more, identical or different, substituents R⁸ and -C(=O)-C₃₋₅ cycloalkyl optionally substituted with one or more, identical or different, substituents R⁸; and
- n is an integer 0, 1, 2 or 3;
or a pharmaceutically acceptable salt, hydrate, polymorph, tautomer, or solvate thereof.

In one embodiment, the compound is of Formula (X) 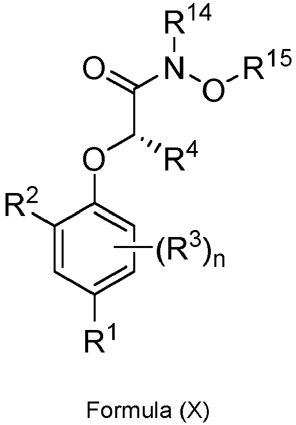 wherein:
- R¹ is selected from the group consisting of F, CI, Br and I;
- R² is selected from the group consisting of H, F, Cl, Br, !, C₁₋₅ alkyl optionally substituted with one or more, identical or different, substituents R⁶, C₂₋₅ alkenyl optionally substituted with one or more, identical or different, substituents R⁶, C₂₋₅ alkynyl optionally substituted with one or more, identical or different, substituents R⁶, C₃₋₅ cycloalkyl optionally substituted with one or more, identical or different, substituents R⁶, phenyl optionally substituted with one or more, identical or different, substituents R⁹, and 5-6 membered aromatic heterocycle optionally substituted with one or more, identical or different, substituents R⁷;
- R³ is selected from the group consisting of deuterium and F;
- R⁴ is selected from the group consisting of C₁₋₅ alkyl, C₂₋₅ alkenyl, C₂₋₅ alkynyl and C₃₋₅ cycloalkyl each of which may be optionally substituted with one or more, identical or different, substituents R⁷;
- R⁶ is independently selected from the group consisting of C₃₋₅ cycloalkyl, -O-C₁₋₅ alkyl, -O-C₃₋₅ cycloalkyl, -S-C₁₋₅ alkyl and -S-C₃₋₅ cycloalkyl each of which may optionally substituted with one or more, identical or different, substituents R⁸, deuterium, F and -CN;
- R⁷ is independently selected from the group consisting of C₃₋₅ cycloalkyl, -O-C₁₋₅ alkyl, -O-C₃₋₅ cycloalkyl, -S-C₁₋₅ alkyl and -S-C₃₋₅ cycloalkyl each of which may optionally substituted with one or more, identical or different, substituents R⁸, deuterium, F and -CN;
- R⁸ is independently selected from the group consisting of deuterium, OH, OMe and F;
- R⁹ is independently selected from the group consisting of deuterium, methoxy, nitro, cyano, CF₃, Cl, Br, I and F;
- R¹⁴ is selected from the group consisting of H, C₁₋₅ alkyl optionally substituted with one or more, identical or different, substituents R¹⁷, C₃₋₅ cycloalkyl optionally substituted with one or more, identical or different, substituents R⁸, -C(=O)-C₁₋₅ alkyl optionally substituted with one or more, identical or different, substituents R⁸ and -C(=O)-C₃₋₅ cycloalkyl optionally substituted with one or more, identical or different, substituents R⁸;
- R¹⁵ is selected from the group consisting of H, C₁₋₅ alkyl optionally substituted with one or more, identical or different, substituents R¹⁶, C₃₋₆ cycloalkyl optionally substituted with one or more, identical or different, substituents R¹⁶, phenyl optionally substituted with one or more, identical or different, substituents R⁹, and 4-6 membered heterocycle optionally substituted with one or more, identical or different, substituents R¹⁷ with the proviso that when R¹⁵ is H then R¹⁴ is not H;
- R¹⁶ is independently selected from the group consisting of F, C!, Br, I, -CN, =O, C₃₋₆ cycloalkyl optionally substituted with one or more, identical or different, substituents R⁸, -O-C₁₋₅ alkyl optionally substituted with one or more, identical or different, substituents R⁸, -O-C₃₋₅ cycloalkyl optionally substituted with one or more, identical or different, substituents R⁸, -S-C₁₋₅ alkyl optionally substituted with one or more, identical or different, substituents R⁸, -S-C₃₋₅ cycloalkyl optionally substituted with one or more, identical or different, substituents R⁸, -SO-C₁₋₅ alkyl optionally substituted with one or more, identical or different, substituents R⁸, -SO-C₃₋₅ cycloalkyl optionally substituted with one or more, identical or different, substituents R⁸, -SO₂-C₁₋₅ alkyl optionally substituted with one or more, identical or different, substituents R⁸, - SO₂-C₃₋₅ cycloalkyl optionally substituted with one or more, identical or different, substituents R⁸, -C(=O)-C₁₋₅ alkyl optionally substituted with one or more, identical or different substituents R⁸, -C(=O)-OC₁₋₅ alkyl optionally substituted with one or more, identical or different, substituents R⁸, -NH-C(=O)-OC₁₋₅ alkyl optionally substituted with one or more, identical or different, substituents R⁸, -C(=O)-C₃₋₅ cycloalkyl optionally substituted with one or more, identical or different, substituents R⁸, -NH-C(=O)-C₁₋₅ alkyl optionally substituted with one or more, identical or different, substituents R⁸, -NH-C(=O)-C₃₋₅ cycloalkyl optionally substituted with one or more, identical or different, substituents R⁸, -C(=O)-NH-C₁₋₅ alkyl optionally substituted with one or more, identical or different, substituents R⁸, -C(=O)-NH-C₃₋₅ cycloalkyl optionally substituted with one or more, identical or different, substituents R⁸, phenyl optionally substituted with one or more, identical or different, substituents R⁹, pyrrolidin-1-yl optionally substituted with one or more, identical or different, substituents R¹⁷ and 4-6 membered heterocycle optionally substituted with one or more, identical or different, substituents R¹⁷;
- R¹⁷ is independently selected from the group consisting of F, C!, Br, I, -CN, =O, C₁₋₅ alkyl optionally substituted with one or more, identical or different, substituents R⁸, C₃₋₆ cycloalkyl optionally substituted with one or more, identical or different, substituents R⁸, -C(=O)-C₁₋₅ alkyl optionally substituted with one or more, identical or different, substituents R⁸, -C(=O)-OC₁₋₅ alkyl optionally substituted with one or more, identical or different, substituents R⁸, -C(=O)-C₃₋₅ cycloalkyl optionally substituted with one or more, identical or different, substituents R⁸, -NH-C(=O)-C₁₋₅ alkyl optionally substituted with one or more, identical or different, substituents R⁸, -NH-C(=O)-C₃₋₅ cycloalkyl optionally substituted with one or more, identical or different, substituents R⁸, -C(=O)-NH-C₁₋₅ alkyl optionally substituted with one or more, identical or different, substituents R⁸ and -C(=O)-NH-C₃₋₅ cycloalkyl optionally substituted with one or more, identical or different, substituents R⁸; and
- n is an integer 0, 1, 2 or 3;
or a pharmaceutically acceptable salt, hydrate, polymorph, tautomer, or solvate thereof.

In one embodiment, the compound is of Formula (XI) 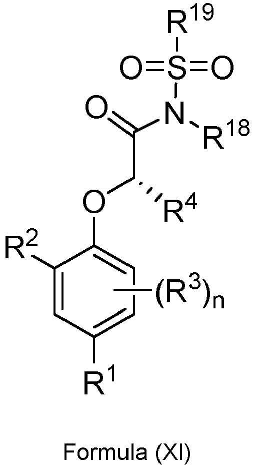 wherein:
- R¹ is selected from the group consisting of F, CI, Br and I;
- R² is selected from the group consisting of H, F, Cl, Br, !, C₁₋₅ alkyl optionally substituted with one or more, identical or different, substituents R⁶, C₂₋₅ alkenyl optionally substituted with one or more, identical or different, substituents R⁶, C₂₋₅ alkynyl optionally substituted with one or more, identical or different, substituents R⁶, C₃₋₅ cycloalkyl optionally substituted with one or more, identical or different, substituents R⁶, phenyl optionally substituted with one or more, identical or different, substituents R⁹, and 5-6 membered aromatic heterocycle optionally substituted with one or more, identical or different, substituents R⁷;
- R³ is selected from the group consisting of deuterium and F;
- R⁴ is selected from the group consisting of C₁₋₅ alkyl, C₂₋₅ alkenyl, C₂₋₅ alkynyl and C₃₋₅ cycloalkyl each of which may be optionally substituted with one or more, identical or different, substituents R⁷;
- R⁶ is independently selected from the group consisting of C₃₋₅ cycloalkyl, -O-C₁₋₅ alkyl, -O-C₃₋₅ cycloalkyl, -S-C₁₋₅ alkyl and -S-C₃₋₅ cycloalkyl each of which may optionally substituted with one or more, identical or different, substituents R⁸, deuterium, F and -CN;
- R⁷ is independently selected from the group consisting of C₃₋₅ cycloalkyl, -O-C₁₋₅ alkyl, -O-C₃₋₅ cycloalkyl, -S-C₁₋₅ alkyl and -S-C₃₋₅ cycloalkyl each of which may optionally substituted with one or more, identical or different, substituents R⁸, deuterium, F and -CN;
- R⁸ is independently selected from the group consisting of deuterium, OH, OMe and F;
- R⁹ is independently selected from the group consisting of deuterium, methoxy, nitro, cyano, CF₃, Cl, Br, I and F;
- R¹⁸ is selected from the group consisting of H, C₁₋₅ alkyl optionally substituted with one or more, identical or different, substituents R⁸, C₃₋₅ cycloalkyl optionally substituted with one or more, identical or different, substituents R⁸, -C(=O)-C₁₋₅ alkyl optionally substituted with one or more, identical or different, substituents R⁸ and -C(=O)-C₃₋₅ cycloalkyl optionally substituted with one or more, identical or different, substituents R⁸;
- R¹⁹ is selected from the group consisting of C₁₋₅ alkyl optionally substituted with one or more, identical or different, substituents R⁸, C₃₋₅ cycloalkyl optionally substituted with one or more, identical or different, substituents R⁸ and phenyl optionally substituted with one or more, identical or different, substituents R⁹;
- n is an integer 0, 1, 2 or 3;
or a pharmaceutically acceptable salt, hydrate, polymorph, tautomer, or solvate thereof.

In one embodiment, R¹ is Cl or Br. In one embodiment, R¹ is Cl.

In one embodiment, R¹ is Br.

In one embodiment, R² is H, F, Cl, Br or I. In one embodiment, R² is H. In one embodiment, R² is F, CI, Br or I. In one embodiment, R² is C₁₋₅ alkyl optionally substituted with one or more, identical or different, substituents R⁶. In one embodiment, R² is selected from the group consisting of methyl, ethyl, n-propyl and isopropyl. In one embodiment, R² is selected from the group consisting of methyl, ethyl, n-propyl and isopropyl wherein the methyl, ethyl, n-propyl or isopropyl group is substituted with one or more, identical or different, substituents R⁶. In one embodiment, R² is -CF₂-C₁₋₄ alkyl optionally substituted with one or more, identical or different, substituents R⁶. In one embodiment, R² is selected from the group consisting of HCF₂-, MeCF₂-, EtCF₂-, ⁱPrCF₂-, ⁿPrCF₂-, ⁿBuCF₂-, ⁱBuCF₂-, ^{t}BuCF₂-, cyclopropylCF₂-. and cyclobutylCF₂-. In one embodiment, R² is MeCF₂- or EtCF₂-. In one embodiment, R² is C₂₋₅ alkenyl optionally substituted with one or more, identical or different, substituents R⁶. In one embodiment, R² is C₂₋₅ alkynyl optionally substituted with one or more, identical or different, substituents R⁶. In one embodiment, R² is C₃₋₅ cycloalkyl optionally substituted with one or more, identical or different, substituents R⁶. In one embodiment, R² is phenyl optionally substituted with one or more, identical or different, substituents R⁹. In one embodiment, R² is a 5-6 membered aromatic heterocycle optionally substituted with one or more, identical or different, substituents R⁷. In one embodiment, R² is 1,2-oxazol-3-yl or 1,2-oxazol-5-yl.

In one embodiment, R³ is deuterium. In one embodiment, R³ is F.

In one embodiment, R⁴ is C₁₋₅ alkyl optionally substituted with one or more, identical or different, substituents R⁷. In one embodiment, R⁴ is selected from the group consisting of methyl, ethyl, n-propyl and isopropyl. In one embodiment, R⁴ is selected from the group consisting of methyl, ethyl, n-propyl and isopropyl wherein the methyl, ethyl, n-propyl or isopropyl group is substituted with one or more, identical or different, substituents R⁷. In one embodiment, R⁴ is -CH₂- and R⁷ is -O-C₁₋₅ alkyl optionally substituted with one or more, identical or different, substituents R⁷. In one embodiment, R⁴ is -CH₂- and R⁷ is -S-C₁₋₅ alkyl optionally substituted with one or more, identical or different, substituents R⁷. In one embodiment, R⁴ is -CH₂- and R⁷ is -O-C₁₋₅ alkyl optionally substituted with one or more, identical or different, substituents R⁸, deuterium, F or -CN. In one embodiment, R⁴ is -CH₂- and R⁷ is -S-C₁₋₅ alkyl optionally substituted with one or more, identical or different, substituents R⁸, deuterium, F or - CN.

In one embodiment, R⁴ is selected from the group consisting of C₁₋₅ alkyl, C₂₋₅ alkenyl, C₂₋₅ alkynyl and C₃₋₅ cycloalkyl each of which may be optionally substituted with one or more, identical or different, substituents R⁷. In one embodiment, R⁴ is C₂₋₅ alkenyl optionally substituted with one or more, identical or different, substituents R⁷. In one embodiment, R⁴ is C₂₋₅ alkynyl optionally substituted with one or more, identical or different, substituents R⁷. In one embodiment, R⁴ is -CH₂-C₂₋₄ alkynyl optionally substituted with one or more, identical or different, substituents R⁷. In one embodiment, R⁴ is C₃₋₅ cycloalkyl optionally substituted with one or more, identical or different, substituents R⁷.

In one embodiment, R⁶ is deuterium. In one embodiment, R⁶ is F. In one embodiment, R⁶ is CN. In one embodiment, R⁶ is C₃₋₅ cycloalkyl optionally substituted with one or more, identical or different, substituents R⁸. In one embodiment, R⁶ is -O-C₁₋₅ alkyl optionally substituted with one or more, identical or different, substituents R⁸. In one embodiment, R⁶ is -O-C₃₋₅ cycloalkyl optionally substituted with one or more, identical or different, substituents R⁸. In one embodiment, R⁶ is -S-C₁₋₅ alkyl optionally substituted with one or more, identical or different, substituents R⁸. In one embodiment, R⁶ is -S-C₃₋₅ cycloalkyl optionally substituted with one or more, identical or different, substituents R⁸.

In one embodiment, R⁷ is deuterium. In one embodiment, R⁷ is F. In one embodiment, R⁷ is CN. In one embodiment, R⁷ is C₃₋₅ cycloalkyl optionally substituted with one or more, identical or different, substituents R⁸. In one embodiment, R⁷ is -O-C₁₋₅ alkyl optionally substituted with one or more, identical or different, substituents R⁸. In one embodiment, R⁷ is -O-C₃₋₅ cycloalkyl optionally substituted with one or more, identical or different, substituents R⁸. In one embodiment, R⁷ is -S-C₁₋₅ alkyl optionally substituted with one or more, identical or different, substituents R⁸. In one embodiment, R⁷ is -S-C₃₋₅ cycloalkyl optionally substituted with one or more, identical or different, substituents R⁸.

In one embodiment, R⁸ is deuterium. In one embodiment, R⁸ is F. In one embodiment, R⁸ is OH. In one embodiment, R⁸ is OMe.

In one embodiment, R⁹ is deuterium. In one embodiment, R⁹ is methoxy. In one embodiment, R⁹ is nitro. In one embodiment, R⁹ is cyano. In one embodiment, R⁹ is CF₃. In one embodiment, R⁹ is CI. In one embodiment, R⁹ is Br. In one embodiment, R⁹ is I. In one embodiment, R⁹ is F.

In one embodiment, R¹⁰ is H. In one embodiment, R¹⁰ is C₁₋₅ alkyl optionally substituted with one or more, identical or different, substituents R⁸. In one embodiment, R¹⁰ is C₃₋₅ cycloalkyl optionally substituted with one or more, identical or different, substituents R⁸. In one embodiment, R¹⁰ is -C(=O)-C₁₋₅ alkyl optionally substituted with one or more, identical or different, substituents R⁸. In one embodiment, R¹⁰ is -C(=O)-C₃₋₅ cycloalkyl optionally substituted with one or more, identical or different, substituents R⁸.

In one embodiment, R¹¹ is C₁₋₅ alkyl optionally substituted with one or more, identical or different, substituents R⁸. In one embodiment, R¹¹ is C₃₋₅ cycloalkyl optionally substituted with one or more, identical or different, substituents R⁸. In one embodiment, R¹¹ is phenyl optionally substituted with one or more, identical or different, substituents R⁹.

In one embodiment, R¹² is -OH. In one embodiment, R¹² is -OC₁₋₅ alkyl optionally substituted with one or more, identical or different, substituents R⁸. In one embodiment, R¹² is -OC₃₋₅ cycloalkyl optionally substituted with one or more, identical or different, substituents R⁸.

In one embodiment, R¹³ is H. In one embodiment, R¹³ is C₁₋₅ alkyl optionally substituted with one or more, identical or different, substituents R⁸. In one embodiment, R¹³ is C₃₋₅ cycloalkyl optionally substituted with one or more, identical or different, substituents R⁸. In one embodiment, R¹³ is -C(=O)-C₁₋₅ alkyl optionally substituted with one or more, identical or different, substituents R⁸. In one embodiment, R¹³ is -C(=O)-C₃₋₅ cycloalkyl optionally substituted with one or more, identical or different, substituents R⁸.

In one embodiment, R¹⁴ is H. In one embodiment, R¹⁴ is C₁₋₅ alkyl optionally substituted with one or more, identical or different, substituents R¹⁷. In one embodiment, R¹⁴ is C₃₋₅ cycloalkyl optionally substituted with one or more, identical or different, substituents R⁸. In one embodiment, R¹⁴ is -C(=O)-C₁₋₅ alkyl optionally substituted with one or more, identical or different, substituents R⁸. In one embodiment, R¹⁴ is -C(=O)-C₃₋₅ cycloalkyl optionally substituted with one or more, identical or different, substituents R⁸.

In one embodiment, R¹⁵ is H with the proviso that when R¹⁵ is H then R¹⁴ is not H. In one embodiment, R¹⁵ is C₁₋₅ alkyl optionally substituted with one or more, identical or different, substituents R¹⁶. In one embodiment, R¹⁵ is C₃₋₆ cycloalkyl optionally substituted with one or more, identical or different, substituents R¹⁶. In one embodiment, R¹⁵ is phenyl optionally substituted with one or more, identical or different, substituents R⁹. In one embodiment, R¹⁵ is 4-6 membered heterocycle optionally substituted with one or more, identical or different, substituents R¹⁷.

In one embodiment, R¹⁶ is F, CI, Br or I. In one embodiment, R¹⁶ is -CN. In one embodiment, R¹⁶ is =O. In one embodiment, R¹⁶ is C₃₋₆ cycloalkyl optionally substituted with one or more, identical or different, substituents R⁸. In one embodiment, R¹⁶ is -O-C₁₋₅ alkyl optionally substituted with one or more, identical or different, substituents R⁸. In one embodiment, R¹⁶ is -O-C₃₋₅ cycloalkyl optionally substituted with one or more, identical or different, substituents R⁸. In one embodiment, R¹⁶ is -S-C₁₋₅ alkyl optionally substituted with one or more, identical or different, substituents R⁸. In one embodiment, R¹⁶ is -S-C₃₋₅ cycloalkyl optionally substituted with one or more, identical or different, substituents R⁸. In one embodiment, R¹⁶ is -SO-C₁₋₅ alkyl optionally substituted with one or more, identical or different, substituents R⁸. In one embodiment, R¹⁶ is -SO-C₃₋₅ cycloalkyl optionally substituted with one or more, identical or different, substituents R⁸. In one embodiment, R¹⁶ is -SO₂-C₁₋₅ alkyl optionally substituted with one or more, identical or different, substituents R⁸. In one embodiment, R¹⁶ is - SO₂-C₃₋₅ cycloalkyl optionally substituted with one or more, identical or different, substituents R⁸. In one embodiment, R¹⁶ is -C(=O)-C₁₋₅ alkyl optionally substituted with one or more, identical or different substituents R⁸. In one embodiment, R¹⁶ is -C(=O)-OC₁₋₅ alkyl optionally substituted with one or more, identical or different, substituents R⁸. In one embodiment, R¹⁶ is -NH-C(=O)-OC₁₋₅ alkyl optionally substituted with one or more, identical or different, substituents R⁸. In one embodiment, R¹⁶ is -C(=O)-C₃₋₅ cycloalkyl optionally substituted with one or more, identical or different, substituents R⁸. In one embodiment, R¹⁶ is -NH-C(=O)-C₁₋₅ alkyl optionally substituted with one or more, identical or different, substituents R⁸. In one embodiment, R¹⁶ is -NH-C(=O)-C₃₋₅ cycloalkyl optionally substituted with one or more, identical or different, substituents R⁸. In one embodiment, R¹⁶ is -C(=O)-NH-C₁₋₅ alkyl optionally substituted with one or more, identical or different, substituents R⁸. In one embodiment, R¹⁶ is -C(=O)-NH-C₃₋₅ cycloalkyl optionally substituted with one or more, identical or different, substituents R⁸. In one embodiment, R¹⁶ is phenyl optionally substituted with one or more, identical or different, substituents R⁹. In one embodiment, R¹⁶ is pyrrolidin-1-yl optionally substituted with one or more, identical or different, substituents R¹⁷. In one embodiment, R¹⁶ is 4-6 membered heterocycle optionally substituted with one or more, identical or different, substituents R¹⁷.

In one embodiment, R¹⁷ is F, CI, Br or I. In one embodiment, R¹⁷ is -CN. In one embodiment, R¹⁷ is =O. In one embodiment, R¹⁷ is C₁₋₅ alkyl optionally substituted with one or more, identical or different, substituents R⁸. In one embodiment, R¹⁷ is C₃₋₆ cycloalkyl optionally substituted with one or more, identical or different, substituents R⁸. In one embodiment, R¹⁷ is -C(=O)-C₁₋₅ alkyl optionally substituted with one or more, identical or different, substituents R⁸. In one embodiment, R¹⁷ is -C(=O)-OC₁₋₅ alkyl optionally substituted with one or more, identical or different, substituents R⁸. In one embodiment, R¹⁷ is -C(=O)-C₃₋₅ cycloalkyl optionally substituted with one or more, identical or different, substituents R⁸. In one embodiment, R¹⁷ is -NH-C(=O)-C₁₋₅ alkyl optionally substituted with one or more, identical or different, substituents R⁸. In one embodiment, R¹⁷ is -NH-C(=O)-C₃₋₅ cycloalkyl optionally substituted with one or more, identical or different, substituents R⁸. In one embodiment, R¹⁷ is -C(=O)-NH-C₁₋₅ alkyl optionally substituted with one or more, identical or different, substituents R⁸. In one embodiment, R¹⁷ is -C(=O)-NH-C₃₋₅ cycloalkyl optionally substituted with one or more, identical or different, substituents R⁸.

In one embodiment, R¹⁸ is H. In one embodiment, R¹⁸ is C₁₋₅ alkyl optionally substituted with one or more, identical or different, substituents R⁸. In one embodiment, R¹⁸ is C₃₋₅ cycloalkyl optionally substituted with one or more, identical or different, substituents R⁸. In one embodiment, R¹⁸ is -C(=O)-C₁₋₅ alkyl optionally substituted with one or more, identical or different, substituents R⁸. In one embodiment, R¹⁸ is -C(=O)-C₃₋₅ cycloalkyl optionally substituted with one or more, identical or different, substituents R⁸.

In one embodiment, R¹⁹ C₁₋₅ alkyl optionally substituted with one or more, identical or different, substituents R⁸. In one embodiment, R¹⁹ is C₃₋₅ cycloalkyl optionally substituted with one or more, identical or different, substituents R⁸. In one embodiment, R¹⁹ is phenyl optionally substituted with one or more, identical or different, substituents R⁹.

In one embodiment, X is N. In one embodiment, X is CR²⁰ wherein R²⁰ is H. In one embodiment, X is CR²⁰ wherein R²⁰ is NH₂. In one embodiment, X is CR²⁰ wherein R²⁰ is C₁₋₅ alkyl optionally substituted with one or more, identical or different, substituents R⁸. In one embodiment, X is CR²⁰ wherein R²⁰ is C₃₋₅ cycloalkyl optionally substituted with one or more, identical or different, substituents R⁸. In one embodiment, X is CR²⁰ wherein R²⁰ is -OC₁₋₅ alkyl optionally substituted with one or more, identical or different, substituents R⁸. In one embodiment, X is CR²⁰ wherein R²⁰ is -OC₃₋₅ cycloalkyl optionally substituted with one or more, identical or different, substituents R⁸. In one embodiment, X is CR²⁰ wherein R²⁰ is -NH-C(=O)C₁₋₅ alkyl optionally substituted with one or more, identical or different, substituents R⁸. In one embodiment, X is CR²⁰ wherein R²⁰ is -NH-SO₂-C₁₋₅ alkyl optionally substituted with one or more, identical or different, substituents R⁸.

In one embodiment, n is 0. In one embodiment, n is 1. In one embodiment, n is 2. In one embodiment, n is 3.

In one embodiment, R²¹ is H with the proviso that when R¹⁵ is H then R²¹ is not H. In one embodiment, R²¹ is C₁₋₅ alkyl optionally substituted with one or more, identical or different, substituents R⁸. In one embodiment, R²¹ is C₃₋₅ cycloalkyl optionally substituted with one or more, identical or different, substituents R⁸. In one embodiment, R²¹ is -C(=O)-C₁₋₅ alkyl optionally substituted with one or more, identical or different, substituents R⁸. In one embodiment, R²¹ is -C(=O)-C₃₋₅ cycloalkyl optionally substituted with one or more, identical or different, substituents R⁸.

In one embodiment, the compound is selected from the list consisting of
(2*S*)-2-(4-bromo-2-fluorophenoxy)-*N*-{1-[(cyclopropylmethoxy)imino]ethyl}propanamide;
(2*S*)-2-(4-bromophenoxy)-*N*-[1-(methoxyimino)ethyl]propanamide;
(2*S*)-2-(4-bromo-2-fluorophenoxy)-*N*-[(4-fluorophenyl)(hydroxyimino)methyl]-3-methylbutanamide;
(2*S*)-2-(4-bromo-2-fluorophenoxy)-*N*-[1-(hydroxyimino)ethyl]-3-methylbutanamide;
(2*S*)-2-(4-bromo-2-fluorophenoxy)-*N*-[1-(hydroxyimino)ethyl]propanamide;
(2*S*)-2-(4-bromophenoxy)-*N*-[1-(hydroxyimino)ethyl]propenamide;
(2*S*)-2-[4-bromo-2-(1,2-oxazol-3-yl)phenoxy]-*N*-cyanopropanamide;
(2*S*)-*N*-cyano-2-(2,4-dibromophenoxy)propanamide;
(2*S*)-2-(4-bromophenoxy)-*N*-cyanopropanamide;
(2*S*)-2-(4-chlorophenoxy)-*N*-cyanopropanamide;
(2*S*)-2-(4-bromophenoxy)-*N*-cyano-3-methylbutanamide;
(2*S*)-2-[4-bromo-2-(1,2-oxazol-3-yl)phenoxy]*-N*-cyclobutoxypropanamide;
(2*S*)-2-[4-bromo-2-(1,2-oxazol-3-yl)phenoxy]-*N*-methoxypropanamide;
(2*S*)-*N*-acetyl-*N*-[(1-acetylazetidin-3-yl)oxy]-2-(4-chlorophenoxy)propanamide;
(2*S*)-*N*-[(1-acetylazetidin-3-yl)oxy]-2-(4-chlorophenoxy)propanamide;
(2*S*)-*N*-(azetidin-3-yloxy)-2-(4-chlorophenoxy)propanamide;
(2*S*)-*N*-acetyl*-N*-[(1-acetylpyrrolidin-3-yl)methoxy]-2-(4-chlorophenoxy)propanamide;
(2*S*)-*N*-[(1-acetylpyrrolidin-3-yl)methoxy]-2-(4-chlorophenoxy)propanamide;
(2*S*)-*N*-[(1-acetylpyrrolidin-3-yl)oxy]-2-(4-bromophenoxy)propanamide;
tert-butyl *N*-(2-{[(2*S*)-2-(4-chlorophenoxy)propanamido]oxy}propyl)carbamate;
(2*S*)-N*-*acetyl-2-(4-chlorophenoxy)-*N*-[(1-acetamidopropan-2-yl)oxy]propanamide;
(2*S*)-2-(4-bromophenoxy)-*N*-(pyrrolidin-3-yloxy)propanamide;
(2*S*)-*N*-acetyl-*N*-[(1-acetylpyrrolidin-3-yl)oxy]-2-(4-bromophenoxy)propanamide;
tert-butyl 3-{[(2*S*)-2-(4-chlorophenoxy)propanamido]oxylazetidine-1-carboxylate;
(2*S*)-2-(4-chlorophenoxy)-*N*-[(pyrrolidin-3-yl)methoxy]propanamide;
tert-butyl 3-({[(2*S*)-2-(4-chlorophenoxy)propanamido]oxy}methyl)pyrrolidine-1-carboxylate;
(2*S*)-*N*-acetyl-*N*-[(1-acetylpyrrolidin-3-yl)oxy]-2-(4-chlorophenoxy)propanamide;
(2*S*)-*N*-[(1-acetylpyrrolidin-3-yl)oxy]-2-(4-chlorophenoxy)propanamide;
(2*S*)-2-(4-chlorophenoxy)-*N*-(pyrrolidin-3-yloxy)propanamide;
(2*S*)-2-(4-chlorophenoxy)-*N*-(2,2-dimethylpropyl)-*N*-hydroxypropanamide;
(2*S*)-2-(4-chlorophenoxy)-*N*-[2-(pyrrolidin-1-yl)ethoxy]propanamide;
(2*S*)-2-(4-chlorophenoxy)-*N*-[2-(2-oxopyrrolidin-1-yl)ethoxy]propanamide;
(2*S*)-*N*-acetyl-2-(4-chlorophenoxy)-*N*-(2-acetamidoethoxy)propanamide;
(2*S*)-2-(4-chlorophenoxy)-*N*-(2-acetamidoethoxy)propanamide;
(2*S*)-2-(4-chlorophenoxy)-*N*-(4,4,4-trifluoro-2-methylbutoxy)propanamide;
(2*S*)-2-(4-chlorophenoxy)-*N*-(3-cyclopentylpropyl)-*N*-hydroxypropanamide;
(2*S*)-2-(4-chlorophenoxy)-*N*-{[(2E)-2-methyl-3-phenylprop-2-en-1-yl]oxy}propanamide;
(2*S*)-2-(4-chlorophenoxy)-*N*-{2-oxo-2-[2-(trifluoromethyl)pyrrolidi*n*-1-yl]ethoxy}propanamide;
tert-butyl *N*-(2-{[(2S)-2-(4-chlorophenoxy)propanamidyl]oxy}ethyl)carbamate;
(2*S*)-2-(4-chlorophenoxy)-*N*-[(2-methyl-1H-imidazol-4-yl)methoxy]propanamide;
tert-butyl 4-({[(2*S*)-2-(4-chlorophenoxy)propanamido]oxy}methyl)-2-methyl-1*H-*imidazole-1-carboxylate;
(2*S*)-2-(4-bromo-2-fluorophenoxy)-*N*-cyclobutoxypropanamide;
(2*S*)-2-(4-bromophenoxy)-*N*-cyclobutoxypropanamide;
(2*S*)-2-(4-chlorophenoxy)-*N*-[2-oxo-2-(pyrrolidin-1-yl)ethoxy]propanamide;
(2*S*)-2-(4-chlorophenoxy)-*N*-[1-(4-fluorophenyl)ethoxy]propanamide;
(2*S*)-2-(4-chlorophenoxy)-*N*-[1-(1,3-thiazol-2-yl)ethoxy]propanamide;
(2*S*)-2-(4-chlorophenoxy)-*N*-{1-[4-(trifluoromethyl)phenyl]ethoxy}propanamide;
(2*S*)-2-(4-chlorophenoxy)-*N*-(2-methanesulfinylethoxy)propanamide;
(2*S*)-2-(4-chlorophenoxy)-*N*-(2-methanesulfonylethoxy)propanamide;
(2*S*)-2-(4-chlorophenoxy)-*N*-[(1,2-oxazol-3-yl)methoxy]propanamide;
(2*S*)-2-(4-chloro-2-fluorophenoxy)-*N*-methoxypropanamide;
(2*S*)-2-[4-bromo-2-(1,3,4-oxadiazol-2-yl)phenoxy]-*N-*(cyclopropylmethoxy)propanamide;
(2*S*)-2-[4-chloro-2-(trifluoromethyl)phenoxy]-*N-*(cyclopropylmethoxy)propanamide;
(2*S*)-*N*-(tert-butoxy)-2-(4-chlorophenoxy)propanamide;
(2*S*)-2-(4-chlorophenoxy)-*N*-[2-(methylsulfanyl)ethoxy]propanamide;
(2*S*)-2-(4-chlorophenoxy)-*N*-(1-phenylethoxy)propanamide;
(2*S*)-2-(4-chlorophenoxy)-*N*-[(1-methyl-1H-imidazol-2-yl)methoxy]propanamide;
(2*S*)-2-(4-chlorophenoxy)-*N*-(2-methoxyethoxy)propanamide;
(2*S*)-2-(4-chloro-2-fluorophenoxy)-*N*-cyclobutoxypropanamide;
(2*S*)-2-[4-bromo-2-(1,3,4-oxadiazol-2-yl)phenoxy]-*N*-methoxypropanamide;
(2*S*)-2-[4-chloro-2-(trifluoromethyl)phenoxy]-*N*-methoxypropanamide;
(2*S*)-*N*-(benzyloxy)-2-(4-chlorophenoxy)propanamide;
(2*S*)-2-(4-chlorophenoxy)-*N*-[(2-methoxycyclopentyl)oxy]propanamide;
(2*S*)-2-(4-chlorophenoxy)-*N*-cyclobutoxy-5-methylhexanamide;
(2*S*)-2-(4-chlorophenoxy)-*N*-cyclobutoxy-*N*-methylpropanamide;
(2*S*)-2-(4-chloro-2-methylphenoxy)-*N*-cyclobutoxypropanamide;
(2*S*)-2-(4-chloro-3-fluorophenoxy)-*N*-cyclobutoxypropanamide;
(2*S*)-2-(4-chloro-2-methylphenoxy)-*N*-methoxypropanamide;
(2*S*)-2-(4-chloro-3-fluorophenoxy)-*N*-methoxypropanamide;
(2*S*)-2-(4-chlorophenoxy)-*N*-cyclobutoxy-4-methylpentanamide;
(2*S*)-2-(4-chlorophenoxy)-*N*-[(3-methylbut-2-en-1-yl)oxy]propanamide;
(2*S*)-2-(4-chlorophenoxy)-*N*-cyclobutoxyhexanamide;
(2*S*)-2-(4-chlorophenoxy)-*N*-[(1,3-oxazol-2-yl)methoxy]propanamide;
(2*S*)-2-(2,4-dibromophenoxy)-*N*-methoxypropanamide;
(2*S*)-2-(4-bromo-2-fluorophenoxy)-*N*-methoxypropanamide;
(2*S*)-2-(4-chlorophenoxy)-*N*-(oxan-2-yloxy)propanam ide;
(2*S*)-2-(4-chlorophenoxy)-*N*-[(1,3-thiazol-2-yl)methoxy]propanamide;
(2*S*)-2-(4-chlorophenoxy)-*N*-(3,3-difluorocyclobutoxy)propanamide;
(2*S*)-2-[4-bromo-2-(1,2-oxazol-5-yl)phenoxy]-*N*-cyclobutoxypropanamide;
(2*S*)-2-[4-bromo-2-(1,2-oxazol-5-yl)phenoxy]-*N*-methoxypropanamide;
(2*S*)-2-(4-chlorophenoxy)-*N*-(cyclopentyloxy)propanamide;
(2*S*)-2-(4-chlorophenoxy)-*N*-(2-cyclopentylethoxy)propanamide;
(2*S*)-2-(4-bromo-2-chlorophenoxy)-*N*-methoxypropanamide;
(2*S*)-2-(4-bromo-2-methylphenoxy)-*N*-methoxypropanamide;
(2*S*)-2-(4-chlorophenoxy)-*N*-(cyclopropylmethoxy)propanamide;
(2*S*)-2-(4-chlorophenoxy)-*N*-(cyclobutylmethoxy)propanamide;
(2*S*)-*N*-(2-aminoethoxy)-2-(4-chlorophenoxy)propanamide;
(2*S*)-2-(4-bromophenoxy)-*N*-methoxy-3-methylbutanamide;
methyl 2-{[(2*S*)-2-(4-chlorophenoxy)propanamido]oxy}acetate;
(2*S*)-2-(4-chlorophenoxy)-*N*-[2-(2-methoxyethoxy)ethoxy]propanamide;
(2*S*)-2-(4-chlorophenoxy)-*N*-cyclobutoxypropanamide;
(2*S*)-2-(4-chlorophenoxy)-*N*-(2-hydroxyethoxy)propanamide;
(2*S*)-2-(4-chlorophenoxy)-*N*-ethoxypropanamide;
(2*S*)-2-(4-chlorophenoxy)-*N*-propoxypropanamide;
(2*S*)-2-(4-chlorophenoxy)-*N*-(propan-2-yloxy)propanamide;
(2*S*)-2-(4-chlorophenoxy)-*N*-methoxypropanamide;
(2*S*)-2-[4-bromo-2-(1,2-oxazol-3-yl)phenoxy]-*N-*(cyclopropanesulfonyl)propenamide;
(2*S*)-2-(4-bromo-2-fluorophenoxy)-*N*-methanesulfonylpropanamide;
(2*S*)-2-(4-bromo-2-fluorophenoxy)-*N*-(cyclopropanesulfonyl)propenamide;
(2*S*)-2-(4-chlorophenoxy)-*N*-methanesulfonylpropanamide;
(2*S*)-2-(4-bromophenoxy)-*N*-methanesulfonyl-3-methylbutanamide;
5-[(1*S*)-1-[4-bromo-2-(1,2-oxazol-3-yl)phenoxy]-3-fluoropropyl]-2H-1,2,3,4-tetrazole;
5-[(1*S*)-1-(4-bromo-2-fluorophenoxy)propyl]-2H-1,2,3,4-tetrazole;
5-[(1*S*)-1-(4-bromo-2-fluorophenoxy)-2-methylpropyl]-2H-1,2,3,4-tetrazole;
5-[(1*R*)-1-(4-bromophenoxy)-2-fluoroethyl]-2H-1,2,3,4-tetrazole;
5-[(1*S*)-1-(4-bromophenoxy)propyl]-2H-1,2,3,4-tetrazole;
5-[(1*S*)-1-(4-bromophenoxy)ethyl]-2H-1,2,3,4-tetrazole;
5-[(1*S*)-1-[4-bromo-2-(1,2-oxazol-3-yl)phenoxy]ethyl]-1H-1,2,3,4-tetrazole;
5-[(1*S*)-1-(4-bromo-2-fluorophenoxy)ethyl]-2H-1,2,3,4-tetrazole;
5-[(1*S*)-1-[4-bromo-2-(1,2-oxazol-5-yl)phenoxy]ethyl]-2H-1,2,3,4-tetrazole;
5-[(1*S*)-1-[4-chloro-2-(1,2-oxazol-3-yl)phenoxy]ethyl]-2H-1,2,3,4-tetrazole;
5-[(1*S*)-1-(4-chloro-2-cyclopropylphenoxy)ethyl]-1H-1,2,3,4-tetrazole;
5-[(1*S*)-1-(4-chloro-2-ethenylphenoxy)ethyl]-1H-1,2,3,4-tetrazole;
5-[(1*S*)-1-(4-chloro-2-ethylphenoxy)ethyl]-1H-1,2,3,4-tetrazole;
5-[(1*S*)-1-(4-chloro-2-methylphenoxy)ethyl]-2H-1,2,3,4-tetrazole;
5-[(1*S*)-1-(4-chlorophenoxy)ethyl]-1H-1,2,3,4-tetrazole;
*N*-{3-[(1*S*)-1-(4-bromo-2-fluorophenoxy)ethyl]-1,2,4-thiadiazol-5-yl}acetamide;
*N*-{3-[(1*S*)-1-(4-bromo-2-fluorophenoxy)ethyl]-1,2,4-thiadiazol-5-yl}methanesulfonamide;
3-[(1*S*)-1-(4-bromo-2-fluorophenoxy)ethyl]-1,2,4-thiadiazol-5-amine;
*N*-{3-[(1*S*)-1-(4-bromo-2-fluorophenoxy)ethyl]-1,2,4-oxadiazol-5-yl}acetamide;
*N*-{3-[(1*S*)-1-(4-bromo-2-fluorophenoxy)ethyl]-1,2,4-oxadiazol-5-yl}methanesulfonamide;
3-[(1*S*)-1-[4-bromo-2-(1,2-oxazol-3-yl)phenoxy]ethyl]-5-(trifluoromethyl)-4H-1,2,4-triazole;
3-[(1*S*)-1-[4-chloro-2-(1,2-oxazol-3-yl)phenoxy]ethyl]-5-(trifluoromethyl)-4H-1,2,4-triazole;
3-[(1*S*)-1-(4-bromo-2-fluorophenoxy)ethyl]-5-methoxy-1,2,4-oxadiazole;
3-[(1*S*)-1-(4-bromo-2-fluorophenoxy)ethyl]-1,2,4-oxadiazol-5-amine;
3-[(1*S*)-1-[4-bromo-2-(1,2-oxazol-3-yl)phenoxy]ethyl]-5-methyl-4H-1,2,4-triazole;
3-[(1*S*)-1-[4-chloro-2-(1,2-oxazol-3-yl)phenoxy]ethyl]-5-methyl-4H-1,2,4-triazole;
3-[(1*S*)-1-(4-bromo-2-fluorophenoxy)ethyl]-5-methyl-4H-1,2,4-triazole;
5-[(1*S*)-1-(4-chlorophenoxy)ethyl]-1H-1,2,4-triazole;
(2*S*)-2-[4-bromo-2-(1,1-difluoroethyl)phenoxy]-*N*-cyanopropanamide;
(2*S*)-2-[4-bromo-2-(1,1-difluoropropyl)-5-fluorophenoxy]-*N*-cyanopropanamide;
(2*S*)-2-[4-bromo-2-(1,1-difluoropropyl)phenoxy]-*N-*methanesulfonylpropanamide;
(2*S*)-2-[4-bromo-2-(1,1-difluoropropyl)phenoxy]-*N-*(cyclopropanesulfonyl)propenamide;
5-[(1*S*)-1-[4-bromo-2-(1,1-difluoroethyl)phenoxy]ethyl]-1H-1,2,3,4-tetrazole;
5-[(1*S*)-1-[4-bromo-2-(1,1-difluoropropyl)phenoxy]ethyl]-1H-1,2,3,4-tetrazole;
5-[(1*S*)-1-(4-bromo-2-cyclobutylphenoxy)-2-methoxyethyl]-1H-1,2,3,4-tetrazole; and
5-[(1*S*)-1-(4-bromo-2-cyclopropylphenoxy)-2-methoxyethyl]-1H-1,2,3,4-tetrazole.

In one embodiment, the compound is of Formula (VIII) wherein R¹ is Br; R² is selected from the group consisting of H and F; R⁴ is C₁₋₅ alkyl optionally substituted with one or more, identical or different, substituents R⁷; R¹⁰ is H; and n, R³, R⁷, R⁸, R⁹, R¹¹ and R¹² are as defined herein.

In one embodiment, the compound is selected from the list consisting of:
(2*S*)-2-(4-bromo-2-fluorophenoxy)-*N*-{1-[(cyclopropylmethoxy)imino]ethyl}propanamide;
(2*S*)-2-(4-bromophenoxy)-*N*-[1-(methoxyimino)ethyl]propanamide;
(2*S*)-2-(4-bromo-2-fluorophenoxy)-*N*-[(4-fluorophenyl)(hydroxyimino)methyl]-3-methylbutanamide;
(2*S*)-2-(4-bromo-2-fluorophenoxy)-*N*-[1-(hydroxyimino)ethyl]-3-methylbutanamide;
(2*S*)-2-(4-bromo-2-fluorophenoxy)-*N*-[1-(hydroxyimino)ethyl]propanamide; and
(2*S*)-2-(4-bromophenoxy)-*N*-[1-(hydroxyimino)ethyl]propenamide.

In one embodiment, the compound is of Formula (IX) wherein R¹ is Br or CI; R² is selected from the group consisting of H, Br and 1,2-oxazol-3-yl; R⁴ is C₁₋₅ alkyl optionally substituted with one or more, identical or different, substituents R⁷; R¹³ is H; and n, R³, R⁷ and R⁸ are as defined herein.

In one embodiment, the compound is selected from the list consisting of:
(2*S*)-2-[4-bromo-2-(1,2-oxazol-3-yl)phenoxy]-*N*-cyanopropanamide;
(2*S*)-*N*-cyano-2-(2,4-dibromophenoxy)propanamide;
(2*S*)-2-(4-bromophenoxy)-*N*-cyanopropanamide;
(2*S*)-2-(4-chlorophenoxy)-*N*-cyanopropanamide;
(2*S*)-2-(4-bromophenoxy)-*N*-cyano-3-methylbutanamide;
(2*S*)-2-[4-bromo-2-(1,1-difluoroethyl)phenoxy]-*N*-cyanopropanamide; and
(2*S*)-2-[4-bromo-2-(1,1-difluoropropyl)-5-fluorophenoxy]-*N*-cyanopropanamide.

In one embodiment, the compound is of Formula (X) wherein R¹ is Br or CI; R² is selected from the group consisting of H, F, Br, C₁₋₅ alkyl optionally substituted with one or more, identical or different, substituents R⁶, 1,3,4-oxadiazol-2-yl and 1,2-oxazol-3-yl; R⁴ is C₁₋₅ alkyl optionally substituted with one or more, identical or different, substituents R⁷; R¹⁴ is selected from the group consisting of H, C₁₋₅ alkyl optionally substituted with one or more, identical or different, substituents R¹⁷ and -C(=O)-C₁₋₅ alkyl optionally substituted with one or more, identical or different, substituents R⁸; and n, R³, R⁶, R⁷, R⁸, R⁹, R¹⁵, R¹⁶, R¹⁷ are as defined herein.

In one embodiment, the compound is selected from the list consisting of:
(2*S*)-2-[4-bromo-2-(1,2-oxazol-3-yl)phenoxy]-*N*-cyclobutoxypropanamide;
(2*S*)-2-[4-bromo-2-(1,2-oxazol-3-yl)phenoxy]-*N*-methoxypropanamide;
(2*S*)-*N*-acetyl-*N*-[(1-acetylazetidin-3-yl)oxy]-2-(4-chlorophenoxy)propanamide;
(2*S*)-*N*-[(1-acetylazetidin-3-yl)oxy]-2-(4-chlorophenoxy)propanamide;
(2*S*)-*N*-(azetidin-3-yloxy)-2-(4-chlorophenoxy)propanamide;
(2*S*)-*N*-acetyl-*N*-[(1-acetylpyrrolidin-3-yl)methoxy]-2-(4-chlorophenoxy)propanamide;
(2*S*)-*N*-[(1-acetylpyrrolidin-3-yl)methoxy]-2-(4-chlorophenoxy)propanamide;
(2*S*)-*N*-[(1-acetylpyrrolidin-3-yl)oxy]-2-(4-bromophenoxy)propanamide;
tert-butyl *N*-(2-{[(2S)-2-(4-chlorophenoxy)propanamido]oxy}propyl)carbamate;
(2*S*)-*N*-acetyl-2-(4-chlorophenoxy)-*N*-[(1-acetamidopropan-2-yl)oxy]propanamide;
(2*S*)-2-(4-bromophenoxy)-*N*-(pyrrolidin-3-yloxy)propanamide;
(2*S*)-*N*-acetyl-*N*-[(1-acetylpyrrolidi*n*-3-yl)oxy]-2-(4-bromophenoxy)propanamide;
tert-butyl 3-{[(2*S*)-2-(4-chlorophenoxy)propanamido]oxy}azetidine-1-carboxylate;
(2*S*)-2-(4-chlorophenoxy)-*N*-[(pyrrolidin-3-yl)methoxy]propanamide;
tert-butyl 3-({[(2*S*)-2-(4-chlorophenoxy)propanamido]oxy}methyl)pyrrolidine-1-carboxylate;
(2*S*)-*N*-acetyl-*N*-[(1-acetylpyrrolidi*n*-3-yl)oxy]-2-(4-chlorophenoxy)propanamide;
(2*S*)-*N*-[(1-acetylpyrrolidin-3-yl)oxy]-2-(4-chlorophenoxy)propanamide;
(2*S*)-2-(4-chlorophenoxy)-*N*-(pyrrolidi*n*-3-yloxy)propanamide;
(2*S*)-2-(4-chlorophenoxy)-*N*-(2,2-dimethylpropyl)-*N*-hydroxypropanamide;
(2*S*)-2-(4-chlorophenoxy)-*N*-[2-(pyrrolidi*n*-1-yl)ethoxy]propanamide;
(2*S*)-2-(4-chlorophenoxy)-*N*-[2-(2-oxopyrrolidi*n*-1-yl)ethoxy]propanamide;
(2S)-*N*-acetyl-2-(4-chlorophenoxy)-*N*-(2-acetamidoethoxy)propanamide;
(2*S*)-2-(4-chlorophenoxy)-*N*-(2-acetamidoethoxy)propanamide;
(2*S*)-2-(4-chlorophenoxy)-*N*-(4,4,4-trifluoro-2-methylbutoxy)propanamide;
(2*S*)-2-(4-chlorophenoxy)-*N*-(3-cyclopentylpropyl)-*N*-hydroxypropanamide;
(2*S*)-2-(4-chlorophenoxy)-*N*-{[(2E)-2-methyl-3-phenylprop-2-en-1-yl]oxy}propanamide;
(2*S*)-2-(4-chlorophenoxy)-*N*-{2-oxo-2-[2-(trifluoromethyl)pyrrolidin-1-yl]ethoxy}propanamide;
tert-butyl *N*-(2-{[(2S)-2-(4-chlorophenoxy)propanamidyl]oxy}ethyl)carbamate;
(2*S*)-2-(4-chlorophenoxy)-*N*-[(2-methyl-1H-imidazol-4-yl)methoxy]propanamide;
tert-butyl 4-({[(2*S*)-2-(4-chlorophenoxy)propanamido]oxy}methyl)-2-methyl-1H-imidazole-1-carboxylate;
(2*S*)-2-(4-bromo-2-fluorophenoxy)-*N*-cyclobutoxypropanamide;
(2*S*)-2-(4-bromophenoxy)-*N*-cyclobutoxypropanamide;
(2*S*)-2-(4-chlorophenoxy)-*N*-[2-oxo-2-(pyrrolidin-1-yl)ethoxy]propanamide;
(2*S*)-2-(4-chlorophenoxy)-*N*-[1-(4-fluorophenyl)ethoxy]propanamide;
(2*S*)-2-(4-chlorophenoxy)-*N*-[1-(1,3-thiazol-2-yl)ethoxy]propanamide;
(2*S*)-2-(4-chlorophenoxy)-*N*-{1-[4-(trifluoromethyl)phenyl]ethoxy}propanamide;
(2*S*)-2-(4-chlorophenoxy)-*N*-(2-methanesulfinylethoxy)propanamide;
(2*S*)-2-(4-chlorophenoxy)-*N*-(2-methanesulfonylethoxy)propanamide;
(2*S*)-2-(4-chlorophenoxy)-*N*-[(1,2-oxazol-3-yl)methoxy]propanamide;
(2*S*)-2-(4-chloro-2-fluorophenoxy)-*N*-methoxypropanamide;
(2*S*)-2-[4-bromo-2-(1,3,4-oxadiazol-2-yl)phenoxy]-*N-*(cyclopropylmethoxy)propanamide;
(2*S*)-2-[4-chloro-2-(trifluoromethyl)phenoxy]-*N-*(cyclopropylmethoxy)propanamide;
(2*S*)-*N*-(tert-butoxy)-2-(4-chlorophenoxy)propanamide;
(2*S*)-2-(4-chlorophenoxy)-*N*-[2-(methylsulfanyl)ethoxy]propanamide;
(2*S*)-2-(4-chlorophenoxy)-*N*-(1-phenylethoxy)propanamide;
(2*S*)-2-(4-chlorophenoxy)-*N*-[(1-methyl-1H-imidazol-2-yl)methoxy]propanamide;
(2*S*)-2-(4-chlorophenoxy)-*N*-(2-methoxyethoxy)propanamide;
(2*S*)-2-(4-chloro-2-fluorophenoxy)-*N*-cyclobutoxypropanamide;
(2*S*)-2-[4-bromo-2-(1,3,4-oxadiazol-2-yl)phenoxy]-*N*-methoxypropanamide;
(2*S*)-2-[4-chloro-2-(trifluoromethyl)phenoxy]-*N*-methoxypropanamide;
(2*S*)-*N*-(benzyloxy)-2-(4-chlorophenoxy)propanamide;
(2*S*)-2-(4-chlorophenoxy)-*N*-[(2-methoxycyclopentyl)oxy]propanamide;
(2*S*)-2-(4-chlorophenoxy)-*N*-cyclobutoxy-5-methylhexanamide;
(2*S*)-2-(4-chlorophenoxy)-*N*-cyclobutoxy-*N*-methylpropanamide;
(2*S*)-2-(4-chloro-2-methylphenoxy)-*N*-cyclobutoxypropanamide;
(2*S*)-2-(4-chloro-3-fluorophenoxy)-*N*-cyclobutoxypropanamide;
(2*S*)-2-(4-chloro-2-methylphenoxy)-*N*-methoxypropanamide;
(2*S*)-2-(4-chloro-3-fluorophenoxy)-*N*-methoxypropanamide;
(2*S*)-2-(4-chlorophenoxy)-*N*-cyclobutoxy-4-methylpentanamide;
(2*S*)-2-(4-chlorophenoxy)-*N*-[(3-methylbut-2-en-1-yl)oxy]propanamide;
(2*S*)-2-(4-chlorophenoxy)-*N*-cyclobutoxyhexanamide;
(2*S*)-2-(4-chlorophenoxy)-*N*-[(1,3-oxazol-2-yl)methoxy]propanamide;
(2*S*)-2-(2,4-dibromophenoxy)-*N*-methoxypropanamide;
(2*S*)-2-(4-bromo-2-fluorophenoxy)-*N*-methoxypropanamide;
(2*S*)-2-(4-chlorophenoxy)-*N*-(oxan-2-yloxy)propanam ide;
(2*S*)-2-(4-chlorophenoxy)-*N*-[(1,3-thiazol-2-yl)methoxy]propanamide;
(2*S*)-2-(4-chlorophenoxy)-*N*-(3,3-difluorocyclobutoxy)propanamide;
(2*S*)-2-[4-bromo-2-(1,2-oxazol-5-yl)phenoxy]-*N*-cyclobutoxypropanamide;
(2*S*)-2-[4-bromo-2-(1,2-oxazol-5-yl)phenoxy]-*N*-methoxypropanamide;
(2*S*)-2-(4-chlorophenoxy)-*N*-(cyclopentyloxy)propanamide;
(2*S*)-2-(4-chlorophenoxy)-*N*-(2-cyclopentylethoxy)propanamide;
(2*S*)-2-(4-bromo-2-chlorophenoxy)-*N*-methoxypropanamide;
(2*S*)-2-(4-bromo-2-methylphenoxy)-*N*-methoxypropanamide;
(2*S*)-2-(4-chlorophenoxy)-*N-*(cyclopropylmethoxy)propanamide;
(2*S*)-2-(4-chlorophenoxy)-*N*-(cyclobutylmethoxy)propanamide;
(2*S*)-*N*-(2-aminoethoxy)-2-(4-chlorophenoxy)propanamide;
(2*S*)-2-(4-bromophenoxy)-*N*-methoxy-3-methylbutanamide;
methyl 2-{[(2*S*)-2-(4-chlorophenoxy)propanamido]oxy}acetate;
(2*S*)-2-(4-chlorophenoxy)-*N*-[2-(2-methoxyethoxy)ethoxy]propanamide;
(2*S*)-2-(4-chlorophenoxy)-*N*-cyclobutoxypropanamide;
(2*S*)-2-(4-chlorophenoxy)-*N*-(2-hydroxyethoxy)propanam ide;
(2*S*)-2-(4-chlorophenoxy)-*N*-ethoxypropanamide;
(2*S*)-2-(4-chlorophenoxy)-*N*-propoxypropanamide;
(2*S*)-2-(4-chlorophenoxy)-*N*-(propan-2-yloxy)propanamide; and
(2*S*)-2-(4-chlorophenoxy)-*N*-methoxypropanamide.

In one embodiment, the compound is of Formula (XI) wherein R¹ is Br or CI; R² is selected from the group consisting of H, F and 1,2-oxazol-3-yl; R⁴ is C₁₋₅ alkyl optionally substituted with one or more, identical or different, substituents R⁷; R¹⁸ is H; R¹⁹ is selected from the group consisting of C₁₋₅ alkyl optionally substituted with one or more, identical or different, substituents R⁸ and C₃₋₅ cycloalkyl optionally substituted with one or more, identical or different, substituents R⁸; and n, R³, R⁶, R⁷, R⁸ and R⁹ are as defined herein.

In one embodiment, the compound is selected from the list consisting of:
(2*S*)-2-[4-bromo-2-(1,2-oxazol-3-yl)phenoxy]-*N-*(cyclopropanesulfonyl)propenamide;
(2*S*)-2-(4-bromo-2-fluorophenoxy)-*N*-methanesulfonylpropanamide;
(2*S*)-2-(4-bromo-2-fluorophenoxy)-*N*-(cyclopropanesulfonyl)propenamide;
(2*S*)-2-(4-chlorophenoxy)-*N*-methanesulfonylpropanamide;
(2*S*)-2-(4-bromophenoxy)-*N*-methanesulfonyl-3-methylbutanamide;
(2*S*)-2-[4-bromo-2-(1,1-difluoropropyl)phenoxy]-*N-*methanesulfonylpropanamide; and
(2*S*)-2-[4-bromo-2-(1,1-difluoropropyl)phenoxy]-*N-*(cyclopropanesulfonyl)propenamide.

In one embodiment, the compound is of Formula (IVa) or Formula (IVb) wherein R¹ is Br or CI; R² is selected from the group consisting of H, F, C₁₋₅ alkyl optionally substituted with one or more, identical or different, substituents R⁶, C₂₋₅ alkenyl optionally substituted with one or more, identical or different, substituents R⁶, C₃₋₅ cycloalkyl optionally substituted with one or more, identical or different, substituents R⁶, and 5-6 membered aromatic heterocycle optionally substituted with one or more, identical or different, substituents R⁷; R⁴ is C₁₋₅ alkyl optionally substituted with one or more, identical or different, substituents R⁷; and n, R³, R⁶, R⁷, R⁸ and R⁹ are as defined herein.

In one embodiment, the compound is selected from the list consisting of:
5-[(1*S*)-1-[4-bromo-2-(1,2-oxazol-3-yl)phenoxy]-3-fluoropropyl]-2H-1,2,3,4-tetrazole;
5-[(1*S*)-1-(4-bromo-2-fluorophenoxy)propyl]-2H-1,2,3,4-tetrazole;
5-[(1*S*)-1-(4-bromo-2-fluorophenoxy)-2-methylpropyl]-2H-1,2,3,4-tetrazole;
5-[(1*R*)-1-(4-bromophenoxy)-2-fluoroethyl]-2H-1,2,3,4-tetrazole;
5-[(1*S*)-1-(4-bromophenoxy)propyl]-2H-1,2,3,4-tetrazole;
5-[(1*S*)-1-(4-bromophenoxy)ethyl]-2H-1,2,3,4-tetrazole;
5-[(1*S*)-1-[4-bromo-2-(1,2-oxazol-3-yl)phenoxy]ethyl]-1H-1,2,3,4-tetrazole;
5-[(1*S*)-1-(4-bromo-2-fluorophenoxy)ethyl]-2H-1,2,3,4-tetrazole;
5-[(1*S*)-1-[4-bromo-2-(1,2-oxazol-5-yl)phenoxy]ethyl]-2H-1,2,3,4-tetrazole;
5-[(1*S*)-1-[4-chloro-2-(1,2-oxazol-3-yl)phenoxy]ethyl]-2H-1,2,3,4-tetrazole;
5-[(1*S*)-1-(4-chloro-2-cyclopropylphenoxy)ethyl]-1H-1,2,3,4-tetrazole;
5-[(1*S*)-1-(4-chloro-2-ethenylphenoxy)ethyl]-1H-1,2,3,4-tetrazole;
5-[(1*S*)-1-(4-chloro-2-ethylphenoxy)ethyl]-1H-1,2,3,4-tetrazole;
5-[(1*S*)-1-(4-chloro-2-methylphenoxy)ethyl]-2H-1,2,3,4-tetrazole;
5-[(1*S*)-1-(4-chlorophenoxy)ethyl]-1H-1,2,3,4-tetrazole;
5-[(1*S*)-1-[4-bromo-2-(1,1-difluoroethyl)phenoxy]ethyl]-1H-1,2,3,4-tetrazole;
5-[(1*S*)-1-[4-bromo-2-(1,1-difluoropropyl)phenoxy]ethyl]-1H-1,2,3,4-tetrazole;
5-[(1*S*)-1-(4-bromo-2-cyclobutylphenoxy)-2-methoxyethyl]-1H-1,2,3,4-tetrazole; and
5-[(1*S*)-1-(4-bromo-2-cyclopropylphenoxy)-2-methoxyethyl]-1H-1,2,3,4-tetrazole.

In one embodiment, the compound is of Formula (V), Formula (VI) or Formula (VII) wherein R' is Br or CI; R² is selected from the group consisting of H, F and 1,2-oxazol-3-yl; R⁴ is C₁₋₅ alkyl optionally substituted with one or more, identical or different, substituents R⁷; and n, R³, R⁶, R⁷, R⁸, R⁹ and R²³ are as defined herein.

In one embodiment, the compound is selected from the list consisting of:
*N*-{3-[(1*S*)-1-(4-bromo-2-fluorophenoxy)ethyl]-1,2,4-thiadiazol-5-yl}acetamide;
*N*-{3-[(1*S*)-1-(4-bromo-2-fluorophenoxy)ethyl]-1,2,4-thiadiazol-5-yl}methanesulfonamide;
3-[(1*S*)-1-(4-bromo-2-fluorophenoxy)ethyl]-1,2,4-thiadiazol-5-amine;
*N*-{3-[(1*S*)-1-(4-bromo-2-fluorophenoxy)ethyl]-1,2,4-oxadiazol-5-yl}acetamide;
*N*-{3-[(1*S*)-1-(4-bromo-2-fluorophenoxy)ethyl]-1,2,4-oxadiazol-5-yl}methanesulfonamide;
3-[(1*S*)-1-[4-bromo-2-(1,2-oxazol-3-yl)phenoxy]ethyl]-5-(trifluoromethyl)-4H-1,2,4-triazole;
3-[(1*S*)-1-[4-chloro-2-(1,2-oxazol-3-yl)phenoxy]ethyl]-5-(trifluoromethyl)-4H-1,2,4-triazole;
3-[(1*S*)-1-(4-bromo-2-fluorophenoxy)ethyl]-5-methoxy-1,2,4-oxadiazole;
3-[(1*S*)-1-(4-bromo-2-fluorophenoxy)ethyl]-1,2,4-oxadiazol-5-amine;
3-[(1*S*)-1-[4-bromo-2-(1,2-oxazol-3-yl)phenoxy]ethyl]-5-methyl-4H-1,2,4-triazole;
3-[(1*S*)-1-[4-chloro-2-(1,2-oxazol-3-yl)phenoxy]ethyl]-5-methyl-4H-1,2,4-triazole;
3-[(1*S*)-1-(4-bromo-2-fluorophenoxy)ethyl]-5-methyl-4H-1,2,4-triazole; and
5-[(1*S*)-1-(4-chlorophenoxy)ethyl]-1H-1,2,4-triazole.

### Methods of treatment

In one aspect, the disclosure relates to the use of compounds of Formula (I) as defined herein in treating, ameliorating and/or preventing a neuromuscular disorder. In one aspect, the disclosure relates to the use of compounds of Formula (I) as defined herein in reversing and/or ameliorating a neuromuscular blockade.

In one aspect, the disclosure relates to the use of compounds of Formula (II) as defined herein in treating, ameliorating and/or preventing a neuromuscular disorder. In one aspect, the disclosure relates to the use of compounds of Formula (II) as defined herein in reversing and/or ameliorating a neuromuscular blockade.

In one aspect, the disclosure relates to the use of compounds of Formula (III) as defined herein in treating, ameliorating and/or preventing a neuromuscular disorder. In one aspect, the disclosure relates to the use of compounds of Formula (III) as defined herein in reversing and/or ameliorating a neuromuscular blockade.

In one aspect, the disclosure relates to the use of compounds of Formula (IVa) and/or Formula (IVb) as defined herein in treating, ameliorating and/or preventing a neuromuscular disorder. In one aspect, the disclosure relates to the use of compounds of Formula (IVa) and/or Formula (IVb) as defined herein in reversing and/or ameliorating a neuromuscular blockade.

In one aspect, the disclosure relates to the use of compounds of Formula (V) as defined herein in treating, ameliorating and/or preventing a neuromuscular disorder. In one aspect, the disclosure relates to the use of compounds of Formula (V) as defined herein in reversing and/or ameliorating a neuromuscular blockade.

In one aspect, the disclosure relates to the use of compounds of Formula (VI) as defined herein in treating, ameliorating and/or preventing a neuromuscular disorder. In one aspect, the disclosure relates to the use of compounds of Formula (VI) as defined herein in reversing and/or ameliorating a neuromuscular blockade.

In one aspect, the disclosure relates to the use of compounds of Formula (VII) as defined herein in treating, ameliorating and/or preventing a neuromuscular disorder. In one aspect, the disclosure relates to the use of compounds of Formula (VII) as defined herein in reversing and/or ameliorating a neuromuscular blockade.

In one aspect, the disclosure relates to the use of compounds of Formula (VIII) as defined herein in treating, ameliorating and/or preventing a neuromuscular disorder. In one aspect, the disclosure relates to the use of compounds of Formula (VIII) as defined herein in reversing and/or ameliorating a neuromuscular blockade.

In one aspect, the disclosure relates to the use of compounds of Formula (IX) as defined herein in treating, ameliorating and/or preventing a neuromuscular disorder. In one aspect, the disclosure relates to the use of compounds of Formula (IX) as defined herein in reversing and/or ameliorating a neuromuscular blockade.

In one aspect, the disclosure relates to the use of compounds of Formula (X) as defined herein in treating, ameliorating and/or preventing a neuromuscular disorder. In one aspect, the disclosure relates to the use of compounds of Formula (X) as defined herein in reversing and/or ameliorating a neuromuscular blockade.

In one aspect, the disclosure relates to the use of compounds of Formula (XI) as defined herein in treating, ameliorating and/or preventing a neuromuscular disorder. In one aspect, the disclosure relates to the use of compounds of Formula (XI) as defined herein in reversing and/or ameliorating a neuromuscular blockade.

In one embodiment, the compound for use in treating, ameliorating and/or preventing a neuromuscular disorder, and/or for use in reversing and/or ameliorating a neuromuscular blockade is selected from the list consisting of
(2S)-2-(4-bromo-2-fluorophenoxy)-*N*-{1-[(cyclopropylmethoxy)imino]ethyl}propanamide;
(2*S*)-2-(4-bromophenoxy)-*N*-[1-(methoxyimino)ethyl]propanamide;
(2*S*)-2-(4-bromo-2-fluorophenoxy)-*N*-[(4-fluorophenyl)(hydroxyimino)methyl]-3-methylbutanamide;
(2*S*)-2-(4-bromo-2-fluorophenoxy)-*N*-[1-(hydroxyimino)ethyl]-3-methylbutanamide;
(2*S*)-2-(4-bromo-2-fluorophenoxy)-*N*-[1-(hydroxyimino)ethyl]propanamide;
(2*S*)-2-(4-bromophenoxy)-*N*-[1-(hydroxyimino)ethyl]propenamide;
(2*S*)-2-[4-bromo-2-(1,2-oxazol-3-yl)phenoxy]-*N*-cyanopropanamide;
(2*S*)-*N*-cyano-2-(2,4-dibromophenoxy)propanamide;
(2*S*)-2-(4-bromophenoxy)-*N*-cyanopropanamide;
(2*S*)-2-(4-chlorophenoxy)-*N*-cyanopropanamide;
(2*S*)-2-(4-bromophenoxy)-*N*-cyano-3-methylbutanamide;
(2*S*)-2-[4-bromo-2-(1,2-oxazol-3-yl)phenoxy]-*N*-cyclobutoxypropanamide;
(2*S*)-2-[4-bromo-2-(1,2-oxazol-3-yl)phenoxy]-*N*-methoxypropanamide;
(2*S*)-*N*-acetyl-*N*-[(1-acetylazetidin-3-yl)oxy]-2-(4-chlorophenoxy)propanamide;
(2*S*)-*N*-[(1-acetylazetidin-3-yl)oxy]-2-(4-chlorophenoxy)propanamide;
(2*S*)-*N*-(azetidin-3-yloxy)-2-(4-chlorophenoxy)propanamide;
(2*S*)-*N*-acetyl-*N*-[(1-acetylpyrrolidin-3-yl)methoxy]-2-(4-chlorophenoxy)propanamide;
(2*S*)-*N*-[(1-acetylpyrrolidin-3-yl)methoxy]-2-(4-chlorophenoxy)propanamide;
(2*S*)-*N*-[(1-acetylpyrrolidin-3-yl)oxy]-2-(4-bromophenoxy)propanamide;
tert-butyl *N*-(2-{[(2*S*)-2-(4-chlorophenoxy)propanamido]oxy}propyl)carbamate;
(2S)-*N*-acetyl-2-(4-chlorophenoxy)-*N*-[(1-acetamidopropan-2-yl)oxy]propanamide;
(2*S*)-2-(4-bromophenoxy)-*N*-(pyrrolidin-3-yloxy)propanamide;
(2*S*)-*N*-acetyl-*N*-[(1-acetylpyrrolidin-3-yl)oxy]-2-(4-bromophenoxy)propanamide;
tert-butyl 3-{[(2*S*)-2-(4-chlorophenoxy)propanamido]oxy}azetidine-1-carboxylate;
(2*S*)-2-(4-chlorophenoxy)-*N*-[(pyrrolidin-3-yl)methoxy]propanamide;
tert-butyl 3-({[(2*S*)-2-(4-chlorophenoxy)propanamido]oxy}methyl)pyrrolidine-1-carboxylate;
(2*S*)-*N*-acetyl-*N*-[(1-acetylpyrrolidin-3-yl)oxy]-2-(4-chlorophenoxy)propanamide;
(2*S*)-*N*-[(1-acetylpyrrolidin-3-yl)oxy]-2-(4-chlorophenoxy)propanamide;
(2*S*)-2-(4-chlorophenoxy)-*N*-(pyrrolidin-3-yloxy)propanamide;
(2*S*)-2-(4-chlorophenoxy)-*N*-(2,2-dimethylpropyl)-*N*-hydroxypropanamide;
(2S)-2-(4-chlorophenoxy)-*N*-[2-(pyrrolidin-1-yl)ethoxy]propanamide;
(2*S*)-2-(4-chlorophenoxy)-*N*-[2-(2-oxopyrrolidin-1-yl)ethoxy]propanamide;
(2*S*)-*N*-acetyl-2-(4-chlorophenoxy)-*N*-(2-acetamidoethoxy)propanam ide;
(2*S*)-2-(4-chlorophenoxy)-*N*-(2-acetamidoethoxy)propanamide;
(2*S*)-2-(4-chlorophenoxy)-*N*-(4,4,4-trifluoro-2-methylbutoxy)propanamide;
(2S)-2-(4-chlorophenoxy)-*N*-(3-cyclopentylpropyl)-N-hydroxypropanamide;
(2*S*)-2-(4-chlorophenoxy)-*N*-{[(2*E*)-2-methyl-3-phenylprop-2-en-1-yl]oxy}propanamide;
(2*S*)-2-(4-chlorophenoxy)-*N*-{2-oxo-2-[2-(trifluoromethyl)pyrrolidin-1-yl]ethoxy}propanamide;
tert-butyl *N*-(2-{[(2*S*)-2-(4-chlorophenoxy)propanamidyl]oxy}ethyl)carbamate;
(2*S*)-2-(4-chlorophenoxy)-*N*-[(2-methyl-1*H*-imidazol-4-yl)methoxy]propanamide;
tert-butyl 4-({[(2*S*)-2-(4-chlorophenoxy)propanamido]oxy}methyl)-2-methyl-1H-imidazole-1-carboxylate;
(2*S*)-2-(4-bromo-2-fluorophenoxy)-*N*-cyclobutoxypropanamide;
(2*S*)-2-(4-bromophenoxy)-*N*-cyclobutoxypropanamide;
(2*S*)-2-(4-chlorophenoxy)-*N*-[2-oxo-2-(pyrrolidin-1-yl)ethoxy]propanamide;
(2*S*)-2-(4-chlorophenoxy)-*N*-[1-(4-fluorophenyl)ethoxy]propanamide;
(2*S*)-2-(4-chlorophenoxy)-*N*-[1-(1,3-thiazol-2-yl)ethoxy]propanamide;
(2*S*)-2-(4-chlorophenoxy)-*N*-{1-[4-(trifluoromethyl)phenyl]ethoxy}propanamide;
(2*S*)-2-(4-chlorophenoxy)-*N*-(2-methanesulfinylethoxy)propanamide;
(2*S*)-2-(4-chlorophenoxy)-*N*-(2-methanesulfonylethoxy)propanamide;
(2*S*)-2-(4-chlorophenoxy)-*N*-[(1,2-oxazol-3-yl)methoxy]propanamide;
(2*S*)-2-(4-chloro-2-fluorophenoxy)-*N*-methoxypropanamide;
(2*S*)-2-[4-bromo-2-(1,3,4-oxadiazol-2-yl)phenoxy]-*N-*(cyclopropylmethoxy)propanamide;
(2*S*)-2-[4-chloro-2-(trifluoromethyl)phenoxy]-*N-*(cyclopropylmethoxy)propanamide;
(2*S*)-*N*-(tert-butoxy)-2-(4-chlorophenoxy)propanamide;
(2*S*)-2-(4-chlorophenoxy)-*N*-[2-(methylsulfanyl)ethoxy]propanamide;
(2*S*)-2-(4-chlorophenoxy)-*N*-(1-phenylethoxy)propanamide;
(2*S*)-2-(4-chlorophenoxy)-*N*-[(1-methyl-1H-imidazol-2-yl)methoxy]propanamide;
(2*S*)-2-(4-chlorophenoxy)-*N*-(2-methoxyethoxy)propanamide;
(2*S*)-2-(4-chloro-2-fluorophenoxy)*-N*-cyclobutoxypropanamide;
(2*S*)-2-[4-bromo-2-(1,3,4-oxadiazol-2-yl)phenoxy]-*N*-methoxypropanamide;
(2*S*)-2-[4-chloro-2-(trifluoromethyl)phenoxy]-*N*-methoxypropanamide;
(2*S*)-*N*-(benzyloxy)-2-(4-chlorophenoxy)propanamide;
(2*S*)-2-(4-chlorophenoxy)-*N*-[(2-methoxycyclopentyl)oxy]propanamide;
(2*S*)-2-(4-chlorophenoxy)-*N*-cyclobutoxy-5-methylhexanamide;
(2*S*)-2-(4-chlorophenoxy)-*N*-cyclobutoxy-*N*-methylpropanamide;
(2*S*)-2-(4-chloro-2-methylphenoxy)-*N*-cyclobutoxypropanamide;
(2*S*)-2-(4-chloro-3-fluorophenoxy)-*N*-cyclobutoxypropanamide;
(2*S*)-2-(4-chloro-2-methylphenoxy)-*N*-methoxypropanamide;
(2*S*)-2-(4-chloro-3-fluorophenoxy)-*N*-methoxypropanamide;
(2*S*)-2-(4-chlorophenoxy)-*N*-cyclobutoxy-4-methylpentanamide;
(2*S*)-2-(4-chlorophenoxy)-*N*-[(3-methylbut-2-en-1-yl)oxy]propanamide;
(2*S*)-2-(4-chlorophenoxy)-*N*-cyclobutoxyhexanamide;
(2*S*)-2-(4-chlorophenoxy)-*N*-[(1,3-oxazol-2-yl)methoxy]propanamide;
(2*S*)-2-(2,4-dibromophenoxy)-*N*-methoxypropanamide;
(2*S*)-2-(4-bromo-2-fluorophenoxy)-*N*-methoxypropanamide;
(2*S*)-2-(4-chlorophenoxy)-*N*-(oxan-2-yloxy)propanamide;
(2*S*)-2-(4-chlorophenoxy)-*N*-[(1,3-thiazol-2-yl)methoxy]propanamide;
(2*S*)-2-(4-chlorophenoxy)-*N*-(3,3-difluorocyclobutoxy)propanamide;
(2*S*)-2-[4-bromo-2-(1,2-oxazol-5-yl)phenoxy]-*N*-cyclobutoxypropanamide;
(2*S*)-2-[4-bromo-2-(1,2-oxazol-5-yl)phenoxy]-*N*-methoxypropanamide;
(2*S*)-2-(4-chlorophenoxy)-*N*-(cyclopentyloxy)propanamide;
(2*S*)-2-(4-chlorophenoxy)-*N*-(2-cyclopentylethoxy)propanamide;
(2*S*)-2-(4-bromo-2-chlorophenoxy)-*N*-methoxypropanamide;
(2*S*)-2-(4-bromo-2-methylphenoxy)-*N*-methoxypropanamide;
(2*S*)-2-(4-chlorophenoxy)-*N*-(cyclopropylmethoxy)propanamide;
(2*S*)-2-(4-chlorophenoxy)-*N*-(cyclobutylmethoxy)propanamide;
(2*S*)-*N*-(2-aminoethoxy)-2-(4-chlorophenoxy)propanamide;
(2*S*)-2-(4-bromophenoxy)-*N*-methoxy-3-methylbutanamide;
methyl 2-{[(2*S*)-2-(4-chlorophenoxy)propanamido]oxy}acetate;
(2*S*)-2-(4-chlorophenoxy)-*N*-[2-(2-methoxyethoxy)ethoxy]propanamide;
(2*S*)-2-(4-chlorophenoxy)-*N*-cyclobutoxypropanamide;
(2*S*)-2-(4-chlorophenoxy)-*N*-(2-hydroxyethoxy)propanamide;
(2*S*)-2-(4-chlorophenoxy)-*N*-ethoxypropanamide;
(2*S*)-2-(4-chlorophenoxy)-*N*-propoxypropanamide;
(2*S*)-2-(4-chlorophenoxy)-*N*-(propan-2-yloxy)propanamide;
(2*S*)-2-(4-chlorophenoxy)-*N*-methoxypropanamide;
(2*S*)-2-[4-bromo-2-(1,2-oxazol-3-yl)phenoxy]-*N-*(cyclopropanesulfonyl)propenamide;
(2*S*)-2-(4-bromo-2-fluorophenoxy)-*N*-methanesulfonylpropanamide;
(2*S*)-2-(4-bromo-2-fluorophenoxy)-*N*-(cyclopropanesulfonyl)propenamide;
(2*S*)-2-(4-chlorophenoxy)-*N*-methanesulfonylpropanamide;
(2*S*)-2-(4-bromophenoxy)-*N*-methanesulfonyl-3-methylbutanamide;
5-[(1*S*)-1-[4-bromo-2-(1,2-oxazol-3-yl)phenoxy]-3-fluoropropyl]-2H-1,2,3,4-tetrazole;
5-[(1*S*)-1-(4-bromo-2-fluorophenoxy)propyl]-2H-1,2,3,4-tetrazole;
5-[(1*S*)-1-(4-bromo-2-fluorophenoxy)-2-methylpropyl]-2H-1,2,3,4-tetrazole;
5-[(1*R*)-1-(4-bromophenoxy)-2-fluoroethyl]-2H-1,2,3,4-tetrazole;
5-[(1*S*)-1-(4-bromophenoxy)propyl]-2H-1,2,3,4-tetrazole;
5-[(1*S*)-1-(4-bromophenoxy)ethyl]-2H-1,2,3,4-tetrazole;
5-[(1*S*)-1-[4-bromo-2-(1,2-oxazol-3-yl)phenoxy]ethyl]-1H-1,2,3,4-tetrazole;
5-[(1*S*)-1-(4-bromo-2-fluorophenoxy)ethyl]-2H-1,2,3,4-tetrazole;
5-[(1*S*)-1-[4-bromo-2-(1,2-oxazol-5-yl)phenoxy]ethyl]-2H-1,2,3,4-tetrazole;
5-[(1*S*)-1-[4-chloro-2-(1,2-oxazol-3-yl)phenoxy]ethyl]-2H-1,2,3,4-tetrazole;
5-[(1*S*)-1-(4-chloro-2-cyclopropylphenoxy)ethyl]-1H-1,2,3,4-tetrazole;
5-[(1*S*)-1-(4-chloro-2-ethenylphenoxy)ethyl]-1H-1,2,3,4-tetrazole;
5-[(1*S*)-1-(4-chloro-2-ethylphenoxy)ethyl]-1H-1,2,3,4-tetrazole;
5-[(1*S*)-1-(4-chloro-2-methylphenoxy)ethyl]-2H-1,2,3,4-tetrazole;
5-[(1*S*)-1-(4-chlorophenoxy)ethyl]-1H-1,2,3,4-tetrazole;
*N*-{3-[(1*S*)-1-(4-bromo-2-fluorophenoxy)ethyl]-1,2,4-thiadiazol-5-yl}acetamide;
*N*-{3-[(1*S*)-1-(4-bromo-2-fluorophenoxy)ethyl]-1,2,4-thiadiazol-5-yl}methanesulfonamide;
3-[(1*S*)-1-(4-bromo-2-fluorophenoxy)ethyl]-1,2,4-thiadiazol-5-amine;
*N*-{3-[(1*S*)-1-(4-bromo-2-fluorophenoxy)ethyl]-1,2,4-oxadiazol-5-yl}acetamide;
*N*-{3-[(1*S*)-1-(4-bromo-2-fluorophenoxy)ethyl]-1,2,4-oxadiazol-5-yl}methanesulfonamide;
3-[(1*S*)-1-[4-bromo-2-(1,2-oxazol-3-yl)phenoxy]ethyl]-5-(trifluoromethyl)-4H-1,2,4-triazole;
3-[(1*S*)-1-[4-chloro-2-(1,2-oxazol-3-yl)phenoxy]ethyl]-5-(trifluoromethyl)-4H-1,2,4-triazole;
3-[(1S)-1-(4-bromo-2-fluorophenoxy)ethyl]-5-methoxy-1,2,4-oxadiazole;
3-[(1*S*)-1-(4-bromo-2-fluorophenoxy)ethyl]-1,2,4-oxadiazol-5-amine;
3-[(1*S*)-1-[4-bromo-2-(1,2-oxazol-3-yl)phenoxy]ethyl]-5-methyl-4H-1,2,4-triazole;
3-[(1*S*)-1-[4-chloro-2-(1,2-oxazol-3-yl)phenoxy]ethyl]-5-methyl-4H-1,2,4-triazole;
3-[(1*S*)-1-(4-bromo-2-fluorophenoxy)ethyl]-5-methyl-4H-1,2,4-triazole;
5-[(1*S*)-1-(4-chlorophenoxy)ethyl]-1H-1,2,4-triazole;
(2*S*)-2-[4-bromo-2-(1, 1-difluoroethyl)phenoxy]-N-cyanopropanamide;
(2*S*)-2-[4-bromo-2-(1,1-difluoropropyl)-5-fluorophenoxy]-*N*-cyanopropanamide;
(2*S*)-2-[4-bromo-2-(1,1-difluoropropyl)phenoxy]-*N-*methanesulfonylpropanamide;
(2*S*)-2-[4-bromo-2-(1,1-difluoropropyl)phenoxy]-*N-*(cyclopropanesulfonyl)propenamide;
5-[(1*S*)-1-[4-bromo-2-(1,1-difluoroethyl)phenoxy]ethyl]-1H-1,2,3,4-tetrazole;
5-[(1*S*)-1-[4-bromo-2-(1,1-difluoropropyl)phenoxy]ethyl]-1H-1,2,3,4-tetrazole;
5-[(1*S*)-1-(4-bromo-2-cyclobutylphenoxy)-2-methoxyethyl]-1H-1,2,3,4-tetrazole; and
5-[(1*S*)-1-(4-bromo-2-cyclopropylphenoxy)-2-methoxyethyl]-1H-1,2,3,4-tetrazole.

In certain embodiments, the compound or the compound for use according to the present disclosure can have >90% enantiomeric excess. In certain embodiments, the compound or the compound for use according to the present disclosure can have >95% e.e.

In one embodiment, the compound or the compound for use according to the present disclosure has been modified in order to increase its half-life when administered to a patient, in particular its plasma half-life.

In one embodiment, the compound or the compound for use according to the present disclosure further comprises a moiety conjugated to said compound, thus generating a moiety-conjugated compound. In one embodiment, said moiety-conjugated compound has a plasma and/or serum half-life being longer than the plasma and/or serum half-life of the non-moiety conjugated compound.

In one embodiment, the moiety conjugated to the compound or compound for use according to the present invention, is one or more type(s) of moieties selected from the group consisting of albumin, fatty acids, polyethylene glycol (PEG), acylation groups, antibodies and antibody fragments.

### Neuromuscular disorders

The compound or compound for use of the present disclosure is used for treating, ameliorating and/or preventing a neuromuscular disorder, or reversing neuromuscular blockade caused by non-depolarizing neuromuscular blocker or antibiotic agent.

The inventors of the present disclosure have shown that inhibition of CIC-1 channels strengthens neuromuscular transmission. CIC-1 function may therefore contribute to muscle weakness in conditions of compromised neuromuscular transmission.

Thus, in one embodiment of the present invention, the compound or the compound for use as described herein inhibits CIC-1 channels. Thus, it is appreciated that compounds and/or compounds for use of Formula (I) inhibit CIC-1 channels.

The neuromuscular disorder may also include neuromuscular dysfunctions.

Neuromuscular disorders include for example disorders with symptoms of muscle weakness and fatigue. Such disorders may include conditions with reduced neuromuscular transmission safety factor. In one embodiment the neuromuscular disorders are motor neuron disorders. Motor neuron disorders are disorders with reduced safety in the neuromuscular transmission. In one embodiment motor neuron disorders are selected from the group consisting of amyotrophic lateral sclerosis (ALS) (Killian JM, Wilfong AA, Burnett L, Appel SH, Boland D. Decremental motor responses to repetitive nerve stimulation in ALS. Muscle Nerve, 1994, 17, 747-754), spinal muscular atrophy (SMA) (Wadman RI, Vrancken AF, van den Berg LH, van der Pol WL. Dysfunction of the neuromuscular junction in spinal muscular atrophy types 2 and 3. Neurology, 2012, 79, 2050-2055), Charcot-Marie Tooth disease (Bansagi B, Griffin H, Whittaker RG, Antoniadi T, Evangelista T, Miller J, Greenslade M, Forester N, Duff J, Bradshaw A, Kleinle S, Boczonadi V, Steele H, Ramesh V, Franko E, Pyle A, Lochmüller H, Chinnery PF, Horvath R. Genetic heterogeneity of motor neuropathies. Neurology, 2017, 28;88(13):1226-1234), X-linked spinal and bulbar muscular atrophy (Yamada, M., Inaba, A., Shiojiri, T. X-linked spinal and bulbar muscular atrophy with myasthenic symptoms. Journal of the Neurological Sciences, 1997, 146, 183-185), Kennedy's disorder (Stevic, Z., Peric, S., Pavlovic, S., Basta, I., Lavrnic, D., Myasthenic symptoms in a patient with Kennedy's disorder. Acta Neurologica Belgica, 2014, 114, 71-73), multifocal motor neuropathy (Roberts, M., Willison, H.J., Vincent, A., Newsom-Davis, J. Multifocal motor neuropathy human sera block distal motor nerve conduction in mice. Ann Neurol. 1995, 38, 111-118), Guillain-Barré syndrome (Ansar, V., Valadi, N. Guillain-Barré Syndrome Prim. Care, 2015, 42, 189-193; poliomyelitis (Trojan, D.A., Gendron, D., Cashman, N.R. Electrophysiology and electrodiagnosis of the post-polio motor unit. Orthopedics, 1991, 14, 1353-1361, and Birk T.J. Poliomyelitis and the post-polio syndrome: exercise capacities and adaptation - current research, future directions, and widespread applicability. Med. Sci. Sports Exerc., 1993, 25, 466-472), post-polio syndrome (Garcia, C.C., Potian, J.G., Hognason, K., Thyagarajan, B., Sultatos, L.G., Souayah, N., Routh, V.H., McArdle, J.J. Acetylcholinesterase deficiency contributes to neuromuscular junction dysfunction in type 1 diabetic neuropathy. Am. J. Physiol. Endocrinol. Metab., 2012, 15, E551 - 561) and sarcopenia (Gilmore K.J., Morat T., Doherty T.J., Rice C.L., Motor unit number estimation and neuromuscular fidelity in 3 stages of sarcopenia. 2017, 55(5):676-684).

Thus, in one embodiment of the present disclosure the neuromuscular disorder is amyotrophic lateral sclerosis (ALS). In another embodiment the neuromuscular disorder is spinal muscular atrophy (SMA). In another embodiment the neuromuscular disorder is Charcot-Marie tooth disease (CMT). In another embodiment the neuromuscular disorder is sarcopenia. In yet another embodiment, the neuromuscular disorder is critical illness myopathy (CIM).

As stated above the neuromuscular disorders include for example disorders with symptoms of muscle weakness and fatigue. Such disorder may for example include diabetes (Burton, A. Take your pyridostigmine: that's an (ethical?) order! Lancet Neurol., 2003, 2, 268).

In one embodiment the compound or the compound for use of the present disclosure is used to prevent neuromuscular disorder. The compound or the compound for use may for example be used prophylactically against nerve gas that is known to cause symptoms of muscle weakness and fatigue (Kawamura, Y., Kihara, M., Nishimoto, K., Taki, M. Efficacy of a half dose of oral pyridostigmine in the treatment of chronic fatigue syndrome: three case reports. Pathophysiology, 2003, 9, 189-194). In one embodiment the compound or the compound for use of the present disclosure is used in the treatment of botulism poisoning (Sellin, L.C., The action of botulinum toxin at the neuromuscular junction, Med Biol., 1981, 59, 11-20. In one disclosure, the compound or the compound for use of the present disclosure is used in the treatment of snake bites (Silva A., Maduwage K., Buckley N.A., Lalloo D.G., de Silva H.J., Isbister G.K., Antivenom for snake venom-induced neuromuscular paralysis, Cochrane Database of Systematic Reviews, 2017, 3, Art. No.: CD012604).

In another embodiment the neuromuscular disorders is chronic fatigue syndrome. Chronic fatigue syndrome (CFS) (Fletcher, S.N., Kennedy, D.D., Ghosh, I.R., Misra, V.P., Kiff, K., Coakley, J.H., Hinds, C.J. Persistent neuromuscular and neurophysiologic abnormalities in long-term survivors of prolonged critical illness. Crit. Care Med. 2003, 31, 1012 - 1016) is the common name for a medical condition characterized by debilitating symptoms, including fatigue that lasts for a minimum of six months in adults. CFS may also be referred to as systemic exertion intolerance disorder (SEID), myalgic encephalomyelitis (ME), post-viral fatigue syndrome (PVFS), chronic fatigue immune dysfunction syndrome (CFIDS), or by several other terms. Symptoms of CFS include malaise after exertion; unrefreshing sleep, widespread muscle and joint pain, physical exhaustion, and muscle weakness.

In another embodiment the neuromuscular disorder is myotubular myopathy (Dowling, J.J. et al, Myotubular myopathy and the neuromuscular junction: a novel therapeutic approach from mouse models, Disease Models & Mechanisms, 2012, 5, 852-859). In another embodiment the neuromuscular disorder is Duchenne muscular dystrophy (van der Pijl, M.M. et al, Characterization of neuromuscular synapse function abnormalities in multiple Duchenne muscular dystrophy mouse models, European Journal of Neuroscience, 2016, 43, 1623-1635.

In a further embodiment the neuromuscular disorder is a critical illness polyneuropathy (Angelini C. Spectrum of metabolic myopathies. Biochim. Biophys. Acta., 2015, 1852, 615 - 621) or CIM (Latronico, N., Bolton, C.F. Critical illness polyneuropathy and myopathy: a major cause of muscle weakness and paralysis. Lancet Neurol. 2011, 10, 931-941). Critical illness polyneuropathy and CIM are overlapping syndromes of widespread muscle weakness and neurological dysfunction developing in critically ill patients.

The neuromuscular disorder may also include metabolic myopathy (Milone, M., Wong, L.J. Diagnosis of mitochondrial myopathies. Mol. Genet. Metab., 2013, 110, 35 - 41) and mitochondrial myopathy (Srivastava, A., Hunter, J.M. Reversal of neuromuscular block. Br. J. Anaesth. 2009, 103, 115 - 129). Metabolic myopathies result from defects in biochemical metabolism that primarily affects muscle. These may include glycogen storage disorders, lipid storage disorder and 3-phosphocreatine storage disorder. Mitochondrial myopathy is a type of myopathy associated with mitochondrial disorder. Symptoms of mitochondrial myopathies include muscular and neurological problems such as muscle weakness, exercise intolerance, hearing loss and trouble with balance and coordination.

In another embodiment the neuromuscular disorder is periodic paralysis, in particular hypokalemic periodic paralysis which is a disorder of skeletal muscle excitability that presents with recurrent episodes of weakness, often triggered by exercise, stress, or carbohydrate-rich meals (Wu, F., Mi, W., Cannon, S.C., Neurology, 2013, 80, 1110-1116 and Suetterlin, K. et at, Current Opinion Neurology, 2014, 27, 583-590) or hyperkalemic periodic paralysis which is an inherited autosomal dominant disorder that affects sodium channels in muscle cells and the ability to regulate potassium levels in the blood (Ammat, T. et at, Journal of General Physiology, 2015, 146, 509-525).

In an embodiment the neuromuscular disorder is a myasthenic condition. Myasthenic conditions are characterized by muscle weakness and neuromuscular transmission failure. Congenital myasthenia gravis (Finlayson, S., Beeson, D., Palace, J. Congenital myasthenic syndromes: an update. Pract. Neurol., 2013, 13, 80 - 91) is an inherited neuromuscular disorder caused by defects of several types at the neuromuscular junction.

Myasthenia gravis and Lambert-Eaton syndrome (Titulaer MJ, Lang B, Verschuuren JJ. Lambert-Eaton myasthenic syndrome: from clinical characteristics to therapeutic strategies. Lancet Neurol. 2011, 10, 1098-107) are examples of myasthenic conditions. Myasthenia gravis is either an autoimmune or congenital neuromuscular disorder that leads to fluctuating muscle weakness and fatigue. In the most common cases, muscle weakness is caused by circulating antibodies that block ACh receptors at the postsynaptic neuromuscular junction, inhibiting the excitatory effects of the neurotransmitter ACh on nicotinic ACh-receptors at neuromuscular junctions (Gilhus, N.E., Owe, J.F., Hoff, J.M., Romi, F., Skeie, G.O., Aarli, J.A. Myasthenia Gravis: A Review of Available Treatment Approaches, Autoimmune Diseases, 2011, Article ID 84739). Lambert-Eaton myasthenic syndrome (also known as LEMS, Lambert-Eaton syndrome, or Eaton-Lambert syndrome) is a rare autoimmune disorder that is characterized by muscle weakness of the limbs. It is the result of an autoimmune reaction in which antibodies are formed against presynaptic voltage-gated calcium channels, and likely other nerve terminal proteins, in the neuromuscular junction.

Thus, in one embodiment of the present disclosure the neuromuscular disorder is myasthenia gravis. In another embodiment the neuromuscular disorder is Lambert-Eaton syndrome.

Neuromuscular blockade is used in connection with surgery under general anaesthesia. Reversing agents are used for more rapid and safer recovery of muscle function after such blockade. Complications with excessive muscle weakness after blockade during surgery can result in delayed weaning from mechanical ventilation and respiratory complications after the surgery. Since such complications have pronounced effects on outcome of the surgery and future quality of life of patients, there is a need for improved reversing agents (Murphy GS, Brull SJ. Residual neuromuscular block: lessons unlearned. Part I: definitions, incidence, and adverse physiologic effects of residual neuromuscular block. Anesth Analg. 2010 111(1):120-8). Thus, in one embodiment, the neuromuscular disorder has been induced by a neuromuscular blocking agent. In one particular embodiment the neuromuscular disorder is muscle weakness caused by neuromuscular blockade after surgery. In another embodiment of the present disclosure the compound or the compound for use is used for reversing and/or ameliorating neuromuscular blockade after surgery. In one embodiment, the neuromuscular blockade is drug induced. In one embodiment the neuromuscular blockade is induced by an antibiotic. In one embodiment the neuromuscular blockade is induced by a non-depolarizing neuromuscular blocker.

### Pharmaceutical formulations

In one embodiment, a composition comprising the compound or the compound for use, according to the present disclosure, is provided. The composition according to the present disclosure may be used for treating, ameliorating and/or preventing a neuromuscular disorder, and/or for use in reversing and/or ameliorating a neuromuscular blockade. Thus, the compositions and compounds described herein can be pharmaceutically acceptable. In one embodiment the composition as described herein is in the form of a pharmaceutical formulation. In one embodiment, the composition as described herein further comprises a pharmaceutically acceptable carrier. Examples of potential formulations and preparations are contained, for example, in the Handbook of Pharmaceutical Excipients as well as Remington's Pharmaceutical Sciences.

### Combination therapy

The composition of the present disclosure may comprise further active ingredients/agents or other components to increase the efficiency of the composition. Thus, in one embodiment the composition further comprises at least one further active agent. It is appreciated that the active agent is suitable for treating, preventing or ameliorating said neuromuscular disorder.

The active agent in certain embodiments can be an acetylcholine esterase inhibitor. Said acetylcholine esterase inhibitor may for example be selected from the group consisting of delta-9-tetrahydrocannabinol, carbamates, physostigmine, neostigmine, pyridostigmine, ambenonium, demecarium, rivastigmine, phenanthrene derivatives, galantamine, piperidines, donepezil, tacrine, edrophonium, huperzine, ladostigil, ungeremine and lactucopicrin.

In one embodiment, the acetylcholine esterase inhibitor is selected from the group consisting of neostigmine, physostigmine and pyridostigmine. In one embodiment the acetylcholine esterase inhibitor is neostigmine or pyridostigmine.

The active agent may also be an immunosuppressive drug. Immunosuppressive drugs are drugs that suppress or reduce the strength of the body's immune system. They are also known as anti-rejection drugs. Immunosuppressive drugs include but are not limited to glucocorticoids, corticosteroids, cytostatics, antibodies and drugs acting on immunophilins. In one embodiment the active agent is prednisone.

The active agent may also be an agent that is used in anti-myotonic treatment. Such agents include for example blockers of voltage gated Na⁺ channels, and aminoglycosides.

The active agent may also be an agent for reversing a neuromuscular blockade after surgery. Such agents include for example neostigmine or sugammadex (Org 25969, tradename Bridion). The active agent may also be an agent for increasing the Ca²⁺ sensitivity of the contractile filaments in muscle. Such agents include tirasemtiv and CK-2127107 (Hwee, D.T., Kennedy, A.R., Hartman, J.J., Ryans, J., Durham, N., Malik, F.I., Jasper, J.R. The small-molecule fast skeletal troponin activator, CK-2127107, improves exercise tolerance in a rat model of heart failure. Journal of Pharmacology and Experimental Therapeutics, 2015, 353, 159 - 168).

The active agent may also be an agent for increasing ACh release by blocking voltage-gated K⁺ channels in the pre-synaptic terminal. Such agent includes 3,4-aminopyridine.

### Methods

In one aspect, the present disclosure relates to a method of treating, preventing and/or ameliorating a neuromuscular disorder, said method comprising administering a therapeutically effective amount of the compound or the compound for use as defined herein to a person in need thereof. In one embodiment, the person is a human being.

In one aspect, the present disclosure relates to a method of reversing and/or ameliorating a neuromuscular blockade, said method comprising administering a therapeutically effective amount of the compound or the compound for use as defined herein to a person in need thereof.

In one aspect, the present disclosure relates to a method for recovery of neuromuscular transmission, said method comprising administering a therapeutically effective amount of the compound or the compound for use as defined herein to a person in need thereof.

The person in need thereof may be a person having a neuromuscular disorder or a person at risk of developing a neuromuscular disorder or a person having symptoms of muscle weakness and/or fatigue. In another embodiment the person in need thereof is a person with reduced neuromuscular transmission safety with prolonged recovery after neuromuscular blockade. Types of neuromuscular disorders are defined herein above. In an embodiment the person has, amyotrophic lateral sclerosis, spinal muscular atrophy, myasthenia gravis or Lambert-Eaton syndrome.

A therapeutically effective amount is an amount that produces a therapeutic response or desired effect in the person taking it. Administration routes, formulations and dosages can be determined by persons of skill in the art.

The method of treatment may be combined with other methods that are known to treat, prevent and/or ameliorate neuromuscular disorders. The treatment method may for example be combined with administration of any of the agents mentioned herein above. In one embodiment the treatment is combined with administration of acetylcholine esterase inhibitor such as for example neostigmine or pyridostigmine.

Another aspect of the disclosure relates to use of a compound as defined herein, for the manufacture of a medicament for the treatment, prevention and/or amelioration of a neuromuscular disorder.

Another aspect relates to use of a compound as defined herein, for the manufacture of a medicament or a reversal agent for reversing and/or ameliorating a neuromuscular blockade after surgery.

### Method of manufacturing

In one aspect, the present disclosure relates to methods of manufacturing compounds or compounds for use according to formula (I).

One method for manufacturing the compounds or compounds for use according to the present disclosure comprises the steps of
a). reacting a compound having formula (GM.I) 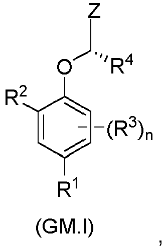 wherein R¹, R², R³, R⁴ and n as defined herein and Z is a carboxylic acid with an amine to generate a compound having formula (GM.II) 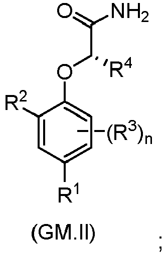
b). dehydrating the product compound of a) to generate a compound having formula (GM.III) 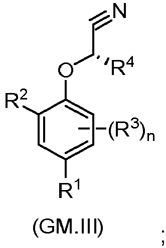 and
c). reacting the product compound of b) with an azide thus generating a compound of Formula (IVa) or (IVb) as defined herein.

A second method for manufacturing the compounds or compounds for use according to the present disclosure comprises the steps of
a). taking a compound having formula (GM.V) 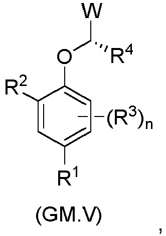 wherein R¹, R², R³, R⁴ and n are as defined herein and when W is an ester or nitrile group reacting said compound with an acid or base or alternatively when W is an alcohol then reacting said compound with an oxidising reagent to generate a compound having formula (GM.VI) 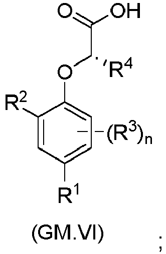 and
b). reacting the product compound of a) with a hydroxylamine derivative thus generating a compound of Formula (X) as defined herein.

Yet a third method for manufacturing the compounds or compounds for use according to the present disclosure comprises the steps of
a). taking a compound having formula (GM.V) 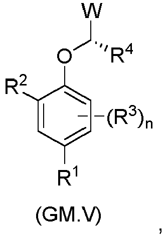 wherein R¹, R², R³, R⁴ and n are as defined herein and when W is an ester or nitrile group reacting said compound with an acid or base or alternatively when W is an alcohol then reacting said compound with an oxidising reagent to generate a compound having formula (GM.VI) 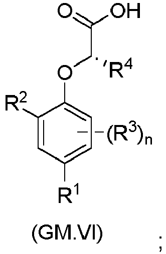 and
b). reacting the product compound of a) with a compound of formula R¹³-NH-CN wherein R¹³ is as defined herein thus generating a compound of Formula (IX) as defined herein.

### Examples

### Materials and methods

### Chemicals

Compounds for testing were obtained from different suppliers including Enamine, Vitas, and CanAm Bioresearch. For synthesis of particular compounds please see below.

### NMR Spectra

¹H-NMR spectra were recorded either on a Bruker AM-300 spectrometer and were calibrated using residual nondeuterated solvent as internal reference. Spectra were processed using Spinworks version 4.0 (developed by Dr. Kirk Marat, Department of Chemistry, University of Manitoba), or on a Bruker 400 MHZ Ultrashield plus equipped with probe BBO 400MHz S1 5mm with Z gradient probe or a Bruker 500 MHz Avance III HD spectrometer, equipped with a Bruker 5mm SmartProbeTM, calibrated using residual non-deuterated solvent as internal reference and spectra processed using topspin version 3.2.7.

### LCMS method 1

Waters Acquity UPLC, X-Select; column: Waters X-Select UPLC C18, 1.7 µm, 2.1 × 30mm. Solvent A: 0.1% formic acid in water; solvent B: 0.1% formic acid in MeCN. Gradient 5-95 % Solvent B over 3 minutes; detector: diode array.

### LCMS method 2

Waters Acquity UPLC, X-Select; column: Waters X-Select UPLC C18, 1.7 µm, 2.1 × 30mm. Solvent A: 0.1% formic acid in water; solvent B: 0.1% formic acid in MeCN. Gradient 5-95 % Solvent B over 10 minutes; detector: diode array.

### Chiral HPLC method

HPLC instrument equipped with Agilent 1200 binary pump, Agilent 1200 variable wavelength detector (UV-vis detector) and a Shodex 150 × 4.5 mm, 3 µm chiral column. Flow rate 0.5 mL/minute. Solvent A: 0.05 % CH₃COOH and 0.2 M NaCl in water. Solvent B: acetonitrile. Chiral HPLC analysis was performed in isocratic conditions (75 % of solvent-A and 25 % of solvent-B) at 280 nm wavelength. Chromatograms were processed using Agilent ChemStation software.

### Chiral SCF method 1

Compounds were analysed using a Waters ACQUITY ultra-performance convergence chromatography (UPC2) system equipped with a binary solvent delivery pump, an auto-sampler, a column oven (CM-30S), a back pressure regulator, and a diode array detector.

### LC/MS System

Samples were analysed my direct inject on a Waters Acquity QDa Mass Detector with a Waters 2695 HPLC. Mass spectra were recorded in ESI scan mode (negative/positive).

### HPLC method

The product was analysed by Waters 2695 HPLC consisting of a Waters 996 photodiode array detector, Kromasil Eternity C18, 5 µm, 4.6 × 150 mm column. Flow rate: 1 mL/minute, run time 20 minutes. Solvent A: methanol; solvent B: 0.1% formic acid in water. Gradient 0-100 % Solvent B over 15 minutes with monitoring at 280 nm.

### General synthetic strategies

Compounds of formula (I) may be synthesized by the following general methods:

### General Method A

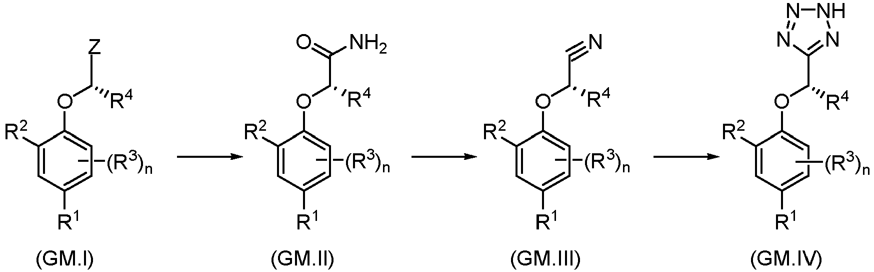

Method A involves the synthesis of compounds of formula (GM.IV), which is an aryloxyalkyl-substituted tetrazole derivative, and -R¹, -R², -R³, -R⁴ and n are as defined in Formula (I) above. Compound (GM.I), wherein -Z represents a group, for example a carboxylic acid functionality, that can be converted into a primary amide by a range of reaction conditions, is available either commercially or synthetically. Compound (GM.I) can be converted into a substituted ether of formula (GM.II) by methods which include the presence of ammonium chloride, a coupling agent such as hexafluorophosphate azabenzotriazole tetramethyl uronium (HATU) and a base such as triethylamine, as well as some two-step methods that include the formation of an amide with dibenzylamine and subsequent conversion into a primary amide. The primary amide functionality in compound (GM.II) can be converted into the corresponding nitrile (GM.III) under dehydrative conditions, including via the use of acetic anhydride, thionyl chloride or phosphorus oxychloride in a suitable solvent such as THF or 1,2-dichloroethane.

The nitrile (GM.III) can be readily converted into the target tetrazole derivative (GM.IV), typically by a cycloaddition reaction under a range of reaction conditions, utilising e.g. trimethylsilyl azide or an alkali metal azide.

Alternative methods involve the reaction of e.g. a suitably protected alcohol, e.g. a benzyl protected tetrazole-CH(R⁴)-OH, with a suitably substituted phenol wherein - R¹, -R², -R³ and n are as defined above under Mitsunobu reaction conditions, followed by deprotection to provide the desired tetrazole derivative GM.IV.

### General Method B

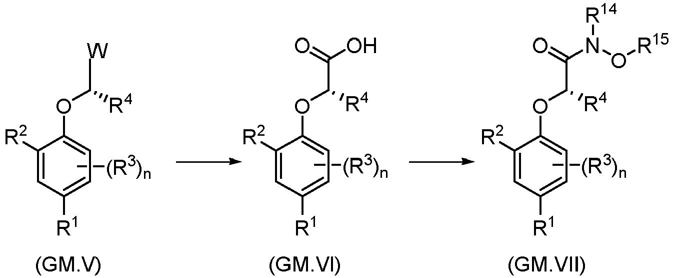

Method B involves the synthesis of compound (GM.VII), an aryloxy-substituted hydroxamic acid ether derivative, wherein -R¹, -R², -R³, -R⁴, -R¹⁴, -R¹⁵ and n are as defined in Formula (I) above. Compound (GM.V), wherein the group -W is a feasible carboxylic acid precursor, which can include an ester, an alcohol, a nitrile, including (GM.III), is available either commercially or synthetically. In the case of the acid precursor being a primary alcohol, it can be oxidised to a carboxylic acid under standard conditions involving potassium permanganate, Jones oxidation conditions, the Heyns oxidation, ruthenium tetroxide or TEMPO. Compound (GM.V) thereby provides varied and facile access to the substituted carboxylic acid (GM.VI), wherein -R¹, -R², - R³ and -R⁴ are as defined above. The target aryloxy substituted hydroxamic acid ether derivative (GM.VII) is readily obtained under reaction conditions that include a coupling procedure between the carboxylic acid (GM.VI), a suitable coupling agent, such as HATU, a suitable dialkylcarbodiimide derivative, or 2-chloro-N-methyl pyridinium iodide, and a hydroxylamine derivative of formula R¹⁵-O-NH-R¹⁴, with -R¹⁴ and -R¹⁵ as defined above, as a nucleophile. An alternative to a coupling reaction is reaction of e.g. the acid chloride derived from compound (GM.VI), by treatment with for example thionyl chloride, with a hydroxylamine derivative of formula R¹⁵-O-NH-R¹⁴ in the presence of a suitable base, e.g. a trialkylamine.

### General Method C

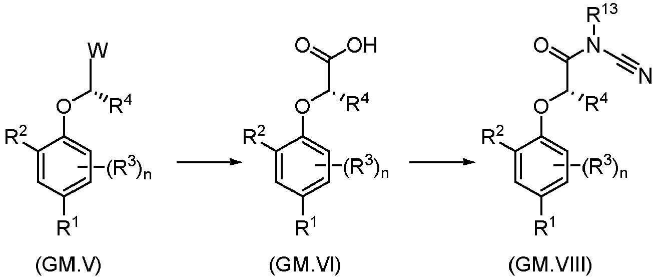

Method C involves the synthesis of compound (GM.VIII), an aryloxy-substituted *N-*cyano derivative, wherein -R¹, -R², -R³, -R⁴, -R¹³ and n are as defined in Formula (I) above. Compound (GM.V), wherein the group -W is a feasible carboxylic acid precursor, which can include an ester, an alcohol, a nitrile, including (GM.III), is available either commercially or synthetically. In the case of the acid precursor being a primary alcohol, it can be oxidised to a carboxylic acid under standard conditions involving potassium permanganate, Jones oxidation conditions, the Heyns oxidation, ruthenium tetroxide or TEMPO. Compound (GM.V) thereby provides varied and facile access to the substituted carboxylic acid XVII, wherein -R¹, -R², -R³ and -R⁴ are as defined above. The target aryloxy substituted N-cyano derivative (GM.VIII) is readily obtained under reaction conditions that include a coupling procedure between the carboxylic acid (GM.VI), involving a suitable coupling agent, such as HATU, a suitable dialkylcarbodiimide derivative or 2-chloro-N-methyl pyridinium iodide, and e.g. a compound of formula R¹³-NH-CN, as a nucleophile, wherein R¹³ is as defined above. An alternative to a coupling reaction is reaction of e.g. the acid chloride derived from compound (GM.VI), by treatment with for example thionyl chloride, with e.g. a compound of formula R¹³-NH-CN, as a nucleophile, wherein R¹³ is as defined above, in the presence of a suitable base, e.g. a trialkylamine.

### Exemplified Compounds

Table 1 below illustrates Example compounds defined by the general Formula (I) which were prepared in >95% purity.

**Table 1: Illustrative Examples of the Invention**

| **Cpd Number** | **IUPAC name** | **Synthesis method** |
|---|---|---|
| A-1 | (2*S*)-2-(4-bromo-2-fluorophenoxy)-*N*-{1-[(cyclopropylmethoxy)imino]ethyl}pro panamide | Example 7 |
| A-2 | (2*S*)-2-(4-bromophenoxy)-*N*-[1-(methoxyimino)ethyl]propanamide | Example 7 |
| A-3 | (2*S*)-2-(4-bromo-2-fluorophenoxy)-*N*-[(4-fluorophenyl)(hydroxyimino)methyl]-3-methylbutanamide | Example 7 |
| A-4 | (2*S*)-2-(4-bromo-2-fluorophenoxy)-*N*-[1-(hydroxyimino)ethyl]-3-methylbutanamide | Example 7 |
| A-5 | (2*S*)-2-(4-bromo-2-fluorophenoxy)-*N*-[1-(hydroxyimino)ethyl]propanamide | Example 7 |
| A-6 | (2*S*)-2-(4-bromophenoxy)-*N*-[1-(hydroxyimino)ethyl]propanamide | Example 7 |
| B-1 | (2*S*)-2-[4-bromo-2-(1,2-oxazol-3-yl)phenoxy]-*N-*cyanopropanamide | General method C Example 3 |
| B-2 | (2*S*)-*N*-cyano-2-(2,4-dibromophenoxy)propanamide | General method C Example 3 |
| B-3 | (2*S*)-2-(4-bromophenoxy)-*N*-cyanopropanamide | General method C Example 3 |
| B-4 | (2*S*)-2-(4-chlorophenoxy)-*N*-cyanopropanamide | General method C Example 3 |
| B-5 | (2*S*)-2-(4-bromophenoxy)-*N*-cyano-3-methylbutanamide | General method C Example 3 |
| B-6 | (2*S*)-2-[4-bromo-2-(1,1-difluoroethyl)phenoxy]-*N-*cyanopropanamide | Example 14 |
| B-7 | (2*S*)-2-[4-bromo-2-(1,1-difluoropropyl)-5-fluorophenoxy]-*N-*cyanopropanamide | Example 13 |
| C-1 | (2*S*)-2-[4-bromo-2-(1,2-oxazol-3-yl)phenoxy]-*N-*cyclobutoxypropanamide | General method B Examples 1 and 2 |
| C-2 | (2*S*)-2-[4-bromo-2-(1,2-oxazol-3-yl)phenoxy]-*N-*methoxypropanamide | General method B Examples 1 and 2 |
| C-3 | (2*S*)-*N*-acetyl-*N-*[(1-acetylazetidin-3-yl)oxy]-2-(4-chlorophenoxy)propanamide | General method B Examples 1 and 2 |
| C-4 | (2S)-*N*-[(1-acetylazetid in-3-yl)oxy]-2-(4-chlorophenoxy)propanamide | General method B Examples 1 and 2 |
| C-5 | (2*S*)-*N*-(azetidin-3-yloxy)-2-(4-chlorophenoxy)propanamide | General method B Examples 1 and 2 |
| C-6 | (2S)-*N*-acetyl-*N*-[(1-acetylpyrrolidin-3-yl)methoxy]-2-(4-chlorophenoxy)propanamide | General method B Examples 1 and 2 |
| C-7 | (2*S*)-*N*-[(1-acetylpyrrolidin-3-yl)methoxy]-2-(4-chlorophenoxy)propanamide | General method B Examples 1 and 2 |
| C-8 | (2S)-N-[(1-acetylpyrrolid in-3-yl)oxy]-2-(4-bromophenoxy)propanamide | General method B Examples 1 and 2 |
| C-9 | tert-butyl N-(2-{[(2S)-2-(4-chlorophenoxy)propanamido]oxy}propyl)carbamate | General method B Examples 1 and 2 |
| C-10 | (2*S*)-*N*-acetyl-2-(4-chlorophenoxy)-*N*-[(1-acetamidopropan-2-yl)oxy]propanamide | General method B Examples 1 and 2 |
| C-11 | (2*S*)-2-(4-bromophenoxy)-*N*-(pyrrolidin-3-yloxy)propanamide | General method B Examples 1 and 2 |
| C-12 | (2S)-*N*-acetyl-*N*-[(1-acetylpyrrolidin-3-yl)oxy]-2-(4-bromophenoxy)propanamide | General method B Examples 1 and 2 |
| C-13 | tert-butyl 3-{[(2S)-2-(4-chlorophenoxy)propanamido]oxy}azetidine-1-carboxylate | General method B Examples 1 and 2 |
| C-14 | (2*S*)-2-(4-chlorophenoxy)-*N*-[(pyrrolidin-3-yl)methoxy]propanamide | General method B Examples 1 and 2 |
| C-15 | tert-butyl 3-({[(2S)-2-(4-chlorophenoxy)propanamido]oxy}methyl)pyrrolidine-1-carboxylate | General method B Examples 1 and 2 |
| C-16 | (2*S*)-*N*-acetyl-*N*-[(1-acetylpyrrolidin-3-yl)oxy]-2-(4-chlorophenoxy)propanamide | General method B Examples 1 and 2 |
| C-17 | (2*S*)-*N*-[(1-acetylpyrrolidin-3-yl)oxy]-2-(4-chlorophenoxy)propanamide | General method B Examples 1 and 2 |
| C-18 | (2*S*)-2-(4-chlorophenoxy)-*N*-(pyrrolidin-3-yloxy)propanamide | General method B Examples 1 and 2 |
| C-19 | (2*S*)-2-(4-chlorophenoxy)-*N*-(2,2-dimethylpropyl)-*N-*hydroxypropanamide | General method B Examples 1 and 2 |
| C-20 | (2*S*)-2-(4-chlorophenoxy)-*N*-[2-(pyrrolidin-1-yl)ethoxy]propanamide | General method B Examples 1 and 2 |
| C-21 | (2*S*)-2-(4-chlorophenoxy)-*N*-[2-(2-oxopyrrolidin-1-yl)ethoxy]propanamide | General method B Examples 1 and 2 |
| C-22 | (2*S*)-*N*-acetyl-2-(4-chlorophenoxy)-*N*-(2-acetamidoethoxy)propanamide | General method B Examples 1 and 2 |
| C-23 | (2*S*)-2-(4-chlorophenoxy)-*N*-(2-acetamidoethoxy)propanamide | General method B Examples 1 and 2 |
| C-24 | (2*S*)-2-(4-chlorophenoxy)-*N*-(4,4,4-trifluoro-2-methylbutoxy)propanamide | General method B Examples 1 and 2 |
| C-25 | (2*S*)-2-(4-chlorophenoxy)-*N*-(3-cyclopentylpropyl)-*N-*hydroxypropanamide | General method B Examples 1 and 2 |
| C-26 | (2*S*)-2-(4-chlorophenoxy)-*N*-{[(2*E*)-2-methyl-3-phenylprop-2-en-1-yl]oxy}propanamide | General method B Examples 1 and 2 |
| C-27 | (2*S*)-2-(4-chlorophenoxy)-*N*-{2-oxo-2-[2-(trifluoromethyl)pyrrolidin-1-yl]ethoxy}propanamide | General method B Examples 1 and 2 |
| C-28 | tert-butyl N-(2-{[(2*S*)-2-(4-chlorophenoxy)propanamidyl]oxy}ethyl)carbamate | General method B Examples 1 and 2 |
| C-29 | (2*S*)-2-(4-chlorophenoxy)-*N*-[(2-methyl-1H-imidazol-4-yl)methoxy]propanamide | General method B Examples 1 and 2 |
| C-30 | tert-butyl 4-({[(2*S*)-2-(4-chlorophenoxy)propanamido]oxy}methyl)-2-methyl-1H-imidazole-1-carboxylate | General method B Examples 1 and 2 |
| C-31 | (2*S*)-2-(4-bromo-2-fluorophenoxy)-*N-*cyclobutoxypropanamide | General method B Examples 1 and 2 |
| C-32 | (2*S*)-2-(4-bromophenoxy)-*N*-cyclobutoxypropanamide | General method B Examples 1 and 2 |
| C-33 | (2*S*)-2-(4-chlorophenoxy)-*N*-[2-oxo-2-(pyrrolidin-1-yl)ethoxy]propanamide | General method B Examples 1 and 2 |
| C-34 | (2*S*)-2-(4-chlorophenoxy)-*N*-[1-(4-fluorophenyl)ethoxy]propanamide | General method B Examples 1 and 2 |
| C-35 | (2*S*)-2-(4-chlorophenoxy)-*N*-[1-(1,3-thiazol-2-yl)ethoxy]propanamide | General method B Examples 1 and 2 |
| C-36 | (2*S*)-2-(4-chlorophenoxy)-*N*-{1-[4-(trifluoromethyl)phenyl]ethoxy}propanamide | General method B Examples 1 and 2 |
| C-37 | (2*S*)-2-(4-chlorophenoxy)-*N*-(2-methanesulfinylethoxy)propanamide | General method B Examples 1 and 2 |
| C-38 | (2*S*)-2-(4-chlorophenoxy)-*N*-(2-methanesulfonylethoxy)propanamide | General method B Examples 1 and 2 |
| C-39 | (2*S*)-2-(4-chlorophenoxy)-*N*-[(1,2-oxazol-3-yl)methoxy]propanamide | General method B Examples 1 and 2 |
| C-40 | (2*S*)-2-(4-chloro-2-fluorophenoxy)-*N*-methoxypropanamide | General method B Examples 1 and 2 |
| C-41 | (2*S*)-2-[4-bromo-2-(1,3,4-oxadiazol-2-yl)phenoxy]-*N-*(cyclopropylmethoxy)propanamide | General method B Examples 1 and 2 |
| C-42 | (2*S*)-2-[4-chloro-2-(trifluoromethyl)phenoxy]-*N-*(cyclopropylmethoxy)propanamide | General method B Examples 1 and 2 |
| C-43 | (2*S*)-*N*-(tert-butoxy)-2-(4-chlorophenoxy)propanamide | General method B Examples 1 and 2 |
| C-44 | (2*S*)-2-(4-chlorophenoxy)-*N*-[2-(methylsulfanyl)ethoxy]propanamide | General method B Examples 1 and 2 |
| C-45 | (2*S*)-2-(4-chlorophenoxy)-*N*-(1-phenylethoxy)propanamide | General method B Examples 1 and 2 |
| C-46 | (2*S*)-2-(4-chlorophenoxy)-*N*-[(1-methyl-1H-imidazol-2-yl)methoxy]propanamide | General method B Examples 1 and 2 |
| C-47 | (2*S*)-2-(4-chlorophenoxy)-*N*-(2-methoxyethoxy)propanamide | General method B Examples 1 and 2 |
| C-48 | (2*S*)-2-(4-chloro-2-fluorophenoxy)-*N-*cyclobutoxypropanamide | General method B Examples 1 and 2 |
| C-49 | (2*S*)-2-[4-bromo-2-(1,3,4-oxadiazol-2-yl)phenoxy]-*N-*methoxypropanamide | General method B Examples 1 and 2 |
| C-50 | (2*S*)-2-[4-chloro-2-(trifluoromethyl)phenoxy]-*N-*methoxypropanamide | General method B Examples 1 and 2 |
| C-51 | (2*S*)-*N*-(benzyloxy)-2-(4-chlorophenoxy)propanamide | General method B Examples 1 and 2 |
| C-52 | (2*S*)-2-(4-chlorophenoxy)-*N*-[(2-methoxycyclopentyl)oxy]propanamide | General method B Examples 1 and 2 |
| C-53 | (2*S*)-2-(4-chlorophenoxy)-*N*-cyclobutoxy-5-methylhexanamide | General method B Examples 1 and 2 |
| C-54 | (2*S*)-2-(4-chlorophenoxy)-*N*-cyclobutoxy-*N-*methylpropanamide | General method B Examples 1 and 2 |
| C-55 | (2*S*)-2-(4-chloro-2-methylphenoxy)-*N-*cyclobutoxypropanamide | General method B Examples 1 and 2 |
| C-56 | (2*S*)-2-(4-chloro-3-fluorophenoxy)-*N-*cyclobutoxypropanamide | General method B Examples 1 and 2 |
| C-57 | (2*S*)-2-(4-chloro-2-methylphenoxy)-*N*-methoxypropanamide | General method B Examples 1 and 2 |
| C-58 | (2*S*)-2-(4-chloro-3-fluorophenoxy)-*N*-methoxypropanamide | General method B Examples 1 and 2 |
| C-59 | (2*S*)-2-(4-chlorophenoxy)-*N*-cyclobutoxy-4-methylpentanamide | General method B Examples 1 and 2 |
| C-60 | (2*S*)-2-(4-chlorophenoxy)-*N*-[(3-methylbut-2-en-1-yl)oxy]propanamide | General method B Examples 1 and 2 |
| C-61 | (2*S*)-2-(4-chlorophenoxy)-*N*-cyclobutoxyhexanamide | General method B Examples 1 and 2 |
| C-62 | (2*S*)-2-(4-chlorophenoxy)-*N*-[(1,3-oxazol-2-yl)methoxy]propanamide | General method B Examples 1 and 2 |
| C-63 | (2*S*)-2-(2,4-dibromophenoxy)-*N*-methoxypropanamide | General method B Examples 1 and 2 |
| C-64 | (2*S*)-2-(4-bromo-2-fluorophenoxy)-*N*-methoxypropanamide | General method B Examples 1 and 2 |
| C-65 | (2*S*)-2-(4-chlorophenoxy)-*N*-(oxan-2-yloxy)propanamide | General method B Examples 1 and 2 |
| C-66 | (2*S*)-2-(4-chlorophenoxy)-*N*-[(1,3-thiazol-2-yl)methoxy]propanamide | General method B Examples 1 and 2 |
| C-67 | (2*S*)-2-(4-chlorophenoxy)-*N*-(3,3-difluorocyclobutoxy)propanamide | General method B Examples 1 and 2 |
| C-68 | (2*S*)-2-[4-bromo-2-(1,2-oxazol-5-yl)phenoxy]-*N-*cyclobutoxypropanamide | General method B Examples 1 and 2 |
| C-69 | (2*S*)-2-[4-bromo-2-(1,2-oxazol-5-yl)phenoxy]-*N-*methoxypropanamide | General method B Examples 1 and 2 |
| C-70 | (2*S*)-2-(4-chlorophenoxy)-*N*-(cyclopentyloxy)propanamide | General method B Examples 1 and 2 |
| C-71 | (2*S*)-2-(4-chlorophenoxy)-*N*-(2-cyclopentylethoxy)propanamide | General method B Examples 1 and 2 |
| C-72 | (2*S*)-2-(4-bromo-2-chlorophenoxy)-*N*-methoxypropanamide | General method B Examples 1 and 2 |
| C-73 | (2*S*)-2-(4-bromo-2-methylphenoxy)-*N*-methoxypropanamide | General method B Examples 1 and 2 |
| C-74 | (2*S*)-2-(4-chlorophenoxy)-*N-*(cyclopropylmethoxy)propanamide | General method B Examples 1 and 2 |
| C-75 | (2*S*)-2-(4-chlorophenoxy)-*N-*(cyclobutylmethoxy)propanamide | General method B Examples 1 and 2 |
| C-76 | (2*S*)-*N*-(2-aminoethoxy)-2-(4-chlorophenoxy)propanamide | General method B Examples 1 and 2 |
| C-77 | (2*S*)-2-(4-bromophenoxy)-*N*-methoxy-3-methylbutanamide | General method B Examples 1 and 2 |
| C-78 | methyl 2-{[(2S)-2-(4-chlorophenoxy)propanamido]oxy}acetate | General method B Examples 1 and 2 |
| C-79 | (2*S*)-2-(4-chlorophenoxy)-*N*-[2-(2-methoxyethoxy)ethoxy]propanamide | General method B Examples 1 and 2 |
| C-80 | (2*S*)-2-(4-chlorophenoxy)-*N*-cyclobutoxypropanamide | General method B Examples 1 and 2 |
| C-81 | (2*S*)-2-(4-chlorophenoxy)-*N*-(2-hydroxyethoxy)propanamide | General method B Examples 1 and 2 |
| C-82 | (2*S*)-2-(4-chlorophenoxy)-*N*-ethoxypropanamide | General method B Examples 1 and 2 |
| C-83 | (2*S*)-2-(4-chlorophenoxy)-*N*-propoxypropanamide | General method B Examples 1 and 2 |
| C-84 | (2*S*)-2-(4-chlorophenoxy)-*N*-(propan-2-yloxy)propanamide | General method B Examples 1 and 2 |
| C-85 | (2*S*)-2-(4-chlorophenoxy)-*N*-methoxypropanamide | General method B Examples 1 and 2 |
| D-1 | (2*S*)-2-[4-bromo-2-(1,2-oxazol-3-yl)phenoxy]-*N-*(cyclopropanesulfonyl)propanamide | Example 8 |
| D-2 | (2*S*)-2-(4-bromo-2-fluorophenoxy)-*N-*methanesulfonylpropanamide | Example 8 |
| D-3 | (2*S*)-2-(4-bromo-2-fluorophenoxy)-*N-*(cyclopropanesulfonyl)propanamide | Example 8 |
| D-4 | (2*S*)-2-(4-chlorophenoxy)-*N*-methanesulfonylpropanamide | Example 8 |
| D-5 | (2*S*)-2-(4-bromophenoxy)-*N*-methanesulfonyl-3-methylbutanamide | Example 8 |
| D-6 | (2*S*)-2-[4-bromo-2-(1,1-difluoropropyl)phenoxy]-*N-*(cyclopropanesulfonyl)propanamide | Example 15 |
| D-7 | (2*S*)-2-[4-bromo-2-(1,1-difluoropropyl)phenoxy]-*N-*methanesulfonylpropanamide | Example 15 |
| E-1 | 5-[(1*S*)-1-[4-bromo-2-(1,2-oxazol-3-yl)phenoxy]-3-fluoropropyl]-2H-1,2,3,4-tetrazole | General method A Examples 4 and 5 |
| E-2 | 5-[(1*S*)-1-(4-bromo-2-fluorophenoxy)propyl]-2H-1,2,3,4-tetrazole | General method A Examples 4 and 5 |
| E-3 | 5-[(1*S*)-1-(4-bromo-2-fluorophenoxy)-2-methylpropyl]-2H-1,2,3,4-tetrazole | General method A Examples 4 and 5 |
| E-4 | 5-[(1*R*)-1-(4-bromophenoxy)-2-fluoroethyl]-2H-1,2,3,4-tetrazole | General method A Examples 4 and 5 |
| E-5 | 5-[(1*S*)-1-(4-bromophenoxy)propyl]-2H-1,2,3,4-tetrazole | General method A Examples 4 and 5 |
| E-6 | 5-[(1*S*)-1-(4-bromophenoxy)ethyl]-2H-1,2,3,4-tetrazole | General method A Examples 4 and 5 |
| E-7 | 5-[(1*S*)-1-[4-bromo-2-(1,2-oxazol-3-yl)phenoxy]ethyl]-1H-1,2,3,4-tetrazole | General method A Examples 4 and 5 |
| E-8 | 5-[(1*S*)-1-(4-bromo-2-fluorophenoxy)ethyl]-2H-1,2,3,4-tetrazole | General method A Examples 4 and 5 |
| E-9 | 5-[(1*S*)-1-[4-bromo-2-(1,2-oxazol-5-yl)phenoxy]ethyl]-2H-1,2,3,4-tetrazole | General method A Examples 4 and 5 |
| E-10 | 5-[(1S)-1-[4-chloro-2-(1,2-oxazol-3-yl)phenoxy]ethyl]-2H-1,2,3,4-tetrazole | General method A Examples 4 and 5 |
| E-11 | 5-[(1*S*)-1-(4-chloro-2-cyclopropylphenoxy)ethyl]-1H-1,2,3,4-tetrazole | General method A Examples 4 and 5 |
| E-12 | 5-[(1*S*)-1-(4-chloro-2-ethenylphenoxy)ethyl]-1H-1,2,3,4-tetrazole | General method A Examples 4 and 5 |
| E-13 | 5-[(1*S*)-1-(4-chloro-2-ethylphenoxy)ethyl]-1H-1,2,3,4-tetrazole | General method A Examples 4 and 5 |
| E-14 | 5-[(1*S*)-1-(4-chloro-2-methylphenoxy)ethyl]-2H-1,2,3,4-tetrazole | General method A Examples 4 and 5 |
| E-15 | 5-[(1*S*)-1-(4-chlorophenoxy)ethyl]-1H-1,2,3,4-tetrazole | General method A Examples 4 and 5 |
| E-16 | 5-[(1*S*)-1-[4-bromo-2-(1,1-difluoroethyl)phenoxy]ethyl]-1H-1,2,3,4-tetrazole | Example 16 |
| E-17 | 5-[(1*S*)-1-[4-bromo-2-(1,1-difluoropropyl)phenoxy]ethyl]-1H-1,2,3,4-tetrazole | Example 16 |
| E-18 | 5-[(1*S*)-1-(4-bromo-2-cyclobutylphenoxy)-2-methoxyethyl]-1H-1,2,3,4-tetrazole | General method A Examples 4 and 5 |
| E-19 | 5-[(1S)-1-(4-bromo-2-cyclopropylphenoxy)-2-methoxyethyl]-1H-1,2,3,4-tetrazole | General method A Examples 4 and 5 |
| F-1 | *N*-{3-[(1*S*)-1-(4-bromo-2-fluorophenoxy)ethyl]-1,2,4-thiadiazol-5-yl}acetamide | Example 6 |
| F-2 | *N*-{3-[(1*S*)-1-(4-bromo-2-fluorophenoxy)ethyl]-1,2,4-thiadiazol-5-yl}methanesulfonamide | Example 6 |
| F-3 | 3-[(1*S*)-1-(4-bromo-2-fluorophenoxy)ethyl]-1,2,4-thiadiazol-5-amine | Example 6 |
| F-4 | *N*-{3-[(1*S*)-1-(4-bromo-2-fluorophenoxy)ethyl]-1,2,4-oxadiazol-5-yl}acetamide | Example 6 |
| F-5 | *N*-{3-[(1*S*)-1-(4-bromo-2-fluorophenoxy)ethyl]-1,2,4-oxadiazol-5-yl}methanesulfonamide | Example 6 |
| F-6 | 3-[(1*S*)-1-[4-bromo-2-(1,2-oxazol-3-yl)phenoxy]ethyl]-5-(trifluoromethyl)-4H-1,2,4-triazole | Example 6 |
| F-7 | 3-[(1*S*)-1-[4-chloro-2-(1,2-oxazol-3-yl)phenoxy]ethyl]-5-(trifluoromethyl)-4H-1,2,4-triazole | Example 6 |
| F-8 | 3-[(1*S*)-1-(4-bromo-2-fluorophenoxy)ethyl]-5-methoxy-1 ,2,4-oxadiazole | Example 6 |
| F-9 | 3-[(1*S*)-1-(4-bromo-2-fluorophenoxy)ethyl]-1,2,4-oxadiazol-5-amine | Example 6 |
| F-10 | 3-[(1*S*)-1-[4-bromo-2-(1,2-oxazol-3-yl)phenoxy]ethyl]-5-methyl-4H-1,2,4-triazole | Example 6 |
| F-11 | 3-[(1*S*)-1-[4-chloro-2-(1,2-oxazol-3-yl)phenoxy]ethyl]-5-methyl-4H-1,2,4-triazole | Example 6 |
| F-12 | 3-[(1*S*)-1-(4-bromo-2-fluorophenoxy)ethyl]-5-methyl-4H-1,2,4-triazole | Example 6 |
| F-13 | 5-[(1*S*)-1-(4-chlorophenoxy)ethyl]-1H-1,2,4-triazole | Example 6 |

### Example 1: (2S)-2-(4-Chlorophenoxy)-N-cyclobutoxypropanamide

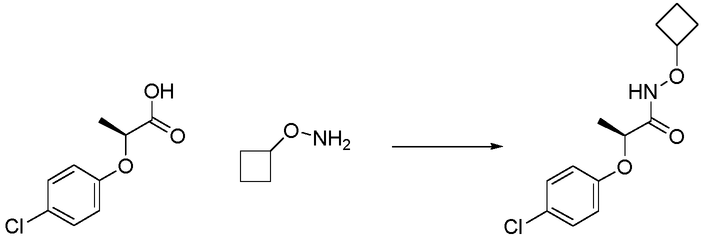

To a solution of (2*S*)-2-(4-chlorophenoxy)propanoic acid (0.16 g, 0.797 mmol, 1.0 eq.) and O-cyclobutylhydroxylamine hydrochloride (0.118 g, 0.957 mmol, 1.2 eq.) in dichloromethane (20 *mL*) at 0°C was added 1-ethyl-3-(3-dimethylaminopropyl)carbodiimide (EDC.HCl) (0.229 g, 1.2 mmol, 1.5 eq.) followed by N,N-diisopropylethylamine (0.124 g, 0.957 mmol, 1.2 eq.). The reaction mixture was stirred overnight at room temperature and extracted with dicholoromethane then washed with water (10 mL) and brine (10 mL). Organic layer was dried over Na₂SO₄, and evaporated *in vacuo.* The crude product was purified by column chromatography on silica gel using (0-5% MeOH/DCM), then by preparative TLC using 1% MeOH/DCM to provide the title compound 32 mg (14% yield).

¹H NMR (300 MHz, CDCl₃) δ 8.85 (s, 1H), 7.25 (d, 2H), 6.80 (d, 2H), 4.65 (m, 1H), 4.48 (m, 1H), 2.30-2.00 (m, 4H), 1.75 (m, 1H), 1.58 (d, 3H), 1.50 (m, 1H).
ES- MS: *m*/*z* 268.3 (M-1).
HPLC Retention time: 13.27 min

### Example 2: (2S)-N-[(1-acetylpyrrolidin-3-yl)oxy]-2-(4-bromophenoxy)propanamide

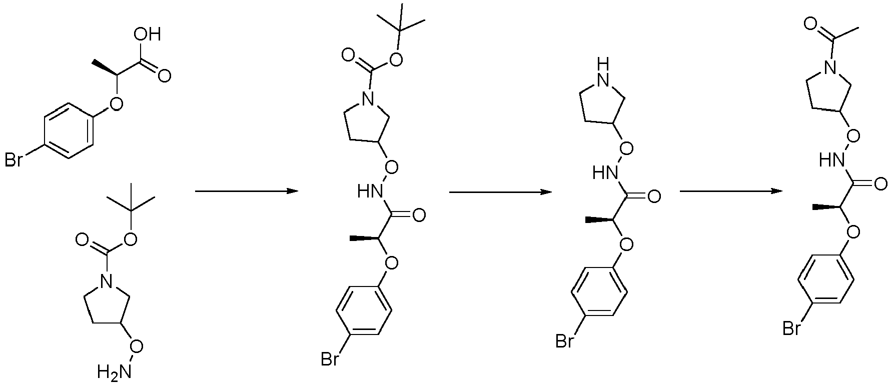

tert-Butyl 3-[[(2S)-2-(4-bromophenoxy)propanoyl]amino]oxypyrrolidine-1-carboxylate

To a solution of (2*S*)-2-(p-bromophenoxy)propionic acid (0.40 g, 1.63 mmol, 1.0 eq.) and *tert*-butyl 3-(aminooxy)-1-pyrrolidinecarboxylate (0.396 g, 1.958 mmol, 1.2 eq.) in dichloromethane (15 mL) at 0°C EDC.HCl (0.469 g, 2.448 mmol, 1.5 eq.) was added, followed by N,N-diisopropylethylamine (0.253 g, 1.958 mmol, 1.2 eq.). The reaction mixture was stirred overnight at room temperature and extracted with dicholoromethane, then washed with water (20 *mL*), and brine (20 mL). The organic layer was dried over Na₂SO₄, filtered and evaporated *in vacuo.* The crude product was purified by column chromatography on silica gel using (10-50% ethylacetate/hexane) to provide the title compound (0.66 g, 94% yield).

¹H NMR (300 MHz, CDCl₃) δ 10.0 (br s, 1H), 7.29 (d, 2H), 6.73 (d, 2H), 4.68-4.41 (m, 2H), 3.67-3.0 (m, 4H), 2.15-1.64 (m, 2H), 1.51 (d, 3H), 1.41-1.28 (br d, 9H).

### (2S)-2-(4-bromophenoxy)-N-pyrrolidin-3-yloxy-propanamide (TFA salt)

Trifluoroacetic acid (1.517 g, 13.31 mmol, 11 equiv.) was added slowly to a solution of *tert*-butyl 3-[[(2S)-2-(4-bromophenoxy)propanoyl]amino]oxypyrrolidine-1-carboxylate (0.52 g, 1.21 mmol, 1 eq.) in dicholoromethane (1 mL) at 0° C. The reaction mixture was stirred at room temperature for 2 h and after completion, dicholoromethane was evaporated to dryness. Residue obtained was again dissolved in dicholoromethane (5 mL × 2) and evaporated to get the title compound 0.535 g (99.6 % yield).

¹H NMR (300 MHz, CDCl₃) δ 12.16-11.47 (br s, 1H), 9.69-9.42 (br s, 1H), 9.13-8.83 (br s, 1H), 7.36 (d, 2H), 6.78 (dd, 2H), 4.86-4.63 (m, 2H), 3.73-3.31 (m, 3H), 3.29-3.05 (br s, 1H), 2.35-1.96 (m, 2H), 1.54 (dd, 3H).

### (2S)-N-r(1-acetylpyrrolidin-3-yl)oxy1-2-(4-bromophenoxy)propanamide

Acetyl chloride (0.093 g, 1.184 mmol, 1.5 eq.) was added to a solution of (2*S*)-2-(4-bromophenoxy)-*N*-pyrrolidin-3-yloxy-propanamide (0.35 g, 0.789 mmol, 1.0 eq.) in dicholoromethane (5 mL) at 0°C, followed by the addition of N,N-diisopropylethylamine (0.306 g, 2.369 mmol, 3 eq.). The reaction mixture was stirred at room temperature for 4 h. Extracted with dicholoromethane, washed with water (15 mL × 2), dried over Na₂SO₄, filtered, and concentrated. The crude product was purified by column chromatography on silica gel using (25-80% ethylacetate/ hexane), then by preparative TLC using ethyl acetate to provide the title compound 20 mg (6.8 % yield).

¹H NMR (300 MHz, CDCl₃) δ 7.42-7.33 (m, 2H), 6.87 (d, 2H), 4.95-4.84 (m, 1H), 4.83-4.70 (m, 1H), 3.81-3.31 (m, 4H), 2.18 (t, 3H), 2.05 (d, 2H), 1.59-1.52 (m, 3H).
LCMS = 371.2 (M+1)
HPLC retention time: 14.51 min

### Example 3: (2S)-2-(4-Bromophenoxy)-N-cyano-3-methyl-butanamide

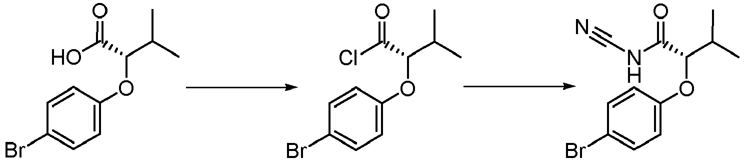

To a stirred solution of (2*S*)-2-(4-bromophenoxy)-3-methylbutanoic acid (1 mmol) in DCM (6 mL) at 0°C, oxalyl chloride (1.2 mmol) was added and the resultant mixture was stirred at room temperature for 2h. The volatiles were removed, and the residue dried under vacuum. This crude material used for next step without purification.

To a stirred solution of cyanamide (3.0 mmol) in THF (5 mL) at 0°C N-ethyldiisopropylamine (DIPEA) (1.5 mmol) was added and after 5 min., (2*S*)-2-(4-bromophenoxy)-3-methylbutanoyl chloride (1.0 mmol) in THF (3 mL) was introduced dropwise and the reaction mixture was stirred at room temperature for 18 h. After completion of the reaction, the crude product was purified by chromatography on silica gel (0-10% EtOAc/ hexane) to afford (2*S*)-2-(4-bromophenoxy)-N-cyano-3-methylbutanamide (0.23 g, 73% yield) as a colourless oil.

¹H NMR (300 MHz, MeOD) δ 7.37 (d, 2H); 6.83 (d, 2H); 4.17 (d, 1H); 2.28-2.11 (m, 1H); 1.06 (d, 6H).

ES-MS: 296 [M-1].

### (2S)-2-(4-Bromophenoxy)-N-cyano-3-methyl-butanamide, sodium salt

To a stirred solution of (*S*)-2-(4-bromophenoxy)-*N*-cyano-3-methylbutanamide (1.0 mmol) in CH₃CN: H₂O (3:1) at room temperature, solid NaHCO₃ (1.1 equiv.) was added and stirred resulting mixture at room temperature for 30 min. After completion of the reaction, volatiles were removed and water (10 mL) was added. The aqueous layer was washed with dichloromethane (2 × 50 mL) to remove any impurities. The aqueous layer was separated and evaporated *in vacuo* to provide the sodium salt which was dried under vacuum for 18 h to provide the desired product (0.236 g, 95 % yield).

¹H NMR (300 MHz, MeOD) δ 7.36 (d, 2H); 6.84 (d, 2H); 4.13 (d, 1H); 2.27-2.12 (m, 1H); 1.07 (d, 6H).

ES-MS: 296 [M-1].

HPLC Retention Time: 12.99 min.

### Example 4: 5-[(1S)-1-(4-bromo-2-fluorophenoxy)ethyl]-2H-tetrazole

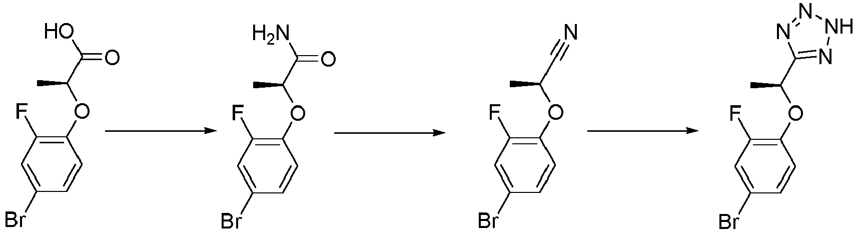

### (2S)-2-(4-bromo-2-fluorophenoxy)propanamide

Hexafluorophosphate azabenzotriazole tetramethyl uronium (HATU) (12.19 g, 32.1 mmol) was added to a stirred suspension of (2S)-2-(4-bromo-2-fluorophenoxy)propanoic acid (7.40 g, 26.7 mmol), ammonium chloride (7.15 g, 134 mmol) and triethylamine (14.90 mL, 107 mmol) in DMF (70 mL, 904 mmol). After 30 min., further ammonium chloride (3.6 g) followed by triethylamine (7.5mL) then HATU (6.1g) were introduced. After a further 45 min. the mixture was added to water (700mL), stirred for 30 min. and the resultant precipitate was filtered, washed with water (3 × 20mL) and dried *in vacuo* at 50°C overnight to provide the desired compound as a solid (4.624g, 17.64 mmol, 66 % yield).

¹H NMR (500 MHz, DMSO-d⁶) δ 7.55 (dd, J = 10.8, 2.4 Hz, 2H), 7.36 - 7.28 (m, 2H), 6.96 (t, J = 8.9 Hz, 1H), 4.67 (q, J = 6.7 Hz, 1H), 1.46 (d, J = 6.7 Hz, 3H).
LCMS method 1: m/z 262/264 (M+H)+ (ES+); at 1.10 min

### (2S)-2-(4-bromo-2-fluorophenoxy)propanenitrile

Trifluoroacetic anhydride (4.23 mL, 29.9 mmol) was added dropwise over 5 min. to a stirred solution of (2*S*)-2-(4-bromo-2-fluorophenoxy)propanamide (6.67 g, 24.94 mmol) and pyridine (4.84 mL, 59.9 mmol) in anhydrous THF (100 mL, 1220 mmol) at 0°C (using an ice/ brine bath) under nitrogen. After 60 min. at 0°C the mixture was quenched by addition of sodium bicarbonate solution (200mL) and extracted with ethyl acetate (200 mL). The organic layer was washed with 0.5 M hydrochloric acid (200mL) then brine (100 mL), dried (MgSO₄) and evaporated *in vacuo* to afford (2*S*)-2-(4-bromo-2-fluorophenoxy)propanenitrile as an oil (5.48g, 21.80 mmol, 87% yield)
¹H NMR (500 MHz, DMSO-d⁶) δ 7.64 (dd, J = 10.7, 2.3 Hz, 1H), 7.44 (ddd, J = 8.8, 2.4, 1.5 Hz, 1H), 7.34 (t, J = 8.8 Hz, 1H), 5.50 (q, J = 6.7 Hz, 1H), 1.72 (d, J = 6.7 Hz, 3H). LCMS method 1: m/z no mass ion observed at 1.48 min

### 5-[(1S)-1-(4-bromo-2-fluorophenoxy)ethyl]-2H-tetrazole, sodium salt

Trimethylsilyl azide (3.56 mL, 26.8 mmol) was added to a stirred solution of (2*S*)-2-(4-bromo-2-fluorophenoxy)propanenitrile (1.685 g, 6.70 mmol) and dibutyltin oxide (1.834 g, 7.37 mmol) in toluene (67.0 mL) and the resulting solution was heated at 110°C for 2 h. and allowed to cool to room temperature. Methanol (5 mL) was added and the solvents were removed under reduced pressure. The residue was purified by column chromatography on silica gel (4 g cartridge) utilizing a 0 - 50% ethyl acetate in isohexane gradient elution. The resulting solid was dissolved in methanol (5 mL) and 1M sodium hydroxide solution (4.30 mL, 4.30 mmol) was added. The mixture was stirred for 10 min. and evaporated under reduced pressure; the residue was suspended in water (20 mL) and freeze dried to provide the title compound as an amorphous solid (1.329 g, 64%).

¹H NMR (500 MHz, DMSO-d⁶) δ 7.42 (dd, J = 10.9, 2.4 Hz, 1H), 7.27 (t, J = 9.0 Hz, 1H), 7.20 (ddd, J = 8.8, 2.4, 1.4 Hz, 1H), 5.64 (q, J = 6.5 Hz, 1H), 1.64 (d, J = 6.5 Hz, 3H).
LCMS method 2: m/z 287.015, 289.015 (M+H)⁺ (ES⁺); 284.938, 286.931 (M-H)⁻ (ES⁻), at 2.99 min

### Example 5: 3-[5-bromo-2-[(1S)-3-fluoro-1-(2H-tetrazol-5-yl)propoxy]phenyl]-isoxazole

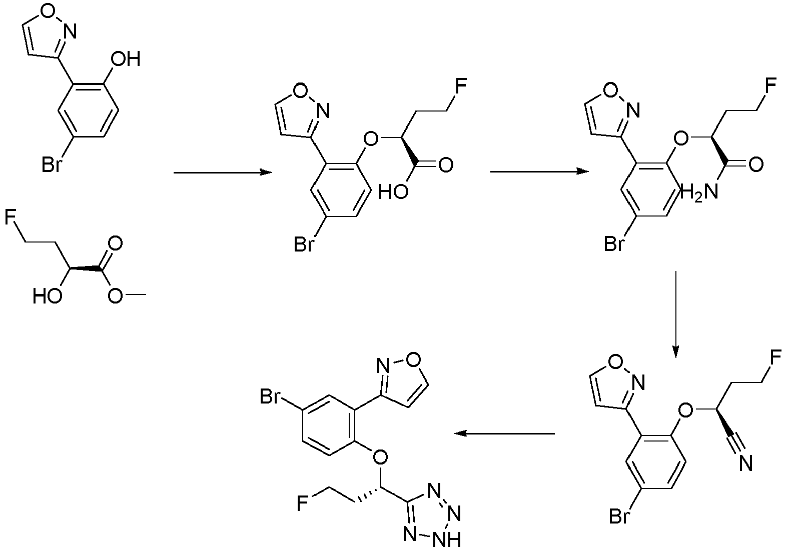

### (2S)-2-(4-bromo-2-(isoxazol-3-yl)phenoxy)-4-fluorobutanoic acid

Diisopropyl azodicarboxylate (DIAD) (0.119 mL, 0.612 mmol) was added to a stirred solution of 4-bromo-2-(isoxazol-3-yl)phenol (105 mg, 0.437 mmol), (*R*)-methyl 4-fluoro-2-hydroxybutanoate (65.5 mg, 0.481 mmol) and triphenylphosphine (161 mg, 0.612 mmol) in anhydrous THF (2 mL, 24.41 mmol). After 1 h. the mixture was cooled to 0°C and methanol (0.5 mL, 12.36 mmol) followed by 1M sodium hydroxide solution (0.547 mL, 0.547 mmol) were added. After a further 1 h. the mixture was diluted with water (20mL), acidified with 1M hydrochloric acid and extracted with ethyl acetate (20mL). The organic phase was extracted with 0.5M sodium hydroxide solution (20mL). The aqueous phase was washed with ethyl acetate (20mL), acidified with 1M hydrochloric acid and extracted with ethyl acetate (20mL). Organic extracts were dried (MgSO₄) and evaporated *in vacuo* to give (2S)-2-(4-bromo-2-(isoxazol-3-yl)phenoxy)-4-fluorobutanoic acid (143 mg, 0.395 mmol, 90% yield) as a gum.

¹H NMR (500 MHz, DMSO-d⁶) δ 13.29 (s, 1H), 9.02 (d, J = 1.7 Hz, 1H), 7.89 (d, J = 2.6 Hz, 1H), 7.64 (dd, J = 8.9, 2.6 Hz, 1H), 7.14 - 7.05 (m, 2H), 5.12 (dd, J = 7.3, 4.7 Hz, 1H), 4.71 - 4.47 (m, 2H), 2.44 - 2.25 (m, 2H).
LCMS method 1: m/z 344/346 (M+H)⁺ (ES⁺); 342/344 (M-H)⁻ (ES⁻), at 1.68 min

### (2S)-2-(4-bromo-2-(isoxazol-3-yl)phenoxy)-4-fluorobutanamide

HATU (177 mg, 0.464 mmol) was added to a stirred suspension of (2S)-2-(4-bromo-2-(isoxazol-3-yl)phenoxy)-4-fluorobutanoic acid (140 mg, 0.387 mmol), ammonium chloride (124 mg, 2.321 mmol) and triethylamine (0.216 mL, 1.548 mmol) in DMF (1mL, 12.91 mmol). After 16 h. extra HATU (177 mg, 0.464 mmol) was added. After a further 30 min., water (10mL) was added and the mixture stirred for 2 h. The resulting precipitate was filtered, washed with water (3 × 1mL) and dried *in vacuo* at 50°C overnight to provide the (2*S*)-2-(4-bromo-2-(isoxazol-3-yl)phenoxy)-4-fluorobutanamide (75 mg, 0.216 mmol, 56% yield) as a solid.

¹H NMR (500 MHz, DMSO-d⁶) δ 9.02 (d, J = 1.6 Hz, 1H), 7.89 (d, J = 2.6 Hz, 1H), 7.80 (s, 1H), 7.67 (dd, J = 8.9, 2.6 Hz, 1H), 7.48 (s, 1H), 7.17 (d, J = 1.6 Hz, 1H), 7.01 (d, J = 9.0 Hz, 1H), 4.82 (dd, J = 7.9, 4.6 Hz, 1H), 4.65 - 4.44 (m, 2H), 2.31 - 2.15 (m, 2H). LCMS method 1: m/z 343/345 (M+H)⁺ (ES⁺); 341/343 (M-H)⁻ (ES⁻), at 1.51 min

### (2S)-2-(4-bromo-2-(isoxazol-3-yl)phenoxy)-4-fluorobutanenitrile

Trifluoroacetic anhydride (0.036 mL, 0.252 mmol) was added to a stirred solution of (2*S*)-2-(4-bromo-2-(isoxazol-3-yl)phenoxy)-4-fluorobutanamide (72 mg, 0.210 mmol) and pyridine (0.041 mL, 0.504 mmol) in anhydrous THF (3.5 mL, 42.7 mmol) at 0°C under nitrogen. After 1 h. the mixture was diluted with water (20mL), acidified with 1M hydrochloric acid and extracted with ethyl acetate (20mL). Organic extracts were washed with sodium bicarbonate solution (20mL) then brine (10mL), dried (MgSO4) and evaporated *in vacuo* to provide (2S)-2-(4-bromo-2-(isoxazol-3-yl)phenoxy)-4-fluorobutanenitrile (67 mg, 0.202 mmol, 96% yield) as an oil.

¹H NMR (500 MHz, DMSO-d⁶) δ 9.06 (d, J = 1.7 Hz, 1H), 7.95 (d, J = 2.5 Hz, 1H), 7.80 (dd, J = 8.9, 2.6 Hz, 1H), 7.44 (d, J = 9.0 Hz, 1H), 7.01 (d, J = 1.7 Hz, 1H), 5.69 (t, J = 6.2 Hz, 1H), 4.78 - 4.59 (m, 2H), 2.58 - 2.49 (m, 2H) (partially obscured by DMSO peak).
LCMS method 1: m/z 325-327 (M+H)⁺ (ES⁺); 323/325 (M-H)⁻ (ES⁻), at 1.78 min

### 3-r5-bromo-2-[(1S)-3-fluoro-1-(2H-tetrazol-5-yl)propoxylphenyl1-isoxazole

Trimethylsilyl amide (0.040 mL, 0.300 mmol) was added to a stirred solution of (2S)-2-(4-bromo-2-(isoxazol-3-yl)phenoxy)-4-fluorobutanenitrile (65 mg, 0.200 mmol) and dibutyltin oxide (54.7 mg, 0.220 mmol) in anhydrous toluene (3.5 mL, 32.9 mmol) under nitrogen. The mixture was heated at 80°C for 16 h. then cooled to room temperature and evaporated *in vacuo.* The residue was purified by column chromatography (12g silica cartridge) with liquid loading in dichloromethane (2mL) followed by elution with a 10-100% gradient of 99/1 ethyl acetate/acetic acid in isohexane. Product-containing fractions were evaporated *in vacuo* and the residue triturated with isohexane/ethyl acetate (1/4, 0.5mL), solvent removed by pipette and the residue dried *in vacuo* at 50°C overnight to afford the title compound as a solid (48 mg, 0.130 mmol, 64.9 % yield)

¹H NMR (400 MHz, DMSO-d⁶) δ 9.03 (d, J = 1.7 Hz, 1H), 7.85 (d, J = 2.6 Hz, 1H), 7.60 (dd, J = 9.0, 2.6 Hz, 1H), 7.22 (d, J = 9.0 Hz, 1H), 7.09 (d, J = 1.7 Hz, 1H), 6.02 (dd, J = 7.6, 5.6 Hz, 1H), 4.72 - 4.45 (m, 2H), 2.65 - 2.38 (m, 2H) (obscured by DMSO).
LCMS method 2: m/z 368/370 (M+H)⁺ (ES⁺); 366/368 (M-H)⁻ (ES⁻), at 3.87 min

### Example 6: N-[3-[(1S)-1-(4-bromo-2-fluoro-phenoxy)ethyl]-1,2,4-thiadiazol-5-yl]acetamide

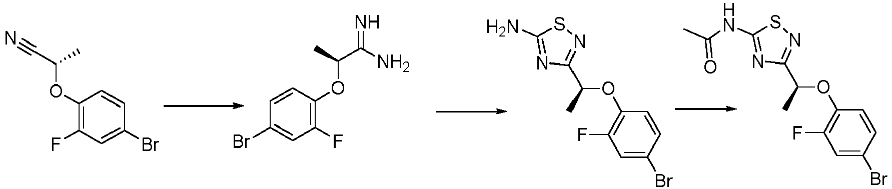

### (2S)-2-(4-bromo-2-fluorophenoxy)propanimidamide

Acetyl chloride (2.331 mL, 32.8 mmol) was added dropwise over 10 min. to anhydrous ethanol (20 mL, 343 mmol) at 20°C under nitrogen. The solution was heated to reflux for 30 min. and cooled to 0°C (using and ice/ brine bath) and (2*S*)-2-(4-bromo-2-fluorophenoxy)propanenitrile (1.00 g, 4.10 mmol) was introduced. After 30 min. the cooling bath was removed and the mixture stirred for a further 16 h. 7M Ammonia solution in methanol (7.02 mL, 49.2 mmol) was added and the mixture maintained at 20°C for 2 h. followed by heating at 50°C for 3 days. The mixture was evaporated *in vacuo* and the residue partitioned between ethyl acetate (100mL) and 0.5M hydrochloric acid (100mL). The aqueous phase was adjusted to pH >10 with solid sodium hydroxide and extracted with ethyl acetate (100mL). The organics were dried (MgSO₄) and evaporated *in vacuo* to provide (2S)-2-(4-bromo-2-fluorophenoxy)propanimidamide (260 mg, 0.637 mmol, 15.5% yield) as a gum.

¹H NMR (500 MHz, DMSO-d⁶) δ 7.57 (dd, J = 10.9, 2.4 Hz, 1H), 7.50 (s, br, 3H), 7.35 (dt, J = 8.9, 1.9 Hz, 1H), 7.03 (t, J = 8.9 Hz, 1H), 4.78 (q, J = 6.5 Hz, 1H), 1.51 (d, J = 6.5 Hz, 3H).
LCMS method 1: m/z 261/263 (M+H)⁺ (ES⁺); at 0.59 min

### 3-((1S)-1-(4-bromo-2-fluorophenoxy)ethyl)-1,2,4-thiadiazol-5-amine

Potassium thiocyanate (89 mg, 0.919 mmol) was added to a stirred solution of (2*S*)-2-(4-bromo-2-fluorophenoxy)propanimidamide (250 mg, 0.613 mmol) and triethylamine (0.256 mL, 1.838 mmol) in methanol (7 mL, 173 mmol) at room temperature. After 30 min. bromine (0.047 mL, 0.919 mmol) was added and the reaction mixture instantly decolourised. After a further 5 min. the mixture was diluted with water (35mL) and sodium sulphite (0.25g) added. The mixture was stirred for 16 h. then extracted with ethyl acetate (35mL). Organics were washed with brine (10mL), dried (MgSO₄) and evaporated *in vacuo.* The residue was subjected to column chromatography (40g silica cartridge) with liquid loading in dichloromethane/isohexane (1:2, 3mL) and elution with a 10-100% ethyl acetate in isohexane gradient to provide an oil that was dried *in vacuo* at 40°C overnight to give 3-((1*S*)-1-(4-bromo-2-fluorophenoxy)ethyl)-1,2,4-thiadiazol-5-amine (178 mg, 0.547 mmol, 89 % yield) as a white solid.

¹H NMR (500 MHz, DMSO-d⁶) δ 8.02 (s, 2H), 7.51 (dd, J = 10.9, 2.4 Hz, 1H), 7.27 (dt, J = 8.8, 1.9 Hz, 1H), 7.06 (t, J = 8.9 Hz, 1H), 5.37 (q, J = 6.4 Hz, 1H), 1.60 (d, J = 6.4 Hz, 3H).
LCMS method 1: m/z 318/320 (M+H)⁺ (ES⁺); 316/318 (M-H)⁻ (ES⁻), at 3.30 min

### N-[3-[(1S)-1-(4-bromo-2-fluoro-phenoxy)ethyl]-1,2,4-thiadiazol-5-yl]acetamide

Acetyl chloride (0.033 mL, 0.468 mmol) was added to a stirred solution of 3-((1*S*)-1-(4-bromo-2-fluorophenoxy)ethyl)-1,2,4-thiadiazol-5-amine (76 mg, 0.234 mmol) and triethylamine (0.072 mL, 0.515 mmol) in anhydrous dichloromethane (3 mL, 46.6 mmol) at room temperature under nitrogen. After 30 min. the mixture was diluted with ethyl acetate (20mL), washed with 1M hydrochloric acid (20mL), sodium bicarbonate solution (20mL) and brine (10mL), dried (MgSO₄) and evaporated *in vacuo.* The residue was subjected to column chromatography (24g silica cartridge) with liquid loading in dichloromethane/ isohexane (1:1, 4mL) followed by elution with a 10-100% gradient of ethyl acetate/acetic acid (99/1) in isohexane. The isolated product was twice further purified by column chromatography (12g silica cartridges) with liquid loading in dichloromethane/isohexane (1:2, 3mL) and elution with gradients of 10-70% then 10-50% ethyl acetate/acetic acid (99/1) in isohexane to provide the title compound (18 mg, 0.047 mmol, 20 % yield) as a gum.

¹H NMR (500 MHz, DMSO-d⁶) δ 13.04 (s, 1H), 7.52 (dd, J = 10.9, 2.4 Hz, 1H), 7.26 (ddd, J = 8.8, 2.4, 1.5 Hz, 1H), 7.06 (t, J = 9.0 Hz, 1H), 5.63 (q, J = 6.5 Hz, 1H), 2.22 (s, 3H), 1.67 (d, J = 6.5 Hz, 3H).

LCMS method 2: m/z 360/362 (M+H)⁺ (ES⁺); 358/360 (M-H)⁻ (ES⁻), at 3.95 min.

### Example 7: (2S)-2-(4-bromo-2-fluorophenoxy)-N-[1-(hydroxyimino)ethyl]-3-methylbutanamide

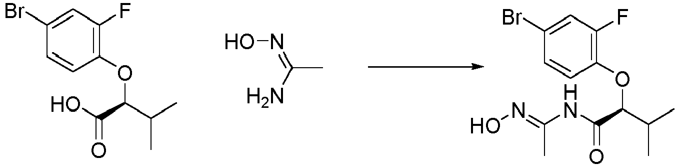

EDC (92 mg, 0.481 mmol) was added to a stirred suspension of (2*S*)-2-(4-bromo-2-fluoro-phenoxy)-3-methyl-butanoic acid (100 mg, 0.344 mmol), (*Z*)-*N*'-hydroxyacetimidamide (30.5 mg, 0.412 mmol) and DMAP (58.8 mg, 0.481 mmol) in DCM (22.10 µl, 0.344 mmol). After 20 hours, LCMS indicated reaction to be ca. 50% complete. Further EDC (92 mg, 0.481 mmol) was added and stirring continued for a futher 2 hours. The reaction solution was loaded directly onto a 24g silica cartridge which was eluted with a 10-100% ethyl acetate in iso-hexane gradient. Product containing fractions were evaporated in vacuo to an oil which was dried overnight in vacuo at 40°C to give the title compound (20 mg).

¹H NMR (500 MHz, DMSO-d⁶) δ 7.56 (dd, J = 10.9, 2.4 Hz, 1H), 7.32 (ddd, J = 8.8, 2.4, 1.5 Hz, 1H), 7.04 (t, J = 9.0 Hz, 1H), 6.49 (d, J = 31.5 Hz, 2H), 4.79 (d, J = 5.0 Hz, 1H), 2.38-2.25 (m, 1H), 1.75 (s, 3H), 1.01 (dd, J = 6.9, 5.2 Hz, 6H).

LCMS method 2: m/z 347.3/349.2 (M+H)⁺ (ES⁺); at 3.86 min.

### Example 8: (2S)-2-(4-bromo-2-fluorophenoxy)-N-(cyclopropanesulfonyl)-propanamide

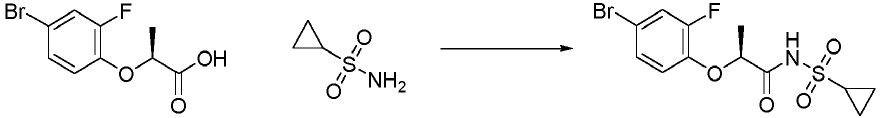

EDC (82 mg, 0.426 mmol) was added to a stirred solution of (2*S*)-2-(4-bromo-2-fluoro-phenoxy)propanoic acid (80 mg, 0.304 mmol), cyclopropanesulfonamide (40.5 mg, 0.335 mmol) and DMAP (52.0 mg, 0.426 mmol) in DCM (4 mL, 62.2 mmol). After 16 hours LCMS indicated completion of reaction. The mixture was loaded directly onto a 24g silica cartridge. The column was eluted with a 10-100% gradient of ethyl acetate containing 1% acetic acid in isohexane. Product-containing fractions were evaporated in vacuo to an oil. The oil was dried overnight in vacuo at 40°C to give the title compound (64mg).

¹H NMR (500 MHz, DMSO-d⁶) δ 12.11 (s, 1H), 7.59 (dd, J = 10.9, 2.4 Hz, 1H), 7.37 (ddd, J = 8.8, 2.4, 1.5 Hz, 1H), 6.93 (t, J = 8.9 Hz, 1H), 4.89 (q, J = 6.7 Hz, 1H), 2.95 (tt, J = 7.7, 5.1 Hz, 1H), 1.53 (d, J = 6.6 Hz, 3H), 1.07 (dddd, J = 12.5, 6.0, 3.5, 1.2 Hz, 4H).

LCMS method 2: m/z 364.1/366.1 (M-H)⁻ (ES⁻); at 3.52 min.

### Example 9: Electrophysiological measurement of compound inhibition of CIC-1 in rat muscle

The investigatory goal of these experiments was to evaluate whether compounds inhibit CIC-1 channels in native tissue of rat skeletal muscle fibres. Apparent CIC-1 affinity was reported by the concentration of compound at which 50% of the compound's full inhibition of CIC-1 was observed (EC₅₀).

CIC-1 Cl⁻ ion channels generate around 80% of the total membrane conductance (Gₘ) in resting skeletal muscle fibres of most animals including rat and human (Bretag, A H. Muscle chloride channels. Physiological Reviews, **1987,** *67*, 618-724). Other ion channels that contribute to Gₘ can therefore be considered negligible, and it is possible to evaluate whether a compound inhibits CIC-1 in rat muscle by comparing Gₘ measurements before and after exposure to a compound. CIC-1 inhibition would in such recordings be reflected by a reduction of Gₘ.

Experimentally, Gₘ was measured in individual fibres of whole rat soleus muscles using a three micro-electrodes technique described in this example and in full detail elsewhere (Riisager *et al.,* Determination of cable parameters in skeletal muscle fibres during repetitive firing of action potentials. Journal of Physiology, **2014,** *592*, 4417-4429). Briefly, intact rat soleus muscles were dissected out from 12-14 week old Wistar rats and placed in an experimental chamber that was perfused with a standard Krebs Ringer solution containing 122 mM NaCl, 25 mM NaHCOs, 2.8 mM KCI, 1.2 mM KH₂PO₄, 1.2 mM MgSO₄, 1.3 mM CaCl₂, 5.0 mM D-glucose. During experiments, the solution was kept at approx. 30°C and continuously equilibrated with a mixture of 95% O₂ and 5% CO₂, pH -7.4. The experimental chamber was placed in Nikon upright microscope that was used to visualize individual muscle fibres and the three electrodes (glass pipettes filled with 2 M potassium citrate). For Gₘ measurements, the electrodes were inserted into the same fibre with known inter-electrode distances of 0.35 - 0.5 mm (V1-V2, X1) and 1.1-1.5 mm (V1-V3, X3) (Figure 1A). The membrane potential of the impaled muscle fibre was recorded by all electrodes. Two of the electrodes were furthermore used to inject 50 ms current pulses of -30 nA. Given the positions of the electrodes, three different inter-electrode distances could be identified (X1-X2, X1-X3, X2-X3) and hence the membrane potential responses to the current injections could be obtained at three distances from the point of current injection. The steady state voltage deflection at each distance was divided by the magnitude of current injected (-30 nA) and the resulting transfer resistances were plotted against inter-electrode distance and the data was fitted to a mono-exponential function from which Gₘ could be calculated using linear cable theory (Figure 1B).

To establish a dose response relationship, Gₘ was first determined in 10 muscle fibres in the absence of compound and then at four increasing compound concentrations with Gₘ determinations in 5-10 fibres at each concentration. The average Gₘ values at each concentration were plotted against compound concentration and the data was fitted to sigmoidal function to obtain an EC₅₀ value (Figure 1C). Table 2 shows the EC₅₀ values for a range of compounds with n values referring to number of experiments that each reflect recordings from around 50 fibres.

**Table 2: Inhibition of CIC-1 ion channel using compounds of the invention**

| **Compound investigated** | **EC₅₀ (µM)** |
|---|---|
| Compound A-4 | 2.2 |
| Compound A-6 | 2.0 |
| Compound B-5 | 19.6 |
| Compound C-22 | 7.8 |
| Compound C-64 | 3.1 |
| Compound C-70 | 4.6 |
| Compound C-74 | 3.4 |
| Compound C-75 | 2.0 |
| Compound C-78 | 3.1 |
| Compound C-80 | 2.6 |
| Compound C-82 | 9.7 |
| Compound C-83 | 3.5 |
| Compound C-84 | 11.5 |
| Compound C-85 | 10.1 |
| Compound E-7 | 6.0 |
| Compound E-8 | 6.6 |
| Compound E-15 | 19.9 |

### Example 10: Measurement of force in an in vitro model

The current disclosure relates to compounds that inhibit CIC-1 ion channels and increase muscle excitability and thereby improve muscle function in clinical conditions where muscle activation is failing. Such conditions result in loss of contractile function of skeletal muscle, weakness and excessive fatigue. In this series of experiments the compounds were tested for their ability to restore contractile function of isolated rat muscle when the neuromuscular transmission had been compromised akin to neuromuscular disorders.

Experimentally, soleus muscles from 4-5 week old rats were isolated with the motor nerve remaining attached. The nerve-muscle preparations were mounted in experimental setups that enabled electrical stimulation of the motor nerve. Stimulation of the motor nerve led to activation of the muscle fibres and ensuing force production that was recorded. The nerve-muscle preparations were also in these experiments incubated in the standard Krebs Ringer (see example 5) and the solution was heated to 30°C and continuously equilibrated with a mixture of 95% O₂ and 5% CO₂, pH ~7.4.

After mounting the nerve-muscle preparation in the experimental setup, the contractile function of the muscle was initially assessed under the control conditions (Figure 2A). Sub-maximal concentration of tubocurarine (115 nM), an acetylcholine receptors antagonist, was then added to the experimental bath to impose partial inhibition of the ability of the motor nerve to activate the muscle fibres. The experimental condition mimics the failing neuromuscular transmission in a range of neuromuscular disorders. After addition of tubocurarine the contractile force declined over the next 90 mins to 10-50 % of the control force. 50 µM) of the test compound was then added and the contractile force recovered despite the continued presence of tubocurarine. To quantify the ability of the compound to restore force the percentage of the initial force that was restored was determined after 40 mins of compound exposure (Figure 2B) and the point increase is reported in Table 3.

**Table 3: Percentage increase of initial force that was restored**

| **Compound investigated** | **Point increase (%)** |
|---|---|
| Compound A-5 | 30% |
| Compound B-6 | 15% |
| Compound B-7 | 37% |
| Compound C-3 | 29% |
| Compound C-8 | 45% |
| Compound C-12 | 18% |
| Compound C-16 | 23% |
| Compound C-22 | 22% |
| Compound C-31 | 56% |
| Compound C-39 | 49% |
| Compound C-40 | 24% |
| Compound C-43 | 20% |
| Compound C-64 | 24% |
| Compound C-65 | 26% |
| Compound C-66 | 21% |
| Compound C-67 | 23% |
| Compound C-71 | 25% |
| Compound C-74 | 22% |
| Compound C-75 | 36% |
| Compound C-78 | 41% |
| Compound C-80 | 47% |
| Compound C-85 | 45% |
| Compound E-1 | 21% |
| Compound E-6 | 21% |
| Compound E-8 | 34% |
| Compound E-12 | 21% |

In conclusion, this example demonstrates that the compounds of the present disclosure are able to increase muscle excitability and thereby improve muscle function in clinical conditions.

### Example 11: Screening of compounds on the human isoform of CIC-1 expressed in CHO cells using automated patch-clamp

The investigatory goal of these experiments was to evaluate how compounds affect the open probability and current amplitude of human CIC-1 channels expressed in CHO cells. Experiments were performed using an automated patch clamp system that allowed high throughput testing of whole cell patches together with both intracellular and extracellular addition of compound.

### Automated voltage clamp measurements

Automated whole cell patch clamp experiments were performed with the Qpatch 16 system (Sophion Bioscience, Ballerup, Denmark) at room temperature. Data acquisition and analysis were performed in the Qassay software (ver. 5.6, Odense).

### Voltage protocol and analysis of whole cell CIC-1 currents

To evoke CIC-1 currents in whole cell patches, the membrane potential was initially stepped from a holding potential of -30 mV to +60 mV for 100 ms and then to various test voltages (sweeps) ranging from +120 mV to -140 mV in steps of 20 mV for 300 ms. To obtain tail currents, the membrane potential was stepped to -100 mV after each test voltage for 300 ms and then relaxed to -30 mV for 2 sec between sweeps (Figure 3).

I/V relationships for whole cell instant and steady state current amplitudes were obtained by plotting average current densities at the beginning and at the end of the 300 ms step against the membrane potential (Figure 4).

In order to determine the relative overall open probability (P₀), the instantaneous tail currents were normalized to the maximal tail current obtained following the most positive voltage step and plotted against the test voltage. Plots of normalized tail currents from each whole cell patch were then fitted to a Boltzmann function allowing determination of half activation voltages (V_{1/2}, Figure 5).

### Solutions

For automated patch clamp experiments extracellular solutions contained: 2 mM CaCl₂, 1 mM MgCl₂, 10 mM HEPES, 4 mM KCI, 145 mM NaCl, 10 mM Glucose, pH adjusted to 7.4 with NaOH (2 M). Osmolality adjusted to -320 using sucrose.

Intracellular solutions contained: 80 mM CsF, 60 mM CsCI, 5/1 mM KOH/EGTA, 10 mM HEPES, 10 mM NaCl, pH adjusted to 7.2 with NaOH (2 M). Osmolality adjusted to -320 mOsm using sucrose.

### Cell line information:

Cells used in patch clamp experiments were Chinese hamster ovary cells (CHO) constitutively expressing human CIC-1 channels. The amino acid sequence encoded by the cDNA used to create this cell line was identical to the translated sequence for GenBank accession number NM_000083.2. Cells were produced by Charles River (Catalogue CT6175, Cleveland OH, USA) in a cryopreserved format. Experiments were performed on the cells directly after thawing (3 × 10⁶ cells used in each experiment).

### Test protocol

To evaluate the compound effect on CIC-1, when applied directly to the intracellular side of the cell membrane, the half activation voltage, V_{1/2}, was determined from whole cell patches with compound added to the intracellular solution and then compared to V_{1/2} determined from control cell patches with only vehicle added to the intracellular solution. Additionally, the effect of extracellular added compound was evaluated by determine V_{1/2} and steady state current amplitudes before and after exchanging the extracellular solution to contain compound.

The difference in half activation voltage of CIC-1 channels, ΔV_{1/2}, was determined as the difference between the cell patches treated intracellularly with compound and control cells patches and is reported in Table 4 below. A positive shift in ΔV_{1/2} is reflecting CIC-1 channel inhibition by the tested compound. P-values of <0.05 is considered significant.

**Table 4: Percentage increase of initial force that was restored**

| **Compound investigated** | **ΔV1/2 (mV)** | **P-value** |
|---|---|---|
| Compound B-2 | 6.2 | 0.04 |
| Compound B-4 | 21.2 | <0.01 |
| Compound B-5 | 26.6 | <0.01 |
| Compound C-8 | 10.3 | <0.01 |
| Compound C-80 | 9.9 | 0.02 |
| Compound E-5 | 13.9 | <0.01 |
| Compound E-8 | 38.4 | <0.01 |

### Example 12: Measurement of In Situ Muscle Contractile Characteristics

Isometric hindlimb force was measured in 12-week old female Lewis rats in the presence and absence of compound.

Rats were placed under anesthesia with isoflurane (2-4%), intubated and subsequently connected to a micro ventilator (Microvent 1, Hallowell EMC, US). Two stimulation electrodes were inserted through the skin to stimulate the sciatic nerve. A small incision was made proximal to the ankle, to expose the Achilles tendon, which was tied by cotton string, and connected to a force transducer (Fort250, World Precision Instruments) with adjustable position (Vernier control). The Achilles tendon was then cut distal to the attached cotton string. The rat was placed on a heated pad, and to prevent movement artefacts from contraction of the ankle dorsiflexors, the foot was fixated by tape on a footplate.

Muscle contractile properties were assessed by applying an electrical current (under supramaximal voltage conditions) to the nerve and recording the force generated by the muscle. The muscle was stretched until maximal force was obtained, when assessed by 2 Hz stimulation. Isometric force was measured every 30 seconds at 12 Hz (Twitch), 10 pulses, and at every 5 minutes at 80 Hz (Tetanic) for 1 second (80 pulses). This stimulation pattern was employed throughout the experiment, expect in few cases where 80 Hz stimulation was replaced by 12 Hz (10 pulses). Neuromuscular transmission was partially inhibited by constant infusion of Cisatracurium (Nimbex, GlaxoSmithKline) at a concentration of 0.1 mg/kg at an adjustable infusion speed, adjusted individually for each animal to obtain a level of inhibition of ca. 50% of the forced generated at 12 Hz stimulation on the 4^{th} pulse. When the level of neuromuscular inhibition was stable, the test article was injected i.v. at the chosen concentration. The effect of test article was assessed on its ability to increase force generated from the stimulation pattern applied. The effect was assessed in the ability to increase force per se (tetanic, 80 Hz, stimulation), and the ratio between individual twitch peaks (12 Hz stimulation). The effect was monitored for at least 1 hour after injection of test article. In addition, the time from injection of test article to maximal effect on force (both twitch and tetanic) was noted and the time for the effect to disappear (return to baseline), if possible. When appropriate the infusion of neuromuscular blocking agent was ceased, with the stimulation pattern continued, and the return of force to control levels was monitored. Animals were sacrificed by cervical dislocation while still fully sedated.

Compound E-8 was dosed 21.6 mg/kg i.v. resulting in an increase in tetanic force of 11%. This demonstrates that compounds of the invention, such as Compounds E-8, can restore force to muscles *in vivo* which have been partially inhibited by a neuromuscular blocker.

### Example 13: Synthesis of (2S)-2-[4-bromo-2-(1,1-difluoropropyl)-5-fluorophenoxy]-N-cyanopropanamide

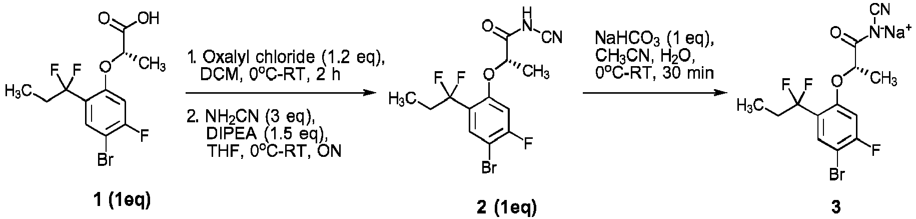

### Step 1: Synthesis of (S)-2-[4-bromo-2-(1,1-difluoropropyl)-5-fluorophenoxyl-1-(cyanoamino)-1-propanone 2

Compound **1** can be prepared according to the method described in Example 15.

To a stirred solution of (S)-2-[4-bromo-2-(1,1-difluoropropyl)-5-fluorophenoxy]propionic acid (210 mg, 0.617 mmol) in DCM at 0°C oxalyl chloride (0.063 ml, 0.741 mmol) was added and stirred resulting mixture at room temperature for 2h. After 2 h, remove the volatiles and dried under vacuum to get the desired product as a colorless solid (0.225 mg, quantitative Yield). This crude material was used for next step without purification. To a stirred solution of cyanamide (75.16 mg, 1.87 mmol) in THF at 0 °C, DIPEA (0.163 ml, 0.938 mmol) was added and after 5 min. (S)-2-[4-Bromo-2-(1,1-difluoropropyl)-5-fluorophenoxy]propionyl chloride (0.225 mg, 0.625 mmol) in THF was added dropwise and stirred the resulting mixture at room temperature overnight. After removal of the solvent, the residue was passed through a short silica gel column (hexane-EtOAc, 25:1, 10:1) to give a desired product (37 mg, 16.24%) as a light-yellow oil.

¹H NMR (300 MHz, CDCl₃) δ 9.71-9.51 (br, 1H), 7.59 (d, 1H), 6.73 (d, 1H), 4.56 (q, 1H), 2.48-2.19 (m, 2H), 1.48 (d, 3H), 0.90 (t, 3H).

¹⁹F NMR (300 MHz, CDCl₃) δ -93.92, -96.95, -101.86.

### Step 2: Synthesis of Sodium salt of (2S)-2-f4-bromo-2-(1,1-difluoropropyl)-5-fluorophenoxyl-N-cyanopropanamide 3

To a stirred solution of (S)-2-[4-bromo-2-(1,1-difluoropropyl)-5-fluorophenoxy]-1-(cyanoamino)-1-propanone (37 mg, 0.101 mmol) in acetonitrile (2 mL) at 0°C was added NaHCO₃ (8.5 mg, 0.101 mmol) in 1 mL of water. The reaction mixture was stirred at room temperature for 30 min, acetonitrile was removed by rotavap and the water layer extracted with DCM (20 mL) to remove unreacted impurities. The aqueous phase was lyophilized to give the desired product as a light yellow solid (17 mg, 43%).

¹H NMR (300 MHz, CD₃OD) δ 7.49 (dd, 1H), 6.68 (d, 1H), 4.46 (q, 1H), 2.48-2.14 (m, 2H), 1.43 (d, 3H), 0.81 (t, 3H).
¹⁹F NMR (300 MHz, CD₃OD) δ -93.80, -99.46, -105.02.
ES-MS: 363.2 [M-1].
HPLC Retention Time: 10.55 min
Chiral HPLC Retention time: 10.85 min, e.e. 93.7%

### Example 14: Synthesis of (2S)-2-[4-bromo-2-(1,1-difluoroethyl)phenoxy]-N-cyanopropanamide

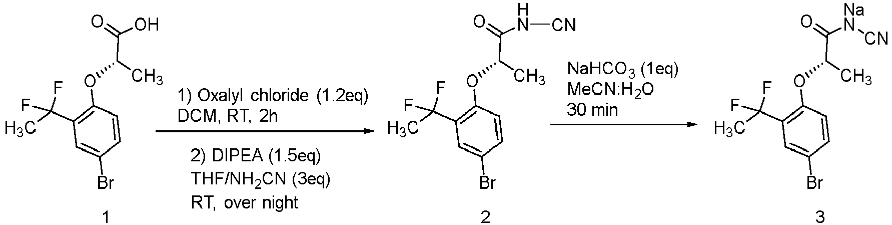

Compound 1 can be prepared according to the method described in Example 15.

### Step 1: Synthesis of (S)-2-(4-bromo-2-(1,1-difluoroethyl)phenoxy)-N-cyanopropanamide

To a stirred solution of (*S*)-2-(4-bromo-2-(1,1-difluoroethyl)phenoxy)propanoic acid (445 mg, 1.44 mmol) in DCM at 0°C oxalyl chloride (0.148 ml, 1.73 mmol) was added and stirred resulting mixture at room temperature for 2h. After 2 h, remove the volatiles and dried under vacuum to get the desired product as a colourless solid (0.471 mg, quantitative Yield). This crude material used for next step without purification.

To a stirred solution of cyanamide (181 mg, 4.31 mmol) in THF at 0 °C, DIPEA (0.37 ml, 2.16 mmol) was added and after 5 min, (S)-2-(4-bromo-2-(1,1-difluoroethyl)phenoxy)propanoyl chloride (471 mg, 1.44 mmol) in THF was added drop wise and stirred the resulting mixture at room temperature overnight. After removal of the solvent, the residue was passed through a short silica gel column (hexane-EtOAc, 25:1, 10:1) to give desired product (39 mg, 8.13%) as a light-yellow oil.

¹H NMR (300 MHz, CDCl₃) δ 7.56 (d, 1H); 7.37 (dd, 1H); 6.71 (d, 1H); 6.59 (br, 1H), 4.66 (q, 1H); 2.00 (t, 3H); 1.55 (d, 3H).

¹⁹F NMR (300 MHz, CDCl₃) δ -85.76, -87.94.

### Step 2: Synthesis of Sodium salt of (2S)-2-[4-bromo-2-(1,1-difluoroethyl)phenoxy]-N-cyanopropanamide

To a stirred solution of (*S*)-2-(4-bromo-2-(1,1-difluoroethyl)phenoxy)-*N*-cyano propanamide (39 mg, 0.12 mmol) in acetonitrile (2 mL) at 0°C was added NaHCO₃ (9.8 mg, 0.117 mmol) in 1 mL of water. The reaction mixture was stirred at room temperature for 30 min., acetonitrile was removed by rotavap and the water layer extracted with DCM (20 mL) to remove unreacted impurities. Water was lyophilized to get desired product as a light yellow solid (26 mg, 62.5%).

¹H NMR (300 MHz, CD₃OD) δ 7.43 (d, 1H); 7.36 (dd, 1H); 6.73 (d, 1H); 4.47 (q, 1H); 1.94 (t, 3H); 1.44 (d, 3H).

¹⁹F NMR (300 MHz, CD₃OD) δ -90.07, -86.60; ES-MS: 332 [M-1].

HPLC Retention Time: 11.15 min.

### Example 15: Synthesis of (S)-2-(4-bromo-2-(1,1-difluoropropyl)phenoxy)-N-(cyclopropylsulfonyl)propanamide

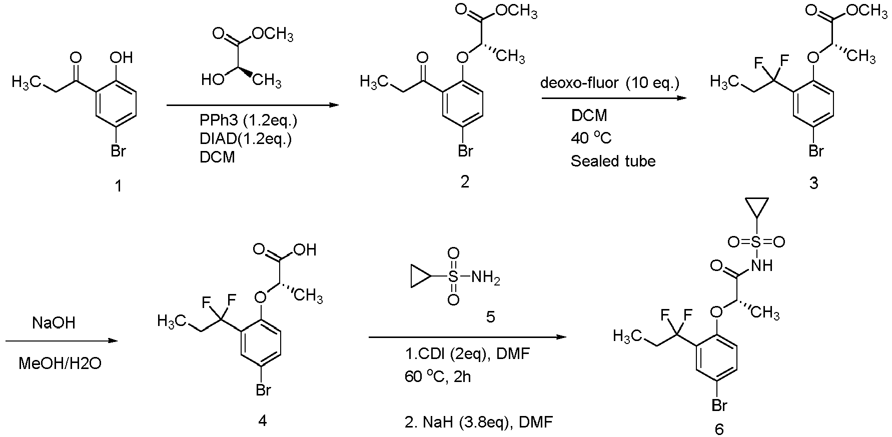

### Step 1: Synthesis of (S)-methyl 2-(4-bromo-2-propionylphenoxy)propanoate 2

DIAD (15.9 g, 78.6 mmol) was slowly added to a stirred solution of 1-(5-bromo-2-hydroxyphenyl)propan-1-one (15.0g, 65.5 mmol), (*R*)-methyl 2-hydroxypropanoate (7.5 g, 72.0 mmol) and triphenylphosphine (20.6g, 78.6 mmol) in 100 mL of dichloromethane previously cooled to 0°C. The ice bath was then removed, and the reaction mixture was stirred at room temperature overnight, then concentrated under reduced pressure. After removal of the solvent, the residue was purified by silica gel column chromatography using 0-20%ethyl acetate/hexane to get desired product **2** (17.3 g, 84.14%). as a light-yellow oil.

¹H NMR (300 MHz, CDCl₃) δ 7.84 (d, 1H), 7.52 (dd, 1H), 6.72 (d, 1H), 4.91 (q, 1H), 3.81 (s, 3H), 3.18 - 3.07 (m, 2H), 1.73 (d, 3H), 1.23 (t, 3H).

### Step 2: Synthesis of (S)-methyl 2-(4-bromo-2-(1,1-difluoropropyl)phenoxy)propanoate 3

To a solution of (*S*)-methyl 2-(4-bromo-2-propionylphenoxy)propanoate (10.0 g, 31.8 mmol) in dry CH₂Cl₂ (30 mL) in a seal tube was added deoxo-fluor (58.7 ml, 318 mmol) and then flushed with argon and the cap sealed. The resulting mixture was stirred at 40°C for 5-7 days. The reaction mixture was poured into ice-cold water (50 mL) and saturated aqueous sodium carbonate was added cautiously, and the mixture stirred for 20 to 30 min. The aqueous layer was extracted with ethyl acetate (2x75 mL). The combined organic extracts were washed with brine (25 mL) and dried (Na₂SO₄). After removal of the solvent, the residue was purified by silica gel column chromatography using 0-20% ethyl acetate/hexane to get desired product **3** (9.4 g, 88.0%) as a light-yellow oil.

¹H NMR (300 MHz, CDCl₃) δ 7.71 - 7.68 (m, 1H), 7.48 (dd, 1H), 6.72 (d, 1H), 4.85 (q, 1H), 3.80 (s, 3H), 2.57 - 2.37 (m, 2H), 1.69 (d, 3H), 0.99 (t, 3H).

¹⁹F NMR (300 MHz, CDCl₃) δ -93.57, -99.02 ppm.

### Step 3: Synthesis of (S)-2-(4-bromo-2-(1,1-difluoropropyl)phenoxy)propanoic acid 4

To a stirred solution of (*S*)-methyl 2-(4-bromo-2-(1,1-difluoropropyl)phenoxy)propanoate (8.0 g, 24.0 mmol) in MeOH/H₂O (50/10 mL) was added NaOH (1.15 g, 28.8 mmol) at 0 °C. The reaction mixture was stirred at room temperature for 1-2 h. The reaction mixture was concentrated. 10 mL of H₂O was added and the mixture cooled to 0 °C then acidified with 1M HCl to pH 2. The product was extracted with EtOAc (3x25 mL), and the organic phase washed with H₂O (25 ml), brine (25 mL), dried over Na2SO4, filtered and concentrated to get compound **4** (6.8 g, 88.0%) as a white solid.

¹H NMR (300 MHz, CDCl₃) δ 8.04 -7.71 (br s, 1H), 7.72 - 7.68 (m, 1H), 7.51 (dd, 1H), 6.76 (d, 1H), 4.88 (q, 1H), 2.54 - 2.32 (m, 2H), 1.74 (d, 3H), 0.99 (t, 3H).

¹⁹F NMR (300 MHz, CDCl₃) δ -94.32, -98.43 ppm.

### Step 4: Synthesis of (S)-2-(4-bromo-2-(1,1-difluoropropyl)phenoxy)-N-(cyclopropylsulfonyl)propanamide 6

(S)-2-(4-Bromo-2-(1,1-difluoropropyl)phenoxy)propanoic acid (0.2 g, 0.62 mmol) and 1,1'-carbonyldiimidazole (0.201 g, 1.4 mmol) in 3 mL of DMF was heated at 60°C for 2 h. The reaction mixture was cooled and cyclopropanesulfonamide (0.376 g, 3.1 mmol) was added followed by sodium hydride (0.094 g, 2.36 mmol). The reaction was stirred at room temperature for 1 h, cooled to 0 °C and quenched with water (5 mL). The aqueous layer was acidified with 6N HCl to pH~2. The product was extracted with ethyl acetate (2x20 mL), and the organic phase washed with water (2x20 mL), brine (10 mL), dried over sodium sulphate and concentrated under reduced pressure. The residue was passed through a silica gel column (MeOH/CH₂Cl₂, 0-2%) to give desired product **6** (0.15 g, 57%) as a colourless gum.

¹H NMR (300 MHz, CDCl₃) δ 8.80 (s, 1H), 7.66 - -7.62(m, 1H), 7.55 - 7.49 (m, 1H), 6.80 (m, 1H), 4.86 (q, 1H), 2.93 - 2.84 (m, 1H), 2.44 - 2.14 (m, 2H), 1.67 (d, 3H), 1.48 -1.38 (m, 1H), 1.31 - 1.21 (m, 1H), 1.16 - 0.96 (m, 5H).

¹⁹F NMR (300 MHz, CDCl₃) δ -92.98, -99.60 ppm.

LC/MS System: MS (ES-): m/z 424.2 (M-H).

HPLC method, retention time: 12.637 min.

### Example 16: Synthesis of 5-[(1S)-1-[4-bromo-2-(1,1-difluoroethyl)phenoxy]ethyl]-1H-1,2,3,4-tetrazole

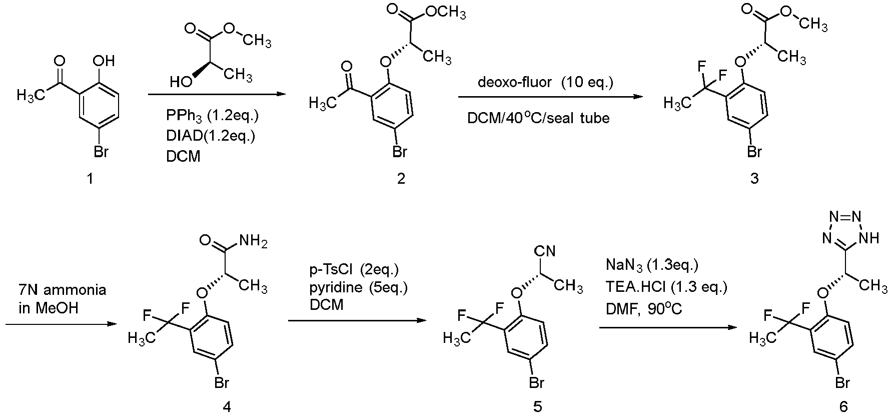

### Step 1: Synthesis of (S)-methyl 2-(2-acetyl-4-bromophenoxy) propanoate

DIAD (6.96 mL) was added slowly to a stirred solution of (*R*)-methyl 2-hydroxypropanoate (3.07 g, 29.44 mmol), 1-(5-bromo-2-hydroxy phenyl)ethanone (6.33 g, 29.44 mmol) and triphenylphosphine (9.28 g, 35.38 mmol) in 150 mL DCM previously cooled to 0°C. The ice bath was then removed, and the mixture stirred at room temperature overnight and then concentrated under reduced pressure. After removal of the solvent, the residue was passed through a short silica gel column (hexane-EtOAc, 25:1, 10:1) to give desired product (7.9 g, 88.9%) as a light-yellow oil.
¹H NMR (300 MHz, CDCl₃) δ 7.83 (d, 1H); 7.48(dd, 1H); 6.67 (d, 1H); 4.87 (q, 1H); 3.75 (s, 3H); 2.67 (s, 3H); 1.68 (d, 3H)

### Step 2: Synthesis of (S)-methyl 2-(4-bromo-2-(1,1-difluoroethyl)phenoxy) propanoate

To a solution of (S)-methyl 2-(2-acetyl-4-bromophenoxy) propanoate (3.99 g, 13.3 mmol) in dry CH₂Cl₂ (40 mL) in a seal tube was added deoxofluor (24.5 ml, 133 mmol, 10 eq) and then flushed with argon and the cap sealed. The resulting mixture was stirred at 40°C for 7-8 days until the starting material was completely consumed. After completion of the reaction, the reaction mixture was poured into ice-cold sat. aqueous sodium bicarbonate and the mixture was stirred for 20 to 30 min. The aqueous layer was extracted with ethyl acetate (2x100 mL). The combined organic extracts were washed with brine then dried (Na₂SO₄). After removal of the solvent, the residue was passed through a short silica gel column (hexane-EtOAc, 25:1, 5:1) to give the desired product (3.55 g, 82.9%) as a colourless oil.

¹H NMR (300 MHz, CDCl₃) δ 7.65 (d, 1H); 7.42 (dd, 1H); 6.66 (d, 1H); 4.79 (q, 1H); 3.75 (s, 3H); 2.04 (d, 3H); 1.64 (d, 3H).

F¹⁹ NMR (300 MHz, CDCl₃) δ -85.67, -89.11.

### Step 3: Synthesis of (S)-2-(4-bromo-2-(1,1-difluoroethyl)phenoxy)propanamide

The mixture of (*S*)-methyl 2-(4-bromo-2-(1,1-difluoroethyl)phenoxy) propanoate (0.452 g, 5.013 mmol) in 7N ammonia in MeOH (20 ml) and catalytic NH₄Cl was stirred at room temperature overnight (the reaction was monitored by TLC). After completion of the reaction, the volatiles were removed *in vacuo* and the product dried under vacuum to get the desired product as a colourless solid (0.435 g, quantitative yield).

¹H NMR (300 MHz, CDCl₃) δ 7.69 (d, 1H); 7.54 (dd, 1H); 6.86 (d, 1H); 6.59 (br, 1H), 5.87 (br, 1H), 4.83 (q, 1H); 2.05 (t, 3H); 1.68 (d, 3H).

¹⁹F NMR (300 MHz, CDCl₃) δ -85.20, -89.72.

### Step 4: (S)-2-(4-bromo-2-(1,1-difluoroethyl)phenoxy)propanenitrile

Pyridine (0.57 ml, 7.05 mmol) was added to a solution of (*S*)-2-(4-bromo-2-(1,1-difluoroethyl)phenoxy)propanamide (0.435 g, 1.41 mmol) and tosyl chloride (538 mg, 2.82 mmol) in DCM. The resulting mixture was stirred at room temperature overnight. After completion of the reaction, aqueous sodium bicarbonate solution was added carefully, and the mixture was stirred for 2 hours. The aqueous layer was extracted with DCM (2x100 mL). The combined organic extracts were washed with brine and dried (Na₂SO₄). After removal of the solvent, the residue was passed through a short silica gel column (hexane-EtOAc, 25:1, 5:1) to give desired product (0.228 g, 55.6%) as a colourless solid.

¹H NMR (300 MHz, CDCl₃) δ 7.75 (d, 1H); 7.62 (dd, 1H); 7.12 (d, 1H); 4.92 (q, 1H); 2.03 (t, 3H); 1.88 (d, 3H).

¹⁹F NMR (300 MHz, CDCl₃) δ -86.06, -88.97.

### Step 5: (S)-5-(1-(4-bromo-2-(1,1-difluoroethyl)phenoxy)ethyl)-1H-tetrazole

Triethylamine hydrochloride (141 mg, 1.02 mmol) was added to a solution of (*S*)-2-(4-bromo-2-(1,1-difluoroethyl)phenoxy)propanenitrile (0.228 g, 0.786 mmol) and sodium azide (66 mg, 1.021 mmol) in DMF, and the resulting mixture was stirred at 90°C for 5 hours. After completion of the reaction, the reaction mixture was quenched with 15% NaOH solution and the aqueous solution was washed with DCM to remove impurities and unreacted materials. The aqueous layer was separated and cooled again to 0°C, then acidified with 3M HCl to pH ~1. The product was extracted with ethyl acetate (2x30 mL), washed with water (20 mL), brine (20 mL), dried over sodium sulphate, filtered and concentrated to get desired product as a white solid (0.212 g, 80.9%).

¹H NMR (300 MHz, CDCl₃) δ 7.70 (d, 1H); 7.52 (dd, 1H); 6.95 (d, 1H); 5.98 (q, 1H); 2.07 (t, 3H); 1.95 (d, 3H).

¹⁹F NMR (300 MHz, CDCl₃) δ -85.05, -88.38.

LC/MS System: ES-MS: 332 [M-1].

HPLC method, retention time: 10.777 min, 99.2% purity @ 280 nm.

## Claims

1. A compound of Formula (I): 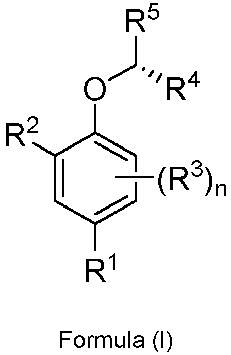 wherein:
- R¹ is selected from the group consisting of F, CI, Br and I;
- R² is selected from the group consisting of H, F, Cl, Br, !, C₁₋₅ alkyl optionally substituted with one or more, identical or different, substituents R⁶, C₂₋₅ alkenyl optionally substituted with one or more, identical or different, substituents R⁶, C₂₋₅ alkynyl optionally substituted with one or more, identical or different, substituents R⁶, C₃₋₅ cycloalkyl optionally substituted with one or more, identical or different, substituents R⁶, phenyl optionally substituted with one or more, identical or different, substituents R⁹, and 5-6 membered aromatic heterocycle optionally substituted with one or more, identical or different, substituents R⁷;
- R³ is selected from the group consisting of deuterium and F;
- R⁴ is selected from the group consisting of C₁₋₅ alkyl, C₂₋₅ alkenyl, C₂₋₅ alkynyl and C₃₋₅ cycloalkyl each of which may be optionally substituted with one or more, identical or different, substituents R⁷;
- R⁵ is selected from the group consisting of 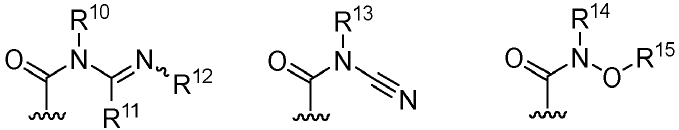 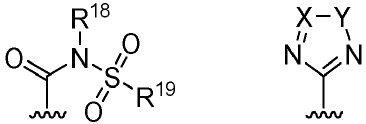
- R⁶ is independently selected from the group consisting of C₃₋₅ cycloalkyl, -O-C₁₋₅ alkyl, -O-C₃₋₅ cycloalkyl, -S-C₁₋₅ alkyl and -S-C₃₋₅ cycloalkyl each of which may optionally substituted with one or more, identical or different, substituents R⁸, deuterium, F and -CN;
- R⁷ is independently selected from the group consisting of C₃₋₅ cycloalkyl, -O-C₁₋₅ alkyl, -O-C₃₋₅ cycloalkyl, -S-C₁₋₅ alkyl and -S-C₃₋₅ cycloalkyl each of which may optionally substituted with one or more, identical or different, substituents R⁸, deuterium, F and -CN;
- R⁸ is independently selected from the group consisting of deuterium, OH, OMe and F;
- R⁹ is independently selected from the group consisting of deuterium, methoxy, nitro, cyano, CF₃, Cl, Br, I and F;
- R¹⁰ is selected from the group consisting of H, C₁₋₅ alkyl optionally substituted with one or more, identical or different, substituents R⁸, C₃₋₅ cycloalkyl optionally substituted with one or more, identical or different, substituents R⁸, -C(=O)-C₁₋₅ alkyl optionally substituted with one or more, identical or different, substituents R⁸ and -C(=O)-C₃₋₅ cycloalkyl optionally substituted with one or more, identical or different, substituents R⁸;
- R¹¹ is selected from the group consisting of C₁₋₅ alkyl optionally substituted with one or more, identical or different, substituents R⁸, C₃₋₅ cycloalkyl optionally substituted with one or more, identical or different, substituents R⁸ and phenyl optionally substituted with one or more, identical or different, substituents R⁹;
- R¹² is selected from the group consisting of -OH, -OC₁₋₅ alkyl optionally substituted with one or more, identical or different, substituents R⁸ and -OC₃₋₅ cycloalkyl optionally substituted with one or more, identical or different, substituents R⁸;
- R¹³ is selected from the group consisting of H, C₁₋₅ alkyl optionally substituted with one or more, identical or different, substituents R⁸, C₃₋₅ cycloalkyl optionally substituted with one or more, identical or different, substituents R⁸, -C(=O)-C₁₋₅ alkyl optionally substituted with one or more, identical or different, substituents R⁸ and -C(=O)-C₃₋₅ cycloalkyl optionally substituted with one or more, identical or different, substituents R⁸;
- R¹⁴ is selected from the group consisting of H, C₁₋₅ alkyl optionally substituted with one or more, identical or different, substituents R¹⁷, C₃₋₅ cycloalkyl optionally substituted with one or more, identical or different, substituents R⁸, -C(=O)-C₁₋₅ alkyl optionally substituted with one or more, identical or different, substituents R⁸ and -C(=O)-C₃₋₅ cycloalkyl optionally substituted with one or more, identical or different, substituents R⁸;
- R¹⁵ is selected from the group consisting of H, C₁₋₅ alkyl optionally substituted with one or more, identical or different, substituents R¹⁶, C₃₋₆ cycloalkyl optionally substituted with one or more, identical or different, substituents R¹⁶, phenyl optionally substituted with one or more, identical or different, substituents R⁹, and 4-6 membered heterocycle optionally substituted with one or more, identical or different, substituents R¹⁷ with the proviso that when R¹⁵ is H then R¹⁴ is not H;
- R¹⁶ is independently selected from the group consisting of F, C!, Br, I, -CN, =O, C₃₋₆ cycloalkyl optionally substituted with one or more, identical or different, substituents R⁸, -O-C₁₋₅ alkyl optionally substituted with one or more, identical or different, substituents R⁸, -O-C₃₋₅ cycloalkyl optionally substituted with one or more, identical or different, substituents R⁸, -S-C₁₋₅ alkyl optionally substituted with one or more, identical or different, substituents R⁸, -S-C₃₋₅ cycloalkyl optionally substituted with one or more, identical or different, substituents R⁸, -SO-C₁₋₅ alkyl optionally substituted with one or more, identical or different, substituents R⁸, -SO-C₃₋₅ cycloalkyl optionally substituted with one or more, identical or different, substituents R⁸, -SO₂-C₁₋₅ alkyl optionally substituted with one or more, identical or different, substituents R⁸, - SO₂-C₃₋₅ cycloalkyl optionally substituted with one or more, identical or different, substituents R⁸, -C(=O)-C₁₋₅ alkyl optionally substituted with one or more, identical or different substituents R⁸, -C(=O)-OC₁₋₅ alkyl optionally substituted with one or more, identical or different, substituents R⁸, -NH-C(=O)-OC₁₋₅ alkyl optionally substituted with one or more, identical or different, substituents R⁸, -C(=O)-C₃₋₅ cycloalkyl optionally substituted with one or more, identical or different, substituents R⁸, -NH-C(=O)-C₁₋₅ alkyl optionally substituted with one or more, identical or different, substituents R⁸, -NH-C(=O)-C₃₋₅ cycloalkyl optionally substituted with one or more, identical or different, substituents R⁸, -C(=O)-NH-C₁₋₅ alkyl optionally substituted with one or more, identical or different, substituents R⁸, -C(=O)-NH-C₃₋₅ cycloalkyl optionally substituted with one or more, identical or different, substituents R⁸, phenyl optionally substituted with one or more, identical or different, substituents R⁹, pyrrolidin-1-yl optionally substituted with one or more, identical or different, substituents R¹⁷ and 4-6 membered heterocycle optionally substituted with one or more, identical or different, substituents R¹⁷;
- R¹⁷ is independently selected from the group consisting of F, C!, Br, I, -CN, =O, C₁₋₅ alkyl optionally substituted with one or more, identical or different, substituents R⁸, C₃₋₆ cycloalkyl optionally substituted with one or more, identical or different, substituents R⁸, -C(=O)-C₁₋₅ alkyl optionally substituted with one or more, identical or different, substituents R⁸, -C(=O)-OC₁₋₅ alkyl optionally substituted with one or more, identical or different, substituents R⁸, -C(=O)-C₃₋₅ cycloalkyl optionally substituted with one or more, identical or different, substituents R⁸, -NH-C(=O)-C₁₋₅ alkyl optionally substituted with one or more, identical or different, substituents R⁸, -NH-C(=O)-C₃₋₅ cycloalkyl optionally substituted with one or more, identical or different, substituents R⁸, -C(=O)-NH-C₁₋₅ alkyl optionally substituted with one or more, identical or different, substituents R⁸ and -C(=O)-NH-C₃₋₅ cycloalkyl optionally substituted with one or more, identical or different, substituents R⁸;
- R¹⁸ is selected from the group consisting of H, C₁₋₅ alkyl optionally substituted with one or more, identical or different, substituents R⁸, C₃₋₅ cycloalkyl optionally substituted with one or more, identical or different, substituents R⁸, -C(=O)-C₁₋₅ alkyl optionally substituted with one or more, identical or different, substituents R⁸ and -C(=O)-C₃₋₅ cycloalkyl optionally substituted with one or more, identical or different, substituents R⁸;
- R¹⁹ is selected from the group consisting of C₁₋₅ alkyl optionally substituted with one or more, identical or different, substituents R⁸, C₃₋₅ cycloalkyl optionally substituted with one or more, identical or different, substituents R⁸ and phenyl optionally substituted with one or more, identical or different, substituents R⁹;
- X is selected from the group consisting of N and CR²⁰;
- Y is selected from the group consisting of NH, O and S;
- R²⁰ is selected from the group consisting of H, NH₂, C₁₋₅ alkyl optionally substituted with one or more, identical or different, substituents R⁸, C₃₋₅ cycloalkyl optionally substituted with one or more, identical or different, substituents R⁸, -OC₁₋₅ alkyl optionally substituted with one or more, identical or different, substituents R⁸, -OC₃₋₅ cycloalkyl optionally substituted with one or more, identical or different, substituents R⁸, -NH-C(=O)C₁₋₅ alkyl optionally substituted with one or more, identical or different, substituents R⁸ and -NH-SO₂-C₁₋₅ alkyl optionally substituted with one or more, identical or different, substituents R⁸;
- n is an integer 0, 1, 2 or 3;
or a pharmaceutically acceptable salt, hydrate, polymorph, tautomer, or solvate thereof.

2. The compound according to claim 1, wherein the compound is of Formula (II) 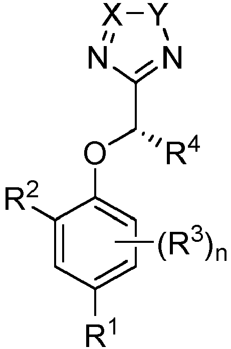 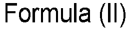 wherein:
- R¹ is selected from the group consisting of F, CI, Br and I;
- R² is selected from the group consisting of H, F, Cl, Br, !, C₁₋₅ alkyl optionally substituted with one or more, identical or different, substituents R⁶, C₂₋₅ alkenyl optionally substituted with one or more, identical or different, substituents R⁶, C₂₋₅ alkynyl optionally substituted with one or more, identical or different, substituents R⁶, C₃₋₅ cycloalkyl optionally substituted with one or more, identical or different, substituents R⁶, phenyl optionally substituted with one or more, identical or different, substituents R⁹, and 5-6 membered aromatic heterocycle optionally substituted with one or more, identical or different, substituents R⁷;
- R³ is selected from the group consisting of deuterium and F;
- R⁴ is selected from the group consisting of C₁₋₅ alkyl, C₂₋₅ alkenyl, C₂₋₅ alkynyl and C₃₋₅ cycloalkyl each of which may be optionally substituted with one or more, identical or different, substituents R⁷;
- R⁶ is independently selected from the group consisting of C₃₋₅ cycloalkyl, -O-C₁₋₅ alkyl, -O-C₃₋₅ cycloalkyl, -S-C₁₋₅ alkyl and -S-C₃₋₅ cycloalkyl each of which may optionally substituted with one or more, identical or different, substituents R⁸, deuterium, F and -CN;
- R⁷ is independently selected from the group consisting of C₃₋₅ cycloalkyl, -O-C₁₋₅ alkyl, -O-C₃₋₅ cycloalkyl, -S-C₁₋₅ alkyl and -S-C₃₋₅ cycloalkyl each of which may optionally substituted with one or more, identical or different, substituents R⁸, deuterium, F and -CN;
- R⁸ is independently selected from the group consisting of deuterium, OH, OMe and F;
- R⁹ is independently selected from the group consisting of deuterium, methoxy, nitro, cyano, CF₃, Cl, Br, I and F;
- X is selected from the group consisting of N and CR²⁰;
- Y is selected from the group consisting of NH, O and S;
- R²⁰ is selected from the group consisting of H, NH₂, C₁₋₅ alkyl optionally substituted with one or more, identical or different, substituents R⁸, C₃₋₅ cycloalkyl optionally substituted with one or more, identical or different, substituents R⁸, -OC₁₋₅ alkyl optionally substituted with one or more, identical or different, substituents R⁸, -OC₃₋₅ cycloalkyl optionally substituted with one or more, identical or different, substituents R⁸, -NH-C(=O)C₁₋₅ alkyl optionally substituted with one or more, identical or different, substituents R⁸ and -NH-SO₂-C₁₋₅ alkyl optionally substituted with one or more, identical or different, substituents R⁸;
- n is an integer 0, 1, 2 or 3;
or a pharmaceutically acceptable salt, hydrate, polymorph, tautomer, or solvate thereof.

3. The compound according to claim 1, wherein the compound is of Formula (III) 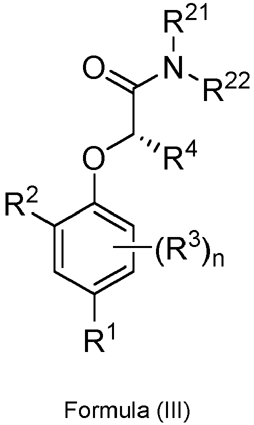 wherein:
- R¹ is selected from the group consisting of F, CI, Br and I;
- R² is selected from the group consisting of H, F, Cl, Br, I, C₁₋₅ alkyl optionally substituted with one or more, identical or different, substituents R⁶, C₂₋₅ alkenyl optionally substituted with one or more, identical or different, substituents R⁶, C₂₋₅ alkynyl optionally substituted with one or more, identical or different, substituents R⁶, C₃₋₅ cycloalkyl optionally substituted with one or more, identical or different, substituents R⁶, phenyl optionally substituted with one or more, identical or different, substituents R⁹, and 5-6 membered aromatic heterocycle optionally substituted with one or more, identical or different, substituents R⁷;
- R³ is selected from the group consisting of deuterium and F;
- R⁴ is selected from the group consisting of C₁₋₅ alkyl, C₂₋₅ alkenyl, C₂₋₅ alkynyl and C₃₋₅ cycloalkyl each of which may be optionally substituted with one or more, identical or different, substituents R⁷;
- R⁶ is independently selected from the group consisting of C₃₋₅ cycloalkyl, -O-C₁₋₅ alkyl, -O-C₃₋₅ cycloalkyl, -S-C₁₋₅ alkyl and -S-C₃₋₅ cycloalkyl each of which may optionally substituted with one or more, identical or different, substituents R⁸, deuterium, F and -CN;
- R⁷ is independently selected from the group consisting of C₃₋₅ cycloalkyl, -O-C₁₋₅ alkyl, -O-C₃₋₅ cycloalkyl, -S-C₁₋₅ alkyl and -S-C₃₋₅ cycloalkyl each of which may optionally substituted with one or more, identical or different, substituents R⁸, deuterium, F and -CN;
- R⁸ is independently selected from the group consisting of deuterium, OH, OMe and F;
- R⁹ is independently selected from the group consisting of deuterium, methoxy, nitro, cyano, CF₃, Cl, Br, I and F;
- R¹¹ is selected from the group consisting of C₁₋₅ alkyl optionally substituted with one or more, identical or different, substituents R⁸, C₃₋₅ cycloalkyl optionally substituted with one or more, identical or different, substituents R⁸ and phenyl optionally substituted with one or more, identical or different, substituents R⁹;
- R¹² is selected from the group consisting of -OH, -OC₁₋₅ alkyl optionally substituted with one or more, identical or different, substituents R⁸ and -OC₃₋₅ cycloalkyl optionally substituted with one or more, identical or different, substituents R⁸;
- R¹⁵ is selected from the group consisting of H, C₁₋₅ alkyl optionally substituted with one or more, identical or different, substituents R¹⁶, C₃₋₆ cycloalkyl optionally substituted with one or more, identical or different, substituents R¹⁶, phenyl optionally substituted with one or more, identical or different, substituents R⁹, and 4-6 membered heterocycle optionally substituted with one or more, identical or different, substituents R¹⁷ with the proviso that when R¹⁵ is H then R²¹ is not H;
- R¹⁶ is independently selected from the group consisting of F, C!, Br, I, -CN, =O, C₃₋₆ cycloalkyl optionally substituted with one or more, identical or different, substituents R⁸, -O-C₁₋₅ alkyl optionally substituted with one or more, identical or different, substituents R⁸, -O-C₃₋₅ cycloalkyl optionally substituted with one or more, identical or different, substituents R⁸, -S-C₁₋₅ alkyl optionally substituted with one or more, identical or different, substituents R⁸, -S-C₃₋₅ cycloalkyl optionally substituted with one or more, identical or different, substituents R⁸, -SO-C₁₋₅ alkyl optionally substituted with one or more, identical or different, substituents R⁸, -SO-C₃₋₅ cycloalkyl optionally substituted with one or more, identical or different, substituents R⁸, -SO₂-C₁₋₅ alkyl optionally substituted with one or more, identical or different, substituents R⁸, - SO₂-C₃₋₅ cycloalkyl optionally substituted with one or more, identical or different, substituents R⁸, -C(=O)-C₁₋₅ alkyl optionally substituted with one or more, identical or different substituents R⁸, -C(=O)-OC₁₋₅ alkyl optionally substituted with one or more, identical or different, substituents R⁸, -NH-C(=O)-OC₁₋₅ alkyl optionally substituted with one or more, identical or different, substituents R⁸, -C(=O)-C₃₋₅ cycloalkyl optionally substituted with one or more, identical or different, substituents R⁸, -NH-C(=O)-C₁₋₅ alkyl optionally substituted with one or more, identical or different, substituents R⁸, -NH-C(=O)-C₃₋₅ cycloalkyl optionally substituted with one or more, identical or different, substituents R⁸, -C(=O)-NH-C₁₋₅ alkyl optionally substituted with one or more, identical or different, substituents R⁸, -C(=O)-NH-C₃₋₅ cycloalkyl optionally substituted with one or more, identical or different, substituents R⁸, phenyl optionally substituted with one or more, identical or different, substituents R⁹, pyrrolidin-1-yl optionally substituted with one or more, identical or different, substituents R¹⁷ and 4-6 membered heterocycle optionally substituted with one or more, identical or different, substituents R¹⁷;
- R¹⁷ is independently selected from the group consisting of F, C!, Br, I, -CN, =O, C₁₋₅ alkyl optionally substituted with one or more, identical or different, substituents R⁸, C₃₋₆ cycloalkyl optionally substituted with one or more, identical or different, substituents R⁸, -C(=O)-C₁₋₅ alkyl optionally substituted with one or more, identical or different, substituents R⁸, -C(=O)-OC₁₋₅ alkyl optionally substituted with one or more, identical or different, substituents R⁸, -C(=O)-C₃₋₅ cycloalkyl optionally substituted with one or more, identical or different, substituents R⁸, -NH-C(=O)-C₁₋₅ alkyl optionally substituted with one or more, identical or different, substituents R⁸, -NH-C(=O)-C₃₋₅ cycloalkyl optionally substituted with one or more, identical or different, substituents R⁸, -C(=O)-NH-C₁₋₅ alkyl optionally substituted with one or more, identical or different, substituents R⁸ and -C(=O)-NH-C₃₋₅ cycloalkyl optionally substituted with one or more, identical or different, substituents R⁸;
- R¹⁹ is selected from the group consisting of C₁₋₅ alkyl optionally substituted with one or more, identical or different, substituents R⁸, C₃₋₅ cycloalkyl optionally substituted with one or more, identical or different, substituents R⁸ and phenyl optionally substituted with one or more, identical or different, substituents R⁹;
- R²¹ is selected from the group consisting of H, C₁₋₅ alkyl optionally substituted with one or more, identical or different, substituents R⁸, C₃₋₅ cycloalkyl optionally substituted with one or more, identical or different, substituents R⁸, -C(=O)-C₁₋₅ alkyl optionally substituted with one or more, identical or different, substituents R⁸ and -C(=O)-C₃₋₅ cycloalkyl optionally substituted with one or more, identical or different, substituents R⁸;
- R²² is selected from the group consisting of -C(R¹¹)=NR¹², -CN, -OR¹⁵ and SO₂R¹⁹; and
- n is an integer 0, 1, 2 or 3;
or a pharmaceutically acceptable salt, hydrate, polymorph, tautomer, or solvate thereof.

4. The compound according to any of claims 1 to 3, wherein R¹ is Cl or Br.

5. The compound according to any of claims 1 to 4, wherein R² is H, F, Cl, Br or I.

6. The compound according to any of claims 1 to 4, wherein R² is a 5-6 membered aromatic heterocycle optionally substituted with one or more, identical or different, substituents R⁷.

7. The compound according to any of claims 1 to 6, wherein R⁴ is C₁₋₅ alkyl optionally substituted with one or more, identical or different, substituents R⁷.

8. The compound according to any of claims 1 to 2 and 4 to 6, wherein X is N and Y is NH.

9. The compound according to any of claims 3 to 6, wherein R²¹ is H.

10. The compound for use according to any one of claims 1 to 9, wherein the compound is selected from the group consisting of:
(2*S*)-2-(4-bromo-2-fluorophenoxy)-*N*-{1-[(cyclopropylmethoxy)imino]ethyl}propanamide;
(2*S*)-2-(4-bromophenoxy)-*N*-[1-(methoxyimino)ethyl]propanamide;
(2*S*)-2-(4-bromo-2-fluorophenoxy)-*N*-[(4-fluorophenyl)(hydroxyimino)methyl]-3-methylbutanamide;
(2*S*)-2-(4-bromo-2-fluorophenoxy)-*N*-[1-(hydroxyimino)ethyl]-3-methylbutanamide;
(2*S*)-2-(4-bromo-2-fluorophenoxy)-*N*-[1-(hydroxyimino)ethyl]propanamide;
(2*S*)-2-(4-bromophenoxy)-*N*-[1-(hydroxyimino)ethyl]propenamide;
(2*S*)-2-[4-bromo-2-(1,2-oxazol-3-yl)phenoxy]-*N*-cyanopropanamide;
(2*S*)-N-cyano-2-(2,4-dibromophenoxy)propanamide;
(2S)-2-(4-bromophenoxy)-*N*-cyanopropanamide;
(2*S*)-2-(4-chlorophenoxy)-*N*-cyanopropanamide;
(2*S*)-2-(4-bromophenoxy)-*N*-cyano-3-methylbutanamide;
(2*S*)-2-[4-bromo-2-(1,2-oxazol-3-yl)phenoxy]-*N*-cyclobutoxypropanamide;
(2*S*)-2-[4-bromo-2-(1,2-oxazol-3-yl)phenoxy]-*N*-methoxypropanamide;
(2*S*)-*N*-acetyl-*N*-[(1-acetylazetidi*n*-3-yl)oxy]-2-(4-chlorophenoxy)propanamide;
(2*S*)-*N*-[(1-acetylazetidi*n*-3-yl)oxy]-2-(4-chlorophenoxy)propanamide;
(2*S*)-*N*-(azetidi*n*-3-yloxy)-2-(4-chlorophenoxy)propanamide;
(2*S*)-*N*-acetyl-*N*-[(1-acetylpyrrolidi*n*-3-yl)methoxy]-2-(4-chlorophenoxy)propanamide;
(2*S*)-*N*-[(1-acetylpyrrolidi*n*-3-yl)methoxy]-2-(4-chlorophenoxy)propanamide;
(2*S*)-*N*-[(1-acetylpyrrolidi*n*-3-yl)oxy]-2-(4-bromophenoxy)propanamide;
tert-butyl *N*-(2-{[(2*S*)-2-(4-chlorophenoxy)propanamido]oxy}propyl)carbamate;
(2*S*)-*N*-acetyl-2-(4-chlorophenoxy)-*N*-[(1-acetamidopropan-2-yl)oxy]propanamide;
(2*S*)-2-(4-bromophenoxy)-*N*-(pyrrolidin-3-yloxy)propanamide;
(2*S*)-*N*-acetyl-*N*-[(1-acetylpyrrolidi*n*-3-yl)oxy]-2-(4-bromophenoxy)propanamide;
tert-butyl 3-{[(2*S*)-2-(4-chlorophenoxy)propanamido]oxylazetidine-1-carboxylate;
(2*S*)-2-(4-chlorophenoxy)-*N*-[(pyrrolidi*n*-3-yl)methoxy]propanamide;
tert-butyl 3-({[(2*S*)-2-(4-chlorophenoxy)propanamido]oxy}methyl)pyrrolidine-1-carboxylate;
(2*S*)-*N*-acetyl*-N*-[(1-acetylpyrrolidi*n*-3-yl)oxy]-2-(4-chlorophenoxy)propanamide;
(2*S*)-*N*-[(1-acetylpyrrolidi*n*-3-yl)oxy]-2-(4-chlorophenoxy)propanamide;
(2*S*)-2-(4-chlorophenoxy)-*N*-(pyrrolidi*n*-3-yloxy)propanamide;
(2*S*)-2-(4-chlorophenoxy)-*N*-(2,2-dimethylpropyl)-*N*-hydroxypropanamide;
(2*S*)-2-(4-chlorophenoxy)-*N*-[2-(pyrrolidi*n*-1-yl)ethoxy]propanamide;
(2*S*)-2-(4-chlorophenoxy)-*N*-[2-(2-oxopyrrolidi*n*-1-yl)ethoxy]propanamide;
(2*S*)-*N*-acetyl-2-(4-chlorophenoxy)-*N*-(2-acetamidoethoxy)propanamide;
(2*S*)-2-(4-chlorophenoxy)-*N*-(2-acetamidoethoxy)propanamide;
(2*S*)-2-(4-chlorophenoxy)-*N*-(4,4,4-trifluoro-2-methylbutoxy)propanamide;
(2*S*)-2-(4-chlorophenoxy)-*N*-(3-cyclopentylpropyl)-*N*-hydroxypropanamide;
(2*S*)-2-(4-chlorophenoxy)-*N*-{[(2E)-2-methyl-3-phenylprop-2-e*n*-1-yl]oxy}propanamide;
(2*S*)-2-(4-chlorophenoxy)-*N*-{2-oxo-2-[2-(trifluoromethyl)pyrrolidi*n*-1-yl]ethoxy}propanamide;
tert-butyl *N*-(2-{[(2*S*)-2-(4-chlorophenoxy)propanamidyl]oxy}ethyl)carbamate;
(2*S*)-2-(4-chlorophenoxy)-*N*-[(2-methyl-1H-imidazol-4-yl)methoxy]propanamide;
tert-butyl 4-({[(2*S*)-2-(4-chlorophenoxy)propanamido]oxy}methyl)-2-methyl-1H-imidazole-1-carboxylate;
(2*S*)-2-(4-bromo-2-fluorophenoxy)-*N*-cyclobutoxypropanamide;
(2*S*)-2-(4-bromophenoxy)-*N*-cyclobutoxypropanamide;
(2*S*)-2-(4-chlorophenoxy)-*N*-[2-oxo-2-(pyrrolidin-1-yl)ethoxy]propanamide;
(2*S*)-2-(4-chlorophenoxy)-*N*-[1-(4-fluorophenyl)ethoxy]propanamide;
(2*S*)-2-(4-chlorophenoxy)-*N*-[1-(1,3-thiazol-2-yl)ethoxy]propanamide;
(2*S*)-2-(4-chlorophenoxy)-*N*-{1-[4-(trifluoromethyl)phenyl]ethoxy}propanamide;
(2*S*)-2-(4-chlorophenoxy)-*N*-(2-methanesulfinylethoxy)propanamide;
(2*S*)-2-(4-chlorophenoxy)-*N*-(2-methanesulfonylethoxy)propanamide;
(2*S*)-2-(4-chlorophenoxy)-*N*-[(1,2-oxazol-3-yl)methoxy]propanamide;
(2*S*)-2-(4-chloro-2-fluorophenoxy)-*N*-methoxypropanamide;
(2*S*)-2-[4-bromo-2-(1,3,4-oxadiazol-2-yl)phenoxy]-*N-*(cyclopropylmethoxy)propanamide;
(2*S*)-2-[4-chloro-2-(trifluoromethyl)phenoxy]-*N-*(cyclopropylmethoxy)propanamide;
(2*S*)-*N*-(tert-butoxy)-2-(4-chlorophenoxy)propanamide;
(2*S*)-2-(4-chlorophenoxy)-*N*-[2-(methylsulfanyl)ethoxy]propanamide
(2*S*)-2-(4-chlorophenoxy)-*N*-(1-phenylethoxy)propanamide;
(2*S*)-2-(4-chlorophenoxy)-*N*-[(1-methyl-1H-imidazol-2-yl)methoxy]propanamide;
(2*S*)-2-(4-chlorophenoxy)-*N*-(2-methoxyethoxy)propanamide;
(2*S*)-2-(4-chloro-2-fluorophenoxy)-*N*-cyclobutoxypropanamide;
(2*S*)-2-[4-bromo-2-(1,3,4-oxadiazol-2-yl)phenoxy]-*N*-methoxypropanamide;
(2*S*)-2-[4-chloro-2-(trifluoromethyl)phenoxy]-*N*-methoxypropanamide;
(2*S*)-*N*-(benzyloxy)-2-(4-chlorophenoxy)propanamide;
(2*S*)-2-(4-chlorophenoxy)-*N*-[(2-methoxycyclopentyl)oxy]propanamide;
(2*S*)-2-(4-chlorophenoxy)-*N*-cyclobutoxy-5-methylhexanamide;
(2*S*)-2-(4-chlorophenoxy)-*N*-cyclobutoxy-*N*-methylpropanamide;
(2*S*)-2-(4-chloro-2-methylphenoxy)-*N*-cyclobutoxypropanamide;
(2*S*)-2-(4-chloro-3-fluorophenoxy)-*N*-cyclobutoxypropanamide;
(2*S*)-2-(4-chloro-2-methylphenoxy)-*N*-methoxypropanamide;
(2*S*)-2-(4-chloro-3-fluorophenoxy)-*N*-methoxypropanamide;
(2*S*)-2-(4-chlorophenoxy)-*N*-cyclobutoxy-4-methylpentanamide;
(2*S*)-2-(4-chlorophenoxy)-*N*-[(3-methylbut-2-e*n*-1-yl)oxy]propanamide;
(2*S*)-2-(4-chlorophenoxy)-*N*-cyclobutoxyhexanamide;
(2*S*)-2-(4-chlorophenoxy)-*N*-[(1,3-oxazol-2-yl)methoxy]propanamide;
(2*S*)-2-(2,4-dibromophenoxy)-*N*-methoxypropanamide;
(2*S*)-2-(4-bromo-2-fluorophenoxy)-*N*-methoxypropanamide;
(2*S*)-2-(4-chlorophenoxy)-*N*-(oxan-2-yloxy)propanamide;
(2*S*)-2-(4-chlorophenoxy)-*N*-[(1,3-thiazol-2-yl)methoxy]propanamide;
(2*S*)-2-(4-chlorophenoxy)-*N*-(3,3-difluorocyclobutoxy)propanamide;
(2*S*)-2-[4-bromo-2-(1,2-oxazol-5-yl)phenoxy]-*N*-cyclobutoxypropanamide;
(2*S*)-2-[4-bromo-2-(1,2-oxazol-5-yl)phenoxy]-*N*-methoxypropanamide;
(2*S*)-2-(4-chlorophenoxy)-*N*-(cyclopentyloxy)propanamide;
(2*S*)-2-(4-chlorophenoxy)-*N*-(2-cyclopentylethoxy)propanamide;
(2*S*)-2-(4-bromo-2-chlorophenoxy)-*N*-methoxypropanamide;
(2*S*)-2-(4-bromo-2-methylphenoxy)-*N*-methoxypropanamide;
(2*S*)-2-(4-chlorophenoxy)-*N*-(cyclopropylmethoxy)propanamide;
(2*S*)-2-(4-chlorophenoxy)-*N*-(cyclobutylmethoxy)propanamide;
(2*S*)-*N*-(2-aminoethoxy)-2-(4-chlorophenoxy)propanamide;
(2*S*)-2-(4-bromophenoxy)-*N*-methoxy-3-methylbutanamide;
methyl 2-{[(2*S*)-2-(4-chlorophenoxy)propanamido]oxy}acetate;
(2*S*)-2-(4-chlorophenoxy)-*N*-[2-(2-methoxyethoxy)ethoxy]propanamide;
(2*S*)-2-(4-chlorophenoxy)-*N*-cyclobutoxypropanamide;
(2*S*)-2-(4-chlorophenoxy)-*N*-(2-hydroxyethoxy)propanamide;
(2*S*)-2-(4-chlorophenoxy)-*N*-ethoxypropanamide;
(2*S*)-2-(4-chlorophenoxy)-*N*-propoxypropanamide;
(2*S*)-2-(4-chlorophenoxy)-*N*-(propan-2-yloxy)propanamide;
(2*S*)-2-(4-chlorophenoxy)-*N*-methoxypropanamide;
(2*S*)-2-[4-bromo-2-(1,2-oxazol-3-yl)phenoxy]-*N-*(cyclopropanesulfonyl)propenamide;
(2*S*)-2-(4-bromo-2-fluorophenoxy)-*N*-methanesulfonylpropanamide;
(2*S*)-2-(4-bromo-2-fluorophenoxy)-*N*-(cyclopropanesulfonyl)propenamide;
(2*S*)-2-(4-chlorophenoxy)-*N*-methanesulfonylpropanamide;
(2*S*)-2-(4-bromophenoxy)-*N*-methanesulfonyl-3-methylbutanamide;
5-[(1*S*)-1-[4-bromo-2-(1,2-oxazol-3-yl)phenoxy]-3-fluoropropyl]-2H-1,2,3,4-tetrazole;
5-[(1*S*)-1-(4-bromo-2-fluorophenoxy)propyl]-2H-1,2,3,4-tetrazole;
5-[(1*S*)-1-(4-bromo-2-fluorophenoxy)-2-methylpropyl]-2H-1,2,3,4-tetrazole;
5-[(1*R*)-1-(4-bromophenoxy)-2-fluoroethyl]-2H-1,2,3,4-tetrazole;
5-[(1*S*)-1-(4-bromophenoxy)propyl]-2H-1,2,3,4-tetrazole;
5-[(1*S*)-1-(4-bromophenoxy)ethyl]-2H-1,2,3,4-tetrazole;
5-[(1*S*)-1-[4-bromo-2-(1,2-oxazol-3-yl)phenoxy]ethyl]-1H-1,2,3,4-tetrazole;
5-[(1*S*)-1-(4-bromo-2-fluorophenoxy)ethyl]-2H-1,2,3,4-tetrazole;
5-[(1*S*)-1-[4-bromo-2-(1,2-oxazol-5-yl)phenoxy]ethyl]-2H-1,2,3,4-tetrazole;
5-[(1*S*)-1-[4-chloro-2-(1,2-oxazol-3-yl)phenoxy]ethyl]-2H-1,2,3,4-tetrazole;
5-[(1*S*)-1-(4-chloro-2-cyclopropylphenoxy)ethyl]-1H-1,2,3,4-tetrazole;
5-[(1*S*)-1-(4-chloro-2-ethenylphenoxy)ethyl]-1H-1,2,3,4-tetrazole;
5-[(1*S*)-1-(4-chloro-2-ethylphenoxy)ethyl]-1H-1,2,3,4-tetrazole;
5-[(1*S*)-1-(4-chloro-2-methylphenoxy)ethyl]-2H-1,2,3,4-tetrazole;
5-[(1*S*)-1-(4-chlorophenoxy)ethyl]-1H-1,2,3,4-tetrazole;
*N*-{3-[(1*S*)-1-(4-bromo-2-fluorophenoxy)ethyl]-1,2,4-thiadiazol-5-yl}acetamide;
*N*-{3-[(1*S*)-1-(4-bromo-2-fluorophenoxy)ethyl]-1,2,4-thiadiazol-5-yl}methanesulfonamide;
3-[(1*S*)-1-(4-bromo-2-fluorophenoxy)ethyl]-1,2,4-thiadiazol-5-amine;
*N*-{3-[(1*S*)-1-(4-bromo-2-fluorophenoxy)ethyl]-1,2,4-oxadiazol-5-yl}acetamide;
*N*-{3-[(1*S*)-1-(4-bromo-2-fluorophenoxy)ethyl]-1,2,4-oxadiazol-5-yl}methanesulfonamide;
3-[(1*S*)-1-[4-bromo-2-(1,2-oxazol-3-yl)phenoxy]ethyl]-5-(trifluoromethyl)-4H-1,2,4-triazole;
3-[(1*S*)-1-[4-chloro-2-(1,2-oxazol-3-yl)phenoxy]ethyl]-5-(trifluoromethyl)-4H-1,2,4-triazole;
3-[(1*S*)-1-(4-bromo-2-fluorophenoxy)ethyl]-5-methoxy-1,2,4-oxadiazole;
3-[(1*S*)-1-(4-bromo-2-fluorophenoxy)ethyl]-1,2,4-oxadiazol-5-amine;
3-[(1*S*)-1-[4-bromo-2-(1,2-oxazol-3-yl)phenoxy]ethyl]-5-methyl-4H-1,2,4-triazole;
3-[(1*S*)-1-[4-chloro-2-(1,2-oxazol-3-yl)phenoxy]ethyl]-5-methyl-4H-1,2,4-triazole;
3-[(1*S*)-1-(4-bromo-2-fluorophenoxy)ethyl]-5-methyl-4H-1,2,4-triazole;
5-[(1*S*)-1-(4-chlorophenoxy)ethyl]-1H-1,2,4-triazole;
(2*S*)-2-[4-bromo-2-(1, 1-difluoroethyl)phenoxy]-*N*-cyanopropanamide;
(2*S*)-2-[4-bromo-2-(1,1-difluoropropyl)-5-fluorophenoxy]-*N*-cyanopropanamide;
(2*S*)-2-[4-bromo-2-(1,1-difluoropropyl)phenoxy]-*N-*methanesulfonylpropanamide;
(2*S*)-2-[4-bromo-2-(1,1-difluoropropyl)phenoxy]-*N-*(cyclopropanesulfonyl)propenamide;
5-[(1*S*)-1-[4-bromo-2-(1,1-difluoroethyl)phenoxy]ethyl]-1H-1,2,3,4-tetrazole;
5-[(1*S*)-1-[4-bromo-2-(1,1-difluoropropyl)phenoxy]ethyl]-1H-1,2,3,4-tetrazole;
5-[(1*S*)-1-(4-bromo-2-cyclobutylphenoxy)-2-methoxyethyl]-1H-1,2,3,4-tetrazole; and
5-[(1*S*)-1-(4-bromo-2-cyclopropylphenoxy)-2-methoxyethyl]-1H-1,2,3,4-tetrazole.

11. The compound according to any one of claims 1 to 10, wherein the compound is an inhibitor of the CIC-1 ion channel.

12. A composition comprising the compound according to any one of claims 1 to 11 and a pharmaceutically acceptable carrier.

13. The compound according to any one of claims 1 to 11 or composition according to claim 12 for use as a medicament.

14. The compound according to any one of claims 1 to 11 or composition according to claim 12 for use in the treatment of symptoms of an indication selected from the group consisting of myasthenia gravis, Lambert-Eaton Syndrome, critical illness myopathy, amyotrophic lateral sclerosis (ALS), spinal muscular atrophy (SMA), critical illness myopathy (CIM), reversal diabetic polyneuropathy, Guillain-Barré syndrome, poliomyelitis, post-polio syndrome, chronic fatigue syndrome, critical illness polyneuropathy, periodic paralysis, sarcopenia, hypokalemic periodic paralysis and hyperkalemic periodic paralysis.

15. The compound according to any one of claims 1 to 11 or composition according to claim 12 for use in reversing and/or ameliorating a neuromuscular blockade.

## Patentansprüche

1. Verbindung der Formel (I): 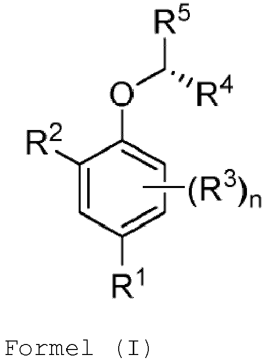 wobei:
- R¹ ausgewählt ist aus der Gruppe bestehend aus F, Cl, Br und I;
- R² ausgewählt ist aus der Gruppe bestehend aus H, F, Cl, Br, I, C₁₋₅-Alkyl, optional substituiert mit einem oder mehreren gleichen oder verschiedenen Substituenten R⁶, C₂₋₅-Alkenyl, optional substituiert mit einem oder mehreren gleichen oder verschiedenen Substituenten R⁶, C₂₋₅-Alkinyl, optional substituiert mit einem oder mehreren gleichen oder verschiedenen Substituenten R⁶, C₃₋₅-Cycloalkyl, optional substituiert mit einem oder mehreren gleichen oder verschiedenen Substituenten R⁶, Phenyl, optional substituiert mit einem oder mehreren gleichen oder verschiedenen Substituenten R⁹ und 5- bis 6-gliedrigem aromatischem Heterocyclus, optional substituiert mit einem oder mehreren gleichen oder verschiedenen Substituenten R⁷;
- R³ ausgewählt ist aus der Gruppe bestehend aus Deuterium und F;
- R⁴ ausgewählt ist aus der Gruppe bestehend aus C₁₋₅-Alkyl, C₂₋₅-Alkenyl, C₂₋₅-Alkinyl und C₃₋₅-Cycloalkyl, die jeweils optional mit einem oder mehreren gleichen oder verschiedenen Substituenten R⁷ substituiert sein können;
- R⁵ ausgewählt ist aus der Gruppe bestehend aus 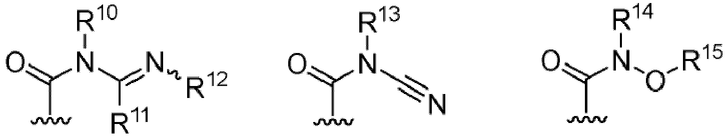 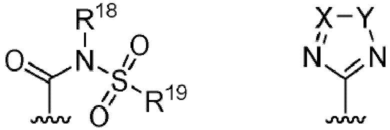
- R⁶ unabhängig ausgewählt ist aus der Gruppe bestehend aus C₃₋₅-Cycloalkyl, -O-C₁₋₅-Alkyl, -O-C₃₋₅-Cycloalkyl, -S-C₁₋₅-Alkyl und -S-C₃₋₅-Cycloalkyl, die jeweils optional mit einem oder mehreren gleichen oder verschiedenen Substituenten R⁸, Deuterium, F und -CN substituiert sein können;
- R⁷ unabhängig ausgewählt ist aus der Gruppe bestehend aus C₃₋₅-Cycloalkyl, -O-C₁₋₅-Alkyl, -O-C₃₋₅-Cycloalkyl, -S-C₁₋₅-Alkyl und -S-C₃₋₅-Cycloalkyl, die jeweils optional mit einem oder mehreren gleichen oder verschiedenen Substituenten R⁸, Deuterium, F und -CN substituiert sein können;
- R⁸ unabhängig ausgewählt ist aus der Gruppe bestehend aus Deuterium, OH, OMe und F;
- R⁹ unabhängig ausgewählt ist aus der Gruppe bestehend aus Deuterium, Methoxy, Nitro, Cyano, CF₃, Cl, Br, I und F;
- R¹⁰ ausgewählt ist aus der Gruppe bestehend aus H, C₁₋₅-Alkyl, optional substituiert mit einem oder mehreren gleichen oder verschiedenen Substituenten R⁸, C₃₋₅-Cycloalkyl, optional substituiert mit einem oder mehreren gleichen oder verschiedenen Substituenten R⁸, -C(=O)-C₁₋₅-Alkyl, optional substituiert mit einem oder mehreren gleichen oder verschiedenen Substituenten R⁸ und -C(=O)-C₃₋₅-Cycloalkyl, optional substituiert mit einem oder mehreren gleichen oder verschiedenen Substituenten R⁸;
- R¹¹ ausgewählt ist aus der Gruppe bestehend aus C₁₋₅-Alkyl, optional substituiert mit einem oder mehreren gleichen oder verschiedenen Substituenten R⁸, C₃₋₅-Cycloalkyl, optional substituiert mit einem oder mehreren gleichen oder verschiedenen Substituenten R⁸ und Phenyl, optional substituiert mit einem oder mehreren gleichen oder verschiedenen Substituenten R⁹;
- R¹² ausgewählt ist aus der Gruppe bestehend aus -OH, -OC₁₋₅-Alkyl, optional substituiert mit einem oder mehreren gleichen oder verschiedenen Substituenten R⁸ und -OC₃₋₅-Cycloalkyl, optional substituiert mit einem oder mehreren gleichen oder verschiedenen Substituenten R⁸;
- R¹³ ausgewählt ist aus der Gruppe bestehend aus H, C₁₋₅-Alkyl, optional substituiert mit einem oder mehreren gleichen oder verschiedenen Substituenten R⁸, C₃₋₅-Cycloalkyl, optional substituiert mit einem oder mehreren gleichen oder verschiedenen Substituenten R⁸, -C(=O)-C₁₋₅-Alkyl, optional substituiert mit einem oder mehreren gleichen oder verschiedenen Substituenten R⁸ und -C(=O)-C₃₋₅-Cycloalkyl, optional substituiert mit einem oder mehreren gleichen oder verschiedenen Substituenten R⁸;
- R¹⁴ ausgewählt ist aus der Gruppe bestehend aus H, C₁₋₅-Alkyl, optional substituiert mit einem oder mehreren gleichen oder verschiedenen Substituenten R¹⁷, C₃₋₅-Cycloalkyl, optional substituiert mit einem oder mehreren gleichen oder verschiedenen Substituenten R⁸, -C(=O)-C₁₋₅-Alkyl, optional substituiert mit einem oder mehreren gleichen oder verschiedenen Substituenten R⁸ und -C(=O)-C₃₋₅-Cycloalkyl, optional substituiert mit einem oder mehreren gleichen oder verschiedenen Substituenten R⁸;
- R¹⁵ ausgewählt ist aus der Gruppe bestehend aus H, C₁₋₅-Alkyl, optional substituiert mit einem oder mehreren gleichen oder verschiedenen Substituenten R¹⁶, C₃₋₆-Cycloalkyl, optional substituiert mit einem oder mehreren gleichen oder verschiedenen Substituenten R¹⁶, Phenyl, optional substituiert mit einem oder mehreren gleichen oder verschiedenen Substituenten R⁹ und 4- bis 6-gliedrigem Heterocyclus, optional substituiert mit einem oder mehreren gleichen oder verschiedenen Substituenten R¹⁷ mit der Maßgabe, dass, wenn R¹⁵ H ist, dann R¹⁴ nicht H ist;
- R¹⁶ unabhängig ausgewählt ist aus der Gruppe bestehend aus F, Cl, Br, I, -CN, =O, C₃₋₆-Cycloalkyl, optional substituiert mit einem oder mehreren gleichen oder verschiedenen Substituenten R⁸, -O-C₁₋₅-Alkyl, optional substituiert mit einem oder mehreren gleichen oder verschiedenen Substituenten R⁸, -O-C₃₋₅-Cycloalkyl, optional substituiert mit einem oder mehreren gleichen oder verschiedenen Substituenten R⁸, -S-C₁₋₅-Alkyl, optional substituiert mit einem oder mehreren gleichen oder verschiedenen Substituenten R⁸, -S-C₃₋₅-Cycloalkyl, optional substituiert mit einem oder mehreren gleichen oder verschiedenen Substituenten R⁸, -SO-C₁₋₅-Alkyl, optional substituiert mit einem oder mehreren gleichen oder verschiedenen Substituenten R⁸, -SO-C₃₋₅-Cycloalkyl, optional substituiert mit einem oder mehreren gleichen oder verschiedenen Substituenten R⁸, -SO₂-C₁₋₅-Alkyl, optional substituiert mit einem oder mehreren gleichen oder verschiedenen Substituenten R⁸, -SO₂-C₃₋₅-Cycloalkyl, optional substituiert mit einem oder mehreren gleichen oder verschiedenen Substituenten R⁸, -C(=O)-C₁₋₅-Alkyl, optional substituiert mit einem oder mehreren gleichen oder verschiedenen Substituenten R⁸, -C(=O)-OC₁₋₅-Alkyl, optional substituiert mit einem oder mehreren gleichen oder verschiedenen Substituenten R⁸, - NH-C(=O)-OC₁₋₅-Alkyl, optional substituiert mit einem oder mehreren gleichen oder verschiedenen Substituenten R⁸, -C(=O)-C₃₋₅-Cycloalkyl, optional substituiert mit einem oder mehreren gleichen oder verschiedenen Substituenten R⁸, -NH-C (=O)-C₁₋₅-Alkyl, optional substituiert mit einem oder mehreren gleichen oder verschiedenen Substituenten R⁸, -NH-C(=O)-C₃₋₅ Cycloalkyl, optional substituiert mit einem oder mehreren gleichen oder verschiedenen R⁸, -C(=O)-NH-C₁₋₅-Alkyl, optional substituiert mit einem oder mehreren gleichen oder verschiedenen Substituenten R⁸, -C(=O)-NH-C₃₋₅-Cycloalkyl, optional substituiert mit einem oder mehreren gleichen oder verschiedenen Substituenten R⁸, Phenyl, optional substituiert mit einem oder mehreren gleichen oder verschiedenen Substituenten R⁹, Pyrrolidin-1-yl, optional substituiert mit einem oder mehreren gleichen oder verschiedenen Substituenten R¹⁷ und 4- bis 6-gliedrigem Heterocyclus, optional substituiert mit einem oder mehreren gleichen oder verschiedenen Substituenten R¹⁷;
- R¹⁷ unabhängig ausgewählt ist aus der Gruppe bestehend aus F, Cl, Br, I, -CN, =O, C₁₋₅-Alkyl, optional substituiert mit einem oder mehreren gleichen oder verschiedenen Substituenten R⁸, C₃₋₆-Cycloalkyl, optional substituiert mit einem oder mehreren gleichen oder verschiedenen Substituenten R⁸, -C(=O)-C₁₋₅-Alkyl, optional substituiert mit einem oder mehreren gleichen oder verschiedenen Substituenten R⁸, -C (=O)-OC₁₋₅₋-Alkyl, optional substituiert mit einem oder mehreren gleichen oder verschiedenen Substituenten R⁸, -C(=O)-C₃₋₅-Cycloalkyl, optional substituiert mit einem oder mehreren gleichen oder verschiedenen Substituenten R⁸, -NH-C(=O)-C₁₋₅-Alkyl, optional substituiert mit einem oder mehreren gleichen oder verschiedenen Substituenten R⁸, -NH-C(=O)-C₃₋₅-Cycloalkyl, optional substituiert mit einem oder mehreren gleichen oder verschiedenen Substituenten R⁸, - C(=O)-NH-C₁₋₅-Alkyl, optional substituiert mit einem oder mehreren gleichen oder verschiedenen Substituenten R⁸ und - C(=O)-NH-C₃₋₅-Cycloalkyl, optional substituiert mit einem oder mehreren gleichen oder verschiedenen Substituenten R⁸;
- R¹⁸ ausgewählt ist aus der Gruppe bestehend aus H, C₁₋₅-Alkyl, optional substituiert mit einem oder mehreren gleichen oder verschiedenen Substituenten R⁸, C₃₋₅-Cycloalkyl, optional substituiert mit einem oder mehreren gleichen oder verschiedenen Substituenten R⁸, -C(=O)-C₁₋₅-Alkyl, optional substituiert mit einem oder mehreren gleichen oder verschiedenen Substituenten R⁸ und -C(=O)-C₃₋₅-Cycloalkyl, optional substituiert mit einem oder mehreren gleichen oder verschiedenen Substituenten R⁸;
- R¹⁹ ausgewählt ist aus der Gruppe bestehend aus C₁₋₅-Alkyl, optional substituiert mit einem oder mehreren gleichen oder verschiedenen Substituenten R⁸, C₃₋₅-Cycloalkyl, optional substituiert mit einem oder mehreren gleichen oder verschiedenen Substituenten R⁸ und Phenyl, optional substituiert mit einem oder mehreren gleichen oder verschiedenen Substituenten R⁹;
- X ausgewählt ist aus der Gruppe bestehend aus N und CR²⁰;
- Y ausgewählt ist aus der Gruppe bestehend aus NH, O und S;
- R²⁰ ausgewählt ist aus der Gruppe bestehend aus H, NH₂, C₁₋₅-Alkyl, optional substituiert mit einem oder mehreren gleichen oder verschiedenen Substituenten R⁸, C₃₋₅-Cycloalkyl, optional substituiert mit einem oder mehreren gleichen oder verschiedenen Substituenten R⁸, -OC₁₋₅-Alkyl, optional substituiert mit einem oder mehreren gleichen oder verschiedenen Substituenten R⁸, - OC₃₋₅-Cycloalkyl, optional substituiert mit einem oder mehreren gleichen oder verschiedenen Substituenten R⁸, -NH-C(=O)C₁₋₅-Alkyl, optional substituiert mit einem oder mehreren gleichen oder verschiedenen Substituenten R⁸ und -NH-SO₂-C₁₋₅-Alkyl, optional substituiert mit einem oder mehreren gleichen oder verschiedenen Substituenten R⁸;
- n eine ganze Zahl 0, 1, 2 oder 3 ist;
oder ein pharmazeutisch verträgliches Salz, Hydrat, Polymorph, Tautomer oder Solvat davon.

2. Verbindung nach Anspruch 1, wobei die Verbindung die Formel (II) aufweist 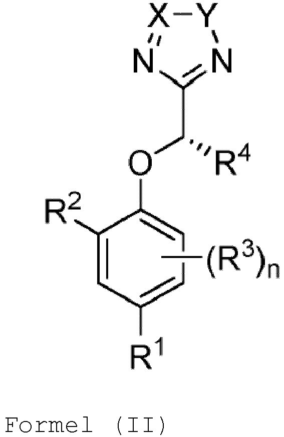 wobei:
- R¹ ausgewählt ist aus der Gruppe bestehend aus F, Cl, Br und I;
- R² ausgewählt ist aus der Gruppe bestehend aus H, F, Cl, Br, I, C₁₋₅-Alkyl, optional substituiert mit einem oder mehreren gleichen oder verschiedenen Substituenten R⁶, C₂₋₅-Alkenyl, optional substituiert mit einem oder mehreren gleichen oder verschiedenen Substituenten R⁶, C₂₋₅-Alkinyl, optional substituiert mit einem oder mehreren gleichen oder verschiedenen Substituenten R⁶, C₃₋₅-Cycloalkyl, optional substituiert mit einem oder mehreren gleichen oder verschiedenen Substituenten R⁶, Phenyl, optional substituiert mit einem oder mehreren gleichen oder verschiedenen Substituenten R⁹ und 5- bis 6-gliedrigem aromatischem Heterocyclus, optional substituiert mit einem oder mehreren gleichen oder verschiedenen Substituenten R⁷;
- R³ ausgewählt ist aus der Gruppe bestehend aus Deuterium und F;
- R⁴ ausgewählt ist aus der Gruppe bestehend aus C₁₋₅-Alkyl, C₂₋₅-Alkenyl, C₂₋₅-Alkinyl und C₃₋₅-Cycloalkyl, die jeweils optional mit einem oder mehreren gleichen oder verschiedenen Substituenten R⁷ substituiert sein können;
- R⁶ unabhängig ausgewählt ist aus der Gruppe bestehend aus C₃₋₅-Cycloalkyl, -O-C₁₋₅-Alkyl, -O-C₃₋₅-Cycloalkyl, -S-C₁₋₅-Alkyl und -S-C₃₋₅-Cycloalkyl, die jeweils optional mit einem oder mehreren gleichen oder verschiedenen Substituenten R⁸, Deuterium, F und -CN substituiert sein können;
- R⁷ unabhängig ausgewählt ist aus der Gruppe bestehend aus C₃₋₅-Cycloalkyl, -O-C₁₋₅-Alkyl, -O-C₃₋₅-Cycloalkyl, -S-C₁₋₅-Alkyl und -S-C₃₋₅-Cycloalkyl, die jeweils optional mit einem oder mehreren gleichen oder verschiedenen Substituenten R⁸, Deuterium, F und -CN substituiert sein können;
- R⁸ unabhängig ausgewählt ist aus der Gruppe bestehend aus Deuterium, OH, OMe und F;
- R⁹ unabhängig ausgewählt ist aus der Gruppe bestehend aus Deuterium, Methoxy, Nitro, Cyano, CF₃, Cl, Br, I und F;
- X ausgewählt ist aus der Gruppe bestehend aus N und CR²⁰;
- Y ausgewählt ist aus der Gruppe bestehend aus NH, O und S;
- R²⁰ ausgewählt ist aus der Gruppe bestehend aus H, NH₂, C₁₋₅-Alkyl, optional substituiert mit einem oder mehreren gleichen oder verschiedenen Substituenten R⁸, C₃₋₅-Cycloalkyl, optional substituiert mit einem oder mehreren gleichen oder verschiedenen Substituenten R⁸, -OC₁₋₅-Alkyl, optional substituiert mit einem oder mehreren gleichen oder verschiedenen Substituenten R⁸, - OC₃₋₅-Cycloalkyl, optional substituiert mit einem oder mehreren gleichen oder verschiedenen Substituenten R⁸, -NH-C(=O)C₁₋₅-Alkyl, optional substituiert mit einem oder mehreren gleichen oder verschiedenen Substituenten R⁸ und -NH-SO₂-C₁₋₅-Alkyl, optional substituiert mit einem oder mehreren gleichen oder verschiedenen Substituenten R⁸;
- n eine ganze Zahl 0, 1, 2 oder 3 ist;
oder ein pharmazeutisch verträgliches Salz, Hydrat, Polymorph, Tautomer oder Solvat davon.

3. Verbindung nach Anspruch 1, wobei die Verbindung die Formel (III) aufweist 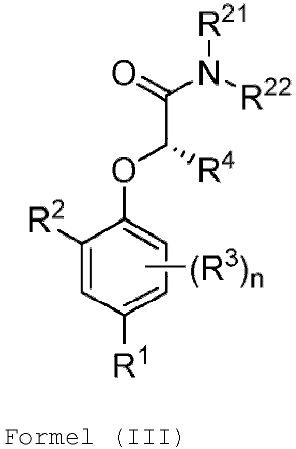 wobei:
- R¹ ausgewählt ist aus der Gruppe bestehend aus F, Cl, Br und I;
- R² ausgewählt ist aus der Gruppe bestehend aus H, F, Cl, Br, I, C₁₋₅-Alkyl, optional substituiert mit einem oder mehreren gleichen oder verschiedenen Substituenten R⁶, C₂₋₅-Alkenyl, optional substituiert mit einem oder mehreren gleichen oder verschiedenen Substituenten R⁶, C₂₋₅-Alkinyl, optional substituiert mit einem oder mehreren gleichen oder verschiedenen Substituenten R⁶, C₃₋₅-Cycloalkyl, optional substituiert mit einem oder mehreren gleichen oder verschiedenen Substituenten R⁶, Phenyl, optional substituiert mit einem oder mehreren gleichen oder verschiedenen Substituenten R⁹ und 5- bis 6-gliedrigem aromatischem Heterocyclus, optional substituiert mit einem oder mehreren gleichen oder verschiedenen Substituenten R⁷;
- R³ ausgewählt ist aus der Gruppe bestehend aus Deuterium und F;
- R⁴ ausgewählt ist aus der Gruppe bestehend aus C₁₋₅-Alkyl, C₂₋₅-Alkenyl, C₂₋₅-Alkinyl und C₃₋₅-Cycloalkyl, die jeweils optional mit einem oder mehreren gleichen oder verschiedenen Substituenten R⁷ substituiert sein können;
- R⁶ unabhängig ausgewählt ist aus der Gruppe bestehend aus C₃₋₅-Cycloalkyl, -O-C₁₋₅-Alkyl, -O-C₃₋₅-Cycloalkyl, -S-C₁₋₅-Alkyl und -S-C₃₋₅-Cycloalkyl, die jeweils optional mit einem oder mehreren gleichen oder verschiedenen Substituenten R⁸, Deuterium, F und -CN substituiert sein können;
- R⁷ unabhängig ausgewählt ist aus der Gruppe bestehend aus C₃₋₅-Cycloalkyl, -O-C₁₋₅-Alkyl, -O-C₃₋₅-Cycloalkyl, -S-C₁₋₅-Alkyl und -S-C₃₋₅-Cycloalkyl, die jeweils optional mit einem oder mehreren gleichen oder verschiedenen Substituenten R⁸, Deuterium, F und -CN substituiert sein können;
- R⁸ ist unabhängig ausgewählt aus der Gruppe bestehend aus Deuterium, OH, OMe und F;
- R⁹ unabhängig ausgewählt ist aus der Gruppe bestehend aus Deuterium, Methoxy, Nitro, Cyano, CF₃, Cl, Br, I und F;
- R¹¹ ausgewählt ist aus der Gruppe bestehend aus C₁₋₅-Alkyl, optional substituiert mit einem oder mehreren gleichen oder verschiedenen Substituenten R⁸, C₃₋₅-Cycloalkyl, optional substituiert mit einem oder mehreren gleichen oder verschiedenen Substituenten R⁸ und Phenyl, optional substituiert mit einem oder mehreren gleichen oder verschiedenen Substituenten R⁹;
- R¹² ausgewählt ist aus der Gruppe bestehend aus -OH, -OC₁₋₅-Alkyl, optional substituiert mit einem oder mehreren gleichen oder verschiedenen Substituenten R⁸ und -OC₃₋₅-Cycloalkyl, optional substituiert mit einem oder mehreren gleichen oder verschiedenen Substituenten R⁸;
- R¹⁵ ausgewählt ist aus der Gruppe bestehend aus H, C₁₋₅-Alkyl, optional substituiert mit einem oder mehreren gleichen oder verschiedenen Substituenten R¹⁶, C₃₋₆-Cycloalkyl, optional substituiert mit einem oder mehreren gleichen oder verschiedenen Substituenten R¹⁶, Phenyl, optional substituiert mit einem oder mehreren gleichen oder verschiedenen Substituenten R⁹ und 4- bis 6-gliedrigem Heterocyclus, optional substituiert mit einem oder mehreren gleichen oder verschiedenen Substituenten R¹⁷ mit der Maßgabe, dass, wenn R¹⁵ H ist, dann R²¹ nicht H ist;
- R¹⁶ unabhängig ausgewählt ist aus der Gruppe bestehend aus F, Cl, Br, I, -CN, =O, C₃₋₆-Cycloalkyl, optional substituiert mit einem oder mehreren gleichen oder verschiedenen Substituenten R⁸, -O-C₁₋₅-Alkyl, optional substituiert mit einem oder mehreren gleichen oder verschiedenen Substituenten R⁸, -O-C₃₋₅-Cycloalkyl, optional substituiert mit einem oder mehreren gleichen oder verschiedenen Substituenten R⁸, -S-C₁₋₅-Alkyl, optional substituiert mit einem oder mehreren gleichen oder verschiedenen Substituenten R⁸, -S-C₃₋₅-Cycloalkyl, optional substituiert mit einem oder mehreren gleichen oder verschiedenen Substituenten R⁸, -SO-C₁₋₅-Alkyl, optional substituiert mit einem oder mehreren gleichen oder verschiedenen Substituenten R⁸, -SO-C₃₋₅-Cycloalkyl, optional substituiert mit einem oder mehreren gleichen oder verschiedenen Substituenten R⁸, -SO₂-C₁₋₅-Alkyl, optional substituiert mit einem oder mehreren gleichen oder verschiedenen Substituenten R⁸, -SO₂-C₃₋₅-Cycloalkyl, optional substituiert mit einem oder mehreren gleichen oder verschiedenen Substituenten R⁸, -C(=O)-C₁₋₅-Alkyl, optional substituiert mit einem oder mehreren gleichen oder verschiedenen Substituenten R⁸, -C(=O)-OC₁₋₅-Alkyl, optional substituiert mit einem oder mehreren gleichen oder verschiedenen Substituenten R⁸, - NH-C(=O)-OC₁₋₅-Alkyl, optional substituiert mit einem oder mehreren gleichen oder verschiedenen Substituenten R⁸, -C(=O)-C₃₋₅-Cycloalkyl, optional substituiert mit einem oder mehreren gleichen oder verschiedenen Substituenten R⁸, -NH-C(=O)-C₁₋₅-Alkyl, optional substituiert mit einem oder mehreren gleichen oder verschiedenen Substituenten R⁸, -NH-C(=O)-C₃₋₅ Cycloalkyl, optional substituiert mit einem oder mehreren gleichen oder verschiedenen R⁸, -C(=O)-NH-C₁₋₅-Alkyl, optional substituiert mit einem oder mehreren gleichen oder verschiedenen Substituenten R⁸, -C(=O)-NH-C₃₋₅-Cycloalkyl, optional substituiert mit einem oder mehreren gleichen oder verschiedenen Substituenten R⁸, Phenyl, optional substituiert mit einem oder mehreren gleichen oder verschiedenen Substituenten R⁹, Pyrrolidin-1-yl, optional substituiert mit einem oder mehreren gleichen oder verschiedenen Substituenten R¹⁷ und 4- bis 6-gliedrigem Heterocyclus, optional substituiert mit einem oder mehreren gleichen oder verschiedenen Substituenten R¹⁷;
- R¹⁷ unabhängig ausgewählt ist aus der Gruppe bestehend aus F, Cl, Br, I, -CN, =O, C₁₋₅-Alkyl, optional substituiert mit einem oder mehreren gleichen oder verschiedenen Substituenten R⁸, C₃₋₆-Cycloalkyl, optional substituiert mit einem oder mehreren gleichen oder verschiedenen Substituenten R⁸, -C(=O)-C₁₋₅-Alkyl, optional substituiert mit einem oder mehreren gleichen oder verschiedenen Substituenten R⁸, -C(=O)-OC₁₋₅-Alkyl, optional substituiert mit einem oder mehreren gleichen oder verschiedenen Substituenten R⁸, -C(=O)-C₃₋₅-Cycloalkyl, optional substituiert mit einem oder mehreren gleichen oder verschiedenen Substituenten R⁸, -NH-C(=O)-C₁₋₅-Alkyl, optional substituiert mit einem oder mehreren gleichen oder verschiedenen Substituenten R⁸, -NH-C(=O)-C₃₋₅-Cycloalkyl, optional substituiert mit einem oder mehreren gleichen oder verschiedenen Substituenten R⁸, - C(=O)-NH-C₁₋₅-Alkyl, optional substituiert mit einem oder mehreren gleichen oder verschiedenen Substituenten R⁸ und - C(=O)-NH-C₃₋₅-Cycloalkyl, optional substituiert mit einem oder mehreren gleichen oder verschiedenen Substituenten R⁸;
- R¹⁹ ausgewählt ist aus der Gruppe bestehend aus C₁₋₅-Alkyl, optional substituiert mit einem oder mehreren gleichen oder verschiedenen Substituenten R⁸, C₃₋₅-Cycloalkyl, optional substituiert mit einem oder mehreren gleichen oder verschiedenen Substituenten R⁸ und Phenyl optional substituiert mit einem oder mehreren gleichen oder verschiedenen Substituenten R⁹;
- R²¹ ausgewählt ist aus der Gruppe bestehend aus H, C₁₋₅-Alkyl, optional substituiert mit einem oder mehreren gleichen oder verschiedenen Substituenten R⁸, C₃₋₅-Cycloalkyl, optional substituiert mit einem oder mehreren gleichen oder verschiedenen Substituenten R⁸, -C(=O)-C₁₋₅-Alkyl, optional substituiert mit einem oder mehreren gleichen oder verschiedenen Substituenten R⁸ und -C(=O)-C₃₋₅-Cycloalkyl, optional substituiert mit einem oder mehreren gleichen oder verschiedenen Substituenten R⁸;
- R²² ausgewählt ist aus der Gruppe bestehend aus -C(R¹¹)=NR¹², - CN, -OR¹⁵ und SO₂R¹⁹; und
- n eine ganze Zahl 0, 1, 2 oder 3 ist;
oder ein pharmazeutisch verträgliches Salz, Hydrat, Polymorph, Tautomer oder Solvat davon.

4. Verbindung nach einem der Ansprüche 1 bis 3, wobei R¹ Cl oder Br ist.

5. Verbindung nach einem der Ansprüche 1 bis 4, wobei R² H, F, Cl, Br oder I ist.

6. Verbindung nach einem der Ansprüche 1 bis 4, wobei R² ein 5-bis 6-gliedriger aromatischer Heterocyclus ist, optional substituiert mit einem oder mehreren gleichen oder verschiedenen Substituenten R⁷.

7. Verbindung nach einem der Ansprüche 1 bis 6, wobei R⁴ C₁₋₅-Alkyl ist, optional substituiert mit einem oder mehreren gleichen oder verschiedenen Substituenten R⁷.

8. Verbindung nach einem der Ansprüche 1 bis 2 und 4 bis 6, wobei X N ist und Y NH ist.

9. Verbindung nach einem der Ansprüche 3 bis 6, wobei R²¹ H ist.

10. Verbindung zur Verwendung nach einem der Ansprüche 1 bis 9, wobei die Verbindung ausgewählt ist aus der Gruppe bestehend aus:
(2S)-2-(4-Brom-2-fluorphenoxy)-N-{1-[(cyclopropylmethoxy)imino]ethyl}propanamid;
(2*S*)-2-(4-Bromphenoxy)-*N*-[1-(methoxyimino)ethyl]propanamid;
(2S)-2-(4-Brom-2-fluorphenoxy)-*N*-[(4-fluorphenyl)(hydroxyimino)methyl]-3-methylbutanamid;
(2*S*)-2-(4-Brom-2-fluorphenoxy)-*N*-[1-(hydroxyimino)ethyl]-3-methylbutanamid;
(2*S*)-2-(4-Brom-2-fluorphenoxy)-*N*-[1-(hydroxyimino)ethyl]propanamid;
(2*S*)-2-(4-Bromphenoxy)-*N*-[1-(hydroxyimino)ethyl]propenamid;
(2*S*)-2-[4-Brom-2-(1,2-oxazol-3-yl)phenoxy]-*N*-cyanopropanamid;
(2*S*)-N-Cyano-2-(2,4-dibromphenoxy)propanamid;
(2*S*)-2-(4-Bromphenoxy)-*N*-cyanopropanamid;
(2*S*)-2-(4-Chlorphenoxy)-*N*-cyanopropanamid;
(2*S*)-2-(4-Bromphenoxy)-*N*-cyano-3-methylbutanamid;
(2*S*)-2-[4-Brom-2-(1,2-oxazol-3-yl)phenoxyl-*N-*cyclobutoxypropanamid;
(2*S*)-2-[4-Brom-2-(1,2-oxazol-3-yl)phenoxy]-*N-*methoxypropanamid;
(2*S*)-*N*-Acetyl-*N*-[(1-acetylazetidin-3-yl)oxyl-2-(4-chlorphenoxy)propanamid;
(2*S*)-*N*-[(1-Acetylazetidin-3-yl)oxy]-2-(4-chlorphenoxy)propanamid;
(2S)-N-(Azetidin-3-yloxy)-2-(4-chlorphenoxy)propanamid;
(2*S*)-*N*-Acetyl-*N*-[(1-acetylpyrrolidin-3-yl)methoxy]-2-(4-chlorphenoxy)propanamid;
(2*S*)-*N*-[(1-Acetylpyrrolidin-3-yl)methoxy]-2-(4-chlorphenoxy)propanamid;
(2*S*)-*N*-[(1-Acetylpyrrolidin-3-yl)oxy]-2-(4-bromphenoxy)propanamid;
tert-Butyl-*N*-(2-{[(2*S*)-2-(4-chlorphenoxy)propanamido]oxy}propyl)carbamat;
(2*S*)-*N*-Acetyl-2-(4-chlorphenoxy)-*N*-[(1-acetamidopropan-2-yl)oxy]propanamid;
(2*S*)-2-(4-Bromphenoxy)-*N*-(pyrrolidin-3-yloxy)propanamid;
(2*S*)-*N*-Acetyl-*N*-[(1-acetylpyrrolidin-3-yl)oxy]-2-(4-bromphenoxy)propanamid;
tert-Butyl-3-{[(2*S*)-2-(4-chlorphenoxy)propanamido]oxy}azetidin-1-carboxylat;
(2*S*)-2-(4-Chlorphenoxy)-*N*-[(pyrrolidin-3-yl)methoxy]propanamid;
tert-Butyl-3-({[(2*S*)-2-(4-chlorophenoxy)propanamido]oxy}methyl)pyrrolidin-1-carboxylat;
(2S)-*N*-Acetyl-*N*-[(1-acetylpyrrolidin-3-yl)oxy]-2-(4-chlorphenoxy)propanamid;
(2*S*)-*N*-[(1-Acetylpyrrolidin-3-yl)oxyl-2-(4-chlorphenoxy)propanamid;
(2*S*)-2-(4-Chlorphenoxy)-*N*-(pyrrolidin-3-yloxy)propanamid;
(2*S*)-2-(4-Chlorphenoxy)-*N*-(2,2-dimethylpropyl)-*N-*hydroxypropanamid;
(2*S*)-2-(4-Chlorphenoxy)-N-[2-(pyrrolidin-1-yl)ethoxy]propanamid;
(2*S*)-2-(4-Chlorphenoxy)-*N*-[2-(2-oxopyrrolidin-1-yl)ethoxy]propanamid;
(2*S*)-*N*-Acetyl-2-(4-chlorphenoxy)-*N*-(2-acetamidoethoxy)propanamid;
(2*S*)-2-(4-Chlorphenoxy)-*N*-(2-acetamidoethoxy)propanamid;
(2*S*)-2-(4-Chlorphenoxy)-*N*-(4,4,4-trifluor-2-methylbutoxy)propanamid;
(2*S*)-2-(4-Chlorphenoxy)-*N*-(3-cyclopentylpropyl)-*N-*hydroxypropanamid;
(2*S*)-2-(4-Chlorphenoxy)-*N*-{ [(2E)-2-methyl-3-phenylprop-2-en-1-yl]oxy}propanamid;
(2*S*)-2-(4-Chlorphenoxy)-*N*-{2-oxo-2-[2-(trifluormethyl)pyrrolidin-1-yl]ethoxy}propanamid;
tert-Butyl-*N*-(2-{[(2*S*)-2-(4-chlorphenoxy)propanamido]oxy}propyl)carbamat;
(2*S*)-2-(4-Chlorphenoxy)-*N*-[(2-methyl-1H-imidazol-4-yl)methoxy]propanamid;
tert-Butyl-4-({[(2*S*)-2-(4-chlorphenoxy)propanamido]oxy}methyl)-2-methyl-1H-imidazol-1-carboxylat;
(2*S*)-2-(4-Brom-2-fluorphenoxy)-*N*-cyclobutoxypropanamid;
(2*S*)-2-(4-Bromphenoxy)-*N*-cyclobutoxypropanamid;
(2*S*)-2-(4-Chlorphenoxy)-*N*-[2-oxo-2-(pyrrolidin-1-yl)ethoxy]propanamid;
(2*S*)-2-(4-Chlorphenoxy)-*N*-[1-(4-fluorphenyl)ethoxy]propanamid;
(2*S*)-2-(4-Chlorphenoxy)-*N*-[1-(1,3-thiazol-2-yl)ethoxy]propanamid;
(2*S*)-2-(4-Chlorphenoxy)-*N*-{1-[4-(trifluormethyl)phenyl]ethoxy}propanamid;
(2*S*)-2-(4-Chlorphenoxy)-*N*-(2-methansulfinylethoxy)propanamid;
(2*S*)-2-(4-Chlorphenoxy)-*N*-(2-methansulfonylethoxy)propanamid;
(2*S)*-2-(4-Chlorphenoxy)-*N*-[(1,2-oxazol-3-yl)methoxy]propanamid;
(2*S*)-2-(4-Chlor-2-fluorphenoxy)-*N*-methoxypropanamid;
(2*S*)-2-[4-Brom-2-(1,3,4-oxadiazol-2-yl)phenoxy]-*N-*(cyclopropylmethoxy)propanamid;
(2*S*)-2-[4-Chlor-2-(trifluormethyl)phenoxy]-*N-*(cyclopropylmethoxy)propanamid;
(2*S*)-*N*-(tert-Butoxy)-2-(4-chlorphenoxy)propanamid;
(2*S*)-2-(4-Chlorphenoxy)-*N*-[2-(methylsulfanyl)ethoxy]propanamid;
(2*S*)-2-(4-Chlorphenoxy)-*N*-(1-phenylethoxy)propanamid;
(2*S*)-2-(4-Chlorphenoxy)-*N*-[(1-methyl-1H-imidazol-2-yl)methoxy]propanamid;
(2*S*)-2-(4-Chlorphenoxy)-*N*-(2-methoxyethoxy)propanamid;
(2*S*)-2-(4-Chlor-2-fluorphenoxy)-*N*-cyclobutoxypropanamid;
(2*S*)-2-[4-Brom-2-(1,3,4-oxadiazol-2-yl)phenoxy]-*N-*methoxypropanamid;
(2*S*)-2-[4-Chlor-2-(trifluormethyl)phenoxy]-*N-*methoxypropanamid;
(2*S*)-*N*-(Benzyloxy)-2-(4-chlorphenoxy)propanamid;
(2*S*)-2-(4-Chlorphenoxy)-*N*-[(2-methoxycyclopentyl)oxy]propanamid;
(2*S*)-2-(4-Chlorphenoxy)-*N*-cyclobutoxy-5-methylhexanamid;
(2*S*)-2-(4-Chlorphenoxy)-*N*-cyclobutoxy-*N*-methylpropanamid;
(2*S*)-2-(4-Chlor-2-methylphenoxy)-*N*-cyclobutoxypropanamid;
(2*S*)-2-(4-Chlor-3-fluorphenoxy)-*N*-cyclobutoxypropanamid;
(2*S*)-2-(4-Chlor-2-methylphenoxy)-*N*-methoxypropanamid;
(2*S*)-2-(4-Chlor-3-fluorphenoxy)-*N*-methoxypropanamid;
(2*S*)-2-(4-Chlorphenoxy)-*N*-cyclobutoxy-4-methylpentanamid;
(2*S*)-2-(4-Chlorphenoxy)-*N*-[(3-methylbut-2-en-1-yl)oxy]propanamid;
(2*S*)-2-(4-Chlorphenoxy)-*N*-cyclobutoxyhexanamid;
(2*S*)-2-(4-Chlorphenoxy)-*N*-[(1,3-oxazol-2-yl)methoxy]propanamid;
(2*S*)-2-(2,4-Dibromphenoxy)-*N*-methoxypropanamid;
(2*S*)-2-(4-Brom-2-fluorphenoxy)-*N*-methoxypropanamid;
(2*S*)-2-(4-Chlorphenoxy)-*N*-(oxan-2-yloxy)propanamid;
(2*S*)-2-(4-Chlorphenoxy)-*N*-[(1,3-thiazol-2-yl)methoxy]propanamid;
(2*S*)-2-(4-Chlorphenoxy)-*N*-(3,3-difluorcyclobutoxy)propanamid;
(2*S*)-2-[4-Brom-2-(1,2-oxazol-5-yl)phenoxy]-*N-*cyclobutoxypropanamid;
(2*S*)-2-[4-Brom-2-(1,2-oxazol-5-yl)phenoxy]-*N-*methoxypropanamid;
(2*S*)-2-(4-Chlorphenoxy)-*N*-(cyclopentyloxy)propanamid;
(2*S*)-2-(4-Chlorphenoxy)-*N*-(2-cyclopentylethoxy)propanamid;
(2*S*)-2-(4-Brom-2-chlorphenoxy)-*N*-methoxypropanamid;
(2*S*)-2-(4-Brom-2-methylphenoxy)-*N*-methoxypropanamid;
(2*S*)-2-(4-Chlorphenoxy)-*N*-(cyclopropylmethoxy)propanamid;
(2*S*)-2-(4-Chlorphenoxy)-*N*-(cyclobutylmethoxy)propanamid;
(2*S*)-*N*-(2-Aminoethoxy)-2-(4-chlorphenoxy)propanamid;
(2*S*)-2-(4-Bromphenoxy)-*N*-methoxy-3-methylbutanamid; Methyl-2-{[(2*S*)-2-(4-chlorphenoxy)propanamido]oxy}acetat;
(2*S*)-2-(4-Chlorphenoxy)-*N*-[2-(2-methoxyethoxy)ethoxy]propanamid;
(2*S*)-2-(4-Chlorphenoxy)-*N*-cyclobutoxypropanamid;
(2*S*)-2-(4-Chlorphenoxy)-*N*-(2-hydroxyethoxy)propanamid;
(2*S*)-2-(4-Chlorphenoxy)-*N*-ethoxypropanamid;
(2*S*)-2-(4-Chlorphenoxy)-*N*-propoxypropanamid;
(2*S*)-2-(4-Chlorphenoxy)-*N*-(propan-2-yloxy)propanamid;
(2*S*)-2-(4-Chlorphenoxy)-*N*-methoxypropanamid;
(2*S*)-2-[4-Brom-2-(1,2-oxazol-3-yl)phenoxy]-*N-*(cyclopropansulfonyl)propenamid;
(2*S*)-2-(4-Brom-2-fluorphenoxy)-*N*-methansulfonylpropanamid;
(2*S*)-2-(4-Brom-2-fluorphenoxy)-*N-*(cyclopropansulfonyl)propenamid;
(2*S*)-2-(4-Chlorphenoxy)-*N*-methansulfonylpropanamid;
(2*S*)-2-(4-Bromphenoxy)-*N*-methansulfonyl-3-methylbutanamid;
5-[(1*S*)-1-[4-Brom-2-(1,2-oxazol-3-yl)phenoxy]-3-fluorpropyl]-2H-1,2,3,4-tetrazol;
5-[(1*S*)-1-(4-Brom-2-fluorphenoxy)propyl]-2H-1,2,3,4-tetrazol;
5-[(1*S*)-1-(4-Brom-2-fluorphenoxy)-2-methylpropyl]-2H-1,2,3,4-tetrazol;
5-[(*1R*)-1-(4-Bromphenoxy)-2-fluorethyl]-2H-1,2,3,4-tetrazol;
5-[(1*S*)-1-(4-Bromphenoxy)propyl]-2H-1,2,3,4-tetrazol;
5-[(1*S*)-1-(4-Bromphenoxy)ethyl]-2H-1,2,3,4-tetrazol;
5-[(1*S*)-1-[4-Brom-2-(1,2-oxazol-3-yl)phenoxy]ethyl]-1H-1,2,3,4-tetrazol;
5-[(1*S*)-1-(4-Brom-2-fluorphenoxy)ethyl]-2H-1,2,3,4-tetrazol;
5-[(1*S*)-1-[4-Brom-2-(1,2-oxazol-5-yl)phenoxy]ethyl]-2H-1,2,3,4-tetrazol;
5-[(1*S*)-1-[4-Chlor-2-(1,2-oxazol-3-yl)phenoxy]ethyl]-2H-1,2,3,4-tetrazol;
5-[(1*S*)-1-(4-Chlor-2-cyclopropylphenoxy)ethyl]-1H-1,2,3,4-tetrazol;
5-[(1*S*)-1-(4-Chlor-2-ethenylphenoxy)ethyl]-1H-1,2,3,4-tetrazol;
5-[(1*S*)-1-(4-Chlor-2-ethylphenoxy)ethyl]-1H-1,2,3,4-tetrazol;
5-[(1*S*)-1-(4-Chlor-2-methylphenoxy)ethyl]-2H-1,2,3,4-tetrazol;
5-[(1*S*)-1-(4-Chlorphenoxy)ethyl]-1H-1,2,3,4-tetrazol;
N*-(3-[(1S)-1-(4-Brom-2-fluorphenoxy)ethyl]-1,2,4-thiadiazol-5-yl}acetamid;*
N*-(3-[(1S)-1-(4-Brom-2-fluorphenoxy)ethyl]-1,2,4-thiadiazol-5-yl}methansulfonamid;*
3-[(1*S*)-1-(4-Brom-2-fluorphenoxy)ethyl]-1,2,4-thiadiazol-5-amin;
N*-[3-[(1S)-1-(4-B-rom-2-fluo-rphenoxy)ethyll-1,2,4-oxadiazol-5-yl}acetamid;*
N*-(3-[(1S)-1-(4-Brom-2-fluorphenoxy)ethyl]-1,2,4-oxadiazol-5-yl}methansulfonamid;*
3-[(1*S*)-1-[4-Brom-2-(1,2-oxazol-3-yl)phenoxy]ethyl]-5-(trifluormethyl)-4H-1,2,4-triazol;
3-[(1*S*)-1-[4-Chlor-2-(1,2-oxazol-3-yl)phenoxy]ethyl]-5-(trifluormethyl)-4H-1,2,4-triazol;
3-[(1*S*)-1-(4-Brom-2-fluorphenoxy)ethyl]-5-methoxy-1,2,4-oxadiazol;
3-[(1*S*)-1-(4-Brom-2-fluorphenoxy)ethyl]-1,2,4-oxadiazol-5-amin;
3-[(1*S*)-1-[4-Brom-2-(1,2-oxazol-3-yl)phenoxy]ethyl]-5-methyl-4H-1,2,4-triazol;
3-[(1*S*)-1-[4-Chlor-2-(1,2-oxazol-3-yl)phenoxy]ethyl]-5-methyl-4H-1,2,4-triazol;
3-[(1*S*)-1-(4-Brom-2-fluorphenoxy)ethyl]-5-methyl-4H-1,2,4-triazol;
5-[(1*S*)-1-(4-Chlorphenoxy)ethyl]-1H-1,2,4-triazol;
(2*S*)-2-[4-Brom-2-(1,1-difluorethyl)phenoxy]-*N*-cyanopropanamid;
(2*S*)-2-[4-Brom-2-(1,1-difluorpropyl)-5-fluorphenoxy]-*N-*cyanopropanamid;
(2*S*)-2-[4-Brom-2-(1,1-difluorpropyl)phenoxy]-*N-*methansulfonylpropanamid;
(2*S*)-2-[4-Brom-2-(1,1-difluorpropyl)phenoxy]-*N-*(cyclopropansulfonyl)propenamid;
5-[(1*S*)-1-[4-Brom-2-(1,1-difluorethyl)phenoxy]ethyl]-1H-1,2,3,4-tetrazol;
5-[(1*S*)-1-[4-Brom-2-(1,1-difluorpropyl)phenoxy]ethyl]-1H-1,2,3,4-tetrazol;
5-[(1*S*)-1-(4-Brom-2-cyclobutylphenoxy)-2-methoxyethyl]-1H-1,2,3,4-tetrazol; und
5-[(1*S*)-1-(4-Brom-2-cyclopropylphenoxy)-2-methoxyethyl]-1H-1,2,3,4-tetrazol.

11. Verbindung nach einem der Ansprüche 1 bis 10, wobei die Verbindung ein Inhibitor des CIC-1-Ionenkanals ist.

12. Zusammensetzung, umfassend die Verbindung nach einem der Ansprüche 1 bis 11 und einen pharmazeutisch verträglichen Träger.

13. Verbindung nach einem der Ansprüche 1 bis 11 oder Zusammensetzung nach Anspruch 12 zur Verwendung als Medikament.

14. Verbindung nach einem der Ansprüche 1 bis 11 oder Zusammensetzung nach Anspruch 12 zur Verwendung bei der Behandlung von Symptomen einer Indikation, ausgewählt aus der Gruppe bestehend aus Myasthenia gravis, Lambert-Eaton-Syndrom, Critical Illness Myopathie, amyotropher Lateralsklerose (ALS), spinaler Muskelatrophie (SMA), Critical Illness Myopathie (CIM), umgekehrte diabetische Polyneuropathie, Guillain-Earré-Syndrom, Poliomyelitis, Post-Polio-Syndrom, chronischem Müdigkeitssyndrom, Critical Illness Polyneuropathie, periodischer Paralyse, Sarkopenie, hypokaliämischer periodischer Paralyse und hyperkaliämischer periodischer Paralyse .

15. Verbindung nach einem der Ansprüche 1 bis 11 oder Zusammensetzung nach Anspruch 12 zur Verwendung beim Umkehren und/oder Lindern einer neuromuskulären Blockade.

## Revendications

1. Composé selon la formule (I) : 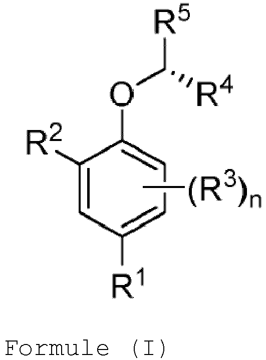 dans laquelle :
- R¹ est choisi dans le groupe constitué de F, de Cl, de Br et de I ;
- R² est choisi dans le groupe constitué de H, de F, de Cl, de Br, de I, de l'alkyle en C₁₋₅ éventuellement remplacé par un ou plusieurs substituants R⁶, identiques ou différents, de l'alcényle en C₂₋₅ éventuellement remplacé par un ou plusieurs substituants R⁶, identiques ou différents, de l'alcynyle en C₂₋₅ éventuellement remplacé par un ou plusieurs substituants R⁶, identiques ou différents, du cycloalkyle en C₃₋₅ éventuellement remplacé par un ou plusieurs substituants R⁶, identiques ou différents, du phényle éventuellement remplacé par un ou plusieurs substituants R⁹, identiques ou différents, et d'un hétérocycle aromatique à 5 ou 6 chaînons éventuellement remplacé par un ou plusieurs substituants R⁷, identiques ou différents ;
- R³ est choisi dans le groupe constitué du deutérium et de F ;
- R⁴ est choisi dans le groupe constitué de l'alkyle en C₁₋₅, de l'alcényle en C₂₋₅, de l'alcynyle en C₂₋₅ et du cycloalkyle en C₃₋₅, chacun d'entre eux pouvant être éventuellement remplacé par un ou plusieurs substituants R⁷ identiques ou différents ;
- R⁵ est choisi dans le groupe constitué de 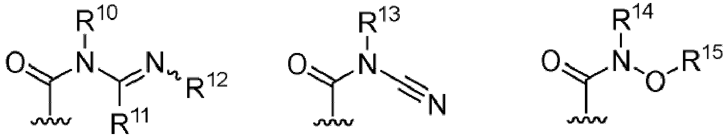 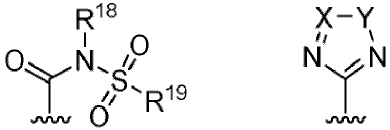
- R⁶ est indépendamment choisi dans le groupe constitué du cycloalkyle en C₃₋₅, de l'alkyle en -O-C₁₋₅, du cycloalkyle en - O-C₃₋₅, de l'alkyle en -S-C₁₋₅ et du cycloalkyle en -S-C₃₋₅, chacun d'entre eux pouvant éventuellement être remplacé par un ou plusieurs substituants R⁸, identiques ou différents, du deutérium, de F et de -CN ;
- R⁷ est indépendamment choisi dans le groupe constitué du cycloalkyle en C₃₋₅, de l'alkyle en -O-C₁₋₅, du cycloalkyle en - O-C₃₋₅, de l'alkyle en -S-C₁₋₅ et du cycloalkyle en -S-C₃₋₅, chacun d'entre eux pouvant éventuellement être remplacé par un ou plusieurs substituants R⁸, identiques ou différents, du deutérium, du F et du -CN ;
- R⁸ est indépendamment choisi dans le groupe constitué du deutérium, de OH, de OMe et de F ;
- R⁹ est indépendamment choisi dans le groupe constitué du deutérium, du méthoxy, du nitro, du cyano, de CF₃, de Cl, de Br, de I et de F ;
- R¹⁰ est choisi dans le groupe constitué de H, de l'alkyle en C₁₋₅ éventuellement remplacé par un ou plusieurs substituants R⁸, identiques ou différents, du cycloalkyle en C₃₋₅ éventuellement remplacé par un ou plusieurs substituants R⁸, identiques ou différents, de l'alkyle en -C(=O)-C₁₋₅ éventuellement remplacé par un ou plusieurs substituants R⁸, identiques ou différents, et du cycloalkyle en -C(=O)-C₃₋₅, éventuellement remplacé par un ou plusieurs substituants R⁸, identiques ou différents ;
- R¹¹ est choisi dans le groupe constitué d'un alkyle en C₁₋₅ éventuellement remplacé par un ou plusieurs substituants R⁸, identiques ou différents, d'un cycloalkyle en C₃₋₅ éventuellement remplacé par un ou plusieurs substituants R⁸, identiques ou différents, et d'un phényle, éventuellement remplacé par un ou plusieurs substituants R⁹, identiques ou différents ;
- R¹² est choisi dans le groupe constitué de -OH, de l'alkyle en -OC₁₋₅, éventuellement remplacé par un ou plusieurs substituants R⁸, identiques ou différents, et du cycloalkyle en
- OC₃₋₅ éventuellement remplacé par un ou plusieurs substituants R⁸, identiques ou différents ;
- R¹³ est choisi dans le groupe constitué de H, de l'alkyle en C₁₋₅ éventuellement remplacé par un ou plusieurs substituants R⁸, identiques ou différents, du cycloalkyle en C₃₋₅ éventuellement remplacé par un ou plusieurs substituants R⁸, identiques ou différents, de l'alkyle en -C(=O)-C₁₋₅ éventuellement remplacé par un ou plusieurs substituants R⁸, identiques ou différents, et du cycloalkyle en -C(=O)-C₃₋₅, éventuellement remplacé par un ou plusieurs substituants R⁸, identiques ou différents ;
- R¹⁴ est choisi dans le groupe constitué de H, de l'alkyle en C₁₋₅ éventuellement remplacé par un ou plusieurs substituants R¹⁷, identiques ou différents, du cycloalkyle en C₃₋₅ éventuellement remplacé par un ou plusieurs substituants R⁸, identiques ou différents, de l'alkyle en -C(=O)-C₁₋₅ éventuellement remplacé par un ou plusieurs substituants R⁸, identiques ou différents, et du cycloalkyle en -C(=O)-C₃₋₅, éventuellement remplacé par un ou plusieurs substituants R⁸, identiques ou différents ;
- R¹⁵ est choisi dans le groupe constitué de H, de l'alkyle en C₁₋₅ éventuellement remplacé par un ou plusieurs substituants R¹⁶, identiques ou différents, du cycloalkyle en C₃₋₆ éventuellement remplacé par un ou plusieurs substituants R¹⁶, identiques ou différents, du phényle éventuellement remplacé par un ou plusieurs substituants R⁹, identiques ou différents, et d'un hétérocycle à 4 à 6 chaînons éventuellement remplacé par un ou plusieurs substituants R¹⁷, identiques ou différents, à condition que, lorsque R¹⁵ est H, alors R¹⁴ n'est pas H ;
- R¹⁶ est indépendamment choisi dans le groupe constitué de F, de Cl, de Br, de I, de -CN, de =O, du cycloalkyle en C₃₋₆ éventuellement remplacé par un ou plusieurs substituants R⁸, identiques ou différents, de l'alkyle en -O-C₁₋₅, éventuellement remplacé par un ou plusieurs substituants R⁸, identiques ou différents, du cycloalkyle en -O-C₃₋₅, éventuellement remplacé par un ou plusieurs substituants R⁸, identiques ou différents, de l'alkyle en -S-C₁₋₅ éventuellement remplacé par un ou plusieurs substituants R⁸, identiques ou différents, du cycloalkyle en -S-C₃₋₅ éventuellement remplacé par un ou plusieurs substituants R⁸, identiques ou différents, de l'alkyle en -SO-C₁₋₅ éventuellement remplacé par un ou plusieurs substituants R⁸, identiques ou différents, du cycloalkyle en-SO-C₃₋₅ éventuellement remplacé par un ou plusieurs substituants R⁸, identiques ou différents, de l'alkyle en -SO₂-C₁₋₅ éventuellement remplacé par un ou plusieurs substituants R⁸, identiques ou différents, du cycloalkyle en -SO₂-C₃₋₅ éventuellement remplacé par un ou plusieurs substituants R⁸, identiques ou différents, de l'alkyle en -C(=O)-C₁₋₅ éventuellement remplacé par un ou plusieurs substituants R⁸, identiques ou différents, de l'alkyle en -C(=O)-OC₁₋₅ éventuellement remplacé par un ou plusieurs substituants R⁸, identiques ou différents, de l'alkyle en -NH-C(=O)-OC₁₋₅ éventuellement remplacé par un ou plusieurs substituants R⁸, identiques ou différents, du cycloalkyle en -C(=O) -C₃₋₅ éventuellement remplacé par un ou plusieurs substituants R⁸, identiques ou différents, de l'alkyle en -NH-C(=O) -C₁₋₅ éventuellement remplacé par un ou plusieurs substituants R⁸, identiques ou différents différents, du cycloalkyle en -NH-C(=O) -C₃₋₅ éventuellement remplacé par un ou plusieurs substituants R⁸, identiques ou différents, de l'alkyle en -C(=O)-NH-C₁₋₅ éventuellement remplacé par un ou plusieurs substituants R⁸, identiques ou différents, du cycloalkyle en -C(=O) -NH-C₃₋₅ éventuellement remplacé par un ou plusieurs substituants R⁸, identiques ou différents, du phényle éventuellement remplacé par un ou plusieurs substituants R⁹, identiques ou différents, du pyrrolidin-1-yle éventuellement remplacé par un ou plusieurs substituants R¹⁷, identiques ou différents, et un hétérocycle à 4 à 6 chaînons éventuellement remplacé par un ou plusieurs substituants R¹⁷, identiques ou différents ;
- R¹⁷ est indépendamment choisi dans le groupe constitué de F, de Cl, de Br, de I, de -CN, de =O, de l'alkyle en C₁₋₅ éventuellement remplacé par un ou plusieurs substituants R⁸, identiques ou différents, du cycloalkyle en C₃₋₆ éventuellement remplacé par un ou plusieurs substituants R⁸, identiques ou différents, de l'alkyle en -C(=O)-C₁₋₅ éventuellement remplacé par un ou plusieurs substituants R⁸, identiques ou différents, de l'alkyle en -C(=O)-OC₁₋₅ éventuellement remplacé par un ou plusieurs substituants R⁸, identiques ou différents, du cycloalkyle en -C(=O)-C₃₋₅ éventuellement remplacé par un ou plusieurs substituants R⁸, identiques ou différents, de l'alkyle en -NH-C(=O)-C₁₋₅ éventuellement remplacé par un ou plusieurs substituants R⁸, identiques ou différents, du cycloalkyle en-NH-C(=O)-C₃₋₅ éventuellement remplacé par un ou plusieurs substituants R⁸, identiques ou différents, de l'alkyle en -C(=O)-NH-C₁₋₅ éventuellement remplacé par un ou plusieurs substituants R⁸, identiques ou différents, et du cycloalkyle en -C(=O)-NH-C₃₋₅ éventuellement remplacé par un ou plusieurs substituants R⁸, identiques ou différents ;
- R¹⁸ est choisi dans le groupe constitué de H, de l'alkyle en C₁₋₅ éventuellement remplacé par un ou plusieurs substituants R⁸, identiques ou différents, du cycloalkyle en C₃₋₅ éventuellement remplacé par un ou plusieurs substituants R⁸, identiques ou différents, de l'alkyle en -C(=O)-C₁₋₅ éventuellement remplacé par un ou plusieurs substituants R⁸, identiques ou différents, et du cycloalkyle en -C(=O)-C₃₋₅, éventuellement remplacé par un ou plusieurs substituants R⁸, identiques ou différents ;
- R¹⁹ est choisi dans le groupe constitué d'un alkyle en C₁₋₅ éventuellement remplacé par un ou plusieurs substituants R⁸, identiques ou différents, d'un cycloalkyle en C₃₋₅ éventuellement remplacé par un ou plusieurs substituants R⁸, identiques ou différents, et d'un phényle, éventuellement remplacé par un ou plusieurs substituants R⁹, identiques ou différents ;
- X est choisi dans le groupe constitué de N et de CR²⁰ ;
- Y est choisi dans le groupe constitué de NH, de O et de S ;
- R²⁰ est choisi dans le groupe constitué de H, de NH₂, de l'alkyle en C₁₋₅ éventuellement remplacé par un ou plusieurs substituants R⁸, identiques ou différents, du cycloalkyle en C₃₋₅ éventuellement remplacé par un ou plusieurs substituants R⁸, identiques ou différents, de l'alkyle en -OC₁₋₅ éventuellement remplacé par un ou plusieurs substituants R⁸, identiques ou différents, du cycloalkyle en -OC₃₋₅ éventuellement remplacé par un ou plusieurs substituants R⁸, identiques ou différents, d'un alkyle en -NH-C(=O)C₁₋₅ éventuellement remplacé par un ou plusieurs substituants R⁸, identiques ou différents, et d'un alkyle en -NH-SO₂-C₁₋₅ éventuellement remplacé par un ou plusieurs substituants R⁸, identiques ou différents ;
- n est un nombre entier 0, 1, 2 ou 3 ;
ou un sel, un hydrate, un polymorphe, un tautomère ou un solvate pharmaceutiquement acceptable de celui-ci.

2. Composé selon la revendication 1, dans lequel le composé est de formule (II) 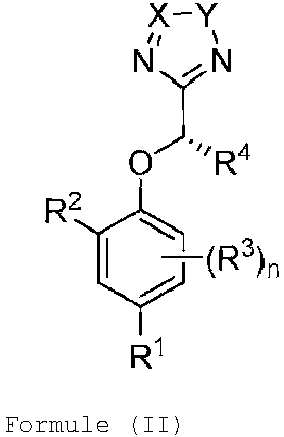 dans laquelle :
- R¹ est choisi dans le groupe constitué de F, de Cl, de Br et de I ;
- R² est choisi dans le groupe constitué de H, de F, de Cl, de Br, de I, de l'alkyle en C₁₋₅ éventuellement remplacé par un ou plusieurs substituants R⁶, identiques ou différents, de l'alcényle en C₂₋₅ éventuellement remplacé par un ou plusieurs substituants R⁶, identiques ou différents, de l'alcynyle en C₂₋₅ éventuellement remplacé par un ou plusieurs substituants R⁶, identiques ou différents, du cycloalkyle en C₃₋₅ éventuellement remplacé par un ou plusieurs substituants R⁶, identiques ou différents, du phényle éventuellement remplacé par un ou plusieurs substituants R⁹, identiques ou différents, et d'un hétérocycle aromatique à 5 ou 6 chaînons éventuellement remplacé par un ou plusieurs substituants R⁷, identiques ou différents ;
- R³ est choisi dans le groupe constitué du deutérium et de F ;
- R⁴ est choisi dans le groupe constitué de l'alkyle en C₁₋₅, de l'alcényle en C₂₋₅, de l'alcynyle en C₂₋₅ et du cycloalkyle en C₃₋₅, chacun d'entre eux pouvant être éventuellement remplacé par un ou plusieurs substituants R⁷, identiques ou différents ;
- R⁶ est indépendamment choisi dans le groupe constitué du cycloalkyle en C₃₋₅, de l'alkyle en -O-C₁₋₅, du cycloalkyle en-O-C₃₋₅, de l'alkyle en -S-C₁₋₅ et du cycloalkyle en -S-C₃₋₅, chacun d'entre eux pouvant éventuellement être remplacé par un ou plusieurs substituants R⁸, identiques ou différents, du deutérium, de F et de -CN ;
- R⁷ est indépendamment choisi dans le groupe constitué du cycloalkyle en C₃₋₅, de l'alkyle en -O-C₁₋₅, du cycloalkyle en-O-C₃₋₅, de l'alkyle en -S-C₁₋₅ et du cycloalkyle en -S-C₃₋₅, chacun d'entre eux pouvant éventuellement être remplacé par un ou plusieurs substituants R⁸, identiques ou différents, du deutérium, de F et de -CN ;
- R⁸ est indépendamment choisi dans le groupe constitué du deutérium, de OH, de OMe et de F ;
- R⁹ est indépendamment choisi dans le groupe constitué du deutérium, du méthoxy, du nitro, du cyano, de CF₃, de Cl, de Br, de I et de F ;
- X est choisi dans le groupe constitué de N et de CR²⁰ ;
- Y est choisi dans le groupe constitué de NH, O et S ;
- R²⁰ est choisi dans le groupe constitué de H, de NH₂, de l'alkyle en C₁₋₅ éventuellement remplacé par un ou plusieurs substituants R⁸, identiques ou différents, du cycloalkyle en C₃₋₅ éventuellement remplacé par un ou plusieurs substituants R⁸, identiques ou différents, de l'alkyle en -OC₁₋₅ éventuellement remplacé par un ou plusieurs substituants R⁸, identiques ou différents, du cycloalkyle en -OC₃₋₅ éventuellement remplacé par un ou plusieurs substituants R⁸, identiques ou différents, d'un alkyle en -NH-C(=O)C₁₋₅ éventuellement remplacé par un ou plusieurs substituants R⁸, identiques ou différents, et d'un alkyle en -NH-SO₂-C₁₋₅ éventuellement remplacé par un ou plusieurs substituants R⁸, identiques ou différents ;
- n est un nombre entier 0, 1, 2 ou 3 ;
ou un sel, un hydrate, un polymorphe, un tautomère ou un solvate pharmaceutiquement acceptable de celui-ci.

3. Composé selon la revendication 1, dans lequel le composé est de formule (III) 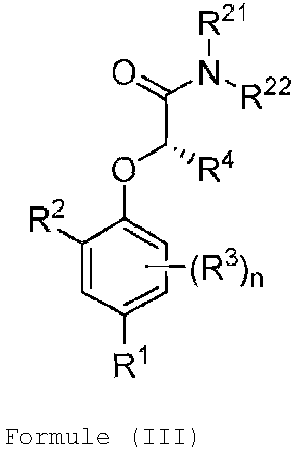 dans laquelle :
- R¹ est choisi dans le groupe constitué de F, de Cl, de Br et de I ;
- R² est choisi dans le groupe constitué de H, de F, de Cl, de Br, de I, de l'alkyle en C₁₋₅ éventuellement remplacé par un ou plusieurs substituants R⁶, identiques ou différents, de l'alcényle en C₂₋₅ éventuellement remplacé par un ou plusieurs substituants R⁶, identiques ou différents, de l'alcynyle en C₂₋₅ éventuellement remplacé par un ou plusieurs substituants R⁶, identiques ou différents, du cycloalkyle en C₃₋₅ éventuellement remplacé par un ou plusieurs substituants R⁶, identiques ou différents, du phényle éventuellement remplacé par un ou plusieurs substituants R⁹, identiques ou différents, et d'un hétérocycle aromatique à 5 ou 6 chaînons éventuellement remplacé par un ou plusieurs substituants R⁷, identiques ou différents ;
- R³ est choisi dans le groupe constitué du deutérium et de F ;
- R⁴ est choisi dans le groupe constitué de l'alkyle en C₁₋₅, de l'alcényle en C₂₋₅, de l'alcynyle en C₂₋₅ et du cycloalkyle en C₃₋₅, chacun d'entre eux pouvant être éventuellement remplacé par un ou plusieurs substituants R⁷, identiques ou différents ;
- R⁶ est indépendamment choisi dans le groupe constitué du cycloalkyle en C₃₋₅, de l'alkyle en -O-C₁₋₅, du cycloalkyle en-O-C₃₋₅, de l'alkyle en -S-C₁₋₅ et du cycloalkyle en -S-C₃₋₅, chacun d'entre eux pouvant éventuellement être remplacé par un ou plusieurs substituants R⁸, identiques ou différents, du deutérium, de F et de -CN ;
- R⁷ est indépendamment choisi dans le groupe constitué du cycloalkyle en C₃₋₅, de l'alkyle en -O-C₁₋₅, du cycloalkyle en-O-C₃₋₅, de l'alkyle en -S-C₁₋₅ et du cycloalkyle en -S-C₃₋₅, chacun d'entre eux pouvant éventuellement être remplacé par un ou plusieurs substituants R⁸, identiques ou différents, du deutérium, de F et de -CN ;
- R⁸ est indépendamment choisi dans le groupe constitué du deutérium, de OH, d'OMe et de F ;
- R⁹ est indépendamment choisi dans le groupe constitué du deutérium, du méthoxy, du nitro, du cyano, du CF₃, de Cl, de Br, de I et de F ;
- R¹¹ est choisi dans le groupe constitué d'un alkyle en C₁₋₅ éventuellement remplacé par un ou plusieurs substituants R⁸, identiques ou différents, d'un cycloalkyle en C₃₋₅ éventuellement remplacé par un ou plusieurs substituants R⁸, identiques ou différents, et d'un phényle, éventuellement remplacé par un ou plusieurs substituants R⁹, identiques ou différents ;
- R¹² est choisi dans le groupe constitué de -OH, d'un alkyle en -OC₁₋₅ éventuellement remplacé par un ou plusieurs substituants R⁸, identiques ou différents, et d'un cycloalkyle en -OC₃₋₅ éventuellement remplacé par un ou plusieurs substituants R⁸, identiques ou différents ;
- R¹⁵ est choisi dans le groupe constitué de H, de l'alkyle en C₁₋₅ éventuellement remplacé par un ou plusieurs substituants R¹⁶, identiques ou différents, du cycloalkyle en C₃₋₆ éventuellement remplacé par un ou plusieurs substituants R¹⁶, identiques ou différents, du phényle éventuellement remplacé par un ou plusieurs substituants R⁹, identiques ou différents, et d'un hétérocycle à 4 à 6 chaînons éventuellement remplacé par un ou plusieurs substituants R¹⁷, identiques ou différents, à condition que, lorsque R¹⁵ est H, alors R²¹ n'est pas H ;
- R¹⁶ est indépendamment choisi dans le groupe constitué de F, de Cl, de Br, de I, de -CN, de =O, du cycloalkyle en C₃₋₆ éventuellement remplacé par un ou plusieurs substituants R⁸, identiques ou différents, de l'alkyle en -O-C₁₋₅, éventuellement remplacé par un ou plusieurs substituants R⁸, identiques ou différents, du cycloalkyle en -O-C₃₋₅, éventuellement remplacé par un ou plusieurs substituants R⁸, identiques ou différents, de l'alkyle en -S-C₁₋₅ éventuellement remplacé par un ou plusieurs substituants R⁸, identiques ou différents, du cycloalkyle en -S-C₃₋₅ éventuellement remplacé par un ou plusieurs substituants R⁸, identiques ou différents, de l'alkyle en -SO-C₁₋₅ éventuellement remplacé par un ou plusieurs substituants R⁸, identiques ou différents, du cycloalkyle en-SO-C₃₋₅ éventuellement remplacé par un ou plusieurs substituants R⁸, identiques ou différents, de l'alkyle en -SO₂-C₁₋₅ éventuellement remplacé par un ou plusieurs substituants R⁸, identiques ou différents, du cycloalkyle en -SO₂-C₃₋₅ éventuellement remplacé par un ou plusieurs substituants R⁸, identiques ou différents, de l'alkyle en -C(=O)-C₁₋₅ éventuellement remplacé par un ou plusieurs substituants R⁸, identiques ou différents, de l'alkyle en -C(=O)-OC₁₋₅ éventuellement remplacé par un ou plusieurs substituants R⁸, identiques ou différents, de l'alkyle en -NH-C (=O)-OC₁₋₅ éventuellement remplacé par un ou plusieurs substituants R⁸, identiques ou différents, du cycloalkyle en -C(=O)-C₃₋₅ éventuellement remplacé par un ou plusieurs substituants R⁸, identiques ou différents, de l'alkyle en -NH-C (=O)-C₁₋₅ éventuellement remplacé par un ou plusieurs substituants R⁸, identiques ou différents, du cycloalkyle en -NH-C(=O)-C₃₋₅ éventuellement remplacé par un ou plusieurs substituants R⁸, identiques ou différents, de l'alkyle en -C(=O)-NH-C₁₋₅ éventuellement remplacé par un ou plusieurs substituants R⁸, identiques ou différents, du cycloalkyle en -C(=O)-NH-C₃₋₅ éventuellement remplacé par un ou plusieurs substituants R⁸, identiques ou différents, du phényle éventuellement remplacé par un ou plusieurs substituants R⁹, identiques ou différents, du pyrrolidin-1-yle éventuellement remplacé par un ou plusieurs substituants R¹⁷, identiques ou différents, et un hétérocycle à 4 à 6 chaînons éventuellement remplacé par un ou plusieurs substituants R¹⁷, identiques ou différents ;
- R¹⁷ est indépendamment choisi dans le groupe constitué de F, de Cl, de Br, de I, de -CN, de =O, de l'alkyle en C₁₋₅ éventuellement remplacé par un ou plusieurs substituants R⁸, identiques ou différents, du cycloalkyle en C₃₋₆ éventuellement remplacé par un ou plusieurs substituants R⁸, identiques ou différents, de l'alkyle en -C(=O)-C₁₋₅ éventuellement remplacé par un ou plusieurs substituants R⁸, identiques ou différents, de l'alkyle en -C(=O)-OC₁₋₅ éventuellement remplacé par un ou plusieurs substituants R⁸, identiques ou différents, du cycloalkyle en -C(=O)-C₃₋₅ éventuellement remplacé par un ou plusieurs substituants R⁸, identiques ou différents, de l'alkyle en -NH-C(=O)-C₁₋₅ éventuellement remplacé par un ou plusieurs substituants R⁸, identiques ou différents, du cycloalkyle en - NH-C(=O)-C₃₋₅ éventuellement remplacé par un ou plusieurs substituants R⁸, identiques ou différents, de l'alkyle en -C(=O)-NH-C₁₋₅ éventuellement remplacé par un ou plusieurs substituants R⁸, identiques ou différents, et du cycloalkyle en -C(=O)-NH-C₃₋₅ éventuellement remplacé par un ou plusieurs substituants R⁸, identiques ou différents ;
- R¹⁹ est choisi dans le groupe constitué d'un alkyle en C₁₋₅ éventuellement remplacé par un ou plusieurs substituants R⁸, identiques ou différents, d'un cycloalkyle en C₃₋₅ éventuellement remplacé par un ou plusieurs substituants R⁸, identiques ou différents, et d'un phényle, éventuellement remplacé par un ou plusieurs substituants R⁹, identiques ou différents ;
- R²¹ est choisi dans le groupe constitué de H, de l'alkyle en C₁₋₅ éventuellement remplacé par un ou plusieurs substituants R⁸, identiques ou différents, du cycloalkyle en C₃₋₅ éventuellement remplacé par un ou plusieurs substituants R⁸, identiques ou différents, de l'alkyle en -C(=O)-C₁₋₅ éventuellement remplacé par un ou plusieurs substituants R⁸, identiques ou différents, et du cycloalkyle en -C(=O)-C₃₋₅, éventuellement remplacé par un ou plusieurs substituants R⁸, identiques ou différents ;
- R²² est choisi dans le groupe constitué de -C(R¹¹)=NR¹²,-CN, -OR¹⁵ et SO₂R¹⁹ ; et
- n est un nombre entier 0, 1, 2 ou 3 ;
ou un sel, un hydrate, un polymorphe, un tautomère ou un solvate pharmaceutiquement acceptable de celui-ci.

4. Composé selon l'une quelconque des revendications 1 à 3, dans lequel R¹ est Cl ou Br.

5. Composé selon l'une quelconque des revendications 1 à 4, dans lequel R² est H, F, Cl, Br ou I.

6. Composé selon l'une quelconque des revendications 1 à 4, dans lequel R² est un hétérocycle aromatique de 5 ou 6 chaînons éventuellement remplacé par un ou plusieurs substituants R⁷, identiques ou différents.

7. Composé selon l'une quelconque des revendications 1 à 6, dans lequel R⁴ est un alkyle en C₁₋₅ éventuellement remplacé par un ou plusieurs substituants R⁷, identiques ou différents.

8. Composé selon l'une quelconque des revendications 1 à 2 et 4 à 6, dans lequel X est N et Y est NH.

9. Composé selon l'une quelconque des revendications 3 à 6, dans lequel R²¹ est H.

10. Composé destiné à être utilisé selon la revendication 1 à 9, dans lequel le composé est choisi dans le groupe constitué de :
(2*S*)-2-(4-bromo-2-fluorophénoxy)-*N*-{1-[(cyclopropylméthoxy)imino]éthyl}propanamide ;
(2*S*)-2-(4-bromophénoxy)-*N*-[1-(méthoxyimino)éthyl]propanamide ;
(2*S*)-2-(4-bromo-2-fluorophénoxy)-*N*-[(4-fluorophényl) (hydroxyimino)méthyl]-3-méthylbutanamide ;
(2*S*)-2-(4-bromo-2-fluorophénoxy)-*N*-[1-(hydroxyimino)éthyl]-3-méthylbutanamide ;
(2*S*)-2-(4-bromo-2-fluorophénoxy)-*N*-[1-(hydroxyimino)éthyl]propanamide ;
(2*S*)-2-(4-bromophénoxy)-*N*-[1-(hydroxyimino)éthyl]propénamide ;
(2*S*)-2-[4-bromo-2-(1,2-oxazol-3-yl)phénoxy]-*N-*cyanopropanamide ;
(2*S*)-*N*-cyano-2-(2,4-dibromophénoxy)propanamide ;
(2*S*)-2-(4-bromophénoxy)-*N*-cyanopropanamide ;
(2*S*)-2-(4-chlorophénoxy)-*N*-cyanopropanamide ;
(2*S*)-2-(4-bromophénoxy)-*N*-cyano-3-méthylbutanamide ;
(2*S*)-2-[4-bromo-2-(1,2-oxazol-3-yl)phénoxy]-*N-*cyclobutoxypropanamide ;
(2*S*)-2-[4-bromo-2-(1,2-oxazol-3-yl)phénoxy]-*N-*méthoxypropanamide ;
(2*S*)-*N*-acétyl-*N*-[(1-acétylazétidin-3-yl)oxy]-2-(4-chlorophénoxy)propanamide ;
(2*S*)-*N*-[(1-acétylazétidin-3-yl)oxy]-2-(4-chlorophénoxy)propanamide ;
(2*S*)-*N*-(azétidin-3-yloxy)-2-(4-chlorophénoxy)propanamide ;
(2*S*)-*N*-acétyl-*N*-[(1-acétylpyrrolidin-3-yl)méthoxy]-2-(4-chlorophénoxy)propanamide ;
(2*S*)-*N*-[(1-acétylpyrrolidin-3-yl) méthoxy]-2-(4-chlorophénoxy)propanamide ;
(2*S*)-*N*-[(1-acétylpyrrolidin-3-yl)oxy]-2-(4-bromophénoxy)propanamide ;
*N*-(2-{[(2*S*)-2-(4-chlorophénoxy)propanamido]oxy}propyl)carbamate de tert-butyle ;
(2*S*)-*N*-acétyl-2-(4-chlorophénoxy)-*N*-[(1-acétamidopropan-2-yl)oxy]propanamide ;
(2*S*)-2-(4-bromophénoxy)-*N*-(pyrrolidin-3-yloxy)propanamide ;
(2*S*)-*N*-acétyl-*N*-[(1-acétylpyrrolidin-3-yl)oxy]-2-(4-bromophénoxy)propanamide ;
3-{[(2*S*)-2-(4-chlorophénoxy)propanamido]oxy}azétidine-1-carboxylate de tert-butyle ;
(2*S*)-2-(4-chlorophénoxy)-*N*-[(pyrrolidin-3-yl)méthoxy]propanamide ;
3-({[(2*S*)-2-(4-chlorophénoxy)propanamido]oxy}méthyl)pyrrolidine-1-carboxylate de tert-butyle ;
(2*S*)-*N*-acétyl-*N*-[(1-acétylpyrrolidin-3-yl)oxy]-2-(4-chlorophénoxy)propanamide ;
(2*S*)-*N*-[(1-acétylpyrrolidin-3-yl)oxy]-2-(4-chlorophénoxy)propanamide ;
(2*S*)-2-(4-chlorophénoxy)-*N*-(pyrrolidin-3-yloxy)propanamide ;
(2*S*)-2-(4-chlorophénoxy)-*N*-(2,2-diméthylpropyl)-*N-*hydroxypropanamide ;
(2*S*)-2-(4-chlorophénoxy)-*N*-[2-(pyrrolidin-1-yl)éthoxy]propanamide ;
(2*S*)-2-(4-chlorophénoxy)-*N*-[2-(2-oxopyrrolidin-1-yl)éthoxy]propanamide ;
(2*S*)-*N*-acétyl-2-(4-chlorophénoxy)-*N*-(2-acétamidoéthoxy)propanamide ;
(2*S*)-2-(4-chlorophénoxy)-*N*-(2-acétamidoéthoxy)propanamide ;
(2*S*)-2-(4-chlorophénoxy)-*N*-(4,4,4-trifluoro-2-méthylbutoxy)propanamide ;
(2*S*)-2-(4-chlorophénoxy)-*N*-(3-cyclopentylpropyl)-*N-*hydroxypropanamide ;
(2*S*)-2-(4-chlorophénoxy)-*N*-{[(2E)-2-méthyl-3-phénylprop-2-en-1-yl]oxy}propanamide ;
(2*S*)-2-(4-chlorophénoxy)-*N*-{2-oxo-2-[2-(trifluorométhyl)pyrrolidin-1-yl]éthoxy}propanamide ;
*N*-(2-{[(2*S*)-2-(4-chlorophénoxy)propanamido]oxy}éthyl)carbamate de tert-butyle ;
(2*S*)-2-(4-chlorophénoxy)-*N*-[(2-méthyl-1H-imidazol-4-yl)méthoxy]propanamide ;
4-({[(2*S*)-2-(4-chlorophénoxy)propanamido]oxy}méthyl)-2-méthyl-1H-imidazole-1-carboxylate de tert-butyle ;
(2*S*)-2-(4-bromo-2-fluorophénoxy)-*N*-cyclobutoxypropanamide ;
(2*S*)-2-(4-bromophénoxy)-*N*-cyclobutoxypropanamide ;
(2*S*)-2-(4-chlorophénoxy)-*N*-[2-oxo-2-(pyrrolidin-1-yl)éthoxy]propanamide ;
(2*S*)-2-(4-chlorophénoxy)-*N*-[1-(4-fluorophényl)éthoxy]propanamide ;
(2*S*)-2-(4-chlorophénoxy)-*N*-[1-(1,3-thiazol-2-yl)éthoxy]propanamide ;
(2*S*)-2-(4-chlorophénoxy)-*N*-{1-[4-(trifluorométhyl)phényl]éthoxy}propanamide ;
(2*S*)-2-(4-chlorophénoxy)-*N*-(2-méthanesulfinyléthoxy)propanamide ;
(2*S*)-2-(4-chlorophénoxy)-*N*-(2-méthanesulfonyléthoxy)propanamide ;
(2*S*)-2-(4-chlorophénoxy)-*N*-[(1,2-oxazol-3-yl)méthoxy]propanamide ;
(2*S*)-2-(4-chloro-2-fluorophénoxy)-*N*-méthoxypropanamide ;
(2*S*)-2-[4-bromo-2-(1,3,4-oxadiazol-2-yl)phénoxy]-*N-*(cyclopropylméthoxy)propanamide ;
(2*S*)-2-[4-chloro-2-(trifluorométhyl)phénoxy]-*N-*(cyclopropylméthoxy)propanamide ;
(2*S*)-*N*-(tert-butoxy)-2-(4-chlorophénoxy)propanamide ;
(2*S*)-2-(4-chlorophénoxy)-*N*-[2-(méthylsulfanyl)éthoxy]propanamide ;
(2*S*)-2-(4-chlorophénoxy)-*N*-(1-phényléthoxy)propanamide ;
(2*S*)-2-(4-chlorophénoxy)-*N*-[(1-méthyl-1H-imidazol-2-yl)méthoxy]propanamide ;
(2*S*)-2-(4-chlorophénoxy)-*N*-(2-méthoxyéthoxy)propanamide ;
(2*S*)-2-(4-chloro-2-fluorophénoxy)-*N*-cyclobutoxypropanamide ;
(2*S*)-2-[4-bromo-2-(1,3,4-oxadiazol-2-yl)phénoxy]-*N-*méthoxypropanamide ;
(2*S*)-2-[4-chloro-2-(trifluorométhyl)phénoxy]-*N-*méthoxypropanamide ;
(2*S*)-*N*-(benzyloxy)-2-(4-chlorophénoxy)propanamide ;
(2*S*)-2-(4-chlorophénoxy)-*N*-[(2-méthoxycyclopentyl)oxy]propanamide ;
(2*S*)-2-(4-chlorophénoxy)-*N*-cyclobutoxy-5-méthylhexanamide ;
(2*S*)-2-(4-chlorophénoxy)-*N*-cyclobutoxy-*N*-méthylpropanamide ;
(2*S*)-2-(4-chloro-2-méthylphénoxy)-*N*-cyclobutoxypropanamide ;
(2*S*)-2-(4-chloro-3-fluorophénoxy)-*N*-cyclobutoxypropanamide ;
(2*S*)-2-(4-chloro-2-méthylphénoxy)-*N*-méthoxypropanamide ;
(2*S*)-2-(4-chloro-3-fluorophénoxy)-*N*-méthoxypropanamide ;
(2*S*)-2-(4-chlorophénoxy)-*N*-cyclobutoxy-4-méthylpentanamide ;
(2*S*)-2-(4-chlorophénoxy)-*N*-[(3-méthylbut-2-en-1-yl)oxy]propanamide ;
(2*S*)-2-(4-chlorophénoxy)-*N*-cyclobutoxyhexanamide ;
(2*S*)-2-(4-chlorophénoxy)-*N*-[(1,3-oxazol-2-yl)méthoxy]propanamide ;
(2*S*)-2-(2,4-dibromophénoxy)-*N*-méthoxypropanamide;
(2*S*)-2-(4-bromo-2-fluorophénoxy) -*N*-méthoxypropanamide ;
(2*S*)-2-(4-chlorophénoxy) -*N*-(oxan-2-yloxy)propanamide ;
(2*S*)-2-(4-chlorophénoxy)*-N*-[(1,3-thiazol-2-yl)méthoxy]propanamide ;
(2*S*)-2-(4-chlorophénoxy)-*N*-(3,3-difluorocyclobutoxy)propanamide ;
(2*S*)-2-[4-bromo-2-(1,2-oxazol-5-yl)phénoxy]-*N-*cyclobutoxypropanamide ;
(2*S*)-2-[4-bromo-2-(1,2-oxazol-5-yl)phénoxy]-*N-*méthoxypropanamide ;
(2*S*)-2-(4-chlorophénoxy)-*N*-(cyclopentyloxy)propanamide ;
(2*S*)-2-(4-chlorophénoxy)-*N*-(2-cyclopentyléthoxy)propanamide ;
(2*S*)-2-(4-bromo-2-chlorophénoxy)-*N*-méthoxypropanamide ;
(2*S*)-2-(4-bromo-2-méthylphénoxy)-*N*-méthoxypropanamide ;
(2*S*)-2-(4-chlorophénoxy)-*N-*(cyclopropylméthoxy)propanamide ;
(2*S*)-2-(4-chlorophénoxy)-*N*-(cyclobutylméthoxy)propanamide ;
(2*S*)-*N*-(2-aminoéthoxy)-2-(4-chlorophénoxy)propanamide ;
(2*S*)-2-(4-bromophénoxy)-*N*-méthoxy-3-méthylbutanamide ;
2-{[(2*S*)-2-(4-chlorophénoxy)propanamido]oxy}acétate de méthyle ;
(2*S*)-2-(4-chlorophénoxy)-*N*-[2-(2-méthoxyéthoxy)éthoxy]propanamide ;
(2*S*)-2-(4-chlorophénoxy)-*N*-cyclobutoxypropanamide ;
(2*S*)-2-(4-chlorophénoxy)-*N*-(2-hydroxyéthoxy)propanamide ;
(2*S*)-2-(4-chlorophénoxy)-*N*-éthoxypropanamide ;
(2*S*)-2-(4-chlorophénoxy)-*N*-propoxypropanamide ;
(2*S*)-2-(4-chlorophénoxy)-*N*-(propan-2-yloxy)propanamide ;
(2*S*)-2-(4-chlorophénoxy)-*N*-méthoxypropanamide ;
(2*S*)-2-[4-bromo-2-(1,2-oxazol-3-yl)phénoxy]-*N-*(cyclopropanesulfonyl)propénamide ;
(2*S*)-2-(4-bromo-2-fluorophénoxy)-*N-*méthanesulfonylpropanamide ;
(2*S*)-2-(4-bromo-2-fluorophénoxy)-*N-*(cyclopropanesulfonyl)propénamide ;
(2*S*)-2-(4-chlorophénoxy)-*N*-méthanesulfonylpropanamide ;
(2*S*)-2-(4-bromophénoxy)-*N*-méthanesulfonyl-3-méthylbutanamide ;
5-[(1*S*)-1-[4-bromo-2-(1,2-oxazol-3-yl)phénoxy]-3-fluoropropyl]-2H-1,2,3,4-tétrazole ;
5-[(1*S*)-1-(4-bromo-2-fluorophénoxy)propyl]-2H-1,2,3,4-tétrazole ;
5-[(1*S*)-1-(4-bromo-2-fluorophénoxy)-2-méthylpropyl]-2H-1,2,3,4-tétrazole ;
5-[(1*R*)-1-(4-bromophénoxy)-2-fluoroéthyl]-2H-1,2,3,4-tétrazole ;
5-[(1*S*)-1-(4-bromophénoxy)propyl]-2H-1,2,3,4-tétrazole ;
5-[(1*S*)-1-(4-bromophénoxy)éthyl]-2H-1,2,3,4-tétrazole ;
5-[(1*S*)-1-[4-bromo-2-(1,2-oxazol-3-yl)phénoxy]éthyl]-1H-1,2,3,4-tétrazole ;
5-[(1*S*)-1-(4-bromo-2-fluorophénoxy)éthyl]-2H-1,2,3,4-tétrazole ;
5-[(1*S*)-1-[4-bromo-2-(1,2-oxazol-5-yl)phénoxy]éthyl]-2H-1,2,3,4-tétrazole ;
5-[(1*S*)-1-[4-chloro-2-(1,2-oxazol-3-yl)phénoxy]éthyl]-2H-1,2,3,4-tétrazole ;
5-[(1*S*)-1-(4-chloro-2-cyclopropylphénoxy)éthyl]-1H-1,2,3,4-tétrazole ;
5-[(1*S*)-1-(4-chloro-2-éthénylphénoxy)éthyl]-1H-1,2,3,4-tétrazole ;
5-[(1*S*)-1-(4-chloro-2-éthylphénoxy)éthyl]-1H-1,2,3,4-tétrazole ;
5-[(1*S*)-1-(4-chloro-2-méthylphénoxy)éthyl]-2H-1,2,3,4-tétrazole ;
5-[(1*S*)-1-(4-chlorophénoxy)éthyl]-1H-1,2,3,4-tétrazole ;
*N*-{3-[(1*S*)-1-(4-bromo-2-fluorophénoxy)éthyl]-1,2,4-thiadiazol-5-yl}acétamide ;
*N*-{3-[(1*S*)-1-(4-bromo-2-fluorophénoxy)éthyl]-1,2,4-thiadiazol-5-yl}méthanesulfonamide ;
3-[(1*S*)-1-(4-bromo-2-fluorophénoxy)éthyl]-1,2,4-thiadiazol-5-amine ;
*N*-{3-[(1*S*)-1-(4-bromo-2-fluorophénoxy)éthyl]-1,2,4-oxadiazol-5-yl}acétamide ;
*N*-{3-[(1*S*)-1-(4-bromo-2-fluorophénoxy)éthyl]-1,2,4-oxadiazol-5-yl}méthanesulfonamide ;
3-[(1*S*)-1-[4-bromo-2-(1,2-oxazol-3-yl)phénoxy]éthyl]-5-(trifluorométhyl)-4H-1,2,4-triazole ;
3-[(1*S*)-1-[4-chloro-2-(1,2-oxazol-3-yl)phénoxy]éthyl]-5-(trifluorométhyl)-4H-1,2,4-triazole ;
3-[(1*S*)-1-(4-bromo-2-fluorophénoxy)éthyl]-5-méthoxy-1,2,4-oxadiazole ;
3-[(1*S*)-1-(4-bromo-2-fluorophénoxy)éthyl]-1,2,4-oxadiazol-5-amine ;
3-[(1*S*)-1-[4-bromo-2-(1,2-oxazol-3-yl)phénoxy]éthyl]-5-méthyl-4H-1,2,4-triazole ;
3-[(1*S*)-1-[4-chloro-2-(1,2-oxazol-3-yl)phénoxy]éthyl]-5-méthyl-4H-1,2,4-triazole ;
3-[(1*S*)-1-(4-bromo-2-fluorophénoxy)éthyl]-5-méthyl-4H-1,2,4-triazole ;
5-[(1*S*)-1-(4-chlorophénoxy)éthyl]-1H-1,2,4-triazole ;
(2*S*)-2-[4-bromo-2-(1,1-difluoroéthyl)phénoxy]-*N-*cyanopropanamide ;
(2*S*)-2-[4-bromo-2-(1,1-difluoropropyl)-5-fluorophénoxy]-*N-*cyanopropanamide ;
(2*S*)-2-[4-bromo-2-(1,1-difluoropropyl)phénoxy]-*N-*méthanesulfonylpropanamide ;
(2*S*)-2-[4-bromo-2-(1,1-difluoropropyl)phénoxy]-*N-*(cyclopropanesulfonyl)propénamide ;
5-[(1*S*)-1-[4-bromo-2-(1,1-difluoroéthyl)phénoxy]éthyl]-1H-1,2,3,4-tétrazole ;
5-[(1*S*)-1-[4-bromo-2-(1,1-difluoropropyl)phénoxy]éthyl]-1H-1,2,3,4-tétrazole ;
5-[(1*S*)-1-(4-bromo-2-cyclobutylphénoxy)-2-méthoxyéthyl]-1H-1,2,3,4-tétrazole ; et
5-[(1*S*)-1-(4-bromo-2-cyclopropylphénoxy)-2-méthoxyéthyl]-1H-1,2,3,4-tétrazole.

11. Composé selon l'une quelconque des revendications 1 à 10, dans lequel le composé est un inhibiteur du canal ionique CIC-1.

12. Composition comprenant le composé selon l'une quelconque des revendications 1 à 11 et un support pharmaceutiquement acceptable.

13. Composé selon l'une quelconque des revendications 1 à 11 ou composition selon la revendication 12 destiné(e) à être utilisé(e) comme médicament.

14. Composé selon l'une quelconque des revendications 1 à 11 ou composition selon la revendication 12 destiné(e) à être utilisé(e) dans le traitement des symptômes d'une indication choisie dans le groupe constitué de la myasthénie grave, du syndrome de Lambert-Eaton, de la myopathie grave, de la sclérose latérale amyotrophique (SLA), de l'atrophie musculaire spinale (AMS), de la myopathie grave (MCI), de la polyneuropathie diabétique réversible, du syndrome de Guillain-Barré, de la poliomyélite, du syndrome post-polio, du syndrome de fatigue chronique, de la polyneuropathie grave, de la paralysie périodique, de la sarcopénie, de la paralysie périodique hypokaliémique et de la paralysie périodique hyperkaliémique.

15. Composé selon l'une quelconque des revendications 1 à 11 ou composition selon la revendication 12 destiné(e) à être utilisé(e) pour inverser et/ou améliorer un blocage neuromusculaire.
